(19)

Europäisches
Patentamt
European
Patent Office
Office européen
des brevets

(11) **EP 1 642 131 B1**

(12) **EUROPEAN PATENT SPECIFICATION**

(45) Date of publication and mention
of the grant of the patent:
**29.10.2008 Bulletin 2008/44**

(51) Int Cl.:
**G01N 33/543** (2006.01)　　**G01N 33/96** (2006.01)

(21) Application number: **04744299.1**

(22) Date of filing: **08.07.2004**

(86) International application number:
**PCT/IB2004/002682**

(87) International publication number:
**WO 2005/003773 (13.01.2005 Gazette 2005/02)**

(54) **STANDARD**

STANDARD

ETALON

(84) Designated Contracting States:
**AT BE BG CH CY CZ DE DK EE ES FI FR GB GR
HU IE IT LI LU MC NL PL PT RO SE SI SK TR**

(30) Priority: **08.07.2003  GB 0315991
11.07.2003  US 486381 P**

(43) Date of publication of application:
**05.04.2006  Bulletin 2006/14**

(73) Proprietor: **Dako Denmark A/S
2600 Glostrup (DK)**

(72) Inventors:
 • **WINTHER, Lars,
c/o DakoCytomation Denmark A/S
2600 Glostrup (DK)**
 • **PII, Kurt,
c/o DakoCytomation Denmark A/S
2600 Glostrup (DK)**

(74) Representative: **Harding, Charles Thomas et al
D Young & Co
120 Holborn
London EC1N 2DY (GB)**

(56) References cited:
　**EP-A- 0 345 953**　　　　**WO-A-00/23799**
　**WO-A-03/016867**

 • **DUNDR M ET AL: "Quantitation of GFP-fusion
proteins in single living cells." JOURNAL OF
STRUCTURAL BIOLOGY, vol. 140, no. 1-3, 2002,
pages 92-99, XP002305339**
 • **BERGQUIST, N. R. ET AL: "Use of polymerized
immunoglobulin particles for specificity control
of fluorescent conjugates" JOURNAL OF
IMMUNOLOGICAL METHODS, vol. 4, no. 2, 1974,
pages 195-202, XP002305340**
 • **POON S S S ET AL: "TELOMERE LENGTH
MEASUREMENTS USING DIGITAL
FLUORESCENCE MICROSCOPY" CYTOMETRY,
vol. 36, no. 4, 1 August 1999 (1999-08-01), pages
267-278, XP009023307**
 • **SLIJEPCEVIC PREDRAG: "Telomere length
measurement by Q-FISH" METHODS IN CELL
SCIENCE, vol. 23, no. 1-3, 2001, pages 17-22,
XP002305341**
 • **NOACK F ET AL: "CD87-POSITIVE TUMOR
CELLS IN BONE MARROW ASPIRATES
IDENTIFIED BY CONFOCAL LASER SCANNING
FLUORESCENCE MICROSCOPY"
INTERNATIONAL JOURNAL OF ONCOLOGY, vol.
15, no. 4, 1999, pages 617-623, XP000929604**
 • **BIKOUE A ET AL: "Stabilised cellular immuno-
fluorescence assay: CD45 expression as a
calibration standard for human leukocytes"
JOURNAL OF IMMUNOLOGICAL METHODS, vol.
266, no. 1-2, 2002, pages 19-32, XP004372268**
 • **http://www.m-w.com/dictionary/coupled**

**Description**

**FIELD**

This invention pertains to the fields of cytology and histology. In particular, the invention is related to the fields of immunohistochemistry and molecular cytogenetics, in particular, the provision of standards for gauging the presence or amount of detectable entities such as in cells, tissues and organs.

**BACKGROUND**

Histological and cytological techniques have been used to analyse biopsies and other tissue samples, as an aid to medical diagnosis. Cytology is the study of the structure of all normal and abnormal components of cells and the changes, movements, and transformations of such components. Cells are studied directly in the living state or are killed (mixed) and prepared by for example embedding, sectioning, or staining for investigation in bright field or electron microscopes.

One well-known cytology procedure is the Papanicolaou test medical procedure used to detect cancer of the uterine cervix. A scraping, brushing, or smear, is taken from the surface of the vagina or cervix and is prepared on a slide and stained for microscopic examination and cytological analysis. The appearance of the cells determines whether they are normal, suspicious, or cancerous.

Histology is the study of groups of specialised cells called tissues that are found in most multi-cellular plants and animals. Histological investigation includes study of tissue death and regeneration and the reaction of tissue to injury or invading organisms. Because normal tissue has a characteristic appearance, histologic examination is often utilised to identify diseased tissue. Immunohistochemistry, IHC, and *in situ* hybridisation, ISH, analysis are useful tools in histological diagnosis and the study of tissue morphology.

Both immunohistochemistry (IHC) and *in situ* hybridisation (ISH), seek to detect a detectable entity in a sample by using specific binding agents capable of binding to the detectable entity. In immunohistochemistry (IHC), the specific binding agent comprises an antibody, and the detectable entity comprises a polypeptide, protein, or epitope comprised therein. In *in situ* hybridisation (ISH), the detectable entity comprises a nucleic acid (including DNA and RNA) in the sample, and the specific binding agent comprises a probe such as a nucleic acid probe. The increasing availability of such antibodies and probes may help in differential diagnosis of diseased and normal tissue. In situ hybridisation and immunochemistry methods are described in detail in Harlow and Lane (Antibodies : A Laboratory Manual).

IHC and ISH techniques require a series of treatment steps conducted on a tissue section mounted on a glass slide or other planar support to highlight by selective staining certain morphological indicators of disease states.

Thus, for example in IHC, a sample is taken from an individual, fixed and exposed to antibodies against the antigen of interest. Further processing steps, for example, antigen retrieval, exposure to secondary antibodies (usually coupled to a suitable enzyme), washing, and to chromogenic enzyme substrates, etc may be necessary to reveal the pattern of antigen binding. There are in general two categories of histological materials: (a) preparations, comprising fresh tissues and/or cells, which generally are not fixed with aldehyde-based fixatives, and (b) fixed and embedded tissue specimens, often archive material.

In ISH, a sample is taken from an individual, fixed and exposed to a probe against the nucleic acid of interest. The detectable entity typically comprises a detectable nucleic acid, such as DNA and RNA, including messenger RNA and siRNA (small interfering RNA or short interfering RNA. Detection of DNA/RNA levels indicates the level of expression of a particular gene, and hence may be used to detect a condition (such as a disease condition) of a cell, tissue, organ or organism. The detectable entity being a nucleic acid is typically denatured to expose binding sites. The probe is typically a double or single stranded nucleic acid, such as a DNA or RNA, and is labelled using radioactive labels such as $^{31}$P, $^{33}$P or $^{32}$S, or non-radioactively, using labels such as digoxigenin, or fluorescent labels, a great many of which are known in the art.

Many methods of fixing and embedding tissue specimens are known, for example, alcohol fixation. However, the most widely used fixing/embedding technique employs formalin-fixation and subsequent paraffin embedding, FFPE. A "typical" FFPE IHC staining procedure may involve the steps of: cutting and trimming tissue, fixation, dehydration, paraffin infiltration, cutting in thin sections, mounting onto glass slides, baking, deparaffination, rehydration, antigen retrieval, blocking steps, applying primary antibody, washing, applying secondary antibody - enzyme conjugate, washing, applying enzyme chromogen substrate, washing, counter staining, cover slipping and microscope examination. Similar steps take place in ISH. The amount of the relevant antigen or other detectable entity such as a nucleic acid detected by such techniques is then assessed to determine whether it is above a certain pre-determined minimum threshold, and therefore diagnostically relevant. Suitable treatment may then be planned for the individual if necessary.

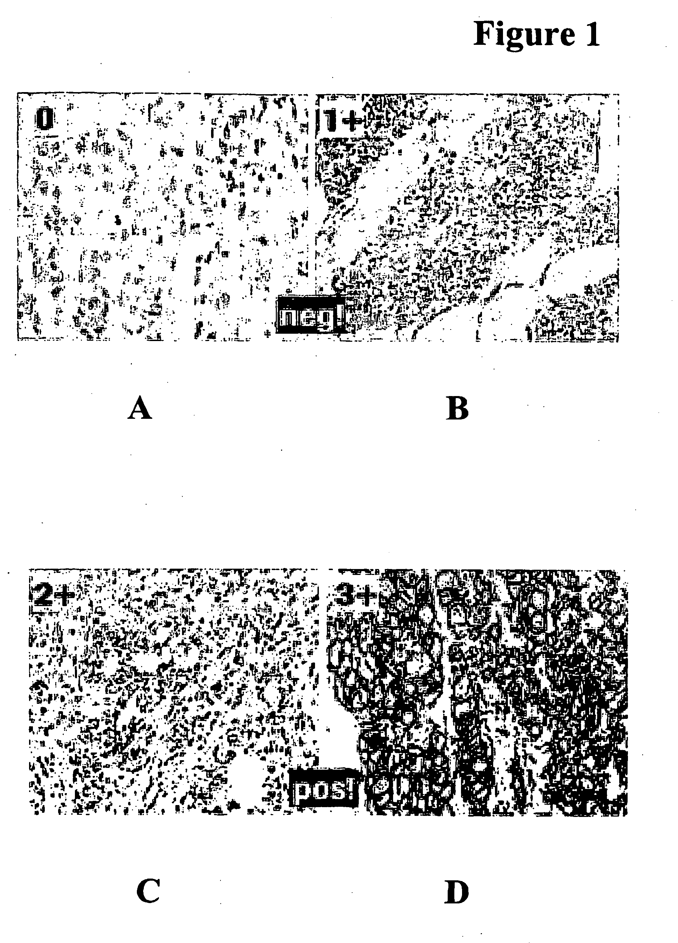

An example of immunohistochemical staining for diagnosis is shown in Figure 1, where tissues are stained for the breast cancer antigen HER2. Tissues which not express the antigen are not stained substantially by anti-HER2 antibody (Figure 1A), while those which do express the protein are stained to a substantial degree by anti-HER2 antibody (Figure 1D).

However, a major problem in such IHC and ISH techniques arises from the necessity of making an accurate determination of whether cells in a tissue being examined express the antigen or detectable entity such as a nucleic acid at a diagnostically significant level or not (i.e., whether the cells are "positive" or "negative" for expression of an antigen). There is a general lack of standardisation of laboratory techniques, leading to the requirement for a subjective judgement of the results. Even small procedural differences can influence the final staining outcome, and the final interpretation of the staining of the same cell population may not be exactly the same from laboratory to laboratory. Even when internal controls (including positive or negative controls, or both) are included in the procedures, variations in the pre-treatment and staining protocols between different workers will cause variations in the internal control. Other analytic sources of error include the quality and quantity of reagents, efficiency of antigen-retrieval and differences in instrumentation.

These problems hinder the assessment of new, alternative, prognostic factors, resulting in contradictory results for most of the prognostic factors in relation to their prognostic value.

The necessity for grading and standardisation has been addressed in the prior art in a number of ways. For example, a diagnostic kit may contain photomicrographs of different sets of cells at various levels of staining. The kit contains an indication that a particular set of cells represents the cut-off or threshold point, with cells matching or exceeding that staining being "positive", with those cells having less staining being "negative". More sophisticated kits may contain actual samples of tissues or cells, which are already stained, on control slides. For example, a kit may include several reference slides with sections of FFPE breast carcinoma cell lines that represent different levels of a breast specific protein expression. An example of such a grading system for the HER2 antigen is shown in Figure 1, where reference staining levels of 0 or 1+ are considered negative (Figures 1A and 1B respectively), while those of 2+ or 3+ are considered positive for that antigen (Figures 1C and 1D respectively).

Written descriptions relating to the pattern or distribution of staining may be included to aid the analysis. For example, Score 0 (negative): No staining is observed, or membrane staining is observed in less than 10 % of tumour cells. Score 1+ (negative): A faint or barely perceptible membrane staining is detected in more than 10 % of the tumour cells. The cells are only stained in part of the membrane. Score 2+ (weakly positive): a weak to moderate complete membrane staining is observed in more than 10% of the tumour cells. Score 3+ (strongly positive): a strong complete membrane staining is observed in more than 10 % of the tumour cells.

However, although it is possible with such kits to establish the reference levels of staining, there exists considerable difficulty in establishing a consistent quality of staining of the samples themselves. This arises from a variety of different factors, including inhomogeneous tissue material, the laborious and complex nature of the procedures, variability in reagent quality (including antibody/probe affinity and specificity), and the subjective nature of the interpretation carried out by the practitioner. Furthermore, other sources of variability in sample staining include the conditions under which tissue samples are collected, processed and stored, variability in epitope retrieval procedures, and enzyme catalysed chromogen precipitation.

As an attempt to solve these problems, it is known in the prior art to include reference sets of unstained tissues or cells with different levels of expression of the relevant antigen (or nucleic acid) with a diagnostic kit. The cells making up the references may comprise biopsy samples (i.e., tissue samples) from known diseased and un-diseased individuals, or individuals which express antigen or relevant detectable nucleic acid at higher than normal levels but are not clinically diseased. Furthermore, tissue culture cells, which may be transfected with expression vectors to enable them to express the antigen or detectable nucleic acid at various levels, may also be used as reference standards. The reference sets comprise slides with formalin fixed and paraffin embedded cells, but are otherwise unstained, and which are embedded in paraffin. The slides are then processed in parallel with the sample to reveal the level and pattern of antigen (or detectable nucleic acid) staining. Finally, the sample is compared to the reference set to determine whether protein or nucleic acid expression levels are diagnostically significant.

Examples of cell lines currently used as reference cells in cytochemistry include the HER2 positive cell line SK-BR-3, the estrogen receptor, ER, positive and progesterone receptor, PR, negative and p53 positive HCC70 cell line, the PR positive and ER negative HCC2218 cell line, the Epidermal Growth Factor Receptor, (EGFR) positive NCI-H23 cell line, the prostate specific antigen (PSA) and androgen receptor positive MDA PCA 2b cell line, and the cytokeratin 19 and the p53 positive, HCC38 cell line. Many human and non-human cell lines are obtainable through various organizations.

However, a problem exists with such reference standards in that it is necessary to specifically identify tissues and cells which express antigen or detectable nucleic acid at the various grading levels, and to obtain them for use. Where tissue culture cells are used, it is necessary to first clone the gene in question, then design and construct appropriate expression vectors. These vectors are then required to be transfected into the cells, and the expression of the gene to be regulated at the right level. As the cell lines are grown continuously and in many laboratories, the level of protein expression can change over time, and may not be have the same protein or mRNA expression from laboratory to laboratory. Both transient and so-called stable transfected cell lines are labile during growth, resulting in changing expression of targets and consequently changing staining level and patterns.

Furthermore, there are issues with the need to include potentially hazardous biological material with the diagnostic kits. Regulatory and ethical problems are associated with the use of material of human origin; such human material is not easy to obtain in large amount from single sources, and may need to be pooled, leading to further variability.

Other techniques known include the use of reference dots made of polymer gels, which are attached to glass slides. The polymer material contains relevant epitopes to be stained. The quality control devices described in WO 00/62064 and Sompuram et al. Clin. Chem., 48 (3), p. 410, 2002 employ surrogate analytic targets, comprising synthetic peptides which resemble the 3D conformation of the epitope to which an antibody binds, which are applied to and coupled to a top surface of a glass slide.

It is known to couple dyes to beads made of polystyrene, which is a man made material, for use in calibrating flow cytometry apparatus. There is no disclosure, however of such polystyrene beads supported by a support medium, such as paraffin or other solids or semi-solids.

It is also known to couple antibodies against a pregnancy indicating hormone to sheep cells, for use in pregnancy tests. A sample of urine from a test subject is added to the sheep cells, and agglutination of the cells is observed visually. Agglutination of the sheep cells indicates the presence of the pregnancy indicating hormone in the sample, and hence pregnancy of the individual.

EP0345953 (Shandon Scientific Limited) describes the use of a test material to facilitate standadisation of immunostaining techniques. The test material comprises pellets of an absorbent gel which are caused to absorb an antigen of interest, and fixed. The pellets are cut from a solidified agar gel using a well cutter of 1.5 or 2.5mm diameter and are installed in individual wells in a block of a gel.

WO91/05263 (Battifora) describes a control which is a section or slice of a medium, in which cells which express a defined amount of a target molecule are embedded. The cells may include tissue culture cells, for example breast cancer cell lines, or transfected cells.

WO 00/23799 (Smith) describes the use of tumour cell lines which express p53 protein as standards for immunohistochemistry. The cells are standardised for quality by the American Type Culture Collection (ATCC). WO 00/23799 does not disclose coupling or chemical coupling of p53 to the cells.

## SUMMARY

We provide generally, according to the invention, reference standards for detectable entities. The detectable entity preferably comprises an entity whose presence or quantity it is desired to establish in a sample. The reference standards preferably contain a known or pre-determined quantity of the detectable entity, which is revealable directly or indirectly.

The reference standards described here are suitable for use in comparison against a sample which is suspected of containing the detectable entity, or in which the quantity of detectable entity is unknown. Comparison of the reference standard against the sample may thus be used to gauge the amount or the presence of the detectable entity in the sample. The reference standards may therefore be used to provide a reference quantity, a reference concentration, a reference signal, etc of the detectable entity, for use in comparisons.

In one embodiment of the invention, a reference standard comprises a compact particle which is a biological, preferably cellular compact particle. We therefore provide according to a 1st aspect of the invention, a reference standard for a detectable entity, the reference standard comprising an embedding medium, a compact particle having a compact shape with a quantity of detectable entity chemically coupled thereto and supported by the embedding medium, in which the compact particle is a biological, preferably cellular compact particle.

This embodiment is distinguished from the sheep cells described above, in that the sheep cells react with the pregnancy indicating hormone in the sample, and thereby indicate its presence by providing a YES / NO result. In contrast, our reference standards are not used for detection *per se,* but provide a passive standard or control against which an assay is to be judged. Furthermore, the detectable entity in our reference standard is chemically coupled to the compact particle of biological, preferably cellular, origin. In contrast, in the prior art the entity to be detected, i.e., the pregnancy indicating hormone, is in the sample and not attached or coupled to the sheep cells. The sheep cells therefore do not, and cannot, perform the function of providing a reference standard.

In a second embodiment of the invention, a reference standard comprises a compact particle which is supported by an embedding medium. We therefore provide, according to a 2nd aspect of the invention, a reference standard for a detectable entity, the reference standard comprising an embedding medium, a compact particle having a compact shape with a quantity of detectable entity coupled thereto and supported by the embedding medium, in which the compact particle is a non-biological compact particle having cell-like dimensions.

The term "cell-like dimension" is explained in detail later in this document, but can range from 1nm to less than 1500 micrometres, preferably around $100\mu m$.

Preferably, a detectable amount of the detectable entity is present in a defined region in a cross section of the reference standard. Preferably, the detectable entity adopts a compact shape, preferably an unextended or non-elongate shape, in the embedding medium. Preferably, the compact shape is such that the ratio of the longest dimension to the shortest dimension is less than 5:1, preferably less than 2:1

In preferred embodiments, the compact shape comprises a particulate, uniform or regular shape. The compact shape may comprise a sphere shape, an ovoid shape, an ellipsoid shape, a disc shape, a cell shape, a pill shape or a capsule shape.

More preferably, the detectable entity is one which the compact particle, such as a cell, does not express naturally. In preferred embodiments, the detectable entity is heterologous to the compact particle. The detectable entity may be chemically coupled to the compact particle where this is a non-biological compact particle.

Preferably, the biological compact particle comprises a cell or a virus. Preferably, the cell or virus does not express the detectable entity. The cell may be selected from the group consisting of: a micro-organism, a bacterial cell, a yeast cell, a eukaryotic cell, an insect cell, an animal cell, a mammalian cell, a mouse cell and a human cell. The cell preferably comprises an insect cell, preferably an Sf9 cell, or a mammalian cell, preferably a Chinese Hamster Ovary (CHO) cell.

Alternatively or in addition the biological compact particle may comprise an organelle. The organelle may comprise a mitochondrion, a plastid, a chloroplast, or a nucleus. The detectable entity is preferably substantially free of cellular material.

Alternatively or in addition, the compact particle may comprise a microbead or a micelle.

In highly preferred embodiments, the compact shape has a dimension of less than $1500\mu m$, preferably less than $1000\mu m$, less than $500\mu m$, less than $250\mu m$, less than $100\mu m$, preferably less than $50\mu m$, more preferably less than $20\mu m$, most preferably less than $10\mu m$.

The defined region may be present in at least one other cross section of the reference standard, preferably comprising a similar amount of detectable entity. The detectable entity may be embedded in the embedding medium.

In preferred embodiments, the detectable entity comprises a diagnostically relevant target. The detectable entity may comprise an antigen, an epitope, a peptide, a polypeptide, a protein, a nucleic acid, or two or more or a plurality of any of the above, or combinations of one or more of the above.

The detectable entity is preferably selected from the group consisting of: a hapten, a biologically active molecule, an antigen, an epitope, a protein, a polypeptide, a peptide, an antibody, a nucleic acid, a virus, a virus-like particle, a nucleotide, a ribonucleotide, a deoxyribonucleotide, a modified deoxyribonucleotide, a heteroduplex, a nanoparticle, a synthetic analogue of a nucleotide, a synthetic analogue of a ribonucleotide, a modified nucleotide, a modified ribonucleotide, an amino acid, an amino acid analogue, a modified amino acid, a modified amino acid analogue, a steroid, a proteoglycan, a lipid, a carbohydrate, a dye, and mixtures, fusions, combinations or conjugates of the above.

Preferably, the detectable entity is selected from the group consisting of a hapten, a biologically active molecule, an antigen, an epitope, a protein, a polypeptide, a peptide, an antibody, a nucleic acid, a virus, a virus-like particle, a nucleotide, a ribonucleotide, a deoxyribonucleotide, a modified deoxyribonucleotide, a heteroduplex, a nanoparticle, a synthetic analogue of a nucleotide, a synthetic analogue of a ribonucleotide, a modified nucleotide, a modified ribonucleotide, an amino acid, an amino acid analogue, a modified amino acid, a modified amino acid analogue, a steroid, a proteoglycan, a lipid, a carbohydrate, a dye, a diagnostically relevant target, preferably selected from the group consisting of an antigen, an epitope, a peptide, a polypeptide, a protein, a nucleic acid, or two or more or a plurality of any of the above, or mixtures, fusions, combinations or conjugates of one or more of the above.

In preferred embodiments, the detectable entity comprises any one or more of HER2, oestrogen receptor (ER), progesterone receptor (PR), p16, Ki-67, c-kit, laminin 5 gamma 2 chain and Epidermal Growth Factor Receptor (EGFR) protein, nucleic acids encoding such, and post-translationally modified forms, preferably phosphorylated forms of such.

The presence and/or quantity of the detectable entity may be revealable by a binding agent, preferably a labelled binding agent, which may be selected from the group consisting of: an antibody, preferably an antibody capable of specific binding to the detectable entity, a nucleic acid such as a DNA or an RNA, preferably a nucleic acid capable of specific binding to the detectable entity, a protein nucleic acid (PNA), a dye, a special stain, Au-chloride, Haematoxylin-Eosin (H & E), Gomori methenamine silver stain (GMS), Periodic Acid-Schiff (PAS) stain, Trichrome Blue, Masson's Trichrome, Prussian Blue, Giemsa, Diff-Quik, Reticulum, Congo Red, Alcian Blue, Steiner, AFB, PAP, Gram, Mucicarmine, Verhoeff-van Gieson, Elastic, Carbol Fuchsin and Golgi's stains.

The presence of the detectable entity in a cell, tissue, organ or organism is in highly preferred embodiments indicative of a disease or a condition. The defined region may include a reference area, the reference area comprising the detectable entity at a pre-defined amount. The amount of the detectable entity in the reference area is preferably compared to the amount of the detectable entity in a sample to determine the presence, quantity or concentration of the detectable entity in the sample.

The reference standard is preferably in the shape of a rectangular box.

According to a 3rd aspect of the invention, we provide a reference standard for a detectable entity, comprising:

(a) an embedding medium in a preferably substantially rectangular box shape; and (b) a cell with a quantity of detectable entity coupled thereto.

The reference standard may comprise two or more compact particles, each having detectable entity attached thereto. It may comprise two or more different detectable entities, each of which is attached to the same or different compact particle. It may comprise two or more compact particles comprising different amounts of detectable entity on each.

In preferred embodiments, a planar section of the reference standard comprises a plurality of areas on which are presented the detectable entity at different density. A planar section of the reference standard may comprise a first area comprising the detectable entity substantially at a diagnostically significant density.

The reference standard may further comprise a control comprising a compact particle which comprises substantially no detectable entity.

The embedding medium is in preferred embodiments selected from the group consisting of: ice, wax, paraffin, acrylic resin, methacrylate resin, epoxy, Epon, Araldite, Lowicryl, K4M and LR White and Durcupan.

According to a 4th aspect of the invention, we provide a planar section, preferably a transverse planar section, preferably of substantially uniform thickness, of a reference standard as described.

According to a 5th aspect of the invention, we provide a support, preferably a slide such as a microscope slide, comprising such a planar section mounted thereon.

According to a 6h aspect of the invention, we provide a kit comprising a reference standard as set out, together with a binding agent capable of specific binding to the detectable entity, optionally together with instructions for use.

According to an 7th aspect of the invention, we provide a reference standard, kit or a planar section as described, in which the reference standard has been stained, preferably with an antibody or a nucleic acid probe.

According to a 8th aspect of the invention, we provide a diagnostic kit for detecting the presence or amount of a detectable entity in a biological sample, comprising: (a) a reference standard, planar section or slide as described; (b) a binding agent capable of specific binding to the detectable entity; and optionally (c) instructions for use.

According to a 9th aspect of the invention, we provide a combination of a reference standard, planar section, support, kit or diagnostic kit as set out together with a therapeutic agent capable of treating or alleviating at least one of the symptoms of a disease or condition in an individual.

Preferably, the individual is diagnosed as suffering from or susceptible to the disease or condition, if the amount of detectable entity in the biological sample or component is similar to or greater than that in the reference standard. Preferably, the binding agent or therapeutic agent comprises an antibody against the detectable entity.

According to an 10th aspect of the invention, we provide use of a reference standard, a planar section or a kit as described, for determining the presence or amount of a detectable entity in a biological sample.

According to a 11th aspect of the invention, we provide a method of comparing the amount of a detectable entity in a biological sample with a reference standard, the method comprising the steps of (a) providing a biological sample and obtaining a first signal indicative of the amount of detectable entity in the biological sample, or a component thereof; (b) providing a reference standard, planar section, support, kit or diagnostic kit as set out above; (c) obtaining a second reference signal indicative of the amount of detectable entity in the reference standard or planar section thereof; and (d) comparing the first signal obtained in (a) against the reference signal.

Preferably, the detectable signal is selected from the group consisting of: radiation, optical density, reflectance, radioactivity, fluorescence, enzymatic activity.

Preferably, the reference standard or planar section thereof is subjected to the same one or more steps or conditions, preferably substantially all, as the biological sample, such as: mounting onto a slide, baking, deparaffination, rehydration, antigen retrieval, blocking, exposure to antibody, exposure to primary antibody, exposure to nucleic acid probe, washing, exposure to secondary antibody-enzyme conjugate, exposure to enzyme substrate, exposure to chromogen substrate, and counter staining.

The biological sample may comprise a cell, tissue or organ, preferably a cell, tissue or organ of an organism suspected of suffering a disease or condition.

According to a 12th aspect of the invention, we provide a method of obtaining an indication suitable for the diagnosis of a disease or a condition in an individual, the method comprising comparing the amount of a detectable entity in a biological sample from an individual or component thereof with a reference standard, by a method according to the 11th aspect of the invention. Preferably, the individual is diagnosed as suffering from or susceptible to the disease or condition, if the amount of detectable entity in the biological sample or component is similar to or greater than that in the reference standard.

According to a 13th aspect of the invention, we provide a method of treatment of a disease or a condition in an individual, the method comprising the steps of diagnosing the disease or condition in an individual by a method according to the 13th aspect of the invention, and administering a therapeutic agent to the individual.

Preferably, the therapeutic agent comprises an antibody capable of binding to the detectable entity.

According to a 14th aspect of the invention, we provide a method of assessing the effectiveness or success of a procedure, the method comprising the steps of: (a) providing a reference standard as set out above, in which a detectable property of the detectable entity is changed as a result of the procedure; (b) conducting the procedure on the reference standard; and (c) detecting a change in the detectable property of the detectable entity.

Preferably, a detectable property of the detectable entity is changed as a result of a successful procedure, which change in the detectable property of the detectable entity is detected to establish that the procedure is successful.

Preferably, a detectable property of the detectable entity is changed as a result of an unsuccessful procedure, which change in the detectable property of the detectable entity is detected to establish that the procedure is not successful. The procedure may be selected from the group consisting of: an *in situ* hybridisation procedure, an immunohistochemical procedure, deparaffination, antigen retrieval, blocking, endogenous biotin blocking, endogenous enzyme blocking, a washing step, incubation with revealing agent such as a primary antibody, incubation with secondary visualisation components, chromogen staining, staining information acquisition and analysis.

Preferably, the procedure is an antigen retrieval procedure, and in which the detectable property of the detectable entity comprises the masking or unmasking of one or more epitopes. Preferably, the detectable entity in the reference standard is modified to mask one or more epitopes, some or all of which are unmasked in an antigen retrieval procedure which is successful.

Preferably, the procedure is an deparaffination procedure, and in which the detectable property of the detectable entity comprises the presence or quantity of detectable entity in the reference standard following the deparaffination procedure. The detectable entity in the reference standard is preferably soluble in the deparaffination medium, and in wich at least a portion, preferably all, of the detectable entity is removed following a successful deparaffination procedure.

According to a 15th aspect of the invention, we provide use of a reference standard as described as an antigen retrieval validation standard, a deparaffination standard, a blocking validation standard, a washing validation standard, a primary antibody validation standard, a secondary antibody validation standard, a calibration standard, or a diagnostic standard.

According to a 16th aspect of the invention, we provide a method of producing a reference standard for a detectable entity, the method comprising the steps of: (a) providing an embedding medium; (b) providing a compact particle having a compact shape, in which the compact particle is a biological, preferably cellular compact particle, preferably a cellular compact particle ; (c) chemically coupling a quantity of detectable entity to the compact particle and (d) supporting or embedding the compact particle in the medium.

According to a 17th aspect of the invention, we provide a method of producing a reference standard for a detectable entity, the method comprising the steps of: (a) providing an embedding medium; (b) providing a compact particle having a compact shape, in which the compact particle is a non-biological compact particle having cell-like dimensions; (c) coupling a quantity of detectable entity to the compact particle and (d) supporting or embedding the compact particle in the medium.

According to a 18th aspect of the invention, we provide a method of producing a reference standard for a detectable entity, the method comprising the steps of: providing a compact particle having a compact shape of biological, preferably cellular origin, chemically coupling a quantity of detectable entity to the compact particle, and supporting or embedding the compact particle in an embedding medium.

According to a 19th aspect of the invention, we provide a method of producing a reference standard for a detectable entity, the method comprising the steps of: providing a compact particle having a compact shape, in which the compact particle is a non-biological compact particle having cell-like dimensions, coupling a quantity of detectable entity to the compact particle, and supporting or embedding the compact particle in an embedding medium.

According to a 20th aspect of the invention, we provide a of producing a reference standard for a detectable entity, the method comprising supporting a compact particle having a compact shape and cell-like dimensions having a quantity of detectable entity chemically coupled thereto in an embedding medium, in which the compact particle is a biological, preferably cellular compact particle, preferably a cellular compact particle.

According to a 21st aspect of the invention, we provide a method of producing a reference standard for a detectable entity, the method comprising supporting a compact particle having a compact shape and cell-like dimensions having a quantity of detectable entity coupled thereto in an embedding medium, in which the compact particle is a non-biological compact particle having cell-like dimensions.

According to a 22nd aspect of the invention, we provide a method of producing a reference standard for a detectable entity, the method comprising the steps of: (a) providing an embedding medium; (b) forming a quantity of detectable entity in a generally compact shape and cell-like dimensions by (i) chemically coupling to a compact particle which is a biological, preferably cellular compact particle, preferably a cellular compact particle, or (ii) coupling to a compact particle which is a non-biological compact particle having cell-like dimensions; and (c) embedding the detectable entity in the embedding medium.

The method may further comprise attaching a quantity of a second detectable entity to the compact particle, or to a second compact particle. It may further comprise attaching a second different quantity of the or each detectable entity to the or each compact particle. The each compact particle may be embedded in the embedding medium.

The or each detectable entity may be covalently coupled to its respective compact particle.

According to a 23rd aspect of the invention, we provide a reference standard for a detectable entity, comprising a detectable entity chemically coupled to a cell and embedded in an embedding medium.

According to a 24th aspect of the invention, we provide a method of producing a reference standard for a detectable entity, the method comprising the steps of providing a cell or a virus, chemically coupling a quantity of detectable entity to the cell or virus, and embedding the cell or virus in an embedding medium.

The cell preferably does not express the detectable entity. Preferably, the cell is selected from the group consisting of: a virus, a bacterial cell, a eukaryotic cell, an insect cell, preferably an Sf9 cell, an animal cell, a mammalian cell, preferably a Chinese Hamster Ovary (CHO) cell, a mouse cell and a human cell.

According to a 25th aspect of the invention, we provide a method of establishing a cellular distribution of detectable entity in a reference standard, the method comprising providing a cell or a component thereof which does not express a detectable entity, chemically coupling a quantity of detectable entity to the cell or component, and supporting the cell or component in an embedding medium.

The reference standard described here may conveniently be referred to as a "Reflector Cell".

## BRIEF DESCRIPTION OF THE FIGURES

Embodiments of the invention are now described in detail with reference by way of example to the accompanying drawings, in which:

Figure 1 shows a typical grading or scoring system of antigen staining for assessment of protein expression. Human breast tissue is stained for HER2 antigen with anti-HER2 antibody using HercepTest™, Dakocytomation code No. K 5204. Figure 1A: Score 0 (negative). No staining is observed, or membrane staining is observed in less than 10% of tumour cells; Figure 1B: Score 1+ (negative). A faint or barely perceptible membrane staining is detected in more than 10% of the tumour cells. The cells are only stained in part of the membrane; Figure 1C: Score 2+ (weakly positive). A weak to moderate complete membrane staining is observed in more than 10 % of the tumour cells; Figure 1D: Score 3+ (strongly positive). A strong complete membrane staining is observed in more than 10 % of the tumour cells.

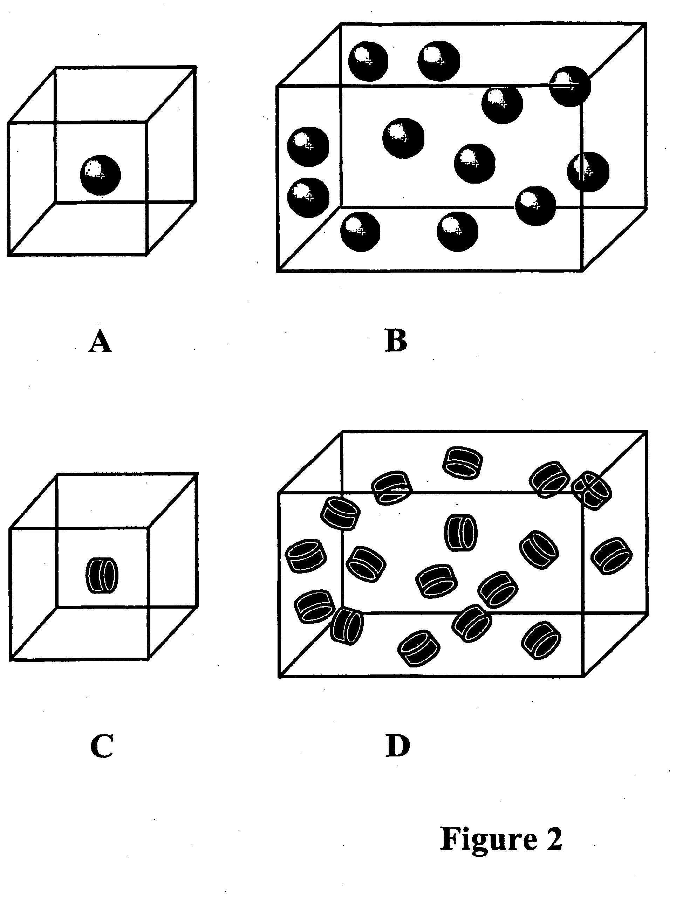

Figure 2 shows an embodiment of the reference standard described here. In this embodiment, a compact particle is modified, for example, chemically, with a detectable entity such as a diagnostically relevant target, and embedded in paraffin blocks. Figure 2A: reference standard comprising a single spherical compact particle; Figure 2B: reference standard comprising multiple spherical compact particles; Figure 2C: reference standard comprising a single compact particle in a flat cylinder shape; Figure 2D: reference standard comprising multiple flat cylinder compact particle, in random orientations. Slices from the reference standard may be IHC or ISH stained as any other "tissue".

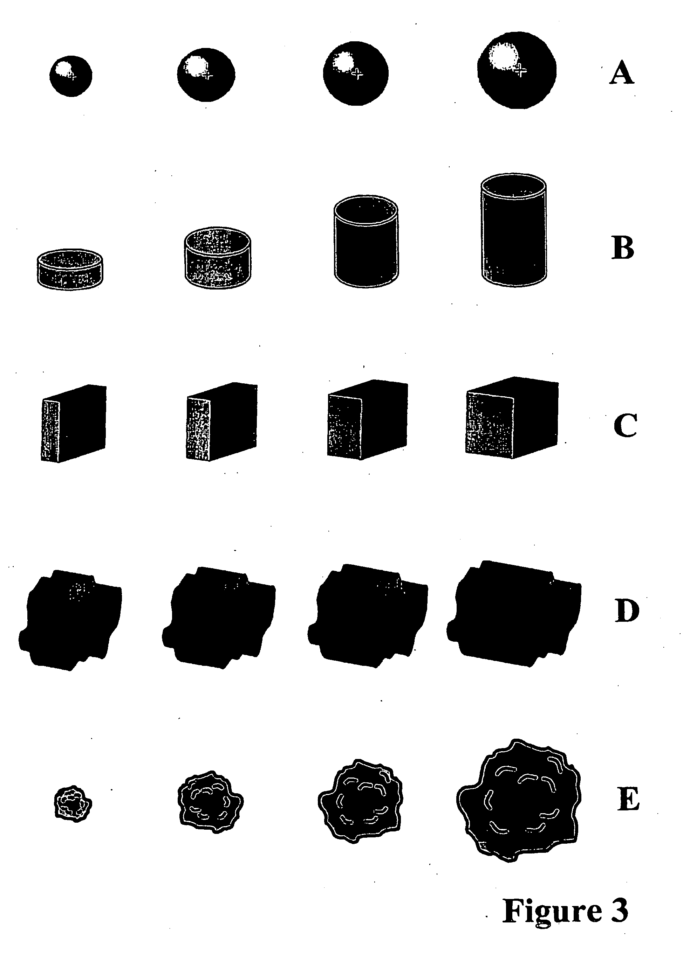

Figure 3 shows examples of compact particle shapes. Figure 3A: compact particle with a spherical shape. Figure 3B: compact particle in a cylindrical shape, including a flat cylinder shape. Figure 3C:compact particle with a tile shape or a cuboid, cube or cubical shape. Figures 3D and 3E show irregularly shaped compact particles.

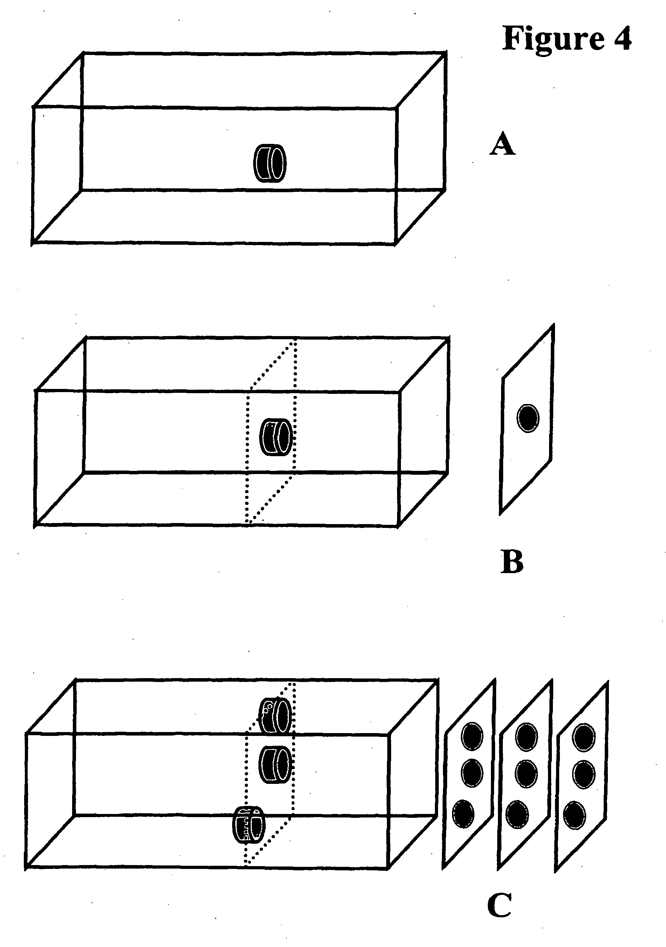

Figure 4 shows an embodiment of a reference standard having an elongate shape. The pattern of detectable entity in the slices and sections through portions of the reference standard shown in dotted lines are also shown. Figure 4A: the reference standard may comprise a single or multiple compact particles, here in a flat cylinder shape, supported in an embedding medium. The compact particle(s) have one or more detectable entities attached to them. Figure 4B: a cross section of the reference standard includes a defined area comprising a quantity of the detectable entity, preferably a defined quantity of detectable entity. Figure 4C: where multiple compact particles are arranged in the reference standard, one or more slices or sections of the reference standard will include more than one defined area comprising a quantity or quantities of the detectable entity.

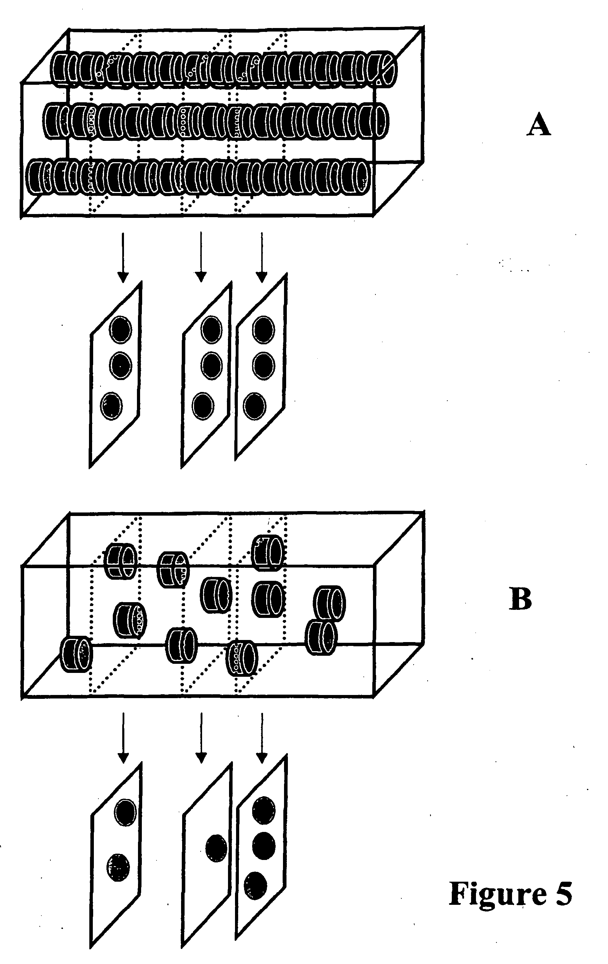

Figure 5 shows that multiple compact particles may be placed in bundles perpendicular to the cutting direction, and cut into thin slices containing uniform reference dots. Figure 5A shows an embodiment of the reference standard in which a plurality of compact particles are arranged in a uniform pattern in the embedding medium. Figure 5B shows an embodiment of the reference standard in which a plurality of compact particles are arranged in a non-uniform pattern in the embedding medium. The pattern of detectable entity in the slices and sections through portions of the reference standard shown in dotted lines are shown below.

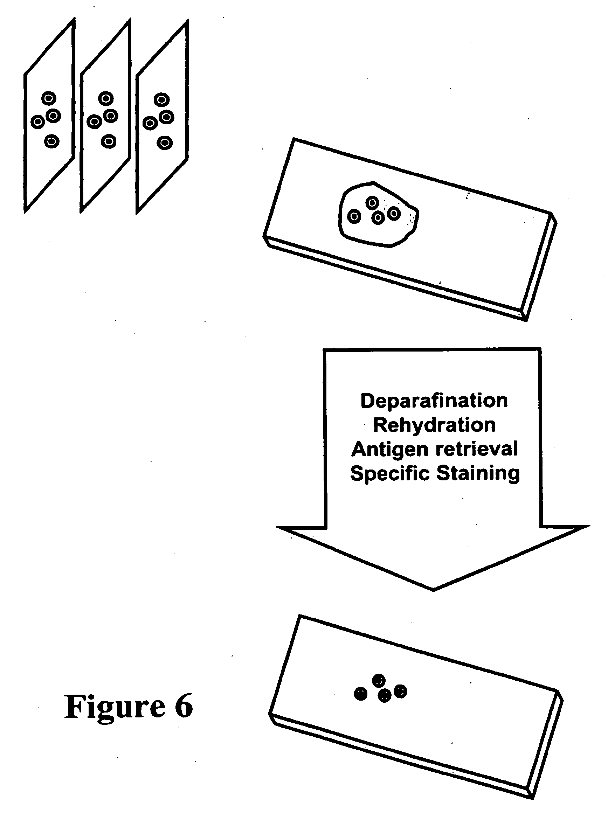

Figure 6 shows an example of how the reference standard, or a slice or section thereof may be processed, preferably in parallel with a biological sample which has been fixed, embedded and sliced.

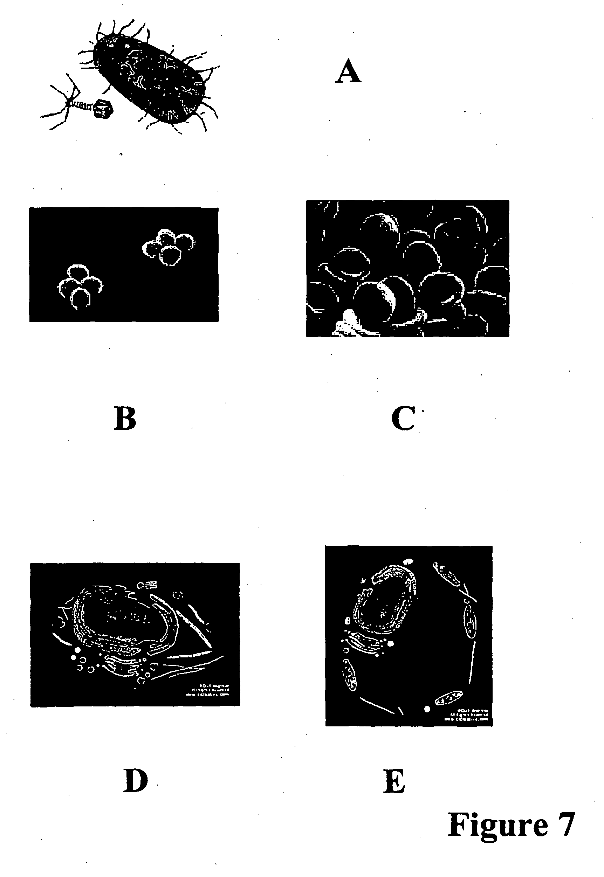

Figure 7 shows a number of examples of compact particles of cellular or biological origin. Figure 7A: bacteriophage or virus, bacteria; Figure 7B: bacterial cells, colonies of bacterial cells; Figure 7C: red blood cells, Figure 7D: an animal cell; Figure 7E: a plant cell.

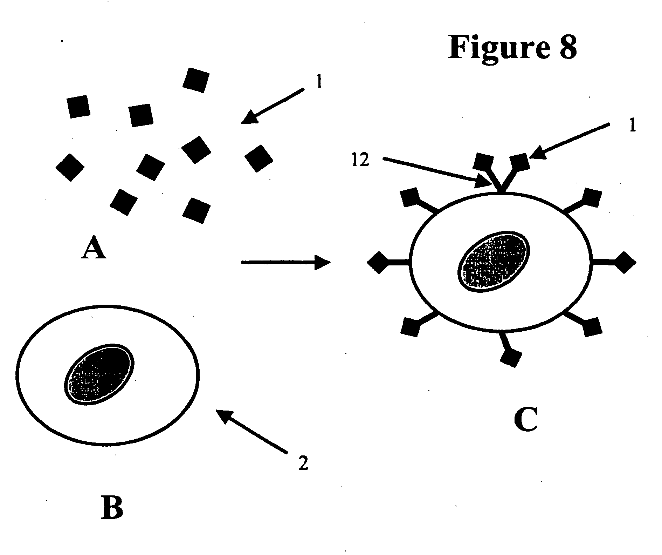

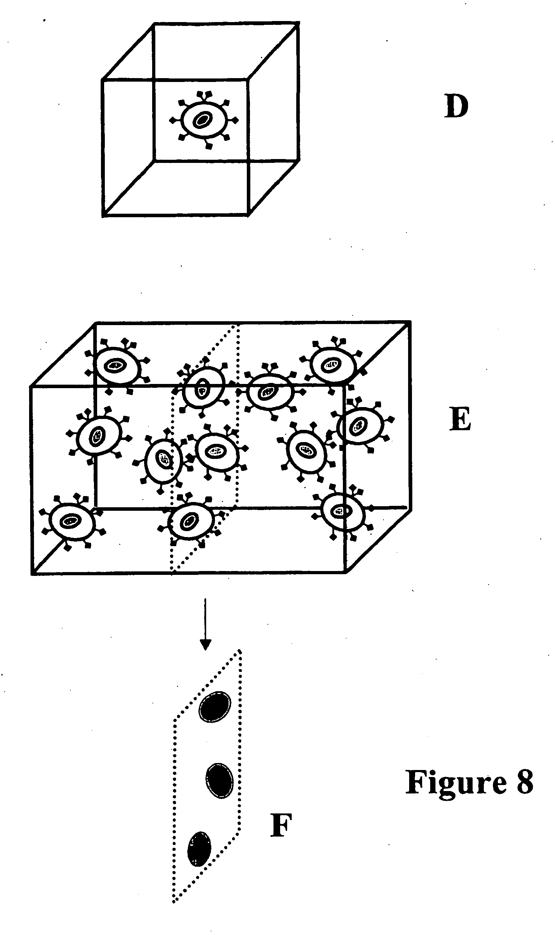

Figure 8 shows the steps in the manufacture of a reference standard which comprises a cellular compact particle; only binding to the surface of the cell is shown. Figure 8A: detectable entity; Figure 8B: cell; Figure 8C: cell with detectable entity attached thereto, optionally via a linker or spacer (12); Figure 8D: reference standard comprising cell with detectable entity attached thereto and supported in a supporting medium; Figure 8E: reference standard comprising plurality of cells with detectable entity attached thereto and supported in a supporting medium; Figure 8F: section or slice through a portion of the reference standard shown in dotted lines, showing defined areas comprising preferably defined quantities of detectable entity.

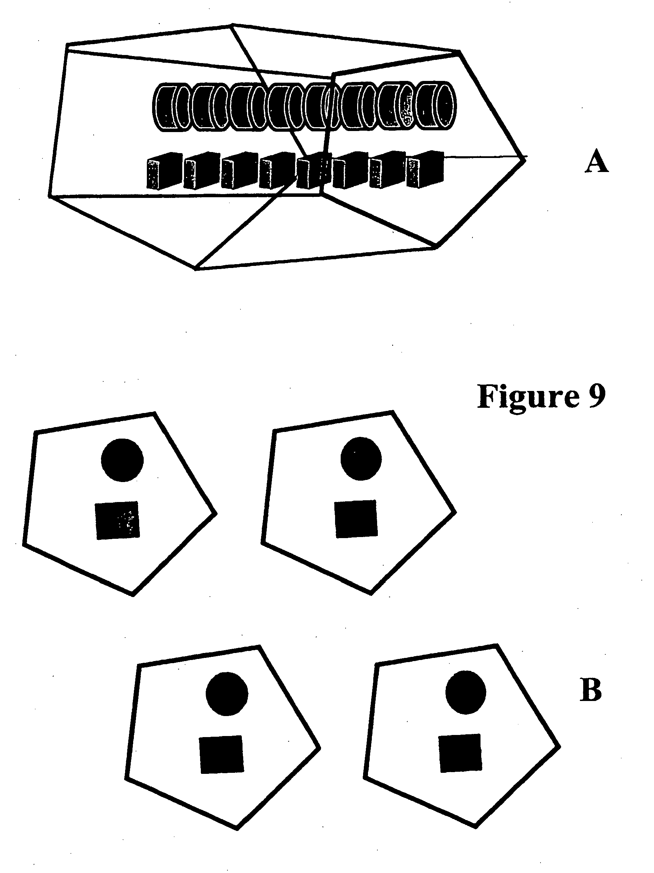

Figure 9A shows a further embodiment of the reference standard, in which the reference standard has a generally elongate shape with a pentagonal cross section. Two sets of differently shaped compact particles are supported by the embedding medium, and each of these is arranged in a linear fashion. Figure 9B shows cross sections of such a reference standard, which comprise two differently shaped areas comprising detectable quantities of the or each detectable entity.

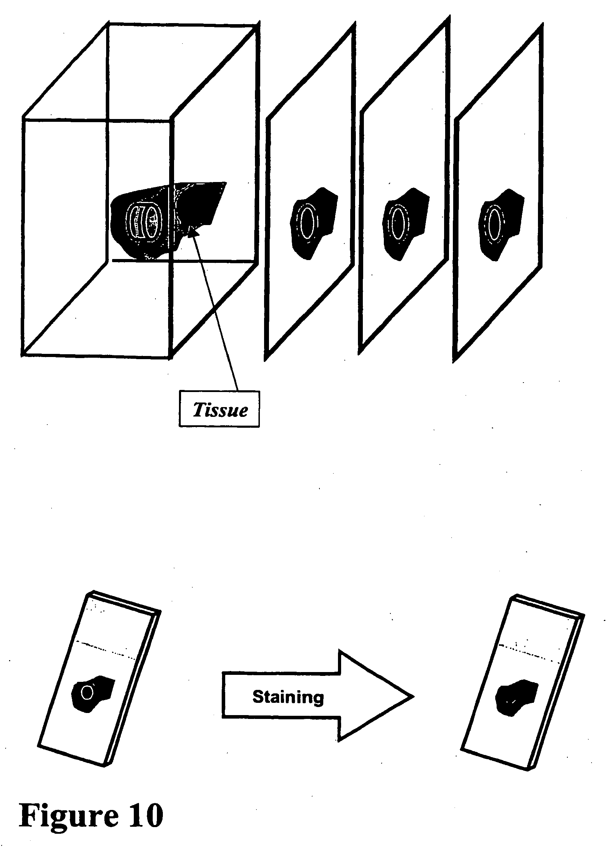

Figure 10 shows an embodiment of the reference standard described here, in which a sample comprising cells, tissues, etc is supported in the supporting medium together with the detectable entity on a compact particle. For example, a tissue sample and a compact particle with a detectable entity attached thereto may be embedded in the same medium. Slices or sections of the reference standard comprise a detectable amount of the detectable entity, together with the tissue, etc.

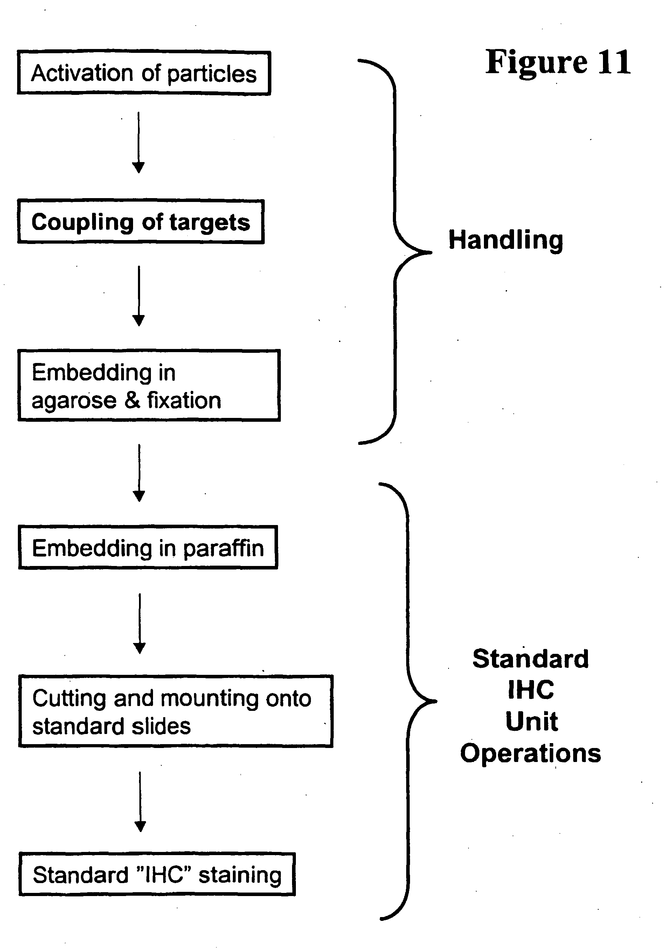

Figure 11 shows a flow chart of a method of producing and handling an embodiment of the reference described here. "Handling": compact particles such as cells are fixed and / or activated, targets comprising detectable entities are coupled to the compact particle, and the resulting coupled compact particle is embedded in agarose and fixed. "Standard IHC Unit Operations": the compact particles are embedded in paraffin, and cut into slices. The slices are mounted onto glass microscope slides, and subject to standard immunohistochemical (IHC) staining procedures.

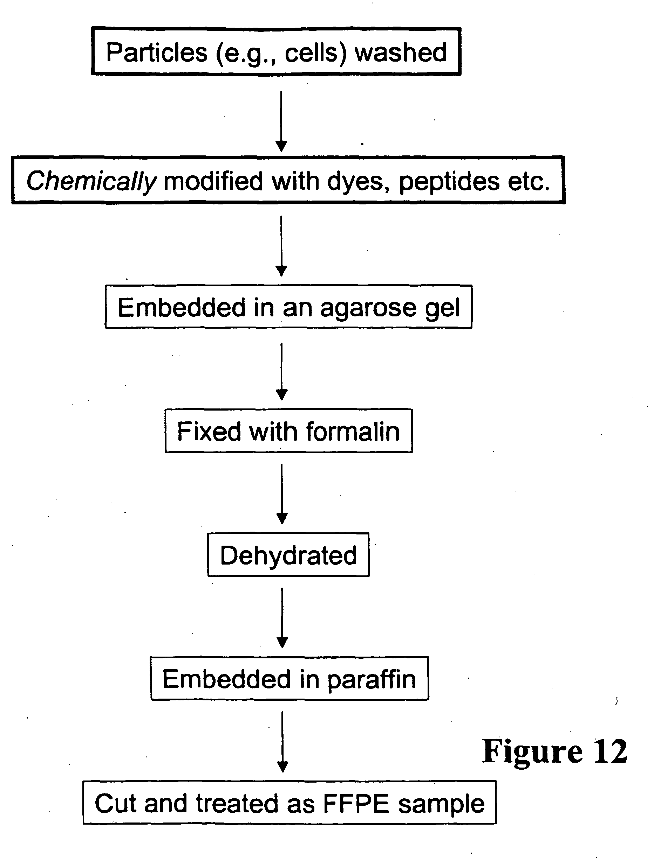

Figure 12 shows a flow chart of a method of producing and handling an embodiment of the reference standard described here. Compact particles such as cells are washed, then chemically modified with a suitable detectable entity such as a peptide or dye. The modified compact particles are optionally embedded in agarose gel, then fixed with formalin. The agarose blocks are then cut into long cylinders, dehydrated and embedded in paraffin. Slices are cut and treated as with other FFPE samples.

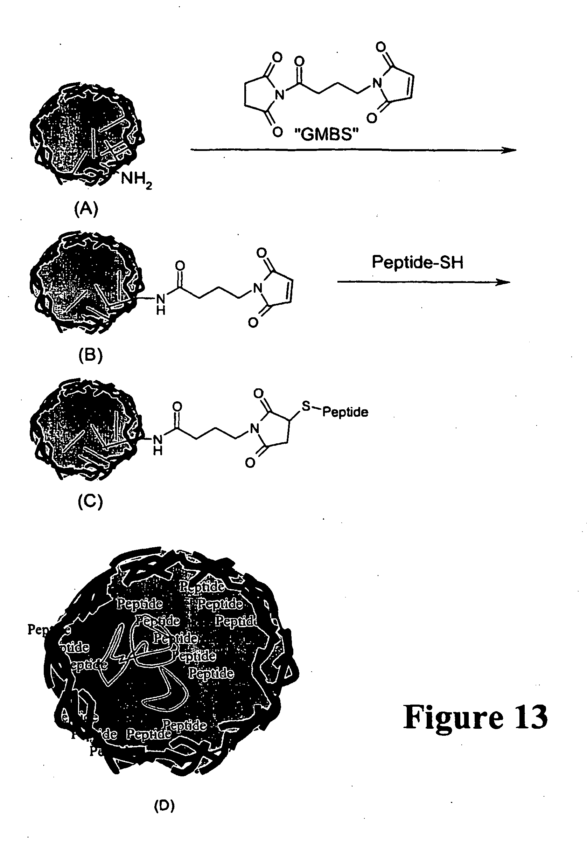

Figure 13 is a schematic illustration of the covalent coupling of sulfhydryl containing peptide to a particle (e.g., cell) using the heterobifunctional cross linker, N-(y-Maleimidobutyryloxy)-succinimide ester ("GMBS"). A fraction of the amino groups on the cells (Figure 13A) react with the NHS ester on the GMBS, resulting in a maleimide functionalised Particle (Figure 13B). Peptide with sulfhydryl group react chemo selectively with the maleimide group in the Particle, resulting in peptides covalently coupled to the Particle. (Figure 13C). The peptide is introduced to various parts of the Particle (Figure 13D), depending on the conditions during coupling.

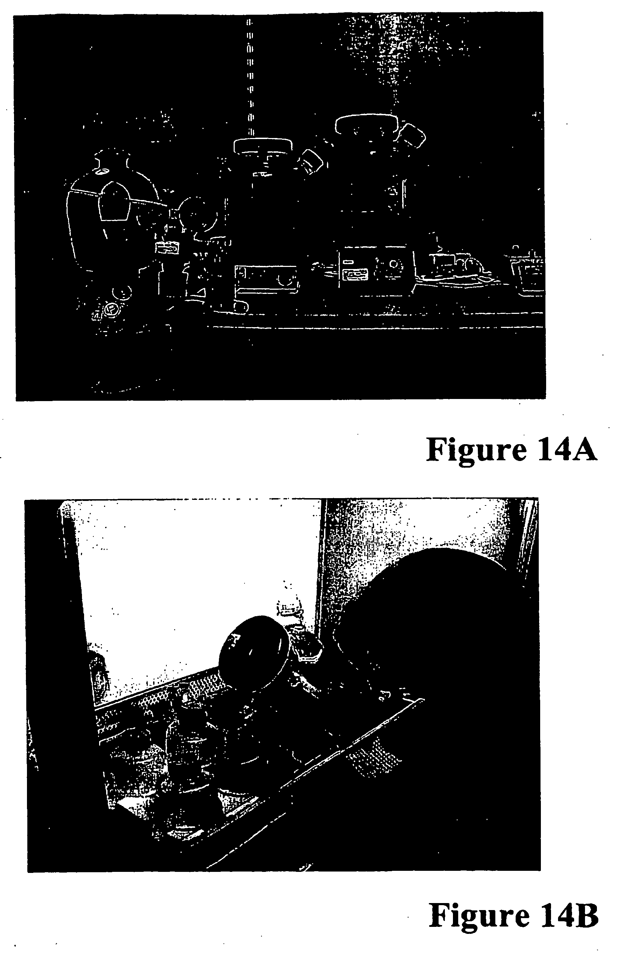

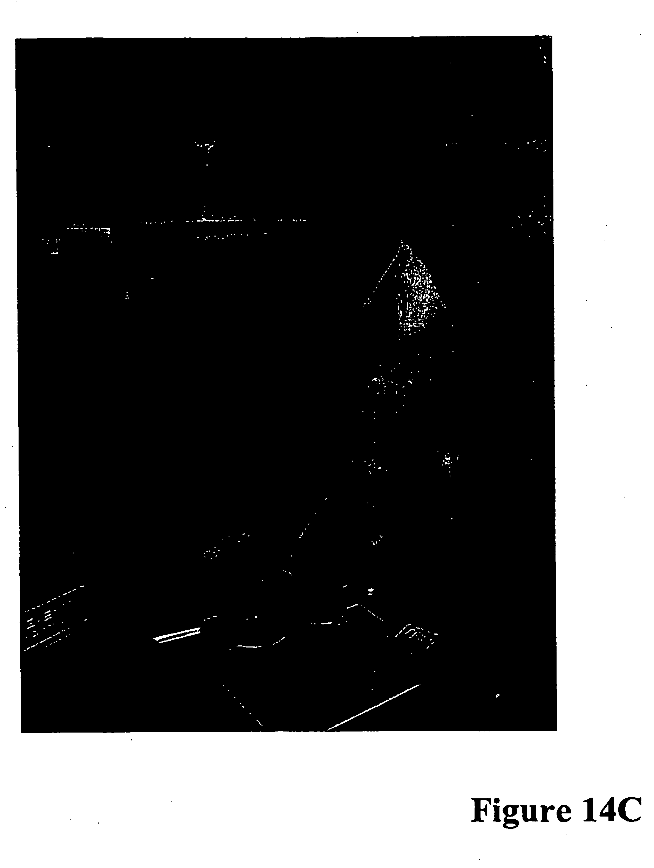

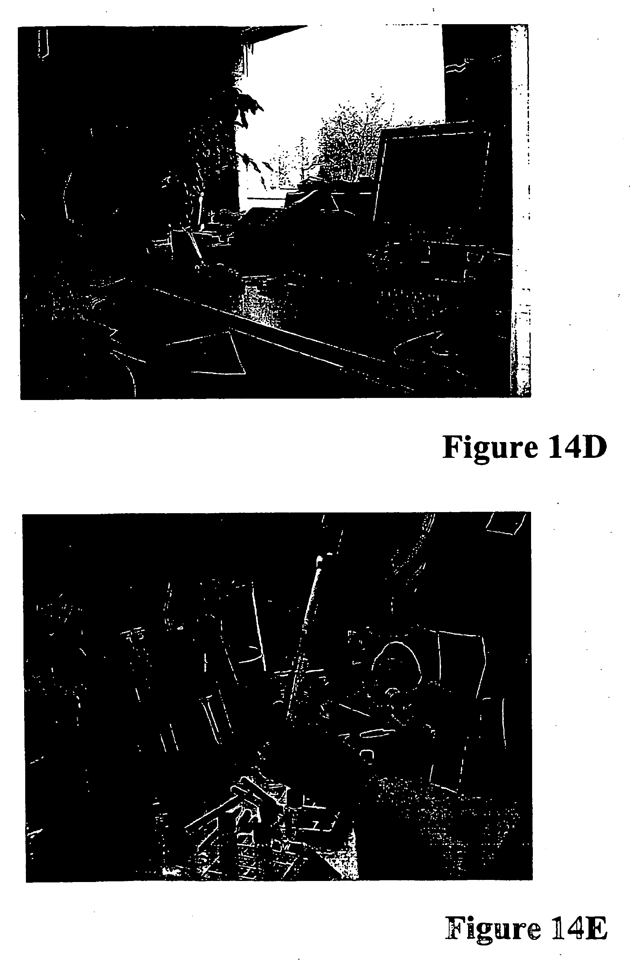

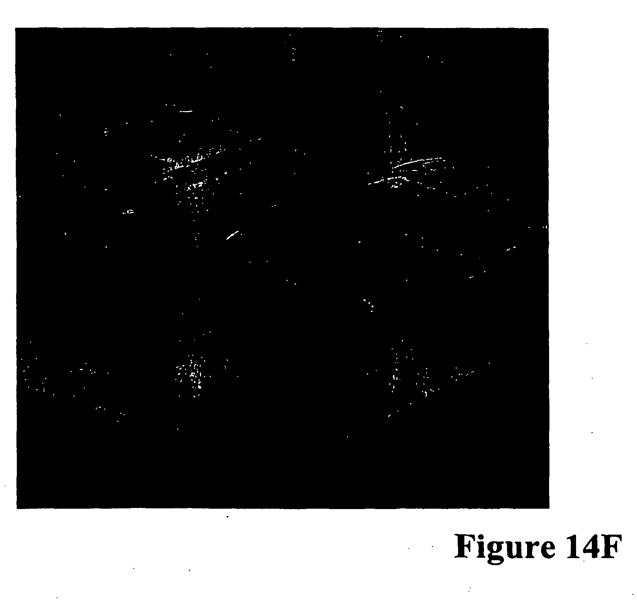

Figure 14 shows photographs illustrating part of the unit operations of making the reference system of the invention using e.g. intact insect sf9 cells and a formalin fixation and paraffin embedding (FFPE) procedure. The cells are grown as suspension culture in a controlled environment (Figure 14A), transferred to centrifuge containers (Figure 14B), and isolated from the media by centrifugation (Figure 14C).
Cells in suspension are washed and counted in a hemacytometer (Figure 14D) before being activation with a heterobifunctional cross linker (Figure 14E), coupling with a peptide, washed and processed as a FFPE preparation. The cells is embedding in agarose cylinders, fixed overnight and wrapped in lens cleansing paper and placed in a histocapsule for easy handling (Figure 14F) before being dehydrated and paraffin infiltrated.

Figure 15 shows the staining of Ki67 peptide modified CHO nuclei treated as a cytospin preparation. Haematoxylin control staining (Figure 15A). Immunovisualized using monoclonal antibody MIB1™ directed against the Ki-67 protein, HRP/Envision and DAB*plus.* (Figure 15B). The photographs are taken at 20x magnification

Figure 16 shows the staining of Ki67 peptide modified CHO nuclei treated in a FFPE preparation. Immunovisualized using monoclonal antibody MIB1™ directed against the Ki-67 protein, HRP/Envision and DAB*plus.* The photograph id taken at 20x magnification

Figure 17 shows the staining of Ki67 peptide modified CHO cell nuclei, treated in a FFPE preparation. A detergent is used during peptide conjugation. Immunovisualized using monoclonal antibody MIB1™ directed against the Ki-67 protein, HRP/Envision and DAB*plus.* The photograph are taken at 20x magnification

Figure 18 shows the staining of Ki67 peptide modified CHO cell nuclei, treated in a cytospin preparation. Immunovisualized using monoclonal antibody MIB1™ directed against the Ki-67 protein, followed by biotinylated Goat anti Mouse secondary antibody and alkaline phosphatase conjugated streptavidin (DAKOcytomation LSAB+) and finally Vector Red chromogenic substrate system. The stained nuclei photographed in a bright field microscope (Figure 18A) and in a fluorescence microscope (Figure 18B).

Figure 19 shows the staining of HER2 peptide modified CHO cells, treated as cytospin or FFPE preparations and staining of HER2 expressing Herceptest reference cells. Immunovisualized using rabbit antibody against the HER2 protein, HRP/Envision and DAB*plus.* Cytospin preparation of not prefixed cells (Figure 19A), prefixed cells (Figure 19B), FFPE preparation of not prefixed cells (Figure 19C), and FFPE preparation of prefixed cells (Figure 19D). FFPE preparation of Herceptest reference cells MDA-175 (Figure 19E) and SK-BR-3 (Figure 19F). The photographs are taken at 20x magnification:

Figure 20 shows the staining of Ki67 or irrelevant peptide modified CHO nuclei, treated in a cytospin preparation. Immunovisualized using monoclonal antibody MIB1™ directed against the Ki-67 protein, HRP/Envision and DAB*plus.* Cells modified with irrelevante HER2 peptide (Figure 20A), Cells modified with low (Figure 20B) and high (Figure 20C) concentration of Ki67 peptide (Figure 20B). The photographs are taken at 20x magnification.

Figure 21 shows the staining of p16 peptide modified and prefixed sf9 cells, treated as a FFPE preparation. The cells are treated with a permeabilization reagent. Immunovisualized using monoclonal antibody p16 clone E6H4, HRP/Envision and DAB*plus.* The photograph are taken at 20x magnification.

Figure 22 shows the staining of p16 peptide modified and prefixed sf9 cells, treated as FFPE preparations. The cells is treated with a permeabilization reagent and coupled with known mixtures of relevant and irrelevant peptides. Immunovisualized using monoclonal antibody p16 clone E6H4, HRP/Envision and DAB*plus.* Stained cells using 30 eq. (Figure 22A), and 10 eq. Irrelevant peptide (Figure 22B). The photographs are taken at 20x magnification

Figure 23 shows the staining of p16 peptide modified and prefixed sf9 cells, treated as FFPE preparations. The cells are treated with a permeabilization reagent and coupled with a known mixture of relevant and irrelevant peptide. Immunovisualized using monoclonal antibody p16 clone E6H4, HRP/Envision and DAB*plus.* Reproduced four times (Figure 23A, B, C and D). The photographs are taken at 20x magnification..

Figure 23E is the average fluorescent signal density for the flourescein tagged targets per cell for each of the four preparations as measured in a flowcytometer.

Figure 24 shows the staining of p16 peptide modified and prefixed sf9 cells, treated as FFPE preparations. The cells are treated with a permeabilization reagent and coupled with a different known mixtures of cross linker and relevant and irrelevant peptide. Immunovisualized using monoclonal antibody p16 clone E6H4, HRP/Envision and DAB*plus.* Figure 24A, B and C are photographs of the stained cells taken at 40 times magnification with (A) 3x excess irrelevant peptide, (B) 6x excess irrelevant peptide and (C) 10x excess irrelevant peptide, respectively.

Figure 25 shows the staining of p16 peptide modified and prefixed sf9 cells, treated as in a ThinPrep (Figure 25A) or Autocyte/TriPath (Figure 25B) cytological preparations. Immunovisualized using monoclonal antibody p16 clone E6H4, HRP/Envision and DAB*plus.* The photographs are taken at 20x magnification

Figure 26 shows flowcytometer diagrams of unstained prefixed HER2/neu modified CHO cells. Distribution of cells in a Forward Scatter (FSC-H) vs. Side Scatter (SSC-H) dot plot (Figure 26A), FL1-H versus counts (Figure 26B), and FSC-H versus FL-H (Figure 26C).

Figure 27 shows flowcytometer diagrams of prefixed HER2/neu modified CHO cells. Distribution of activated CHO cells immuno-labelled with FITC conjugated Rabbit $F(ab)_2$ negative control IgG pool (DAKO X0929) and a fluorescein isothiocyanate (FITC) labelled Swine $F(ab)_2$ Anti-Rabbit Ig antibody

Figure 28 shows flowcytometer diagrams of prefixed HER2/neu modified CHO cells. Distribution of activated CHO cells labelled with HER2/neu peptide and immuno-labelled with Rabbit Anti-HER2/*neu* antibody and a fluorescein isothiocyanate (FITC) labelled Swine F(ab)$_2$ Anti-Rabbit Ig.

Figure 29 shows flowcytometer diagrams of mildly prefixed HER2/neu modified CHO cells. Flow diagrams of prefixed HER2/neu modified CHO cells. Distribution of cells in a Forward Scatter (FSC-H) vs. Side Scatter (SSC-H) dot plot (Figure 29A), FL1-H versus counts (Figure 29B), and FSC-H versus FL-H (Figure 29C).

Figure 30 shows flowcytometer diagrams of prefixed HER2/neu modified CHO cells. Distribution of activated CHO cells immuno-labelled with FITC conjugated Rabbit F(ab)$_2$ negative control IgG pool (DAKO X0929) and a fluorescein isothiocyanate (FITC) labelled Swine F(ab)$_2$ Anti-Rabbit Ig antibody

Figure 31 shows flow diagrams of prefixed HER2/neu modified CHO cells. Distribution of activated CHO cells labelled with HER2/neu peptide and immuno-labelled with Rabbit Anti-HER2/*neu* antibody and a fluorescein isothiocyanate (FITC) labelled Swine F(ab)$_2$ Anti-Rabbit Ig.

Figure 32 shows flowcytometer single parameter linear histogram showing calibrator beads (Dakocytomation Fluorospheres) containing six different fluorescence intensities used for daily monitoring of the flow cytometer and standardisation of the signal.

Figure 33 shows flow diagrams of prefixed HER2/neu modified CHO cells using high concentration of cross linker. Distributions of unstained cells in a Forward Scatter (FSC-H) vs. Side Scatter (SSC-H) dot plot (Figure 33A), FL1-H versus counts (Figure 33B), and FSC-H versus FL-H (Figure 33C).

Figure 34 shows flow diagrams of prefixed HER2/neu modified CHO cells using high (1.00 $\mu$M GMBS) concentration of cross linker. Distributions of CHO cells immuno-labelled with Rabbit F(ab)$_2$ negative control IgG pool and a fluorescein isothiocyanate labelled Swine F(ab)$_2$ Anti-Rabbit Ig antibody

Figure 35 shows flow diagrams of prefixed irrelevant (HER3) peptide modified CHO cells using high concentration of cross linker. Distributions of CHO cells immuno-labelled with fluorescein isothiocyanate labelled Rabbit Anti-HER2/*neu* antibody.

Figure 36 shows flow diagrams of prefixed HER2/neu modified CHO cells using low (0.04 $\mu$M GMBS) concentration of cross linker. Distributions of CHO cells immuno-labelled with fluorescein isothiocyanate labelled Rabbit Anti-HER2/*neu* antibody.

Figure 37 shows flow diagrams of prefixed HER2/neu modified CHO cells using medium (0.20 $\mu$M GMBS) concentration of cross linker. Distributions of CHO cells immuno-labelled with fluorescein isothiocyanate labelled Rabbit Anti-HER2/neu antibody.

Figure 38 shows flow diagrams of prefixed HER2/neu peptide modified CHO cells using high (1.00 $\mu$M GMBS) concentration of cross linker. Distributions of CHO cells immuno-labelled with fluorescein isothiocyanate labelled Rabbit Anti-HER2/neu antibody.

Figure 39 shows flowcytometer diagrams of unstained prefixed HER2/neu peptide modified sf9 cells. Distribution of cells in a Forward Scatter (FSC-H) vs. Side Scatter (SSC-H) dot plot (Figure 39A), FL1-H versus counts (Figure 39B), and FSC-H versus FL-H (Figure 39C).

Figure 40 shows flowcytometer diagrams of unstained prefixed HER2/neu peptide modified sf9 cells. Distributions of sf9 cells immuno-labelled with Rabbit F(ab)$_2$ negative control IgG pool and a fluorescein isothiocyanate labelled Swine F(ab)$_2$ Anti-Rabbit Ig antibody

Figure 41 shows flowcytometer diagrams of unstained prefixed PhosphoHER2 peptide modified sf9 cells. Distribution of cells in a Forward Scatter (FSC-H) vs. Side Scatter (SSC-H) dot plot (Figure 39A), FL1-H versus counts (Figure 39B), and FSC-H versus FL-H (Figure 39C).

Figure 42 shows flowcytometer diagrams of unstained prefixed PhosphoHER2 peptide modified sf9 cells. Distributions of sf9 cells immuno-labelled with Rabbit F(ab)$_2$ negative control IgG pool and a fluorescein isothiocyanate labelled Swine F(ab)$_2$ Anti-Rabbit Ig antibody

Figure 43 shows flowcytometer diagrams of unstained prefixed HER2/neu peptide modified sf9 cells. Distribution of activated sf9 cells labelled with HER2/*neu* peptide and immuno-labelled with Rabbit Anti-HER2/*neu* antibody and a fluorescein isothiocyanate (FITC) labelled Swine F(ab)$_2$ Anti-Rabbit Ig.

Figure 44 shows flowcytometer diagrams of unstained prefixed PhosphorHER2 peptide modified sf9 cells. Distribution of activated sf9 cells labelled with *HER2*/*neu* peptide and immuno-labelled with mouse anti PhosphorHER2 (DAK-H2-PY-1248) antibody and a fluorescein isothiocyanate (FITC) labelled rabbit F(ab)$_2$ Anti-Mouse Ig.

Figure 45 shows the DAB staining of PEGA resin: (A) coupled with 1 mM Fitc, (B) coupled with 10 mM FITC, (C) negative antibody control on PEGA coupled with 10 mM Fitc, (D) negative EnVision control and (E) unmodified PEGA resin, immunovisualized using a antibody conjugate directed against the Fitc, HRP/Envision and DAB*plus* and taken at 20 times magnification in a bright field microscope. (F) is the 10mM FITC modified resin, and (G) is the unmodified resin, both taken in a fluorescent microscope, at 10 times magnification.

Figure 46 shows multiple pictures of the DAB staining of PEGA resin modified with 0.5 mg Rabbit IgG/ml immunovisualized using a antibody conjugate directed against the Fitc, HRP/Envision and DAB*plus* and taken at (A) 20 times and (B) 10 time magnification.

Figure 47 shows multiple pictures of the DAB staining of PEGA resin modified with 1.5 mg Rabbit IgG/ml immunovisualized using a antibody conjugate directed against the Fitc, HRP/Envision and DAB*plus* and taken at (A) 20 times and (B) 10 time magnification.

Figure 48 shows the control DAB staining of PEGA resin. (A) EnVision negative control using goat anti mouse conjugates, (B) unmodified Mini Leak matrix, all taken at 10 times magnification.

Figure 49 shows the DAB staining of Mini Leak coupled with (A) 0,0 g/l, (B) 0,01 g/l, (C) 0,05g/l, (D) 0,10g/l, (E) 0,15g/l or (F) 0,19g/l HER2 target peptide, respectively. Immunovisualized using HercepTest™ with a monoclonal antibody directed against the HER2 protein, HRP/Envision and DAB*plus.* All photomicrographs were taken at 20 times magnification in a bright field microscope.

Figure 50 shows various control stainings of modified Mini leak matrix cells and reference cells. (A) Negative primary antibody control and (B) Envision negative control on HER2/p16 modi fed Minileak cells, (C) unmodified Mini leak matrix stained with the Herceptest and (D), (E)F and (F) HercepTest™ control slides.

Figure 51 shows the DAB staining of Mini Leak coupled with (A) 0,0 g/l, (B) 0,19g/l, (C) 0,15g/l, (D) 0,10g/l, (E) 0,05g/l or (F) 0,01g/l p16 target peptide, respectively. Immunovisualized using CINtec™ p16$^{INK4a}$ cytology kit with a monoclonal antibody directed against the p16 protein, HRP/Envision and DAB*plus.* All photomicrograph were taken at 20 times magnification in a bright field microscope.

Figure 52 shows various control stainings of modified Mini leak matrix cells. (A) Negative primary antibody control and (B) Envision negative control on HER2/pl6 modified Minileak cells, and (C) unmodified Mini leak matrix stained with the CINtec™ p16$^{INK4a}$ kit.

## DETAILED DESCRIPTION

The practice of the present invention will employ, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; J. M. Polak and James O'D. McGee, 1990, In situ Hybridization: Principles and Practice; Oxford University Press; M. J. Gait (Editor), 1984, Oligonucleotide Synthesis: A Practical Approach, Irl Press; D. M. J. Lilley and J. E. Dahlberg, 1992, Methods of Enzymology: DNA Structure Part A: Synthesis and Physical Analysis of DNA Methods in Enzymology, Academic Press; Using Antibodies : A Laboratory Manual : Portable Protocol NO. I by Edward Harlow, David Lane, Ed Harlow (1999, Cold Spring Harbor Laboratory Press, ISBN 0-87969-544-7);

Antibodies : A Laboratory Manual by Ed Harlow (Editor), David Lane (Editor) (1988, Cold Spring Harbor Laboratory Press, ISBN 0-87969-314-2), 1855. Handbook of Drug Screening, edited by Ramakrishna Seethala, Prabhavathi B. Fernandes (2001, New York, NY, Marcel Dekker, ISBN 0-8247-0562-9); and Lab Ref: A Handbook of Recipes, Reagents, and Other Reference Tools for Use at the Bench, Edited Jane Roskams and Linda Rodgers, 2002, Cold Spring Harbor Laboratory, ISBN 0-87969-630-3.

## REFERENCE STANDARD

We describe novel reference standards which may be used to standardise and grade samples in any staining procedure, including immunohistochemical (IHC) or *in situ* hybridisation (ISH) procedures, and flow cytometry procedures, as well as methods of using and making the reference standards described here. Our reference systems may be manufactured using relatively simple procedures.

A reference standard as described here comprises at least the following components: (i) a quantity of a relevant detectable entity, and (ii) a compact particle having a compact shape. The detectable entity is coupled or chemically coupled to the compact particle. The compact particle may in some embodiments comprise a biological, preferably cellular compact particle. In other embodiments, the compact particle comprises a non-biological compact particle. The reference standard further comprises an embedding medium which supports the compact particle, and may preferably in the form of a block.

Biological compact particles are anatomical, histological or cellular in nature. They are typically entities such as viruses, cells, tissues, organelles, etc. They are derived typically directly from biological material, and may be used in such a state. However, some limited processing may have taken place. Typically, this is limited to simple steps such as isolation from other similar entities (e.g., separating a cell of interest from another cell), washing, permeabilisation etc. Biological compact particles have not undergone significant or substantial purification or refinement. Biological materials have some or more characteristics, typically substantially all characteristics, of the origin material, such as size, shape, colour, composition, etc. In preferred embodiments, biological compact particles retain at least the structure, preferably the cytoskeletal structure, of the origin material. Biological compact particles are preferred embodiments, such as use as reference standards in flow cytometry, where analysis is dependent on the ability to scatter light, and it is difficult to mimic the scattering characteristics of true cells using synthetic material.

Biological compact particles are not purified to any substantial extent that their components are separated from each other. As such, biological compact particles specifically do not include purified components which may once have formed part of biological material, such as agar or agarose. Biological compact particles are therefore typically complex and heterogenous in composition.

Non-biological compact particles, on the other hand, are molecular in nature. They are typically chemical or biochemical entities such as molecules or compounds, including complex molecules and macromolecules such as polypeptides, nucleic acids, etc. Examples of non-biological compact particles include microbeads, agar chunks, silica particles, etc. They are typically homogenous in composition, and are usually pure or isolated from other chemical or biochemical entities.

Non-biological compact particles are cheap and easy to make and handle. They may carry less risk to the handler than biological material. Embodiments where non-biological compact particles are preferred include use as procedural validation standards, where the mimicking of cell characteristics is less important than, say, flow cytometry.

In some embodiments, the compact particle has cell-like dimensions, by which we mean that the compact particle has in at least in some aspects, one or more features of a cell. Such features preferably correspond to external features of a cell, preferably dimension, such as such as size, shape, geometry. In highly preferred embodiments, the dimension comprises length, height, width, radius, etc. Preferably, the dimension is the maximum distance between any two points of the compact particle. Accordingly, in preferred embodiments, the compact particle has a maximum dimension which corresponds to that of a typical cell. In particular, the compact particle preferably has a feature of dimension of a cell or tissue, that is to say, the compact particle is comparable in size to the cell or tissue. Specific dimensions which are preferred are set out below.

Preferably, the presence of the cell-like dimension in the compact particle enables it to mimic the cell in at least some respects so that, when examined next to a cell or tissue, as the case may be, the compact particle appears similar to that cell or tissue. The similarity extends to, but does not encompass or include, the presence or absence of then detectable entity. That being so, a comparison between the compact particle and the cell or tissue will enable the observer to determine the minimal difference between the two, this difference being the presence or absence of the detectable entity in or on the cell or tissue. The compact particle can therefore be used as a reference standard for a detectable entity present or not present in the cell or tissue.

The detectable entity is attached by being chemically coupled, preferably chemically coupled, to the compact shape. The compact particle has a shape which its compact, non-elongate or unextended. It may comprise a "biological" compact particle, such as a cell, tissue, organelle, etc, or it may comprise a "non-biological" compact particle, such as a microbead, agarose bead or a latex bead. "Biological" and "non-biological" compact particles are described in more detail below. In some embodiments, the compact particle comprises a cell. In preferred embodiments, the cell does not express the detectable entity. Examples of such compact particles are provided in greater detail below.

Referring to the Figures, Figure 2A shows a reference standard according to this document, having a cuboid shape. The reference standard comprises a compact particle having a compact shape, in this case a sphere, supported by an embedding medium. Figure 2C shows a reference standard in a cuboid shape comprising a compact particle in the shape of a disc or flat cylinder. A quantity of detectable entity is attached to the compact particle, as described in further detail below (not shown in Figures 2).

The reference standard may comprise a single compact particle (Figures 2A and 2C) or it may comprise more than one compact particle, for example, a plurality of compact particles (Figures 2B and 2D). In the latter case, where the compact particle is asymmetric, or non-uniform in shape, the compact particles may be in the same orientation relative to each other, or as shown in Figure 2D, in different orientations. Where more than one compact particle is supported by the embedding medium, the compact particles may have the same shape, or different shapes (e.g., a reference standard comprising a spherical compact particle and a disc or flat cylinder shaped compact particle). A reference standard comprising two differently shaped compact particles is illustrated in Figure 9A and a cross section of such a standard in Figure 9B.

The compact particle can be of any shape, so long as this is a "compact" shape, as defined below. Examples of compact particle shapes are provided in detail below. The shapes may be regular or non-regular, amorphous or uniform.

Figure 3 shows examples of such shapes. Figure 3A shows a compact particle with a spherical shape, which is the most "compact" shape of all possible shapes. Figure 3B shows a compact particle in a cylindrical shape, including a flat cylinder shape. Figure 3C shows a compact particle with a tile shape or a cuboid, cube or cubical shape. Figures 3D and 3E show irregularly shaped compact particles.

The compact particles may harbour the same detectable entity, or different detectable entities. There may be one, two, three, four, five or more, or a plurality of detectable entities in the reference standard. These may be attached by being coupled or chemically coupled to the same compact particle, or to different compact particles. The same or different detectable entities may be coupled in the same or different quantities, to the or each compact particle. Various combinations can be envisaged by the skilled reader.

In preferred embodiments, the reference standard is such that a quantity of the detectable entity, preferably a detectable amount of the detectable entity, is present in a defined region, preferably a reference area, in a cross section of the reference standard. This is illustrated in Figure 4.

Referring to Figure 4, Figure 4A shows an embodiment of a reference standard, here in the shape of a rectangular box. Figure 4B shows a transverse section of the reference standard. A quantity (preferably a detectable amount) of the detectable entity is disposed in a defined area in the cross section. Where more than one compact particle is suspended in the embedding medium, more than one defined area comprising a quantity of the or each detectable entity may be present in the or each cross section, as shown in Figure 4C.

The compact particles, with the detectable entity or entities coupled or chemically coupled thereto, may be arranged in the embedding medium. Thus, the compact particles may form a bundle, or a bunch, or any sort of defined pattern within the reference standard. As shown in Figure 5A, for example, the compact particles may be arranged in a linear fashion, head to tail, or in a row. Multiple lines or rows of compact particles may be formed. Figure 9A also shows a reference standard comprising a number of compact particles (here in two different shapes) arranged in a line.

Different transverse sections of such a reference standard along the long axis of the reference standard show similar patterns of defined areas or reference areas comprising quantities of the detectable entity, as shown in Figure 5A (lower portion). This is also illustrated in Figure 9B, where two differently shaped defined areas or reference areas (here a square and a circular shape) are produced in cross section, as a result of the reference standard comprising two differently shaped compact particles.

Where the pattern of arrangement of compact particles is uniform along the length of the reference standard, this results in a configuration where each slice or section gives the same or similar pattern of reference areas. This may be preferred for manufacturing purposes.

Alternatively, or in addition, the compact particles may be disposed in a more loose pattern or even randomly in the reference standard. As shown in Figure 5B, the compact particles are supported by the embedding medium in the reference in a non-uniform manner. In this case, the three sample sections or slices taken of the reference standard show different patterns of reference areas in the cross sections.

The detectable entity is one whose presence and preferably quantity is revealable, that is, its presence is demonstrable or its amount is measurable, either directly or indirectly. It may be visible to the naked eye, or visible with the aid of magnification such as the use of a microscope. The detectable entity may be visible only when stained with a dye. The detectable entity which is embedded or supported may take a variety of forms, as described in further detail below. The detectable entity is preferably one which is detectable by use of a binding agent, which is capable of binding to the detectable entity and revealing its presence. The detectable entity may in particular comprise protein, peptide or polypeptide, or nucleotide, nucleic acids, in particular, DNA and RNA.

Further details on detectable entities suitable for use in the reference standard described here are provided later in this document.

Preferably, the reference standard is such that a cross section of it includes a defined area comprising a known or pre-defined amount of detectable entity. Where the quantity of the detectable entity in the reference standard is known, it may be compared with that in the sample to establish the quantity or quality of the detectable entity in that sample, or preferably cells comprised in the sample.

In highly preferred embodiments, slices or sections of the reference standard are taken. These slices or sections should be treated as part of the invention described here.

Such slices or sections comprise defined areas comprising the detectable entity, as shown in Figures 4B, 4C, 5A and 5B. Multiple slices or sections may be made from a reference standard. The slices or sections may be treated like any section from FFPE material, to reveal the detectable entity. Treatment of the slices or sections of the reference standard together (preferably in parallel with) the sample ensures uniformity, and reduces or eliminates variations due to differences in protocols, materials, etc as discussed earlier in this document.

In preferred embodiments, the sections or slices are taken through one or more, preferably all, of these steps, preferably in parallel with the relevant FFPE section. Figure 6 shows an example of how the reference standard, or a slice or section thereof (for example, the planar slices generated in Figures 4B, 4C, 5A, 5B, 8F, 9 and 10 above) may be processed, preferably in parallel with a biological sample which has been fixed, embedded and sliced. The planar slices are mounted on glass microscope slides, and the paraffin-embedding medium removed. The slide with a planar slice mounted on it is then rehydrated and may be subjected to standard antigen retrieval techniques. The slide is then stained using specific antibodies to reveal the presence and distribution of the antigen. Counterstaining and secondary staining, optionally with chromogenic substrate where the secondary stain is enzyme linked, may also be carried out.

The slices or sections do not need to be of uniform thickness, but may be. It will also be apparent that the staining intensity can be changed by the thickness of the slice. For example, various staining intensities could be obtained by using slices of different thickness. Thus, the staining level may be varied by varying the thickness of the section and therefore the amount of material comprising detectable entity per area. As will be appreciated, this is a very simple method of controlling the staining intensity.

The reference standards as described here provide simple means to establish a "standard", in other words, an established value of a measurable property, by which a sample or test item may be judged. They may be used as colour standards, such as fluorescence standards and chemi luminescence standards, position standards, quantity standards, quality standards, or as diagnostic standards.

In particular, the reference standards described here allow the status or condition of a sample, or any components of the sample, to be determined. In some preferred embodiments, they allow the detection of whether a sample comprises diagnostically relevant levels of a particular relevant antigen. Preferably, the reference standards as described here are used to gauge levels or amounts of detectable entity in tissues or preferably cells comprised in biological samples.

The level, quantity etc of the detectable entity in the sample may in preferred embodiments be indicative of a "condition" or status of a cell or tissue comprised therein. Such level, quantity etc is therefore preferably indicative of the condition of the organism from which the cell or tissue is derived. The condition may be any state of the cell, tissue, organ or organism, whose detection may be desirable. Included are conditions which the cell, etc may adopt, as part of normal biological processes, such as part of a developmental or differentiation program. Although the term "condition" includes physiologically normal or undisturbed conditions, it preferably refers to non-physiologically standard conditions.

Preferred non-physiologically standard conditions include disease conditions, or any conditions which lead to disease. Preferably, the terms "diagnostically relevant level", "diagnostically relevant quantity" and "diagnostically relevant amount" should be taken to mean a level, quantity or amount of a detectable entity in a sample, which is indicative of a condition or disease in an individual from which the sample is taken.

The diagnostically relevant amount of a detectable entity will depend on the particular disease or condition in question, and may be established as standards. It will be understood that the skilled person will be aware of such standards, and will be able to determine the relevant diagnostically relevant amount depending on the disease or condition.

**COMPACT SHAPE**

The reference standard described here comprises the detectable entity in a generally compact shape in the embedding medium. This is achieved by coupling or chemically coupling the detectable entity to a compact particle having a compact shape, and supporting this in the embedding medium. The detectable entity therefore has or adopts a generally compact shape in the reference standard as described here.

Examples of compact particles, which have compact shapes, are given in this and the following section.

It will be appreciated from the above that the "compact shape" does not refer to the specific shape of the molecules or atoms making up the detectable entity, as these can be of any shape. Rather, the term should be taken to refer to the general disposition or localisation of the detectable entity within the embedding medium. For example, the mass or bulk of the detectable entity, for example, in a contiguous state, preferably has an "compact shape" as it is disposed in the embedding medium.

By "compact", we mean a shape which is not generally elongate. In other words, "compact" shapes are those which are generally non-elongate or unextended, or which are preferably not extended in any one dimension. The compact shape may be one which is not generally spread out, or not long or spindly. Therefore, such "compact shapes" generally possess linear dimensions which may be generally similar, or which do not differ by a large amount.

Preferably, the ratio of any two dimensions of the compact shape is 5:1 or less, more preferably 4:1 or less, most preferably 3:1, 2.5:1, 2.4:1, 2.3:1, 2.2:1, 2.1:1, 2:1, 1.9:1, 1.8:1, 1.7:1, 1.6:1, 1.5:1, 1.4:1, 1.3:1, 1.2:1, 1.1:1,or less. Preferably, no two pairs of dimensions have a ratio of 5:1 or more.

In highly preferred embodiments, the longest dimension of the compact shape is less than five times the shortest dimension of the compact shape. In some embodiments, the longest dimension of the compact shape is not significantly greater than the shortest dimension, i.e., the shape is relatively uniform.

The "longest dimension" as the term is used in this document should be taken to mean the length of the major axis, i.e., the axis containing the longest line that can be drawn through the compact particle. Similarly, the "shortest dimension" is the length of the minor axis, which is the axis containing the shortest line that can be drawn through the compact particle

In the examples of compact particle shapes shown in Figure 3, for example, each of the shapes has a longest dimension which is less than 5 times its shortest dimension.

In particular, regular shapes in which the linear dimensions are approximately the same, or are comparable, or in which the ratio of the longest dimension to the shortest dimension is less than 5:1 are included in the reference standards described here. In highly preferred embodiments, therefore, the above ratios relate to the ratio of the longest dimension to the shortest dimension. In some embodiments, the ratio of two dimensions (preferably the longest dimension to the shortest dimension) is less than 1.1:1, preferably 1:1 (i.e., a regular or uniform shape).

Therefore, where applicable, preferably the length of the shape is less than 5x its width or diameter, preferably less than 4x its width or diameter, preferably less than 3x, most preferably, less than 2x or less.

Alternatively, or in addition, a "compact shape" may be defined mathematically, by using any one or more of the measures described below.

*Circularity Ratio*

One measure of "compactness" of two-dimensional shapes is given by the Circularity Ratio (4 x pi x area) to (perimeter)$^2$, viz:

$$\text{Circularity Ratio} = \frac{4\pi * area}{(perimeter) * (perimeter)}$$

In a highly preferred embodiment, the compact particle and/or the detectable entity has a compact shape as determined by the Circularity Ratio, as discussed below.

Using the Circularity Ratio as a compactness measure, a circle - which is the two-dimensional shape which is the most compact - has a compactness of 1. Objects that have more extended, or more or less complicated or irregular boundaries have lower compactness. For example, an ellipse has a lower compactness than a circle using this measure, and a square has a compactness $\pi/4$. A rectangle with a length 5 times its width has a compactness of $\pi/7.2$.

This measure of compactness using the Circularity Ratio may be applied to one or more cross sections of a three-dimensional shape to determine the overall compactness of the shape. Thus, using this measure, a shape is "compact" if none of its cross-sections has a compactness as measured by the Circularity Ratio of $\pi/7.2$ or more. Preferably, the cross section with the greatest compactness, as measured by the Circularity Ratio, has a compactness substantially less than $\pi/7.2$. In highly preferred embodiments, the majority, or preferably substantially all, of its cross sections, have a compactness as measured by the Circularity Ratio, of less than $\pi/7.2$.

Although the Circularity Ratio is used, in a highly preferred embodiment, as a gauge of compactness of the compact particle, other measures of compactness, for example, the Form Ratio, the C Ratio and the Radius Ratio, may be used in alternative embodiments.

*Form Ratio*

An alternative measure of "compactness" of two-dimensional shapes is given by the Form Ratio (4 x area) to (pi * length²), viz:

$$\text{Form Ratio} = \frac{4 * area}{\pi * (L) * (L)},$$ where L is the length of a line joining the area's two most distant points (i.e.,

the longest dimension). Using the Form Ratio as a compactness measure, a circle - which is the two-dimensional shape which is the most compact - has a compactness of 1. Objects that have more extended, or more or less complicated or irregular boundaries have lower compactness. For example, an ellipse has a lower compactness than a circle using this measure, and a square has a compactness $2/\pi$. A rectangle with a length 5 times its width has a compactness of $10/13\pi$.

This measure of compactness using the Form Ratio may be applied to one or more cross sections of a three-dimensional shape to determine the overall compactness of the shape. Thus, using this measure, a shape is "compact" if none of its cross-sections has a compactness as measured by the Form Ratio of $10/13\pi$ or more. Preferably, the cross section with the greatest compactness, as measured by the Form Ratio, has a compactness substantially less than $10/13\pi$. In highly preferred embodiments, the majority, or preferably substantially all, of its cross sections, have a compactness as measured by the Form Ratio, of less than $10/13\pi$.

*C Ratio*

An alternative measure of "compactness" of two-dimensional shapes is given by the C Ratio (area) to (pi * radius²), viz:

$$\text{C Ratio} = \frac{area}{\pi * (R) * (R)},$$ where R is the radius of the smallest circle which will surround the shape. Using the C Ratio as a compactness measure, a circle - which is the two-dimensional shape which is the most compact - has a compactness of 1. Objects that have more extended, or more or less complicated or irregular boundaries have lower compactness. For example, an ellipse has a lower compactness than a circle using this measure, and a square has a compactness $2/\pi$. A rectangle with a length 5 times its width has a compactness of $10/13\pi$.

This measure of compactness using the C Ratio may be applied to one or more cross sections of a three-dimensional shape to determine the overall compactness of the shape. Thus, using this measure, a shape is "compact" if none of its cross-sections has a compactness as measured by the C Ratio of $10/13\pi$ or more. Preferably, the cross section with the greatest compactness, as measured by the C Ratio, has a compactness substantially less than $10/13\pi$. In highly preferred embodiments, the majority, or preferably substantially all, of its cross sections, have a compactness as measured by the C Ratio, of less than $10/13\pi$.

*Radius Ratio*

An alternative measure of "compactness" of two-dimensional shapes is given by the Radius Ratio (r) to (R), viz:

$$\text{Radius Ratio} = \frac{r}{R},$$ where r is the radius of the largest circle which will fit inside the shape, and R is the radius of the smallest circle which will surround the shape. Using the Radius Ratio as a compactness measure, a circle - which is the two-dimensional shape which is the most compact - has a compactness of 1. Objects that have more extended, or more or less complicated or irregular boundaries have lower compactness. For example, an ellipse has a lower compactness than a circle using this measure, and a square has a compactness $\sqrt{2}/2$. A rectangle with a length 5 times its width has a compactness of $1/\sqrt{26}$.

This measure of compactness using the Radius Ratio may be applied to one or more cross sections of a three-dimensional shape to determine the overall compactness of the shape. Thus, using this measure, a shape is "compact" if none of its cross-sections has a compactness as measured by the Radius Ratio of $1/\sqrt{26}$ or more. Preferably, the cross section with the greatest compactness, as measured by the Radius Ratio, has a compactness substantially less than $1/\sqrt{26}$. In highly preferred embodiments, the majority, or preferably substantially all, of its cross sections, have a compactness as measured by the Radius Ratio, of less than $1/\sqrt{26}$.

*Shape*

The compact shape may comprise a regular solid, a sphere, a spheroid, an oblate spheroid, a flattened spheroid, an ellipsoid, a cube, a cone, a cylinder, or a polyhedron. Preferred polyhedra include simple polyhedra or regular polyhedra. Polyhedra include, for example, a hexahedron, holyhedron, cuboid, deltahedron, pentahedron, tetradecahedron, polyhedron, tetraflexagon, trapezohedron, truncated polyhedron, geodesic dome, heptahedron and hexecontahedron. Any of the above shapes are preferably such that they are "compact", according to the definition provided above. For example, where the shape comprises an oblate spheroid, this has the appropriate oblateness such that the spheroid is compact, and not elongate.

In preferred embodiments, the compact shape may comprise a balloon shape, a cigar shape, a sausage shape, a disc shape, a teardrop shape, a ball shape or an elliptical shape, so long as the dimensions are as given above. The compact shape may also comprise a sphere shape, a cube shape, a cuboid shape, a tile shape, an ovoid shape, an ellipsoid shape, a disc shape, a cell shape, a pill shape, a capsule shape, a flat cylinder shape, a bean shape, a drop shape, a globular shape, a pea shape, a pellet shape, etc.

Some examples of compact particle shapes are shown in Figure 3.

The compact shape may be non-linear, and may have a curved orientation comprising one or more curved portions. It may be curly or wavy in shape, and still retain a compact shape.

The compact shape may be uniform in cross section, or non-uniform in cross section. For example, the compact shape may comprise a lens shape or a sausage shape. In some embodiments, the compact shape has a uniform cross section across at least a substantial portion of the compact shape, preferably substantially the length of the compact shape. The cross sectional area of the compact shape is therefore in such embodiments substantially the same across all portions of the detectable entity. However, embodiments where the detectable entity is not uniformly distributed along the compact shape may be envisaged.

The cross sectional profile of the compact shape, i.e., the defined area comprising the detectable entity in cross section, may have a variety of configurations. Preferably, the defined area is circular or elliptical or ellipsoid in shape. However, the defined area may have a pill shape, a regular shape or an irregular shape. Different cross sectional profiles may be established by use of appropriately profiled compact shapes. Preferably, the cross sectional profile is similar to the size or shape of a cell, or both, for example, a prokaryotic cell or a eukaryotic cell, preferably an animal cell and most preferably a human cell. That is to say, the cross sectional profile preferably has a cross sectional profile of cell-like dimensions.

Preferably, the cross sectional profile of the compact shape, or the shortest dimension of the compact shape, has a diameter of (or a greatest dimension of) less than 1500μm, 1400μm, 1300μm, 1200μm, 1000μm, 900μm, 800μm, 700μm, 600μm, 500μm, preferably less than 400μm, more preferably less than 300μm, more preferably less than 200μm, more preferably between about 0.5 μm to 100 μm, more preferably between 1 μm and 100 μm, even more preferably between 10 μm to 20 μm and most preferably between 2 μm to 20 μm. Particularly preferred embodiments are those with diameters or greatest dimensions of, or substantially of, 1 μm, 2 μm, 3 μm, 4μm, 5μm, 6μm, 7μm, 8μm, 9μm, 10μm, 11μm, 12μm, 13μm, 14μm, 15μm, 16μm, 17μm, 18μm, 19μm or 20μm.

In highly preferred embodiments, the cross sectional profile of the compact shape is in the order of size of a typical eucaryotic or prokaryotic cell, that is to say, between 1μm and 100μm.

It will be appreciated that these sizes are not limiting, and the user will know to vary the size depending on the application. Furthermore, where the reference standard comprises more than one detectable entity, or more than one compact shape, or both, it will be appreciated that each of these may independently have the features and properties set out above. Furthermore, where the reference standard comprises two or more compact shape, these may be stained at the same density, or at different densities.

**Compact Particle**

As noted above, the reference standard comprises a detectable entity coupled or chemically coupled to a compact particle. The compact particle serves as a framework, substrate or support, for the detectable entity, and allows the detectable entity to maintain a compact shape in the embedding medium. The compact particle may also help retain the form of the detectable entity, or its morphology, distribution or concentration constant in the embedding medium. The distribution or disposition of the detectable entity may generally follow the shape of the compact particle. The compact particle also presents the detectable entity for detection.

Where the term "compact particle" is used in this document, the term "particle" in this phrase should not be mean that the "compact particle" is necessarily very small or minute, nor that it is a part of some larger entity. Indeed, the "particle" can be as large as necessary, provided it performs its functions in the reference standard, as described elsewhere in this document. The particle, however, is required to have a "compact" shape as described above. Preferably, the compact particle has a compact shape when supported by the embedding medium in the reference standard, but preferably, it also has a compact shape when not so supported (e.g., before or after coupling, fixing, etc).

The compact particle may comprise any material, so long as it has the physical properties which allow it to serve its purposes as described above, for example as a point of attachment for the detectable entity. The compact particle may therefore comprise material which is stiff, rigid, malleable, solid, or otherwise, for this purpose. It may comprise a solid material, or a semi-solid, gel, etc material. The material is at least reactive to allow coupling or chemical coupling of the detectable entity, or capable of being made reactive by an activator, but may otherwise comprise a generally inert substance. The compact particle may comprise a composite, such that more than one material may make up the compact particle. For example, the core of the compact particle may comprise a different material from surface portions. Thus, the core of the compact particle may comprise a generally inert material, while the surface portions may comprise material reactive for coupling or chemical coupling of the detectable entity.

The compact particle may be natural in origin, or synthetic. Natural and synthetic materials and sources for obtaining them are well known in the art. Preferably, the compact particle will have at least some mechanical resistance, at least some resistance to chemical attack, or to heat treatment, or any combination of these.

In preferred embodiments, a compact particle having one or more detectable entities at the same or different concentrations are embedded in a paraffin-embedding medium. A flowchart showing how a reference standard according to this embodiment may be made is shown in Figures 11 and 12. The agarose-embedding step, described in further detail below, is optional.

More than one compact particle may be present in the reference standard. For example, two or more compact particle, each comprising a different detectable entity may be employed. Furthermore, different quantities of the same detectable entity may be provided on different compact particle. Finally, more than one detectable entity may be conjugated or coupled or chemically coupled to a compact particle. Multiple compact particle, each comprising a single, two or more detectable entities, at identical or different quantities, may be used.

The or each compact particle comprising the or each detectable entity may be disposed through substantially the reference standard, or at least through a portion of it. For example, where the reference standard is in the form of a rectangular box, the or each compact particle may be disposed at points from one end to another (i.e., across substantially the entire length of the rectangular box).

Preferably, the compact particle has a diameter of between about 0.5 $\mu$m to 100 $\mu$m, more preferably between 1 $\mu$m and 100 $\mu$m, even more preferably between 10 $\mu$m to 20 $\mu$m and most preferably between 2 $\mu$m to 20 $\mu$m. Other diameters are possible, for example, less than 1500$\mu$m, less than 1000$\mu$m, less than 500$\mu$m, less than 200$\mu$m, etc.

The detectable entity or each compact particle may be dyed using any one of various dyes known in the art, for easier identification and/or location. Where two or more compact particle are comprised in the reference standard, each of the compact particle is dyed is preferably dyed using a different colour, to allow easier distinction between the compact particles.

As described above, the or each compact particle may be embedded in the same embedding medium as the sample (for example, a cell or tissue to be assayed) itself, at the same time, before, or after, the sample is embedded in that medium. In preferred embodiments, the or each compact particle is co-embedded in an embedding medium comprising paraffin. Preferably, such compact particle embedding takes place as part of the paraffin embedding process of the sample in FFPE.

The compact particle may comprise a "biological" object, or a "non-biological" object. In preferred embodiments, the compact particle comprises a biological compact particle.

## BIOLOGICAL COMPACT PARTICLE / CELLULAR COMPACT PARTICLE

In some embodiments, the compact particle is derived from, or consists of, a biological compact particle.

By "biological" compact particles we mean those which have one or more of the following general characteristics: they are not synthetic or man-made, they are derived from a biological object, and they comprise, are derived from, or consist of, objects which are or once were living.

Preferably, the biological compact particle is an anatomical, histological or cellular portion of a living organism, whether a virus, cell, tissue, etc. Preferably, where the compact particle is derived from something that is or was living, the compact particle preferably maintains the morphology of that living thing. That is to say, where derivation or processing takes place, that derivation or processing is preferably simple or minimal in nature. In particular, the biological compact particle does not comprise a purified or semipurified chemical or biochemical component of a biological object or a living thing, e.g., it does not comprise a purified protein or macromolecule for example.

Examples of biological compact particles include a tissue, a cell, any portion or product of a cell, such as an organelle, nucleus, mitochondrion, plastid, chloroplast, etc. Further examples of c biological ompact particles include tissues or aggregations of cells.

The biological compact particle may in preferred embodiments comprise a cellular compact particle, by which term we mean that it is derived from or comprises a cell. A cellular compact particle may comprise or be derived from whole cells, or portions of cells, such as an organelle or any other sub-cellular compartment. Examples of these include cellular structures, such as nuclei, mitochondria, vacuoles, cell walls, cell membranes, organelles, cytoskeleton, etc.

Preferably, the cellular compact particle does not express, naturally or otherwise, the detectable entity.

The cellular compact particle may be derived from any part of the body, and from any organism of any species. In a highly preferred embodiment, the cellular compact particle comprises a cell. The cell may be a prokaryotic cell, or a eukaryotic cell. Included are single celled organisms, commonly referred to as micro-organisms. Compact particles which may be used therefore include a bacterium, a yeast, a fungus, a protozoan, an amoeba, an alga, etc. Also included as cellular compact particles are viruses of various kinds, such as bacteriophages, retroviruses, poxviruses, adenoviruses, etc.

Cells from multicellular organisms, from whichever portion, may also be employed. Cellular compact particles include plant cells, animal cells, insect cells, fish cells, mammalian cells, mouse cells, primate cells, human cells, etc. The cell may be derived from any portion of the organism, from any tissue or organ, such as the skin, heart, brain, muscle, breast, bone, fibroblast, vascular system, blood system, lymph system, etc. The cell may be isolated from the organism and used directly, or the cell may be a cultured cell, whether in primary culture or secondary culture. Methods of primary and secondary culture of tissue cultured cells are well known in the art.

Examples of cellular compact particles suitable for use in the reference standard described here are illustrated in Figure 7: bacteriophage or virus, bacteria (Figure 7A), bacterial cells, colonies of bacterial cells (Figure 7B), red blood cells (Figure 7C), an animal cell (Figure 7D) and a plant cell (Figure 7E).

The compact particle preferably does not comprise the detectable entity in its natural state, but rather the detectable entity is coupled or chemically coupled to it for use in the reference standard. For example, where the compact particle comprises or is derived from a cell, the cell is one which does not normally contain the detectable entity. Thus, the cell is preferably one which does not express the detectable entity, whether because the gene for the detectable entity is not expressed (where the detectable entity comprises a polypeptide or nucleic acid), or the detectable entity is not a normal product of such a cell (where for example the detectable entity comprises a metabolic product), or the cell is from a different organism, for example, a different species, from a cell which normally comprises the detectable entity.

The cell or a portion thereof may be used intact, or it may be treated or processed before or after attachment by coupling or chemically coupling of the detectable entity. Preferably, the cellular compact particle maintains cellular morphology after treatment. For example, the cellular compact particle may be permeabilised for example by use of detergents, or fixed using a fixative. Means of cell permeabilisation are well known in the art, and include use of digitonin, Triton X100, etc. The cellular compact particle may comprise a cytoskeletal structure. The detectable entity may be coupled or chemically coupled to any relevant portion of the cellular compact particle, such as its surface or interior. The detectable may preferably be coupled or chemically coupled to the surface of the cellular compact particle, such as the surface of the cell, for example, to plasma membrane molecules such as lipids, phospholipids, cell surface proteins, receptors, membrane bound receptors, etc.

The detectable entity may be targeted to the location at which it is desired by means of a binding portion, which is preferably capable of recognising, targeting or attaching to the desired location. The binding portion may recognise, attach to, or target, by means of charge, pH, ionic interactions, non-ionic interactions, hydrophobic interactions, active transport, etc. The binding portion may comprise a probe, such as a nucleic acid probe capable of recognising and binding to another nucleic acid present at or in the location. For example, the detectable entity may be attached to the cell surface by comprising a binding portion capable of binding to a cell surface entity, such as an antibody.

The binding portion may comprise an antibody, or a portion thereof, capable of specific binding to a protein expressed in or at the desired location, such as a cell surface protein such as a receptor. The antibody or portion thereof may be attached or coupled to the detectable entity by any number of ways, for example as described below, or as a fusion protein with the detectable entity.

The detectable entity may also be attached to the cell, by being inserted into the plasma membrane in the same fashion as a transmembrane protein. For this purpose, the detectable entity may be modified by attaching or coupling a membrane inserting sequence, for example, a 7TM sequence or a bacteriorhodopsin membrane sequence to it.

Alternatively, or in addition, the detectable entity may be attached to other portions of the cell, for example internal portions or any cellular component, particularly where permeabilisation has taken place. The cell is preferably fixed prior to coupling or chemical coupling. In some cases, there is scope for modifying the shape of the cell somewhat, before fixing, coupling or chemical coupling or supporting in the embedding medium. For example, a chemical or biological signal may be provided to the cell to enable it to change shape. Other treatments, for example, protease treatment of monolayer cell cultures, enable the cells to round up into a generally compact shape.

Examples of particularly preferred cells include Sf9 cells, which are a line of insect cells derived from the fall armyworm *Spodoptera frugiperda.* Sf9 cells are well known in the art, for use as baculoviral expression hosts, and methods of culturing, treating and processing such cells are also generally known (see for example, O'Reilly, D.R., L.K. Miller, and V.A. Luckow, (1994). Baculovirus Expression Vectors).

Sf9 was cloned by G.E. Smith and C.L. Cherry in 1983 from the parent line, IPLB-SF 21 AE, which was derived from pupal ovarian tissue of the fall armyworm, Spodoptera frugiperda, by Vaughn, et al., in 1977 (Vaughn JL, et al. The establishment of two cell lines from the insect Spodoptera frugiperda (Lepidoptera; Noctuidae). In Vitro 13: 213-217, 1977). Sf9 cells may be cultured in a propagation medium comprising Grace's Insect Medium with L-glutamine and supplemented with: 500 mg/L calcium chloride 2800 mg/L potassium chloride 3330 mg/L lactalbumin hydrolysate 3330 mg/L yeastolate 10% Heat-inactivated fetal bovine serum (previously tested for insect cell culture) at a temperature of 28 degrees C. The cells may be subcultured by gently resuspending cells in the spent culture medium by pipetting across the monolayer or by hitting the flask against the palm of the hand (the latter is only preferable when working with larger flasks). If many floating cells are present before subculturing, the old medium and the floating cells may be discarded and the medium replaced before subculture. Cells should be incubated at 28 degrees C. A subcultivation ratio of 1:5 or greater is recommended.

Sf9 Insect Cells may be obtained from the American Type Culture Collection, as catalogue number CRL-1711. In addition, such cells are commercially available from, for example, BD Biosciences Pharmingen (catalogue number 551407) as Ready-Plaque™ Sf9 Cells from Novagen (catalogue number 70033-3) and as TriEx™ Sf9 Cells, also from Novagen (catalogue number 71023-3). Media for growing Sf9 cells include EX-CELL™ 420 (JRH Biosciences, Inc.), Sf-900 II SFM (Life Technologies, Inc.), HyQSFX-Insec™ (HyClone Laboratories, Inc.), and High Five™ (Invitrogen Corporation).

The cellular compact particle may also comprise an animal cell, such as a mouse cell. In preferred embodiments, the compact particle comprises a Chinese Hamster Ovary (CHO) cell. The Chinese Hamster Ovary cell line comprises adherent epithelial cells, and was initiated from a biopsy of an ovary of an adult Chinese hamster (*Cricetulus griseus*) by T. T. Puck in 1957 (Puck TT, et al. Genetics of somatic mammalian cells III. Long-term cultivation of euploid cells from human and animal subjects. J. Exp. Med. 108: 945-956, 1958).

Cells from this cell line, as well as any of the derivatives of it, may be used as compact particles according to the methods and compositions described here. Thus, for example, the cell line CHO-K 1 (American Type Tissue Collection catalogue number: CCL-61) is derived as a subclone from the parental Puck CHO cell line, and require proline in the medium for growth. CHO-K1 may be propagated in Ham's F12K medium with 2 mML-glutamine adjusted to contain 1.5 g/L sodium bicarbonate., 90%; fetal bovine serum, 10% at a temperature of 37 degrees C. The cells may be subcultured by removing the medium, and rinsing with 0.25% trypsin, 0.03% EDTA solution. The solution is removed and an additional 1 to 2 ml of trypsin-EDTA solution added. The flask is allowed to sit at room temperature (or at 37 degrees C) until the cells detach. Fresh culture medium is added, aspirated and dispensed into new culture flasks. A subcultivation ratio of 1:4 to 1:8 is recommended for this cell line.

Figure 8 illustrates a process for the production of a reference standard as described here, in which a cellular compact particle is employed. As shown in Figure 8A, a quantity of detectable entity 1 is provided, by any means suitable, for example, purification from natural sources, recombinant expression, etc. A compact entity shown in Figure 8B comprising a cell 2, or more than one cell, is also provided by any suitable means (for example, methods of culturing, purifying or cloning cells are well known in the art).

The quantity of detectable entity 1 is then coupled or chemically coupled to the cell 2, resulting in a compact entity comprising a cell, with a detectable entity coupled or chemically coupled to it (Figure 8C). In preferred embodiments, the detectable entity is coupled or chemically coupled to the cell by being chemically coupled, for example using a cross-linker as described in further detail below. Also as described in further detail below, the detectable entity may be directly chemically coupled to the cell, or as shown in the Figure, the detectable entity may be coupled or chemically coupled to the cell via a spacer 12.

The cell with the detectable entity coupled or chemically coupled thereto may itself be used as a reference standard. Alternatively, or in addition, the cellular compact particle with the detectable entity coupled or chemically coupled to it is supported or embedded in an embedding medium (Figure 8D). In preferred embodiments of the reference standard, a number of cellular compact particles are supported in the embedding medium (see Figure 8E). Slices or sections of such a reference standard may be taken, and used for the purposes described here. Such slices or sections comprise a detectable amount of the detectable entity in a defined region in a cross section of the reference standard (Figure 8F).

*Non-Biological Compact Particles*

In an alternative embodiment, the compact particle comprises a "non-biological" object.

Non-biological compact particles, in contrast to the histological or cellular nature of biological compact particles, are molecular in nature. They are typically chemical or biochemical entities such as molecules or compounds, including complex molecules and macromolecules such as polypeptides, nucleic acids, etc. Examples of non-biological compact particles include microbeads, agar chunks, silica particles, etc. They are typica homogenous in composition, and are usually pure or isolated from other chemical or biochemical entities.

Non-biological compact particles are cheap and easy to make and handle. They may carry less risk to the handler than biological material. Embodiments where non-biological compact particles are preferred include use as procedural validation standards, where the mimicking of cell characteristics is less important than, say, flow cytometry.

The non-biological compact particle may ultimately have a biological origin - thus, it may be a purified component of biological material, such as serum protein, cellulose, agarose, etc. Non-biological objects may include those that have been purified, or refined, from a biological source, such as agar and agarose. It does not however retain any substantial histological or cellular characteristics, in particular structural characteristics of the origin material such as cell shape and cell size. The "non-biological" compact particle in preferred embodiments is therefore free or substantially free of cellular material, including tissue.

In highly preferred embodiments, the non-biological compact particle may be a "non-cellular compact particle", i.e., one which does not comprise or is derived from a cell.

Such a non-biological or non-cellular compact particle may therefore comprise a synthetic material, or a non-naturally occurring material. Various compact particles of various shapes are known in the art, and include for example, beads of various kinds. Particularly preferred embodiments of compact particles include microbeads, such as agarose beads, polyacrylamide beads, silica gel beads, etc

*Beads*

Beads or microbeads suitable for use include those which are used for gel chromatography, for example, gel filtration media such as Sephadex. Suitable microbeads of this sort include Sephadex G-10 having a bead size of 40-120 (Sigma Aldrich catalogue number 27,103-9), Sephadex G-15 having a bead size of 40-120$\mu$m (Sigma Aldrich catalogue number 27,104-7), Sephadex G-25 having a bead size of 20-50$\mu$m (Sigma Aldrich catalogue number 27,106-3), Sephadex G-25 having a bead size of 20-80$\mu$m (Sigma Aldrich catalogue number 27,107-1), Sephadex G-25 having a bead size of 50-150$\mu$m (Sigma Aldrich catalogue number 27,109-8), Sephadex G-25 having a bead size of 100-300 $\mu$m (Sigma Aldrich catalogue number 27,110-1), Sephadex G-50 having a bead size of 20-50$\mu$m (Sigma Aldrich catalogue number 27,112-8), Sephadex G-50 having a bead size of 20-80$\mu$m (Sigma Aldrich catalogue number 27,113-6), Sephadex G-50 having a bead size of 50-150$\mu$m (Sigma Aldrich catalogue number 27,114-4), Sephadex G-50 having a bead size of 100-300$\mu$m (Sigma Aldrich catalogue number 27,115-2), Sephadex G-75 having a bead size of 20-50$\mu$m (Sigma Aldrich catalogue number 27,116-0), Sephadex G-75 having a bead size of 40-120$\mu$m (Sigma Aldrich catalogue number 27,117-9), Sephadex G-100 having a bead size of 20-50$\mu$m (Sigma Aldrich catalogue number 27,118-7), Sephadex G-100 having a bead size of 40-120$\mu$m (Sigma Aldrich catalogue number 27,119-5), Sephadex G-150 having a bead size of 40-120$\mu$m (Sigma Aldrich catalogue number 27,121-7), and Sephadex G-200 having a bead size of 40-120$\mu$m (Sigma Aldrich catalogue number 27,123-3).

Sepharose beads, for example, as used in liquid chromatography, may also be used. Examples are Q-Sepharose, S-Sepharose and SP-Sepharose beads, available for example from Amersham Biosciences Europe GmbH (Freiburg, Germany) as Q Sepharose XL (catalogue number 17-5072-01), Q Sepharose XL (catalogue number 17-5072-04), Q Sepharose XL (catalogue number 17-5072-60), SP Sepharose XL (catalogue number 17-5073-01), SP Sepharose XL (catalogue number 17-5073-04) and SP Sepharose XL (catalogue number 117-5073-60) etc.

*Micelles*

The non-biological or non-cellular compact particle may also comprise a micelle. The micelle may have a monolayer or a bilayer. It may have protein or other molecules inserted therein, which may serve as points of coupling or chemical coupling of the compact particle. Micelles may be made by first selecting one or more surfactants. For example, two different surfactants such as block copolymers of propylene oxide and ethylene oxide and polyoxyethylene sorbitan monolaurate may be used. However, one, two, three or more surfactants can be utilized. Suitable surfactants also include derivatives of capryl imidzoline, alkyl polyglycol ethers, polyoxyalkylene lanolins, block copolymers of propylene oxide and ethylene oxide, and polyoxyethylene sorbitan monolaurate.

The surfactants are then dispersed in a water base. Surfactants selected for the present composition are made up of molecules that have a hydrophilic end group and a hydrophobic hydrocarbon tail. The hydrophilic group has a propensity for water while the hydrophobic tail possesses an aversion for water. Thus, above a certain concentration, the surfactant molecules tend to associate with one another in a group whereby the hydrophilic group is exposed to the water and are configured such that they form a generally circular or spherical configuration while the hydrophobic tails extend inwardly and associate with each other, perhaps in an intertwined relationship. Effectively, this forms clusters of surfactant molecules which are called micelles

It is postulated that the hydrophilic portion of the micelles forms a shell or a continuous mixture around an interior area that is occupied by the hydrophobic tails of the surfactant molecules. Thus, this creates or gives rise to a shell type structure.

## DETECTABLE ENTITY

In highly preferred embodiments, the detectable entity is one which is heterologous to the compact particle. That is to say, the compact particle is not in its natural state associated with the detectable entity.

Thus, for example, where the compact particle comprises a cellular compact particle, the detectable entity is one which is not normally expressed by the cell, but is made to be coupled or chemically coupled to it by manipulation or human intervention, such as for example chemical coupling. The methods and compositions described here therefore do not make use of cells which naturally express a particular detectable entity, such as cancer cell lines, nor does it include cells which have been induced to express a particular detectable entity by transfection.

Preferably, the cell does not include the detectable entity in its natural state, in whatever part of the cell cycle, or developmental stage, and expression of the detectable entity is preferably not inducible. Preferably, the cell is not a transfected cell, i.e., the cell does not include genetic material which has been artificially introduced therein. Thus, the detectable entity is not expressed by the cell by virtue of it being transfected, but rather added externally to the context of the cell by manipulation.

The detectable entity is one whose presence and preferably quantity is revealable, that is, its presence is demonstrable or its amount is measurable, either directly or indirectly. In general, the detectable entity can be anything which is capable of producing a detectable signal, or a revealable signal, whether by itself, naturally or when stimulated to do so.

The detectable entity may produce a signal alone or in conjunction with one or more other entities, for example, when contacted with revealing agents such as antibodies and/or secondary antibodies. The detectable entity may itself be labelled, as discussed elsewhere, using any of a variety of labels, for example, radioactive and non-radioactive labels, as known in the art. Further discussion of this aspect is contained in the section "Detection and Visualisation" below.

The reference standard preferably comprises the detectable entity in a relatively pure state, that is, substantially isolated from other molecules or compounds. The detectable entity is preferably free or substantially free from cellular components with which it may be normally associated. For example, the detectable entity may be in isolated form.

The detectable entity is preferably non-cellular in nature, but not necessarily non-cellular in origin. By this we mean that the detectable entity may originate from the cell, but is preferably one which has undergone some processing, for example purification or concentration, to isolate the detectable entity from at least one other cellular component. In particular, the detectable entity does not for example comprise raw cellular material or whole cells. Preferably, the detectable entity does not comprise substantial amounts of cellular structures, such as cell walls, cell membranes, organelles, cytoskeleton, etc. Preferably, in such embodiments, the detectable entity comprises "molecular" components in a relatively pure state, compared to their environment within the cell.

In other words, the detectable entity preferably comprises non-cellular material and/or non-cellular components. It preferably does not comprise substantial amounts of cellular material, for example, it is "cell-free". For this purpose, chemical synthesis or recombinant production of detectable entity is preferred.

The nature of the binding agent will depend on the detectable entity, but may comprise a non-specific or a preferably a specific binding agent. Thus, non-specific binding agents such as dyes, for example, dyes typically used to colour fabrics, may be employed as binding agents. However, specific binding agents are preferred.

The detectable entity may comprise a "small molecule", such as simple inorganic or organic compounds. The detectable entity may in particular comprise a hapten, such as di-nitrophenol (DNP). The detectable entity may include dyes, such as fluorescent dyes.

In highly preferred embodiments, the detectable entity comprises a nucleic acid, such as DNA, RNA, PNA or LNA, or a polypeptide, such as a protein or antigen, or other epitope-comprising polypeptide. Detectable entity may further comprise a peptide, such as a modified peptide, including an acetylated, methylated, deletion mutated peptide, etc. Reference standards comprising protein, etc detectable entities are preferred for innmohistochemistry (IHC), while reference standards comprising nucleic acids, etc are preferred for *in situ* hybridisation (ISH).

Where the detectable entity comprises a nucleic acid, the binding agent may in particular comprise a nucleic acid probe. For this purpose, it may comprise a nucleic acid, such as DNA or RNA, or a derivative thereof, such as Peptide nucleic acid, PNA or Locked nucleic acid, LNA. The nucleic acid probe is preferably capable of specifically hybridising to a sequence in the detectable entity, preferably under stringent conditions. In particular, the nucleic acid probe may comprise at least a sequence complementary to a sequence in the detectable entity. Preferably, the nucleic acid binding agent or probe comprises a single stranded portion, or is denatured to expose binding sites.

Where the detectable entity comprises a protein such as an antigen, the binding agent preferably comprises any molecule capable of specifically binding to the protein. In particular, the binding agent may comprise an antibody (whether monoclonal or polyclonal) capable of specifically binding to the antigen.

In the above examples, nucleic acids are typically detected by binding agents comprising nucleic acids, while proteins are typically detected by antibodies. It will be appreciated, however, that detectable entity - binding agent pairs may be chosen based on protein nucleic acid interactions. Thus, it is known for example that nucleic acid binding proteins such as zinc finger proteins, HLH proteins, etc can bind to specific nucleic acids sequences. Thus, a nucleic acid binding protein may be used as a binding agent to detect a detectable entity comprising a cognate nucleic acid, and a nucleic acid may be used as a binding agent to detect a detectable entity comprising a cognate nucleic acid binding protein.

Preferably the detectable entity is selected from the group consisting of a protein, a polypeptide, a peptide, a phosphylated peptide, a phosphorylated peptide, a glucated peptide, a glycopeptide, a nucleic acid, a virus, a virus-like particle, a nucleotide, a ribonucleotide, a synthetic analogue of a nucleotide, a synthetic analogue of a ribonucleotide, a modified nucleotide, a modified ribonucleotide, an amino acid, an amino acid analogue, a modified amino acid, a modified amino acid analogue, a steroid, a proteoglycan, a lipid and a carbohydrate or a combination thereof (for example, chromosomal material comprising both protein and DNA components or a pair or set of effectors, wherein one or more convert another to active form, for example catalytically).

In preferred embodiments, the detectable entity comprises a polypeptide, or a nucleic acid. In highly preferred embodiments, the detectable entity suitably comprises an indicator of a state of a cell such as an indicator of the health or disease state of the cell.

For example, the presence or amount of the detectable entity may serve to indicate that the cell is in a healthy, normal or functional state. Preferably, however, the detectable entity is such that it is an indicator of an abnormal state of the cell, for example, a diseased state. In other words, if the detectable entity is present in a cell or tissue, it may be inferred that the cell or tissue or organ or individual comprising this is diseased. The detectable entity may be diagnostic of a single disease, or a number of diseases, or a syndrome such as AIDS, or a medical condition. Preferably, the disease, syndrome or condition is a treatable one.

Preferably, the presence, quantity or concentration of the detectable entity in a cell or tissue is detected to provide an indication of a condition of the cell or tissue, preferably a pathological condition of the cell or tissue. Therefore, in embodiments where the reference standard is employed as a diagnostic standard, the detectable entity may comprise any diagnostically relevant entity.

Thus, in this preferred embodiment, the detectable entity is essentially a marker of a pathological condition or a disease marker, the presence or quantity of which in a cell indicates that the cell is or is likely to be diseased. The presence of the detectable entity may be diagnostic, or it may serve as merely an indicator, which together with other indicators, for example a panel of indicators, points to the likelihood of disease. The quantity of the detectable entity in the cell or tissue may be significant, i.e., whether or not it is above a threshold level which is indicative of disease.

Preferably, the disease comprises cancer. The detectable entity is therefore preferably a cancer marker or cancer protein or cancer nucleic acid. A number of cancer and cancer related proteins are known in the art, for example, *ras*, BRCA1, HER2, *ATM, RhoC*, telomerase, etc. Carcinoembryonic antigen (CEA) is associated with digestive tract cancers (for example of the colon) as well as other malignant and non-malignant disorders. Examples of other cancer markers are set out below, and it will be appreciated that nucleic acids encoding these may also be used as detectable entities:

Prostate specific antigen (PSA) levels are elevated in prostate cancers and are sometimes enlarged prostate conditions (for example BPH) or prostatis.

CA 19.9 is mainly associated with gastrointestinal cancers. Sometimes increased values have also been observed in those patients with metastasis and in non-malignant conditions for example hepatitis, cirrhosis, pancreatitis.

HER2 is associated with breast cancers. Increased values of the HER2 protein, overexpression, are often associated with rapid growth of the tumour cells that may lead to metastatic conditions. Metastatic patents who overexpress HER2 protein may benefit from HERCEPTIN therapy (therapy with anti-HER2 antibodies).

Elevated CA 125 values are often associated with cancer of the ovaries. However non-malignant conditions such as endometriosis, first semester pregnancy, ovarian cysts or pelvic inflammatory disease can also cause elevated CA 125 levels. The link between family history and incidence of cancer has been well reported.

CA 15.3 values are often elevated in patients with breast cancers. When there is a history of cancer among family members, patients may be advised to also do a breast mammogram. Besides breast cancer, other non-malignant conditions (for example cirrhosis, benign diseases of ovaries & breast) have also been known to cause elevated CA 15.3 levels.

Alpha fetoprotein (AFP) levels are often elevated in liver cancers (hepatocellular) and testicular cancers (non-

**EP 1 642 131 B1**

seminomatous). Raised levels are also present during pregnancy or some gastrointestinal cancers. AFP is also used in combination with other tests as a screening test for open neural tube defects.

Nasopharygeal carcinoma (NPC) is a non-lymphatous, non-glandular, squamous cell carcinoma arising from the epithelial cells of the nasopharynx. It is the most common form of nasophryngeal cancer, with a higher incidence in adults. Some clinical symptoms include nose or ear problems, blood in nasal mucous, neck lumps, enlarged lymph nodes (usually cervical) and sensation of nasal obstruction. Epstein Bar virus (EBV) has been shown to have a direct relationship with NPC where it can be detected in NPC tumours and patients with NPC tend to have higher titres of EBV specific antibodies than the general population. Early detection through screening usually results in favourable prognosis.

Tissue Inhibitor of Metalloproteinase-1 (TIMP-1) also known as metalloproteinase inhibitor 1, fibroblast collagenase inhibitor, collagenase inhibitor and erythroid potentiating activity (EPA). Overexpression of hepatic TIMP-1 has been reported to block the development of TAg-induced hepatocellular carcinomas by inhibiting growth and angiogenesis. TIMP-1 is associated with e.g. non-small lung cancer (NSCLS), Malignant Melanoma and Chondrosarcoma.

Tumour suppressor proteins, such as p53 and Rb may also be used as detectable entities.

The detectable entities may comprise immunoglobulin Kappa and Lambda light chains. The detectable entity or entities may comprise one or more prognostic markers like estrogen receptor (ER) alfa and beta and progesteron receptor (PR), p53 protein, Proliferation related proteins like Ki-67 and Proliferating cell nuclear antigen (PCNA). The detectable entity or entities may comprise one or more cell adhesion molecules like Cadherin E, and tumor suppressor proteins like p16, p21, p27 and Rb. The detectable entity or entities may comprise one or more hematologic factors like CD3, CD15, CD20, CD30, CD34, CD45, CD45RO, CD99, Kappa and Lambda light chains and factor VIII. Furthermore, any of the following may be used as a detectable entity: CD3, CD4, CD5, CD8, CD13, CD14, CD19; CD34 class I, CD34 class II, CD34 class III, CD45, CD45RO, CD64, CD117, p16, p19, p21, p53 protein, proliferating cell nuclear antigen, Ki67 antigen, epithelial antigen, Epithelial membrane antigen, Estrogen receptor, Progesteron receptor, Glycophorin A, HLA-ABC antigen, HLA-DP/DQ/DR antigen, Herpes Simplex 1 & 2 (HSV 1&2), Papillomavirus antigen, HIV-1, 2 antigen, adenovirus antigen, Hepatitis B virus surface antigen, Helicobacter Pylori antigen, Chlamydia antigen, Chlamydia Pneumoniae antigen and CMV antigen.

The detectable entity or entities may comprise one or more epithelial differentiation markers like Prostate specific antigen (PSA), Prostate specific alkaline phosphatase (PSAP), cytokeratin, epithelial membrane antigen, carcinoembryonic antigen (CEA), polymorphic epithalial mucin, mesenchymal differentiation markers, Desmin, Actin, Vimentin, Collagen type IV. The detectable entity or entities may comprise one or more melanocytic markers like S-100, HMB45. The detectable entity or entities may comprise one or more of markers as CD117, CD133, CD45, CD4, CD8, CD19, CD20, CD56, CD13, CD33, CD235a, CD15, BerEP4, Neuron specific enolase, Glial fibrillary acidic protein, Chromogranin, Synaptophysin, c-Kit, Epidermal Growth Factor Receptors (EGFR), HER2/neu, HER3, and HER4 and their ligand like EGF and TGF-alfa and other receptor protein-tyrosine kinases like the Insulin Receptor (IR), the Platelet-derived growth factor receptor (PDGFR), and the Vascular endothelial growth factor receptors (VEGFR-1, VEGFR-2, and VEGFR-3), Apoptosis related proteins like M30, Bcl-2, p53, caspases and Fas.

In one embodiment, the detectable entity comprises c-kit. The CD117 antigen, c-kit or KIT, is a 145 kDa transmembrane protein which belongs to the class III receptor tyrosine kinase family. The intracytoplasmic tyrosine kinase domain is split by a long hydrophilic insert between the ATP-binding region and the phosphotransferase active site (Fleishman RA. Trends Genet 1993;9:285-90.). The extracellular region consists of five immunoglobulin-like domains where the second and third loops are thought to be involved in ligand binding (Blechman JM, et al J Biol Chem 1993; 268:4399-4406). The natural ligand of c-kit has been termed stem cell factor, steel factor (SLF), or mast cell growth factor. c-kit is expressed on 1-4% of normal bone marrow cells (Papayannopoulou et al, Blood 1991;78:1403-12; Bühring H-J, Ullrich A, Schaudt K, Müller CA, Busch FW. The product of the proto-oncogene c-kit).

The majority of positive marrow cells (50-70%) coexpress CD34 and comprise progenitor cells and their precursors of all haematopoietic lineages (Kikutani et al in: Schlossman et al., editors. Leukocyte typing V. White cell differentiation antigens. Proceedings of the 5th International Workshop and Conference; 1993 Nov 3-7; Boston, USA. Oxford, New York, Tokyo: Oxford University Press; 1995. p. 1855-64; Bühring et al, in: Schlossman SF, Boumsell L, Gilks W, Harlan JM, Kishimoto T, Morimoto C, et al., editors. Leukocyte typing V. White cell differentiation antigens. Proceedings of the 5th International Workshop and Conference; 1993 Nov 3-7; Boston, USA. Oxford, New York, Tokyo: Oxford University Press; 1995. p. 1882-8). c-kit is almost exclusively associated with immature stages of haematopoiesis, development of melanocytes, osteoclast differentiation, and Langerhans cell differentiation. c-kit is expressed on mast cells and is found to be expressed on the blasts of patients with AML, but is absent from most ALL blasts (Bühring et al, Br J Haematol 1992;82:287-94.).

In another embodiment, the detectable entity comprises a laminin, preferably laminin 5 gamma 2 chain.

Laminins are large heterotrimeric basement membrane glycoproteins composed of an $\alpha$, a $\beta$ and a $\gamma$ chain. At present five $\alpha$ chains, three $\beta$ chains, and three $\gamma$ chains are known to form at least 15 different isoforms. The laminin-5 protein, consisting of the $\alpha 3$, $\beta 3$ and $\gamma 2$ chains, is initially synthesized as a 460 kDa precursor, which undergoes specific proteolytic processing to a smaller form after secretion into the extracellular matrix. Laminin-5 $\gamma 2$ chain is a unique isoform

25

as the expression of its subunits is restricted to epithelial tissues, where it is part of the epithelial anchoring system and cell locomotion. Laminin-5 γ2 chain protein is essential for the adhesion of basal keratinocytes to the underlying basement membrane, functioning as an adhesion ligand for the integrins α3β1, α6β1 and α6β4 (Decline et al, J Cell Sci 2000;114: 811-231; Salo, S, Function of the γ2 chain in epithelial adhesion and migration, and expression in epithelial cells and carcinomas (academic dissertation). Oulu: Oulu Univ.; 1999). Accumulating data suggest increased laminin-5 γ2 chain protein expression in a number of different human carcinomas, with its expression being characteristic of cancer cells with a budding cell phenotype (Salo, S, supra). Several studies indicate that laminin-5 γ2 chain protein expression can serve as a marker for invasive cancer in various types of squamous cell carcinomas (Skyldberg et al, J Natl Cancer Inst 1999;91:1882-7; Nordström et al, Int J Gynecol Cancer 2002;12:105-9), colon adenocarcinomas (Pyke et al, Cancer Res 1995;55:4132-9), and lung adenocarcinomas (Määttä et al, J Pathol 1999;188:361-8).

In preferred embodiments, the detectable entity comprises any one or more of HER2, oestrogen receptor (ER), progesterone receptor (PR), p16, Ki-67, c-kit, laminin 5 gamma 2 chain and Epidermal Growth Factor Receptor (EGFR) protein, nucleic acids encoding such, and post-translationally modified forms, preferably phosphorylated forms of such.

HER2, also known as NEW and ERBB2, is described in detail in Coussens et al, Science. 1985 Dec 6;230 (4730):1132-9; Spivak-Kroizman et al, J Biol Chem. 1992 Apr 25;267(12):8056-63; King et al, J Biol Chem. 1986 Aug 5;261(22):10073-8; and Plowman et al, Proc Natl Acad Sci USA. 1990 Jul;87(13):4905-9.

Oestrogen receptor is known in the art, and is described in detail in, for example, Ponglikitmongkol, et al, EMBO J. 1988 Nov;7(11):3385-8; Lazennec et al., Gene. 1995 Dec 12;166(2):243-7; Waterman et al., Mol Endocrinol. 1988 Jan;2(1):14-21; Green, et al Nature 320: 134-139, 1986; Greene, et al Science 231: 1150-1154, 1986.

Progesterone receptor, PGR or PR, is described in detail in, for example, Misrahi et al, Biochem. Biophys. Res. Commun. 143: 740-748, 1987; Conneely et al, J Soc Gynecol Investig. 2000 Jan-Feb;7(1 Suppl):S25-32; Mote et al, J Clin Endocrinol Metab. 1999 Aug;84(8):2963-71.

P16 is also known as CDKN2, CDK4 INHIBITOR, MULTIPLE TUMOR SUPPRESSOR 1; MTS 1, TP16, p16 (INK4), p16(INK4A), p19(ARF) and p14(ARF). It is described in detail in, for example, Bogenrieder et al, Hautarzt. 1998 Feb;49(2):91-100; Uchida et al, Leuk Lymphoma. 1998 Mar;29(1-2):27-35 and Geradts et al, Cancer Res. 1995 Dec 15;55(24):6006-11.

Ki-67 is described in detail in, for example, Schluter et al, J. Cell. Biol. 123: 513-522, 1993.

Epidermal Growth Factor Receptor (EGFR) is also known as V-ERB-B AVIAN ERYTHROBLASTIC LEUKEMIA VIRAL ONCOGENE HOMOLOG; ONCOGENE ERBB; ERBB1; SPECIES ANTIGEN 7 and S7. It is described in detail in, for example,

Carlin et al, Proc Natl Acad Sci USA. 1982 Aug;79(16):5026-30; Kondo et al, Cytogenet Cell Genet. 1983;35 (1):9-14; Revis-Gupta et al, Proc Natl Acad Sci USA. 1991 Jul 15;88(14):5954-8; Huang et al, J Biol Chem. 2003 May 23;278(21):18902-13. Epub 2003 Mar 14.

Nucleic acids encoding any of these may also be used as detectable entities.

It will be appreciated that it is not strictly necessary to use the proteins of the above detectable entities in the reference standards as described here, and that it is possible to detect the antigens by use of nucleic acids encoding the proteins. Therefore, it should be appreciated that the reference standard may comprise a detectable entity which is a nucleic acid capable of encoding any of the polypeptide detectable entities as described above. Needless to say, the binding agent or revealing agent in this case would preferably comprise a nucleic acid, preferably a nucleic acid capable of specific binding to at least a portion of the nucleic acid detectable entity, preferably a complementary nucleic acid.

Where the detectable entity comprises a polypeptide, it may be unmodified, or comprise one or more post-translational modifications, such as the addition of a carbohydrate (glycosylation), ADP-ribosyl (ADP ribosylation), fatty acid (prenylation, which includes but is not limited to: myrisoylation and palmitylation), ubiquitin (ubiquitination) protein phosphorylation and dephosphorylation and sentrin (sentrinization; a ubiquitination-like protein modification).

Preferably, the post-translational modification comprises phosphorylation. For example, the detectable entity may comprise phosphorylated HER2 or phosphorylated Epidermal Growth Factor Receptor (EGFR). The detectable entity may further comprise phosphorylated c-kit, phosphorylated laminin 5 gamma 2 chain, phosphorylated oestrogen receptor (ER), phosphorylated progesterone receptor (ER), phosphorylated p16, and/or phosphorylated Ki-67. Phosphorylated detectable entities are particularly preferred where the reference standard is being used in flow cytometry applications.

The detectable entity may also suitably comprise an antigen, preferably a diagnostically relevant antigen, which is detectable by binding to a relevant antibody. Any suitable antigen may be employed, and a skilled person will know which antigens are suitable for which diagnostic purposes.

As used herein, the term "detectable entity" includes but is not limited to an atom or molecule, wherein a molecule may be inorganic or organic, a hapten, a biological effector molecule and/or a nucleic acid encoding an agent such as a biological effector molecule, a protein, a polypeptide, a peptide, a modified peptide, an acetylated peptide, a methylated peptide, a mutant peptide, a deleted peptide, a phosphylated peptide, a phosphorylated peptide, a glucated peptide, a glycopeptide, a nucleic acid, a virus, a virus-like particle, a nucleotide, a ribonucleotide, a nucleic acid, a DNA, an RNA, a peptide nucleic acid (PNA), locked nucleic acid (LNA), a synthetic analogue of a nucleotide, a synthetic analogue of a ribonucleotide, a modified nucleotide, a modified ribonucleotide, an amino acid, an amino acid analogue, a modified amino acid, a modified amino acid analogue, a steroid, a proteoglycan, a lipid and a carbohydrate. The detectable entity may be in solution or in suspension (for example, in crystalline, colloidal or other particulate form). The detectable entity may be in the form of a monomer, dimer, oligomer, etc, or otherwise in a complex.

It will be appreciated that it is not necessary for a single detectable entity to be used, and that it is possible to use two or more detectable entities in the reference standard. Accordingly, the term "detectable entity" also includes mixtures, fusions, combinations and conjugates, of atoms, molecules etc as disclosed herein. For example, a detectable entity may include but is not limited to: a nucleic acid combined with a polypeptide; two or more polypeptides conjugated to each other; a protein conjugated to a biologically active molecule (which may be a small molecule such as a prodrug); or a combination of any of these with a biologically active molecule.

In preferred embodiments, the detectable entity comprises a "biological effector molecule" or "biologically active molecule". These terms refer to an entity that has activity in a biological system, including, but not limited to, a protein, polypeptide or peptide including, but not limited to, a structural protein, an enzyme, a cytokine (such as an interferon and/or an interleukin) an antibiotic, a polyclonal or monoclonal antibody, or an effective part thereof, such as a F(ab)2, F(ab') or Fv fragment, which antibody or part thereof may be natural, synthetic or humanised, a peptide hormone, a receptor, a signalling molecule or other protein; a nucleic acid, as defined below, including, but not limited to, an oligonucleotide or modified oligonucleotide, an antisense oligonucleotide or modified antisense oligonucleotide, cDNA, genomic DNA, an artificial or natural chromosome (for example a yeast artificial chromosome) or a part thereof, RNA, including mRNA, tRNA, rRNA or a ribozyme, or a peptide nucleic acid (PNA), locked nucleic acid (LNA), a virus or virus-like particles; a nucleotide or ribonucleotide or synthetic analogue thereof, which may be modified or unmodified; an amino acid or analogue thereof, which may be modified or unmodified; a non-peptide (for example, steroid) hormone; a proteoglycan; a lipid; or a carbohydrate. Small molecules, including inorganic and organic chemicals, are also of use as detectable entities. In a particularly preferred embodiment, the biologically active molecule is a pharmaceutically active detectable entity, for example, an isotope.

The detectable entity may emit a detectable signal, such as light or other electromagnetic radiation. The detectable entity may be a radio-isotope as known in the art, for example $^{32}$P or $^{35}$S or $^{99}$Tc, or a molecule such as a nucleic acid, polypeptide, or other molecule as explained below conjugated with such a radio-isotope. The detectable entity may be opaque to radiation, such as X-ray radiation. The detectable entity may also comprise a targeting means by which it is directed to a particular cell, tissue, organ or other compartment within the body of an animal. For example, the detectable entity may comprise a radiolabelled antibody specific for defined molecules, tissues or cells in an organism.

The detectable entity may comprise a dye, including azo dyes, organic pigments, cibacron blue, etc.

It will be appreciated that the detectable entity may comprise one or more entities as set out above, and in particular may comprise two or more or a plurality of any of the above entities, or combinations of one or more of the above entities.

## ATTACHMENT OF DETECTABLE ENTITY

the detectable entity is attached to a compact particle having a compact shape by coupling or chemical coupling, and the compact particle comprising the entity is supported in the embedding medium. Preferably, the compact particle comprising the detectable entity is embedded in the embedding medium.

The detectable entity is coupled or chemically coupled to a compact particle. The coupling or chemical coupling, etc between the detectable entity and the compact particle may be permanent or transient, and may involve covalent or non-covalent interactions (including hydrogen bonding, ionic interactions, hydrophobic forces, Van der Waals interactions, etc).

In some embodiments, the detectable entity is chemically coupled to the compact particle by a linker, as known in the art and described in further detail below. Use of such chemical coupling enables the amount of detectable entity to be coupled in an accurate manner to the compact particle.

The term "attached" implies a permanent or semi-permanent association between the compact particle and the detectable entity. The detectable entity is coupled or chemically coupled to the compact particle. This coupling or chemical coupling may be transient or loose, so long as that association is sufficient for the detectable entity to adopt a shape which is substantially defined by the positioning and/or shape of compact particle during and subsequent to supporting or embedding. All that is important is that the compact particle holds the detectable entity in place during the step or steps of supporting or preferably embedding in the medium.

The compact particle may merely retain the detectable entity, or otherwise prevent it from diffusing or being washed away. The retention may be transient, or it may persist through a substantial portion of the supporting or embedding step, preferably throughout the course of that step.

In preferred embodiments, the detectable entity is covalently coupled to the compact particle. In such preferred embodiments, the detectable entity is chemically coupled or cross-linked to one or more molecules making up the compact particle. Preferred methods of chemical coupling are described in further detail below, in the section headed "Coupling". Needless to say, the amount of detectable entity coupled to the compact particle in this embodiment will control how much detectable entity is presented in cross-section, and hence the staining level achieved.

*Spacers*

Furthermore, in certain embodiments, it may be desirable to include spacing means between the detectable entity and the compact particle, or components of the compact particle. Such spacing means may suitably comprise linkers or spacers as known in the art. The purpose of the spacing means is to space the detectable entity from the compact particle (or other component of the compact particle), to avoid for example steric hindrance and to promote detection of the detectable entity. Accordingly, depending on the application, the use of shorter or longer spacers may be preferred.

The spacing means may comprise linkers or spacers which are polymers of differing lengths (the length of which may be controlled by controlling the degree of polymerisation). Numerous spacers and linkers are known in the art, and the skilled person will know how to choose and use these, depending on the application. The skilled person will also know what spacer length to use.

The spacers may be made for example of polyethylenglycol, PEG derivatives or polyalkanes or homo poly amino acids. Dextrans and dendrimers, as known in the art, may also be used. In particular, the linkers or spacers may comprise nucleotide polymers (nucleic acids, polynucleotides, etc) or amino acid polymers (proteins, peptides, polypeptides, etc).

For example, where the detectable entity comprises a peptide or polypeptide, this may be synthesised or expressed (e.g., as a fusion protein) together with additional amino acid residues (these making up the spacer or linker). The linker or spacer need not be contiguous with the detectable entity, however, but may itself be coupled to it, whether covalently or non-covalently (as described above) using any suitable means, for example by use of the cross-linkers described below.

It will be appreciated that where peptides are used as spacers, the configuration and length of the peptide spacers is limited only by the peptide synthesis methods themselves. Thus, for example, the flexibility of peptide synthesis methods allows the synthesis of long, short and branched peptides, including peptides with natural and un-natural occurring amino acids. In particular, the use of branched spacers, for example, branched peptide structures, is desirable, as it enables more than one molecule of a detectable entity to be attached to a spacer or linker. Furthermore, spacers with branched or tree-structures enable the coupling or attachment of more than one type of detectable entity. For example, a first detectable entity may be coupled to one arm of the spacer, a second detectable entity coupled to a second arm, etc. Furthermore, different quantities of the same or different detectable entity may be coupled to each arm.

**EXAMPLE PROTOCOL**

This section sets out a short overview of a preferred non-limiting procedure by which a detectable entity may be coupled or chemically coupled to the compact particle having a compact shape.

The process of making the reference standard using e.g. intact insect sf9 cells and a formalin fixation and paraffin embedding (FFPE) procedure includes one or more of the following procedural steps: (1) The cells are grown as suspension culture in spinner flasks in media in a controlled environment before being isolated from the media by centrifugation and washed (Figure 14A, B and C). (2) Cells in suspension are counted in a hemacytometer (Figure 14D).

(3) The cells in suspension are activated with a heterobifunctional cross linker (Figure 14E), washed by repeated centrifugation and resuspension, coupling with a peptide, washed again and counted, before being processed as a FFPE preparation. (4) The cells are embedded in agarose cylinders and fixed overnight, followed by wrapping in lens clearising paper and placed in a histocapsule for easy handling (Figure 14F). (5) The gel embedded cells is dehydrated by sequential treating with ethanol/water mixtures followed by xylene and subsequent infiltration with melted paraffin and casting of the final blocks. (6) The paraffin blocks are cut on a microtome, mounted on slides, deparafinated, immunovizualized and evaluated in a microscope as standard tissue samples.

For cytological preparations, the cell suspension after Step 3 is mounted on slides using a Cytospin, an Autocyte/TriPath or ThinPrepTM procedure, followed by immuno visualization and evaluation in a microscope as standard samples. For cytological preparations evaluated in a flowcytometer, the diluted cell suspension is immunovizualized using fluorescent-labelled reagents and evaluated in a routine flow cytometer.

**SWELLING**

In some embodiments, the compact particle may be at least partially swollen before or during association with the detectable entity. In preferred embodiments, the compact particle may be allowed to partially or completely swell before or during chemical coupling to the detectable entity.

Although the term "swelling" may in some contexts be taken as reference to the uptake of solvents in a insoluble polymer gel, our use of the term is meant to be more general, and specifically including mechanical, physical or chemical treatment to increase the volume or surface area or accessibility to sites, preferably internal sites, of the compact particle such as a microbead.

Swelling of the compact particle enables the detectable entity to access interior of the compact particle as well as surface portions. The degree of penetration of the detectable entity into core portions of the compact particle, and hence coupling thereto, may then be modulated or controlled. The resulting different modulated distribution patterns of the detectable entity in the compact particle may be used for different detectable entities, depending on their distribution in the cell.

The compact particle may be swollen by various means. For example, the compact particle may treated mechanically. The compact particle may be opened up mechanically, such as by teasing apart. The degree of swelling or shrinking may be controlled by controlling the amount of mechanical action or teasing apart, as the case may be.

Preferably, however, the compact particle is allowed to swell by being exposed to a swelling agent, the absorption or adsorption of which enables the volume of the compact particle to be changed, preferably increased. A preferred example of a swelling agent is water. Where a swelling agent is used, the degree of swelling may be modulated by various methods. For example, the amount of swelling agent, such as water, which is exposed to a fixed amount of compact particle may be varied. Furthermore, the time or temperature, or both, of exposure of the swelling agent or water to the compact particle may also be varied, in conjunction with, or instead of controlling the amount exposed.

In preferred embodiments, the coupling between the detectable entity and the compact particle is such that it optimally takes place in a non-aqueous medium such as an organic medium. A preferred organic medium is toluene, xylene, dichloromethan, acetone or dimthylformamide, as these are compatible with preferred coupling and activation reagents, for example vinylsulfone, azlactones, cyanuric chloride, dichlorotriazine, chlorotriazine, isocyanates, N-hydroxyl succinimide esters, aldehydes, epoxides, carbonyl diimadazole, cyanogenbromide, tresylchloride, bromoacetyl and alkyl bromide.

In such a case, the amount or degree of swelling may conveniently be controlled by adding amounts of different solvents into the non-aqueous medium. In other words, the extent of swelling may be controlled by allowing coupling in a mixture of non-aqeuous medium and water in varying proportions or by mixing different non aquous solvents like toluene and alcohols.

Mixtures of solvents, whether organic, inorganic, water miscible, water immiscible, etc, may also be used. The solvent mixtures could be any mixable solvents - for example preferable alcohols and water, acetone and water, alcohols and toluene, toluene and dimethylformamide - or any mixture thereof, which are compatible with the compact particle and the coupling chemistry used.

In such a case, the amount or degree of swelling may conveniently be controlled by adding amounts of water into the non-aqueous medium. In other words, the extent of swelling may be controlled by allowing coupling in a mixture of non-aqeuous medium and water in varying proportions. The more water present, the higher the amount of swelling.

The compact particle may be allowed to swell substantially completely during or before coupling. The detectable entity then has access to the interior or core of the compact particle, and can couple thereto, resulting in at least some staining in such core portions. In extreme cases, the detectable entity is coupled to substantially all portions in a cross section of the compact particle. Exposure to a relevant antibody results in homogenous staining (i.e., staining at both core and peripheral regions of the compact particle). The cross section profile of the compact particle and reference standard therefore has a uniform distribution of detectable entity. Such an embodiment is preferred where the detectable entity is known to have a distribution in both the cell membrane as well as the cytoplasm, or even across substantially all portions of the cell.

Where the detectable entity is known or suspected to have some distribution in the cytoplasm, but perhaps the majority being present in the cell membrane, such a distribution or pattern of staining may be mimicked by enabling or allowing the compact particle to swell partially during or after coupling. Partial swelling produces compact particle in which cross sections have substantially more detectable entity at surface portions compared to core portions. Antibody staining is non-homogenous, and is concentrated on the peripheral or surface regions of the compact particle, with limited internal staining.

Where swelling is not allowed to take place, the molecules of the detectable entity will only react with and couple to surface portions of the compact particle. The detectable entity is only able to access and couple to the peripheral or surface portions of the compact particle. No coupling takes place in the internal or core portion of the compact particle, resulting in staining which is substantially restricted to the surface (dense surface staining, no internal staining).

This results in a compact particle with a cross sectional distribution of detectable entity which is substantially restricted to outer portions of the compact particle, with substantially no staining in interior or core portions of the compact particle. The resulting profile will therefore have a "ring" shape. Such a ring shape may be useful where the detectable entity is known in a cell to be restricted to the cell surface, as the staining pattern will in both cases be similar. Reference standards as described in which no swelling is allowed to take place may therefore be usefully employed as standards to gauge the presence, quantity and/or distribution of a detectable entity which is a membrane protein or a cell-surface receptor, for example.

Furthermore, it will be appreciated that the different distributions achieved as described above by modulating swelling may be employed for the purposes of monitoring cell entry of an agent. Thus, for example, they can be used to track whether a particular agent, such as a drug, is capable of passing through the cell membrane and penetration into the cell. The efficiency of a membrane translocation sequence (MTS) such as *Drosophila* Antennapaedia protein or HIV TAT (or their fragments) may be monitored this way.

The compact shape of the compact particle, and hence the detectable entity, is established in the medium in a number of ways. Most simply, the medium is formed around the compact particle comprising the detectable entity coupled or chemically coupled thereto. For example, an embedding medium such as paraffin may be treated with heat and melted, and the molten embedding medium poured around the compact particle; once solidified, the embedding medium will encase the compact particle within it. In such situations, it may be desirable during the process for the compact particle to be held in place with a scaffold or other support for example. Such a scaffold may have the facility to carry more than one compact particle, preferably multiple compact particles in a particular pattern. Once the embedding medium has solidified, the scaffold may be released from the compact particle or compact particles.

Furthermore, the compact particle may be embedded or supported in another medium prior to being embedded in the embedding medium. Such an embodiment is illustrated in the flowchart shown in Figures 11 and 12, which include an agarose embedding step. In this embodiment, the compact particle is held in place in the context of an agarose gel, which may be melted and poured around the compact particle to encase it. The compact particle may be held in place during this procedure with a scaffold, as described above. A strip or portion or block of the agarose gel may then be cut which includes the encased compact particle. The agarose block is then itself embedded in the embedding medium, for example, by melting, pouring and solidifying the embedding medium as described above. The advantage of such an embodiment is that the agarose gel is handled more easily compared to the bare compact particle. Needless to say, the agarose gel should have adequate stiffness, and concentrations of agarose of between 0.5 % to 1 %, 2 %, 3 %, 4 %, 5 % or more may be required.

It will also be apparent that compact particles embedded in agarose (without paraffin embedding) may be themselves used as the reference standard. In such an embodiment, the embedding medium is agarose itself. The agarose block containing embedded compact particles may be cut into sections as described elsewhere in this document, to produce slices or sections for subsequent fixing and staining procedures. For this purpose, the agarose blocks may be frozen, so that slicing is made easier.

## REFERENCE STANDARD USES

As described above, the reference standards as described here provide simple means to establish a "standard", in other words, an established value of a measurable property of a detectable entity. The same or another property, of the same, similar, or different entity in a ample or test item may also be measured, and the values may be compared.

In the most general sense, the reference standard enables the presence of the detectable entity to be revealed. Thus, for some purposes, it is often enough to simply obtain information on the presence of the detectable entity in the reference standard. However, for other purposes, information on one or more characteristics of the detectable entity may be desired. Thus, for example, characteristics such as a dimension, quantity, quality, colour, orientation, position, reactivity (or any combination of any two or more these), etc may be determined. The reference standards may also be used to validate one or more procedures in a method.

### Colour Standard

In one embodiment, the colour of the detectable entity is detected or determined. Thus, for example, it may be desired to have a colour standard in a series of staining experiments. In this case, the reference standard described here may be used to provide a "standard" colour, by inclusion of a detectable entity which has, or is stained to provide, a pre-determined colour.

Use of such a "colour standard" enables an operator to judge whether a sample, which may be stained, is similar to or different from the "standard" colour. For example, the sample when stained may be expected to produce a certain blue colour if positive, and the reference standard may therefore comprise a detectable entity which has or may be stained to produce such a blue colour. The colour in the sample may thus be compared to the blue colour provided by the reference standard to establish whether the sample should be regarded as positive or not.

Furthermore, the "colour standard" may also be used for example for calibration of optical machinery. Colour drifts or errors in colour detection which occur during use of optical machinery may therefore be prevented or adjusted for by comparing the response of the machinery to the standard colour and suitable adjustment if necessary. Two or more "standard" colours, for example of different wavelengths, may be included in the reference standard for more precise calibration.

*Position Standard*

The position of the detectable entity within the reference standard may be detected or determined. Thus, the area comprising the expected colour may be detected by an operator or by machinery, to establish a reference point for a grid location in the sample, for example. The distance between such a reference point and a point in the sample may easily be measured, to provide information on distance, area or volume within the reference standard or sample. The reference standard or position standard may comprise two or more such position standards, preferably three or more position standards. Use of multiple colour locations, of the same or different colours, enables higher accuracy of dimensioning, positioning and navigation through triangulation.

*Quantity / Quality Standard*

In other embodiments, the quantity of the detectable entity is detected or determined. This is most readily achieved by reaction with the binding agent, and the binding agent may be labelled for this purpose. Preferably, the binding agent binds to the detectable entity in a stoichiometric fashion. The intensity of the staining by the binding agent then gives information on the quantity or amount of detectable entity. However, it will be appreciated that other characteristics of the staining, and not just the intensity, may be just as important or useful.

*Validation Standard*

In addition to the other uses described elsewhere in this document, the reference standard described here may be used for validating or verifying one or more procedural steps in a method. By validation and verification we specifically refer to a process by which the success, effectiveness or efficiency of a procedural step is measured.
In general, we disclose a method of validation of a procedure, the method comprising providing a reference standard for a detectable entity as disclosed in this document, applying the procedure to the reference standard or a portion thereof (in particular a slice or section of it), and detecting a change in a property of the detectable entity as a result of the procedure. The property of the detectable entity which is changed is preferably one which is indicative of the success or failure (or relative success or failure) of the procedure. In particular, the detectable entity may be modified in such a way that the procedure, where successful, removes the modification. Alternatively, or in addition, the detectable entity may be modified by the procedure. In each case, the modification is one which is easily detectable as a means to detect the success or failure of the procedure.
Therefore, we disclose a method of assessing the effectiveness or success of a procedure, the method comprising the steps of: (a) providing a reference standard as described in this document, in which a detectable property of the detectable entity is changed as a result of the procedure; (b) conducting the procedure on the reference standard; and (c) detecting a change in the detectable property of the detectable entity.
In one embodiment, a detectable property of the detectable entity is changed as a result of a successful procedure, which change in the detectable property of the detectable entity is detected to establish that the procedure is successful. Alternatively, or in addition, a detectable property of the detectable entity is changed as a result of an unsuccessful procedure, which change in the detectable property of the detectable entity is detected to establish that the procedure is not successful.
We also disclose the use of a reference standard as described in this document, as an antigen retrieval validation standard, a deparaffination standard, a blocking validation standard, a washing validation standard, a primary antibody validation standard, a secondary antibody validation standard, a calibration standard, or a diagnostic standard. The detectable entity preferably comprises a property which is detectable, and which is changed as a result of the procedure, i.e., whether the procedure is successful or unsuccessful.
In particular, the reference standard described here may be used to validate any one or more of the steps employed in traditional IHC staining procedures. Such steps may include: Removal of paraffin, antigen retrieval (AR), blocking, endogenous biotin blocking (for example where a biotin based visualisation system is used), endogenous enzyme blocking (for example phosphatase or peroxidase activity), one or more washing steps, incubation with revealing agent such as a primary antibody, incubation with secondary visualisation components, chromogen staining (for example, enzyme catalysed), staining information acquisition and analysis.
In particular, the reference standard may be used to validate: i) Verifying the ability or functionality of the visualisation system to stain the cell population in the particular staining procedure, ii), verifying the ability or functionality of the primary antibody to stain the cell population in the particular staining procedure, iii) defining a staining threshold intensity for counting positively stained cells, iv) defining the diagnostic threshold intensity ratio between two or more stained populations, v) or verifying the function of individual reagents in the staining protocol, for example antigen retrieval, washing efficiency, blocking of peroxidase activity, and secondary visualisation reagents.

In highly preferred embodiments, the reference standard may be used as a validation standard for the steps of the addition of the primary antibody, the antigen retrieval step, the addition of the secondary visualization reagents, and staining information acquisition and analysis.

*General Procedural Validation Standard*

In one particular embodiment, the detectable entity may comprise streptavidin or avidin. The streptavidin or biotin may be bound to the compact particle. The resulting reference standard may then be used as a simple indicator for the correct addition of certain reagents, for example incubation with the correct primary antibody. By adding for example a small amount of biotinylated mouse antibody to the other vice un-labelled primary antibody solution, the visualization system will stain the reference standard positive if the correct primary antibody is used on the particular slide.

In another particular embodiment, the detectable entity may comprise an immunoglobulin, for example a rabbit or mouse immunoglobulin or antibody. The immunoglobulin could for example be bound to the compact particle in embodiments employing these. The ability of the secondary visualization systems to recognise and stain rabbit or mouse antibodies may thereby be validated.

*Antigen Retrieval Validation Standard*

The reference standard may be used as an antigen retrieval validation standard. For this purpose, we disclose a method of assessing the effectiveness or success of an antigen retrieval procedure, the method comprising the steps of: (a) providing a reference standard as described in this document, in which a detectable property of the detectable entity is changed as a result of the antigen retrieval procedure, in which the detectable property of the detectable entity comprises the masking or unmasking of one or more epitopes; (b) conducting the antigen retrieval procedure on the reference standard; and (c) detecting a change in the detectable property of the detectable entity.

In highly preferred embodiments, the detectable entity in the reference standard is modified to mask one or more epitopes, some or all of which are unmasked in an antigen retrieval procedure which is successful.

Antigen retrieval ("AR") procedures can be standardized or monitored by employing reference standards comprising detectable entities which cannot normally be stained or in which the staining level depends strongly on the antigen retrieval process.

The detectable entity may be modified such that it completely or partially loses its antigenicity. In other words, one or more epitopes in the detectable entity may be masked artificially or naturally. Correct antigen retrieval unmasks the epitope(s) or antigens, and is revealed by correct staining of the detectable entity in subsequent procedures.

The masking or loss of antigenicity (which may affect one or more epitopes) may be effected chemically. For example, the same immunoglobulin modified compact particle as described above could be fixed with for example formaldehyde. In particular, the detectable entity could be "masked" by over fixation with formaldehyde, resulting in the loss of most or all of the epitopes and low diffusion in the reference material.

Paraformaldehyde or any other fixatives as known in the art may also be used. Derivatives with acetylating, alkylating or otherwise masking reagents may also be employed for this purpose. Numerous methods are known from the organic chemistry literature, for example masking using Schiff Bases, esters, ethers or hemiacetal derivatives.

The masking may also be effected immunologically, by for example, the use of a suitable masking antibody or other binding agent. The reference material may therefore contain chemically and/or immunologically masked targets for either the primary antibody or the secondary visualization system.

Demasking or deprotection can be achieved by either chemoselective antigen retrieval or random antigen retrieval. Masked targets, which will only be stained when excessive antigen retrieval procedures are used, may also be employed

Such a reference standard can be used as an indicator of correct antigen retrieval. Correct antigen retrieval procedure will demask the immunoglobulin and stain this embodiment of the reference standard. Compact particles with other proteins or peptides could be fixed with for example formaldehyde and thereby totally lose their antigenicity. Such a reference standard will be indicative of correct antigen retrieval. Correct antigen retrieval procedure will demask the immunoglobulin and stain this embodiment of the reference standard.

*Deparaffination Standard*

The reference standard may be used as validation standard for deparaffination, i.e., for the step of removal of paraffin.

For this purpose, we disclose a method of assessing the effectiveness or success of a deparaffination procedure, the method comprising the steps of: (a) providing a reference standard as described in this document, in which a detectable property of the detectable entity is changed as a result of the deparaffination, procedure, in which the detectable property of the detectable entity comprises the presence or quantity of detectable entity in the reference standard following the deparaffination procedure; (b) conducting the antigen retrieval procedure on the reference standard; and (c) detecting a change in the detectable property of the detectable entity.

In highly preferred embodiments, the detectable entity in the reference standard is soluble in the deparaffination medium, and in which at least a portion, preferably all, of the detectable entity is removed following a successful deparaffination procedure.

Thus, reference material with non-covalently coupled or chemically coupled and water insoluble targets detected by for example the secondary visualization systems can indicate insufficient deparaffination if they are stained.

For example, in traditional IHC procedures, deparaffination is done by washing with toluene or for example citrus or coconut oil followed by rehydration in alcohol/water solutions. Thus, a reference standard used for such a purpose may comprise detectable entities which are easily detached or removed or dislodged from their positions within the supporting medium. For example, they may be loosely coupled or chemically coupled, for example, by non-covalent means, to the compact particles in certain embodiments. For such purposes, it is desirable that the detectable entity be water insoluble. If the deparaffination step is conducted properly, then the detectable entity should not be revealed by the reagents such as secondary visualisation systems.

Furthermore, a marker such as a dye may be added to the paraffin. Such a dye would preferably stain the detectable entity, or the compact particle on which the detectable entity is coupled or chemically coupled. Where insufficient deparaffination has taken place, the presence of the dye will be easily visible to the human eye (or easily detected by machinery such as image analysis systems). This will indicate that insufficient deparaffnation has taken place.

*Blocking Validation Standard*

The reference standard described here may also be employed for validating any blocking steps, for example, blocking of endogenous activity, such as endogenous biotin activity or enzymatic activity.

Thus, for example, biotin or other haptens, which are naturally deposited in tissue, need to be blocked before one can use a visualization system using biotin or other haptens. It is because of the presence of endogenous biotin that secondary (for example goat anti mouse or goat anti rabbit) visualization systems are preferred, as they produce less "background" noise. For validating blocking, the reference material can contain biotin (or other haptens like Digoxigenin or DNP) used in the visualization system, which may be bound to the compact particle where present. Positive staining of this reference material will indicate insufficient biotin blocking - due to for example non-reactive reagents, inefficient mixing/diffusion on the slide or too short incubation time.

The reference material may also contain covalently bound enzyme used in the enzyme catalysed staining system as the detectable entity. The typical enzymes used are peroxidase or phosphatase. For example, the detectable entity may comprise horseradish peroxidase. Alternatively, or in addition, compact particles modified with for example horseradish peroxidase may be used for validating correct and efficient endogenous peroxide blocking. If the reference standard remains unstained after the last peroxide chromogen step, the blocking will be determined as being efficient.

Thus, positive staining of this reference material will indicate insufficient latent enzyme blocking - due to for example non-reactive reagents, reversible blocking, inefficient mixing/diffusion on the slide or too short incubation time. The material could be combined with the antigen retrieval reference material.

*Washing Validation Standard*

The reference standard may also be used to validate the efficiency of any number of washing steps, for example, washing with buffer. The reference material could include a detectable entity which is insoluble in organic solvent (for example, toluene insoluble) and partly water insoluble for either the primary or secondary visualization system. The target should not be covalently bound in the reference material. It could be bound by ionic pairing or metal complex binding or by simply adsorption.

The target could have a large molecular weight in order to test the ability of washing buffers to diffuse into the material and remove the target. The molecular weight cut off ("MwCO") of the reference material should be matched with the Mw of the targets by e.g selection of pore size or the degree of fixation.

Positive staining of this reference material will indicate insufficient washing - either due to for example number of washing steps, type of buffer used, inefficient mixing on the slide or too low temperature or diffusion times. For this purpose, the mounted sample tissue section and the reference material should preferably have the same thickness.

Alternatively, the target could be substituted with a detectable high molecular weight dye trapped in the reference material. The dye will be removed by efficient washing - and only be present if the washing is insufficient or ineffective.

Alternatively, a reference standard comprising a compact particle (or detectable entity) made of a partly water soluble material could be used. If the compact particle or detectable entity disappears, the washing was efficient.

*Primary Antibody Validation Standard*

The reference standard may be used as a standard for any incubation step, for example, the step of incubation with the correct primary antibody. One of the most critical human errors in the IHC staining is incubation with the wrong antibody.

The reference standard for use in validation of correct primary antibody addition or incubation could therefore comprise a detectable entity which is capable of binding (preferably specific binding) to a binding partner. The binding partner would be added to the primary antibody solution as a "marker", which would bind or specifically attach to the detectable entity and therefore indicate that the correct primary antibody was used. It will be appreciated that the primary antibody may be sold in a form which comprises the "marker".

As a particular example, the detectable entity may comprise or consist of streptavidin, and the "marker" comprise or consist of biotin. For example, the primary antibody may be supplemented with a biotinylated irrelevant mouse antibody, which would specifically attach to a detectable entity in the reference standard, for example, a detectable entity comprising or consisting of biotin. Where slices or sections of the reference standard are taken (as is preferred), the reference dot would be stained positive by the visualization system, if the correct antibody was used. It will be appreciated that the primary antibody could itself be modified with biotin, and function as the "marker", so that an additional "marker" is not strictly necessary.

*Secondary Antibody Validation Standard*

For use in validating secondary antibody addition, the reference material could contain covalently bound mouse or rabbit antibodies or fractions hereof in various density. Positive and graded staining of this reference material will validate the functionality of the secondary visualization system. The same reference material could be combined with the reference material useful for validating the antigen retrieval step.

*Calibration Standard*

Besides analysis of the various reference materials for graded primary or secondary staining, the reference system could consist of or contain permanent colours and physical shapes suited for calibrating cameras, optics and software algorithms.

Therefore, in yet another aspect, the reference standard can serve as a calibrator for any equipment, for example digital image processing equipment or any automatic image analysis system. This may be achieved for example by defining a particular colour, intensity or a particular number of events. This is particularly useful in automated scanners and microscopes. By combining a dyed material with an immunological or special staining, orientation and navigation on the slide microscope may be made easier.

Such reference material could help to define sizes, colours, colour spectra, boundaries between stained areas, counterstaining level and background.

It will be appreciated from the above that it is possible to construct a reference standard which is capable of being used to validate more than one of the procedural steps. For example, we disclose reference standards which are capable of validating 1, 2, 3, 4, 5, 6, 7, 8, 9, 10, 11, 12 or more procedural steps in any method. Such reference standards may suitable comprise a plurality (or more than one) detectable entity, which may be the same or different, as described above. The arrangement of the detectable entities in the reference standard is preferably such that the resulting slice or section taken of the reference standard comprises more than one "dot" or reference area, suitably arranged in an array.

Such reference standards may give rise to slices or sections which comprise for example a "spot array" of 9 different reference materials. For example, they may comprise spots with targets for the primary antibody in different density - giving graded staining levels; spots with lightly and graded fixed targets for the secondary visualization system - general validation of the antigen retrieval step; spots with lightly and graded fixed targets for the primary antibody - validation of the specific target antigen retrieval step or "threshold" antigen retrieval necessary for staining; spot with "over fixed" targets for the secondary visualization system - general validation of the antigen retrieval step; spots with targets for the secondary visualization system (for example mouse or rabbit Abs) in different density - general validation of the secondary visualization system; spots with peroxidase and/or phosphatase activity - validation of the endogen enzyme blocking step; spots with bound biotin - validation of the endogen biotin-blocking step (if for example LSAB type of visualization systems are used; spots with non-covalently bound and somewhat high molecular weight targets for the secondary visualization system - general validation of the washing steps; spots with distinct, homogeneous and permanent shape, size and colour - Calibration of the automatic image analysis system.

In highly preferred embodiments, the reference standard comprise an array of reference "spots" on the same slide as the patient sample tissue. In such an embodiment, it is possible for validation to be done automatically by the image analysis software.

**DIAGNOSTIC STANDARDS**

In highly preferred embodiments, the reference standard is or may be used as a diagnostic standard. By this we mean that the detectable entity is detected for the purpose of revealing a condition of a cell, preferably a physiological or medical condition of the cell. It will however be appreciated that in some or more cases, the mere detection of a property of the detectable entity may be insufficient in itself to provide a medical diagnosis. Therefore, it will be appreciated that in some cases it may be desirable to conduct other tests in order to establish diagnosis.

In embodiments where the reference standard is employed as a diagnostic standard, the detectable entity suitably comprises an indicator of a state of a cell such as an indicator of the health or disease state of the cell, for example, a disease marker. Thus, a detectable entity may indicate, by its presence or quantity in a relevant sample, the state of the organism (such as its state of health or state of disease) from which the sample is taken. Disease markers, in particular, cancer markers, are discussed in more detail below.

Preferably, the reference standard is such that a cross section of it includes a defined area comprising an amount of detectable entity. This is shown in the illustration in Figures 4B, 5A, 5B, 8F, 9B and 10. The amount of detectable entity in the reference standard or cross section may be compared with that in the sample to establish whether the latter is present in identical or similar amounts, or in lesser amounts, or in greater amounts, than that in the standard. However, it will be appreciated that while the reference standard described here is most useful to detect the presence and/or quantity of a detectable entity in a sample, it may be used more simply to indicate whether a detectable entity in a sample is the same as, or different from, the detectable entity of the reference standard.

The quantity or amount of detectable entity in the reference standard or cross section is preferably a known or pre-determined quantity. The quantity may be varied by controlling the quantity which is coupled or chemically coupled to or retained in the compact particle, as described in further detail below. Where slices or sections of the reference standard are taken, variation in quantity may also be achieved by changing the thickness (and hence the amount of detectable entity captured) of the section or slice.

In highly preferred embodiments, the quantity of a detectable entity in the reference standard comprises a diagnostically relevant quantity.

In contrast to the prior art, accurate and known amounts of detectable entity may be incorporated in the reference standard as described. Furthermore, sample-to-sample variation as present in the prior art may be overcome. The result is a more precise grading system.

In some embodiments, different quantities of the detectable entity are present in the reference standard. In a preferred embodiment, the reference standard comprises two or more quantities of the same detectable entity, in separate compact particles. Preferably, a range of quantities of increasing amounts of the detectable entity is provided, preferably in an arithmetic or more preferably a logarithmic range. Preferably, the range encompasses a diagnostically or clinically relevant amount of detectable entity, i.e., an amount of detectable entity, which if-present in the sample at about or above that amount, indicates that the sample contains, or is likely to contain, cells or tissues which are diseased or prone to disease.

Comparison of the amounts present in a cell or tissue or other biological sample with the reference standard according to this embodiment will provide an indication of whether the particular sample is "positive" or "negative" for the particular detectable entity. Where the detectable entity comprises a disease antigen, such as a cancer antigen (or expresses DNA/RNA, including messenger RNA, derived from a cancer gene) its presence or quantity may be used as a diagnostic for a relevant disease. We therefore provide a method of diagnosis of a disease in an individual, comprising comparing the presence or amount (or both) of a detectable entity in a biological sample from the individual with the amount in a reference standard as described here. Preferably, the comparison is done against a clinically relevant amount of detectable entity in the reference standard (i.e., an amount in a sample at or above which the presence of a disease is indicated, suspected or diagnosed).

In a particular embodiment, the detectable entity comprises HER2 antigen, the cell or tissue comprises breast tissue, and the disease which is diagnosed, or its presence indicated, comprises breast cancer. The detectable entity may also comprise other antigens, instead of or in addition to HER2, preferably selected from the group consisting of: oestrogen receptor (ER), progesterone receptor (ER), p16, Ki-67 or Epidermal Growth Factor Receptor (EGFR) (see also section "Detectable entity"). Mixtures of any two or more of these may be used. The detectable entity may comprise a nucleic acid encoding any of the above, or any detectable entity as specified in the section "Detectable Entity"; in particular, such reference standards comprising nucleic acids are useful for standardising *in situ* hybridisation. In particular, the reference standard may comprise a nucleic acid cancer marker, such as DNA/RNA cancer markers (for example an cancer mRNA marker).

Preferably, the reference standard comprises, in addition to a clinically or diagnostically relevant amount of detectable entity, a negative control, i.e. a defined area or volume or path comprising no detectable entity, or detectable entity at an undetectable quantity, for example in compact or elongate shape.

The diagnosed disease may be optionally treated, by administration of an appropriate therapeutic agent. Such an agent or drug may be one which is known to be effective for treating such a disease. In particular, the therapeutic agent may comprise an antibody, preferably an antibody against the detectable entity. Such an antibody may comprise the same antibody which was used for staining and detecting the detectable entity in the reference standard and/or the biological sample, or it may be a variant of it, such as a humanised antibody, or a single chain antibody such as an ScFv.

In particular, the detectable entity may comprise HER2, the binding agent may comprise any anti-HER2 antibody and the therapeutic agent may comprise a humanised anti-HER2 antibody such as Herceptin (Trastuzumab, Genentech). The detectable entity may comprise a HER2 nucleic acid, such as a HER2 RNA, HER2 mRNA or a HER2 DNA. The detectable entity may comprise any molecule such as a nucleic acid capable of binding to the HER2 nucleic acid, and may in particular comprise a sequence complementary to at least a portion of the HER2 nucleic acid.

HER2 and Herceptin are described in a number of publications, including Pegram M, Hsu S, Lewis G, et al. Inhibitory effects of combinations of HER-2/neu antibody and chemotherapeutic agents used for treatment of human breast cancers. Oncogene. 1999;18:2241-2251; Argiris A, DiGiovanna M. Synergistic interactions between tamoxifen and Herceptin. Proc Am Assoc Cancer Res. 2000;41:718. Abstract 4565; Pietras RJ, Fendly BM, Chazin VR, et al. Antibody to HER-2/neu receptor blocks DNA repair after cisplatin in human breast and ovarian cancer cells. Oncogene. 1994;9:1829-1838; Baselga J, Norton L, Albanell J, et al. Recombinant humanized anti-HER2 antibody (Herceptinú) enhances the antitumor activity of paclitaxel and doxorubicin against HER2/neu overexpressing human breast cancer xenografts. Cancer Res. 1998;58:2825-2831; Sliwkowski MX, Lofgren JA, Lewis GD, et al. Nonclinical studies addressing the mechanism of action of trastuzumab (Herceptin). Semin Oncol. 1999;26(suppl 12):60-70; Lewis GD, Figari I, Fendly B, et al. Differential responses of human tumor cell lines to anti-p185HER2 monoclonal antibodies. Cancer Immunol Immunother. 1993;37:255-263 and Pegram MD, Baly D, Wirth C, et al. Antibody dependent cell-mediated cytotoxicity in breast cancer patients in Phase III clinical trials of a humanized anti-HER2 antibody. Proc Am Assoc Cancer Res. 1997;38:602. Abstract 4044.

In other embodiments, more than one detectable entity is present in the reference standard. In particular, we envisage a plurality of different types of detectable entity in a single reference standard. Where more than one detectable entity is present in the reference standard, each of these may be in or on or coupled or chemically coupled to the same one compact particle, or more than one compact particle may be present. In the latter case, the or each compact particle may comprise one or more different detectable entities. The detectable entities may be present in the same or different quantities, in the or each compact particle.

Therefore, two or more different detectable entities may be supported in the embedding medium, at least one of which is coupled or chemically coupled to a compact particle. Preferably, all the different detectable entities are coupled or chemically coupled to compact particles. The reference standard may comprise a single or more than one quantity of a first detectable entity, and a single or more than one quantity of a second detectable entity. Multiple detectable entities, at the same or different quantities, may be present in the reference standard. Where more than one detectable entity is present, the reference medium preferably comprises at least one detectable entity at a quantity or amount which is diagnostically or clinically significant.

The reference standard may therefore comprise a quantity of first detectable entity therein coupled or chemically coupled to a compact particle. The reference standard may further comprise a quantity of a second detectable entity attached or coupled to the same compact particle, or in a second compact particle. Furthermore, the reference standard may comprise a second different quantity of the or each detectable entity attached or coupled to the compact particle, or in a second or further compact particle. The multiple same or different detectable entities and / or quantities of such may be mixed or separated in space and / or time.

For example, the detectable entities may comprise HER2, together with another cancer antigen, such as *ras*, or in particular a breast cancer antigen such as a BRCA1 or BRCA2 protein. Alternatively or in addition, the reference standard comprises a quantity of tumour suppressor protein such as retinoblastoma (Rb) protein. The detectable entities may also comprise nucleic acids, such as HER2 nucleic acids, and other cancer antigen nucleic acids. The detectable entities may comprise one or more polypeptide detectable entities, together with one or more nucleic acid detectable entities.

Such embodiments of reference standards comprising a plurality of detectable entities are useful in applications where testing of a sample is conducted using more than one binding agent. Thus, we envisage the use of such reference standards in testing using "banks" or "panels" of antibodies/probes, for example. Such testing may be used to detect the presence or absence, or relative or absolute levels of different detectable entities in a sample, each of which may be diagnostically relevant. Such relative or comparative information is typically more useful than a single presence/ absence test. Multiple testing in this way may be used to generate a "profile" of the patient or individual in question. Profiles generated from individuals suffering from (or suspected of suffering from), a disease or condition may be compared against matching profiles of "normal" or non-affected individuals. The profiles, or information generated by comparing profiles, may be used to diagnose (or aid in the diagnosis of) a disease or condition in that individual for the purpose of selecting the best possible treatment ("individualised therapy").

In embodiments where more than one compact particle is present, it may be advantageous to arrange the compact particles in a bundle, or more than one bundle. The compact particles may be arranged in such a way that it makes it easy to find the different reference dots and clusters. For example, an asymmetrical pattern of compact particle bundles can help the user to orientate the slide correctly.

Needless to say, where more than one detectable entity is present in the reference standard, the two or more detectable entities may be present in different compact particles. Alternatively, more than one detectable entity may be present in a single compact particle. For example, more than one detectable entity may be conjugated or coupled or chemically coupled to a compact particle. Multiple compact particles, each comprising a single, two or more detectable entities, may be used.

The or each detectable entity is present in an compact particle in the reference standard; this may be achieved by various means as described in detail further below.

The reference standard, or slices or sections of it may be packaged in a kit. The kit may comprise a binding agent, such as an antibody, which is capable of specific binding to the detectable entity. The kit may comprise a microtome block with the reference standard, slices or sections mounted thereon. The kit may further comprise other reagents, such as detection, washing, or processing reagents, as well as instructions for use. The kit may further comprise instructions for use, or other indicia, for example scoring aids such as charts or photographs of diseased and/or normal tissue.

The kit may also comprise a therapeutic agent which is capable of treating or at least alleviating at least one of the symptoms of a disease. We also provide a combination of a reference standard as described here, or a section or slice thereof, together with a therapeutic agent.

In particular embodiments, the disease is one whose presence in an individual is indicated or suspected where a biological sample comprises a diagnostically relevant amount of detectable entity. In particular, the therapeutic agent may comprise an antibody against the detectable entity, or a nucleic acid capable of biding to the detectable entity, any other therapeutic agent which is known to be effective in treating or preventing the disease. For example, a kit or combination for detecting breast cancer may comprise a reference standard in which the detectable entity comprises HER2 antigen or HER2 nucleic acid. The kit may further comprise anti-HER2 antibody for detecting of that antigen, and may also further comprise any breast cancer drug, for example, Herceptin (a humanised monoclonal antibody which targets HER2 protein in metastatic breast cancer patients). Other embodiments of the kit include any antigen selected from the group consisting of oestrogen receptor (ER), PR, p16, Ki-67 or Epidermal Growth Factor Receptor (EGFR) (see also section "Detectable entity").

In preferred embodiments, the detectable entity is molecular in nature (see description below), and the reference standard is therefore substantially free of cellular material. However, in some embodiments, cellular components, for example parts of a cell (for example, any subcellular compartment or organelle such as the nucleus, mitochondria, chloroplast, vacuole, etc) or whole cells themselves may be incorporated in the reference standard.

**FLOW CYTOMETRY STANDARDS**

The reference systems described here may be used, for example, as a reference standard for applications such as Fluorescence Activated Cell Sorter (FACS). In such applications, where the reference standard may preferably comprise cellular or biological compact particles, the embedding medium need not be present. That is to say, where cells or cellular components are used as compact particles for chemical coupling of detectable entity, the cells or cellular components themselves may be regarded as refererence standards.

Flow cytometry is a system for measuring cells, beads or particles as they move in a liquid stream, in the so-called flow cell, through a laser or light beam past a sensing area. The relative light scattering and colour discriminated fluorescence of the particles is measured. In the flow cytometer, different cells can be identified by their distinct cell morphology like density, shape and size. Tissue morphology as such is not visible from the data obtained from flow cytometer as the cells are broken up.

A flow cytometer consists in general of a light source, flow cell, optics to focus light of different colours onto a detector, signal amplifier and processor and a computer to record and analyse data. Lasers are used as the preferred light source in modem flow cytometers. The most common laser used is the argon-ion laser. This produces a major line at 488 nm, which gives a source of blue light for excitation of e.g. fluorescein, phycoerythrin, and tandem conjugates and for propium iodide used in DNA measurements. In the flow cell, cells are aligned by hydrodynamic focusing, so that they pass through the laser beams one at a time.

Light scatter is utilised to identify the cell or particle population of interest, while the measurement of fluorescence intensity provides specific information about individual cells. Individual cells held in the stream of fluid are passed through one or more laser beams. The cells scatter the laser light, which at the same time make fluorescent dyes emit light at various frequencies. Photomultiplier tubes (PMT) convert light to electrical signals and cell data is collected.

What makes flow cytometry such a powerful technique is its ability to measure several parameters on many thousands of individual cells in a very short time, by measurement of their fluorescence and the way in which they scatter light. As an example, using blue light for excitation, it is possible to measure red, green and orange fluorescence and the amount of light scattered, both forward and at right angles to the beam, on each cell in a population of thousands.

Many instruments can measure at least five different parameters. As all the parameters cannot be combined for display simultaneously in a correlated fashion, a system called gating is employed. Regions of interest - or "Gates"- are defined, enabling selection of specific cell populations for display of further parameters. A flow cytometer can be used to analyse sub-populations of cells, which have been fluorescently labelled, with speed and accuracy. Sorting on the basis of other features, e.g. size, is also possible.

Flow cytometry instruments simultaneously generate three types of data: 1) Forward scatter (FSc) gives the approximate cell or particle size, 2) Side or Orthogonal scatter (SSc) gives the cell or particle complexity or granularity, and 3) fluorescent labelling is used to investigate e.g. cell structure and function. Forward and side scatter are used for preliminary identification of cells. In a peripheral blood sample, for example, lymphocyte, monocyte, and granulocyte populations can be defined on the basis of forward and side scatter. Forward and side scatter are used to exclude debris and dead cells. Particles, for example, can be identified by their size and/or their fluorescence.

Cell or particle populations may be represented on single or dual parameter histograms. Light scatter and fluorescence signals may be analysed after linear or logarithmic amplification. Once the population of cells or particles to be analysed has been identified, the fluorescence associated with bound antibodies or dyes is determined after the background fluorescence has been established.

Some flow cytometers are able to physically sort cells or particles into specific populations. This is most commonly done by electrostatic deflection of charged droplets containing a cell. The flow cell is vibrated and causes the liquid stream to break up into small droplets as it leaves the exit nozzle. At the moment a cell or particle of interest is inside the droplet currently being formed, the flow cell is charged - thus charging the droplet. The stream of droplets then passes through a pair of electrically charged plates, and droplets that are charged (containing the cells or particles of interest) are deflected into a collection vessel.

The electric field created between the plates can direct the cells or particles towards one of several user-specified collection receptacles. Uncharged droplets flow into a waste vessel. Analysis of concentrations of cells or subsets of cells, often referred to as "absolute counting", can be of further interest for medical diagnostics or monitoring the status of cells in cell cultures or other biotechnological processes.

The flow cytometer is able to rapidly screen large numbers of cells far beyond the capacity of traditional pathological or cytological methods. The information obtained aids in the diagnosis, classification, and prognosis of a variety of diseases. The applications to which flow cytometry can be applied have expanded rapidly from cell sorting, to measurement of cell surface antigens, and analysis of DNA to aid the interpretation of malignant disorders.

Common uses for flow cytometry in the routine clinical laboratory include immunophenotyping of hematopoietic neoplasms, immune status evaluation, especially quantificaation of CD4+ T-cells in HIV positive patients, and DNA cell cycle analysis of solid tumours. Different cell populations that compose the hematopoietic system express distinctly different cell surface antigens at various stages of maturation. By detecting and measuring these expressed antigens, flow cytometry can aid in the classification of the cell lineage of leukaemia and lymphoma.

Although not intended to be an independent diagnostic modality, flow cytometry is often able to sub-classify haematopoietic malignancies beyond the capabilities of traditional morphologic and cytochemical techniques.

The most common routine uses of flow cytometry have been measurement of surface antigens (markers) by immunofluorescent labelling using monoclonal antibodies. The markers commonly used are total B-cells, total T-cells and subsets of T-cells. The markers for total T-cells, Helper T-cells and suppressor T-cells have been assigned the cluster differentiation (CD) categories of CD3, CD4, and CD8, respectively. This spectrum of markers, of which there are more than 45 in all, are used for clinical classification of immunodeficiency states, lymphoid leukaemias, autoimmune diseases and for monitoring their response to therapy.

For example, CD4 and CD8 measurements are especially useful to monitor the progression of AIDS, as the CD4+ cells are depleted by infection by HIV, whereas the CD8+ cells persist. The absolute number of CD4+ cells is also a marker of progression of HIV infection to more overt AIDS. The CD4/CD8 ratio can also be used to assess the success of immunosuppressive therapy with cyclosporin A in transplant patients.

For immune status evaluation, typically sub-populations of lymphocytes are identified and quantified by the flow cytometer by utilising monoclonal antibodies to various cell surface antigens. Patients with acquired or congenital immunodeficiency disease and patients on immunosuppressive drug therapy exhibit characteristic alterations in lymphocyte populations.

The typical direct staining procedure for flow cytometry may include one or several of the following steps besides washing and mixing steps: Fixation of the cells with e.g. buffered formaldehyde, permeabilisation, addition of fluorescently labelled target specific reagent, incubation, centrifugation, aspiration of the supernatant from the cell pellet, resuspension, dilution and analysis on flow cytometer.

The advantages of flowcytometry are numerous; it does however have a number of disadvantages, the main one being the fact that the data obtained is not absolutely quantitative, as the signal strength for each fluorescent colour depends on e.g. PMT settings, exact laser conditions, and compensation algorithm. Another disadvantage is that the volume of the cell suspension analysed is not directly measured. Therefore, the absolute concentration - i.e. cells per volume - is not being directly measured.

The absolute concentration of cells in a sample can e.g. be measured by adding a known volume of a solution of fluorescent-tagged polymer beads to the sample. The concentration or number of polymer beads has been established by another method. By counting the number of polymer beads and cells in the flowcytometer, the absolute concentration of cells - or "absolute count" as it is often referred to - can be calculated. A known example of this technique is the TrueCount (BD Bioscience) system.

Light emitted from the fluorescent dyes are detected in one or several of the channels, the FL1, FL2, FL3 channels etc. As some of the fluorescent dyes have emission spectra, which can be detected in several channels, it is often necessary to correct the obtained data by compensating for the signal spill over into several channels.

Compensation has been done in the prior art by using beads made of a solid polymer material, often polystyrene. The polystyrene beads are embedded with the dyes in question or functionalized on the surface with the dye. The spill over between the detector channels can then be measured and the compensation algorithm adjusted to the present instrument setting.

Flowcytometry has become indispensable in both the routine and research laboratory. As new fluorescent dyes are found and monoclonal antibodies are produced to even more antigens, the applications to which flowcytometry can be applied will continue to increase.

As indicated by the descriptions above, the technique is complex and utilises molecules with high affinities for specific staining. The full potential of flowcytometer analysis has not been fully utilized, as comparison of diagnostic results can be difficult to interpret from laboratory to laboratory due to lack of standardization and references. Thus, improvement of the existing means, in particular with regard to internal reference material, is continuously sought.

The reference standard described in this document can be used to calibrate the staining level for a particular diagnostic target or ratio of diagnostic targets. The use of an internal control reflects and accounts for variations in the pre-treatment and staining protocols.

Besides the ability to verify and calibrate the staining procedures, the staining level and the staining reagent quality, as in e.g. the slide based applications like IHC and cytology, the reference standard has several specific advantageous properties for use in flowcytometer applications. These are described in detail below:

*Dual Applications*

The same reference standard can be stained and measured in both slide-based techniques and in a flowcytometer. The data from two independent techniques can be combined to further strengthen the validity of the analysis and calibrate the different techniques in relation to each other.

It should be understood, that as the reference standard can adopt the shape of a cell, it is capable of mimicking any one or more properties of the cell, such as shape, size, scattering ability, etc. Therefore, the technical imperfections of using polymer beads could be avoided using the reference standard using a cellular support as decribed here.

In the following, some additional preferred embodiments are described:

*Compensation*

The reference standard may be used as a compensation standard. Dyes embedded inside solid beads like e.g. polystyrene latex particles have distorted fluorescent spectra as compared to the dyes used as tags on e.g. antibodies in solution during the staining of samples. Also, fluorescent dyes like e.g. RPE, APC or their tandem conjugates attached or tagged to the surface of polymers or other solid particles may have distorted spectra due to surface interactions. During the subsequent sample analysis, the compensation setting may therefore not represent the true spill over of signals between channels.

By using cell materials as the basis of the reference standard, i.e., employing a detectable entity chemically coupled to a support which is of biological or cellular origin, one can use dyes in the correct surroundings and at conditions almost identical to the actual analysis.

Of particular interest is the possibility of using compensation standards tagged with the same lot or batch of fluorescent reagent used in the subsequent sample analysis. Thereby the compensation settings can be calculated based on dye compensation references with the same properties as during the analysis.

*Sorting*

The reference standard may be used as a sorting standard. The reference standard may therefore be used to verify and monitor the quality of flow cytometer sorting. As the reference material can be cell like, and stained as e.g. true patient samples, the quality of sorting can be compared from run to run and from laboratory to laboratory.

This will be of special importance for clinical sorting, e.g. sorting of specific T-cells, in which the quality of the sorting is of tremendous importance and need to be documented in relation to a standard. Also, sorting of cells in low concentration will need very accurate optimisation and validation using cell standards.

Solid beads like e.g. polystyrene latex particles have a disadvantage compared to the reference standards described here in that they may not truly represent e.g. patient cells during optimisation and validation of sorting experiments.

*Enumeration or Absolute Counting*

The reference standard may be used for absolute counting calibration in a way similar to the use of solid beads. It may therefore be used as an enumeration standard or as an absolute counting standard.

Absolute counting is especially of importance for analysis in connection with transplantation (CD34 counts) and for analysis of rare events, like platelet counting or minimal residual decease. By combining e.g. fluorescent-tagged target or targets attached to the reference standard, it will be possible to e.g. immunologically label the target in flowcytometry.

By further measuring the number or concentration of reference cells by other methods, the same reference standard can calibrate the flow cytometry analysis with respect to e.g. counting, staining level and compensation.

## REFERENCE STANDARD SHAPE

The reference standard described here may take any suitable shape.

The reference standard may have an amorphous shape, for example, an elongate amorphous shape, but preferably it has a defined shape. It may take the form generally of a sphere, but preferably, the reference standard is of polyhedral shape.

More preferably, the reference standard has substantially a shape selected from the group consisting of: a regular polyhedron, a prism, a right prism, a regular right prism, a cuboid, a rectangular box and a cube. The reference standard preferably has an elongate shape, such that it possesses a long axis.

In highly preferred embodiments, the reference standard has a cuboid shape. A cuboid as the term is used here refers to a closed box composed of three pairs of rectangular faces placed opposite each other and joined at right angles to each other, also known as a rectangular parallelepiped. The cuboid is also a right prism, a special case of the parallelepiped, and corresponds to what in everyday parlance is known as a (rectangular) "box".

Where slices or sections are taken of the reference standard, these are preferably taken at planes which are substantially at right angles to the orientation of the reference standard, i.e., longitudinally. Alternatively, or in combination, the sections or slices may be taken transversely with respect to the orientation of the reference standard.

## EMBEDDING MEDIUM

The reference standard comprises an embedding medium in which the compact particle is supported. Any material or medium which is capable of embedding and supporting the detectable entity may be used as an "embedding medium".

Preferably, such an embedding medium is capable of supporting the detectable entity so that it retains its shape, position, or configuration substantially. In preferred embodiments, the embedding medium supports the detectable entity in compact shape. In preferred embodiments, the detectable entity is maintained in position within the embedding medium, i.e., it does not move or shift position. The embedding medium may comprise a rigid or semi-rigid material such as a solid, semi-solid, or a gel. In highly preferred embodiments the embedding medium does not include a liquid, such as water or a buffer. Preferably, the embedding medium is solid or semi-solid.

The embedding medium may be comprised of a matrix or web, or network or grid, on which the detectable entity is supported, for example. The matrix, web etc may be comprised of any suitable fibrous material, for example, carbon fibre or fibreglass. A loose matrix, web, etc may be employed, providing a substantially open structure. Alternatively, a more dense structure may be preferred in certain embodiments.

The embedding medium preferably surrounds or envelopes at least a portion of the detectable entity, preferably its entirety. Any embedding medium as known in the art, such as an immunohistochemical (IHC) or an *in situ* hybridisation (ISH) embedding medium, may be employed for this purpose. Polymers, preferably made by polymerisation of monomers, may be used, as described below. Preferred examples of such embedding media comprise paraffin and agarose.

The embedding medium may be homogenous, or it may be heterogeneous and comprise other material. This "other material" may comprise cells, parts of cells, tissue fragments, dyes, granular materials, etc.

The purpose of including this other material is to produce a "ground" or "background" in any slice or section taken of the reference standard; the presence of "ground" enables the defined region comprising the detectable entity to be more easily detected or located, i.e., it increases the contrast. Furthermore, the presence of the "ground" enables the viewer to make a more accurate determination of the presence or quantity of the detectable entity within the embedding medium. This is because of the well known "optical effect", in which the eye perceives two signals of identical intensities as being different intensities, depending on the background. Thus, for example, in a clinical sample, the signal to be detected by the eye (e.g., a cell which is stained) may have a background comprising other cells, other cells of different types, blood vessels, bone tissue, etc. Accordingly, the use of the other material within the embedding medium ensures that the eye will perceive signals from the reference standard of a similar intensity to those from the sample in question as being similar or identical, rather than of different intensity.

The embedding medium may further comprise orientation means. The orientation means may comprise any visible indication within the medium which aids or enables the user to determine its orientation or direction or position. The orientation means may in particular comprise reticulations, or a network of lines, within the medium. The orientation means may comprise one or more generally parallel lines in one or more planes. Preferably, a three-dimensional network of such planes each comprising parallel lines is included. The matrix may therefore include visible structures that look like for example chicken wire net to help the user to navigate over the slide, as pathologists are trained to look for "chicken wire" structures.

The embedding medium may comprise biological material, such as cells, tissues, organs, etc, or any part of these, such as organelles, cellular structures, etc. The biological material may completely surround the detectable entity, or be positionally spaced from it. In either configuration, a planar section or slice will comprise a defined region comprising the detectable entity, together with a section of the tissue, etc. In the former configuration, the defined region is located within the section of the tissue, and may allow easier comparisons to be made between the two.

The embedding medium may comprise any suitable material, for example, a material which is used to embed tissues or samples in immunohistochemistry, such as paraffin.

Preferably, the embedding medium is inert. More preferably, the embedding medium is transparent to radiation, preferably transparent to visible light.

While paraffin is the most commonly used embedding medium, any other type of suitable embedding medium may be used. Included are materials such as Araldite M, Dammar Resin, Divinylbenzene,, Durcupan (Fluka), Epoxy Embedding Medium, Ethylene glycol dimethacrylate, Glycol methacrylate, Histocryl embedding resin, Lowicryl HM20, Butyl methacrylate, Hydroxypropyl methacrylate, Methyl methacrylate, Paraffin wax, Paraplast. Embedding media may be formed from polymerisation of monomers such as methacrylic acid monomer and styrene monomer. Further details of forming embedding media through polymerisation are provided in the section headed "Polymers" below.

The embedding medium may comprise ice, i.e., a frozen section comprising frozen water in which the tissue, etc is embedded. Therefore, in certain embodiments, the compact particle comprising the detectable entity coupled or chemically coupled thereto is supported in the same embedding medium as the sample tissue, cell, organ itself. Such embodiments are advantageous because only a single cut section needs to be handled, instead of one for the reference standard and one for the sample.

Such embodiments of the reference standards may be made achieved by embedding a compact particle with a detectable entity coupled or chemically coupled thereto as described below in the same embedding medium as the sample. It will be appreciated that the compact particle may be embedded in the embedding medium at the same time, before, or after, the sample is embedded in that medium. In other words, the compact particle with the detectable entity may be embedded in the embedding medium at any time relative to the embedding of the sample in the embedding medium. Preferably, the compact particle with the detectable entity is embedded as part of the paraffin embedding process in FFPE.

## SECTIONS AND MOUNTING

The reference standard may be provided in a single piece, and used without further processing. Preferably, however, the reference standard may be cut into slices or sections, and these sections or slices being used as standards themselves. As illustrated in Figures 4B, 4C, 5A, 5B, 8F, 9 and 10, the slices or sections comprise defined areas comprising the detectable entity, and multiple slices or sections may be made from a reference standard.

It will be appreciated that the defined areas in the slices or sections which comprise the detectable entity have a compact shape and are themselves supported by the supporting medium, the slices or sections may themselves preferably be treated as "reference standards" as described in this document.

The slices or sections may be taken using any suitable means (for example, a microtome such as a bench microtome or a rocking microtome). The thickness of the slices or sections are typically those of standard microtome slices. Preferably, the slices or sections are between about 0.5 to 300 $\mu$m, preferably between 5 to 200 $\mu$m, preferably between about 10 to 100 $\mu$m, preferably between about 1 to 100 $\mu$m, preferably between about 20 to 30 $\mu$m, most preferably between about 2 to 10 $\mu$m thick. In a highly preferred embodiment, the slices or sections are 5 $\mu$m thick or thereabouts.

Multiple sections or slices of the reference standard may be taken in a similar manner as for any FFPE sample. The resulting section or slice of the reference standard may be treated in the same way as any other fixed and embedded tissue or cell sample.

The slice or section of the reference standard may be mounted on a suitable support to aid handling. Such a support may suitably comprise a slide, such as a microscope slide, made of glass or other material. The section or slice of the reference section may be mounted in a similar manner to a section of a FFPE embedded material. Such mounting techniques are known in the art. The section or slice may be mounted in a temporary, permanent or semi-permanent manner, and may in particular be fixed to the support, by adhesive or surface tension for example.

The slice or section may also be placed on a liner, which is typically a thin planar piece of material able to support the slice or section. The slice or section may be shipped or sold with the liner, either one slice or section on a single piece of liner, or a plurality of slices or sections. For example, such a liner may be made of paper, cardboard, plastic, cellulose acetate, etc. The surface of the liner may be treated to prevent adhesion of the slice or section, for example, by silicone. A preferred embodiment of such a liner therefore comprises siliconized paper. Use of such a liner enables the slice or section to be easily mounted on a microscope slide, preferably next to the sample from the patient.

The methods for attaching or mounting sections to slides include using clean slides and relying on the capillary attraction and no adhesives. Other techniques include glues like egg-white glycerine, glycerine-gelatine mixtures, polyvinyl acetate glue, chrome-alum gelatine and poly lysine coating. Heating or "burning" of the section as a means of facilitating mounting of the section should be used with caution, as the tissue can be destroyed.

The support may comprise indicia to aid the quantitation of the detectable entity. Such indicia may preferably be located in the vicinity of the area comprising the detectable entity. The indicia may relate to quantity of detectable entity, or the grade assigned to that quantity or the nature of detectable entity

The amount of staining in the slice or section may then be compared to that in the sample to provide an assessment of the significance of the level of staining of the latter. It will be appreciated that although it is preferred that the slice or section be taken of the reference standard be subject to the processing procedures, it may be desirable in some embodiments for the reference standard itself to be taken through processing.

**DETECTION AND VISUALISATION**

The reference standard may comprise the detectable entity in an already visualisable state; in other words a detectable entity in a form which does not need to be processed further to identify its presence or quantity. The detectable label may therefore comprise a label such as a fluorescent or radioactive label, or otherwise tagged with an agent which is capable of emitting radiation. The label preferably emits light; most preferably, the light is emitted as a result of fluorescence. The detectable entity may be detected by detecting signal emission (or a change in signal emission) by the detectable label, and the detection may further comprise exciting the detectable label and monitoring fluorescence emission. The amount of the signal emitted may be measured to indicate the amount of detectable entity present.

In preferred embodiments, however, the reference standard comprises a detectable entity which is not substantially modified from its native form, for example, unlabelled. In such embodiments, the presence and/or quantity of the detectable entity is only revealed on further processing of the reference standard, for example, by application of the steps conventionally taken to reveal a detectable entity in a biological sample.

Thus, application of a primary revealing means such as a binding agent which binds to the detectable entity, preferably specifically, may be used. The detectable entity, the binding agent, or the combination of the two may be further detected by the use of secondary revealing means. Where the binding agent comprises an antibody and the detectable entity comprises an antigen, the antibody, antigen, or the antigen-antibody complex, may be revealed by a secondary antibody. The secondary antibody may be conjugated to an enzyme, which is capable of producing a signal when reacted to a chromogenic substrate. It will be appreciated that any step or series of steps which may be used to reveal, detect or quantify a detectable entity in a biological sample may be applied to the reference standard or its sections. Preferably, such step(s) are those which are conventionally employed in immunohistochemistry, for example, detection steps to detect the entity in FFPE sections.

The section or slice of the reference standard may in particular be subjected to any one or more of the procedures used to stain a FFPE embedded sample or section thereof. Thus, the slice or section of the reference standard is intended to be, and may be subjected to any one or more of the typical post embedding procedures used to stain tissue samples, in order to reveal the antigen. In preferred embodiments, the section or slice is subjected to all or substantially all of such procedures. Preferably, therefore, the section or slice is subjected to any one or more, preferably all, of the following:

mounting onto a slide, baking, deparaffination, rehydration, antigen retrieval, blocking, exposure to antibody, exposure to primary antibody, washing, exposure to secondary antibody-enzyme conjugate, exposure to enzyme substrate, exposure to chromogen substrate, and counter staining.

Baking refers to gently heating the slide with the paraffin slice on it. The paraffin partly melts away and the tissue sticks to the glass surface.

In preferred embodiments, the detectable entity is exposed to an antibody, and binding of antibody visualised in any of several ways. For a general introduction to different immunocytochemistry visualization techniques, see for example Lars-Inge Larsson "Immunocytochemistry: Theory and Practice", CRC Press inc., Baca Raton, Florida, 1988, ISBN 0-8493-6078-1, and John D. Pound (ed); "Immunochemical Protocols, vol 80", in the series: "Methods in Molecular Biology", Humana Press, Totowa, New Jersey, 1998, ISBN 0-89603-493-3.

The most commonly used detection methods in immunohistochemistry are direct visualisation of fluorescence or gold particles and enzyme mediated colorimetric detection.

For direct fluorescent studies, the labels can for example be 5-(and 6)-carboxyfluorescein, 5- or 6-carboxyfluorescein, 6-(fluorescein)-5-(and 6)-carboxamido hexanoic acid, fluorescein isothiocyanate (FITC), rhodamine, tetramethylrhodamine, and dyes such as Cy2, Cy3, and Cy5, optionally substituted coumarin including AMCA, PerCP, phycobiliproteins including R-phycoerythrin (RPE) and allophycoerythrin (APC), Texas Red, Princeton Red, Green fluorescent protein (GFP) and analogues thereof, and conjugates of R-phycoerythrin or allophycoerythrin and for example Cy5 or Texas Red, and and inorganic fluorescent labels based on semiconductor nanocrystals (like quantum dot and Qdot™ nanocrystals), and time-resolved fluorescent labels based on lanthanides like Eu3+ and Sm3+.

Colloidal gold or silver can be used as direct labels for immunocytochemical studies for electron microscopy and light microscopy. Amplification of the signal can be obtained by further silver enhancement of the colloidal gold particles.

The general enzymatic methods use labelled avidin or streptavidin-biotin (LAB or LSAB), avidin or streptavidin-biotin complex (ABC), enzyme anti-enzyme complex (PAP and APAAP), direct dextran polymer based antibody-enzyme complex (EPOS, DakoCytomation); indirect dextran polymer based antibody-enzyme complex (EnVision, DakoCytomation) or double bridge enzyme anti-enzyme complex.

The enzymatic staining uses enzymatic labels such as horse radish peroxidase (HRP), alkaline phosphatase (AP), beta-galactosidase (GAL), glucose-6-phosphate dehydrogenase, beta-N-acetylglucosaminidase, invertase, Xanthine Oxidase, firefly luciferase and glucose oxidase (GO).

Examples of commonly used substrates for horse radish peroxidase include 3,3'-diaminobenzidine (DAB), diaminobenzidine with nickel enhancement, 3-amino-9-ethylcarbazole (AEC), Benzidine dihydrochloride (BDHC), Hanker-Yates reagent (HYR), Indophane blue (IB), tetramethylbenzidine (TMB), 4-chloro-1-naphtol (CN), □-naphtol pyronin (□-NP), o-dianisidine (OD), 5-bromo-4-chloro-3-indolylphosphate (BCIP), Nitro blue tetrazolium (NBT), 2-(p-iodophenyl)-3-p-nitrophenyl-5-phenyl tetrazolium chloride (INT), tetranitro blue tetrazolium (TNBT), 5-bromo-4-chloro-3-indoxyl-beta-D-galactoside/ferro-ferricyanide (BCIG/FF).

Examples of commonly used substrates for Alkaline Phosphatase include Naphthol-AS-B1-phosphate/fast red TR (NABP/FR), Naphthol-AS-MX-phosphate/fast red TR (NAMP/FR), Naphthol-AS-B1-phosphate/fast red TR (NABP/FR), Naphthol-AS-MX-phosphate/fast red TR (NAMP/FR), Naphthol-AS-B1-phosphate/new fuschin (NABP/NF), bromochloroindolyl phosphate/nitroblue tetrazolium (BCIP/NBT), 5-Bromo-4-chloro-3-indolyl-b-d-galactopyranoside (BCIG).

One of the most potent detection systems is the catalysed reporter deposition (CARD); this amplification method is based on the deposition of labelled tyramide on tissue through the enzymatic action of HRP. After HRP-immunostaining, labelled tyramide is applied and bound near the site of HRP-activity. The bound and labelled tyramide is then visualised by traditional fluorescence or colorimetric enzyme mediated detection.

The labelling compounds mentioned above can in general be applied to both probes and antibodies (or any other substance used to detect a desired target).

The method of viewing the stained specimens includes bright field microscopes or scanners, fluorescent microscopes or scanners, transmission electron microscope (TEM) or scanning electron microscope (SEM).

Automated staining systems have been introduced to reduce cost, increase uniformity of slide preparation, reduce laborious routine work and most significantly reduce procedural human errors.

The current automated systems can handle any immunochemical assay including assays relying on immunofluorescence, indirect immunoassay procedures, enzyme or gold staining methods. They perform all steps of the immunohistochemical assay irrespective of complexity or their order, at the prescribed time and temperature.

Immunocytochemistry techniques have traditionally used specific antibodies for identification and visualisation of specific antigens. The technique is complex, many steps and molecules with high affinities for specific staining are needed.

"Special stains", described in a separate section below may also be used, either alone or in combination with immunohistochemical visualisation.

**STAINING AND IMMUNOSTAINING**

In the following, some of the individual steps in a staining procedure are described. Each of, some of, or all of these steps may be applied to the reference standard, or a slice or section thereof.

Fixatives are needed to preserve cells and tissues in a reproducible and life-like manner. To achieve this, tissue blocks, sections, or smears are immersed in a fixative fluid, or in the case of smears, are dried. Fixatives stabilise cells and tissues thereby protecting them from the rigors of processing and staining techniques.

Any suitable fixing agent may be used, for example, ethanol, acetic acid, picric acid, 2-Propanol, 3,3'-Diaminobenzidine tetrahydrochloride Dihydrate, Acetoin (mixture of monomer) and dimer, Acrolein, Crotonaldehyde( (cis+trans), Formaldehyde, Glutaraldehyde, Glyoxal, Potassium dichromate, Potassium permanganate, Osmium tetroxide, Paraformaldehyde, Mercuric chloride, Tolylene-2,4-diisocyanate, Trichloroacetic acid, Tungstic acid. Preferred types of fixative include formalin (aqueous formaldehyde) and neutral buffered formalin (NBF) is among the most commonly used. Other preferred fixatives include glutaraldehyde, acrolein, carbodiimide, imidates, benzoequinone, osmic acid and osmium tetraoxide.

Fresh biopsy specimens, cytological preparations (including touch preparations and blood smears), frozen sections and tissues for immunohistochemical analysis are commonly fixed in organic solvents, including ethanol, acetic acid, methanol and/or acetone.

**ANTIBODIES**

In preferred embodiments, an antibody capable of binding to the detectable entity is employed to reveal its presence.

Antibodies comprise immunoglobulin molecules. Immunoglobulin molecules are in the broadest sense members of the immunoglobulin superfamily, a family of polypeptides comprising the immunoglobulin fold characteristic of antibody molecules, which contains two $\beta$ sheets and, usually, a conserved disulphide bond. Members of the immunoglobulin superfamily are involved in many aspects of cellular and non-cellular interactions *in vivo*, including widespread roles in the immune system (for example, antibodies, T-cell receptor molecules and the like), involvement in cell adhesion (for example the ICAM molecules) and intracellular signalling (for example, receptor molecules, such as the PDGF receptor). The methods described here of detecting detectable entities and of using the reference standard may therefore make use of any immunoglobulin superfamily molecule which is capable of binding to a target. Peptides or fragments derived from immunoglobulins may also be used.

Antibodies, as used herein, refers to complete antibodies or antibody fragments capable of binding to a selected target, and including Fv, ScFv, F(ab') and F(ab')$_2$, monoclonal and polyclonal antibodies, engineered antibodies including chimeric, CDR-grafted and humanised antibodies, and artificially selected antibodies produced using phage display or alternative techniques. Small fragments, such as Fv and ScFv, possess advantageous properties for diagnostic and therapeutic applications on account of their small size and consequent superior tissue distribution. Preferably, the antibody is a single chain antibody or ScFv.

The antibodies may be altered antibodies comprising an effector protein such as a toxin or a label. Use of labelled antibodies allows the imaging of the distribution of the antibody *in vivo*. Such labels may be radioactive labels or radioopaque labels, such as metal particles, which are readily visualisable within the body of a patient. Moreover, they may be fluorescent labels (such as the ones described here) or other labels which are visualisable on tissue samples removed from patients. Antibodies with effector groups may be linked to any association means as described above.

Antibodies may be obtained from animal serum, or, in the case of monoclonal antibodies or fragments thereof, produced in cell culture. Recombinant DNA technology may be used to produce the antibodies according to established procedure, in bacterial, yeast, insect or preferably mammalian cell culture. The selected cell culture system preferably secretes the antibody product.

Growing of hybridoma cells or mammalian host cells in vitro is carried out in suitable culture media, which are the customary standard culture media, for example Dulbecco's Modified Eagle Medium (DMEM) or RPMI 1640 medium, optionally replenished by a mammalian serum, for example foetal calf serum, or trace elements and growth sustaining supplements, for example feeder cells such as normal mouse peritoneal exudate cells, spleen cells, bone marrow macrophages, 2-aminoethanol, insulin, transferrin, low density lipoprotein, oleic acid, or the like. Multiplication of host cells which are bacterial cells or yeast cells is likewise carried out in suitable culture media known in the art, for example for bacteria in medium LB, NZCYM, NZYM, NZM, Terrific Broth, SOB, SOC, 2 x YT, or M9 Minimal Medium, and for yeast in medium YPD, YEPD, Minimal Medium, or Complete Minimal Dropout Medium.

Use of insect cells as hosts for the expression of proteins has advantages in that the cloning and expression process is relatively easy and quick. In addition, there is a high probability of obtaining a correctly folded and biologically active protein when compared to bacterial or yeast expression. Insect cells may be cultured in serum free medium, which is cheaper and safer compared to serum containing medium. Recombinant baculovirus may be used as an expression vector, and the construct used to transfect a host cell line, which may be any of a number of lepidopteran cell lines, in particular *Spodoptera frugiperda Sf9*, as known in the art. Reviews of expression of recombinant proteins in insect host cells are provided by Altmann et al. (1999), Glycoconj J 1999, 16, 109-23 and Kost and Condreay (1999), Curr Opin Biotechnol, 10, 428-33.

*In vitro* production provides relatively pure antibody preparations and allows scale-up to give large amounts of the desired antibodies. Techniques for bacterial cell, yeast, insect and mammalian cell cultivation are known in the art and include homogeneous suspension culture, for example in an airlift reactor or in a continuous stirrer reactor, or immobilised or entrapped cell culture, for example in hollow fibres, microcapsules, on agarose microbeads or ceramic cartridges.

Large quantities of the desired antibodies can also be obtained by multiplying mammalian cells *in vivo.* For this purpose, hybridoma cells producing the desired antibodies are injected into histocompatible mammals to cause growth of antibody-producing tumours. Optionally, the animals are primed with a hydrocarbon, especially mineral oils such as pristane (tetramethyl-pentadecane), prior to the injection. After one to three weeks, the antibodies are isolated from the body fluids of those mammals. For example, hybridoma cells obtained by fusion of suitable myeloma cells with antibody-producing spleen cells from Balb/c mice, or transfected cells derived from hybridoma cell line Sp2/0 that produce the desired antibodies are injected intraperitoneally into Balb/c mice optionally pre-treated with pristane, and, after one to two weeks, ascitic fluid is taken from the animals.

The foregoing, and other, techniques are discussed in, for example, Kohler and Milstein, (1975) Nature 256: 495-497; US 4,376,110; Harlow and Lane, Antibodies: a Laboratory Manual, (1988) Cold Spring Harbor, incorporated herein by reference. Techniques for the preparation of recombinant antibody molecules is described in the above references and also in, for example, EP 0623679; EP 0368684 and EP 0436597.

The cell culture supernatants are screened for the desired antibodies, preferentially by immunofluorescent staining of cells expressing the desired target by immunoblotting, by an enzyme immunoassay, for example a sandwich assay or a dot-assay, or a radioimmunoassay.

For isolation of the antibodies, the immunoglobulins in the culture supernatants or in the ascitic fluid may be concentrated, for example by precipitation with ammonium sulphate, dialysis against hygroscopic material such as polyethylene glycol, filtration through selective membranes, or the like. If necessary and/or desired, the antibodies are purified by the customary chromatography methods, for example gel filtration, ionexchange chromatography, chromatography over DEAE-cellulose and/or immunoaffinity chromatography, for example affinity chromatography with the a protein containing a target or with Protein-A.

Antibodies generated according to the foregoing procedures may be cloned by isolation of nucleic acid from cells, according to standard procedures. Usefully, nucleic acids variable domains of the antibodies may be isolated and used to construct antibody fragments, such as scFv.

The methods described here preferably employ recombinant nucleic acids comprising an insert coding for a heavy chain variable domain and/or for a light chain variable domain of antibodies. By definition such nucleic acids comprise coding single stranded nucleic acids, double stranded nucleic acids consisting of the coding nucleic acids and of complementary nucleic acids thereto, or these complementary (single stranded) nucleic acids themselves.

Furthermore, nucleic acids encoding a heavy chain variable domain and/or for a light chain variable domain of antibodies can be enzymatically or chemically synthesised nucleic acids having the authentic sequence coding for a naturally-occurring heavy chain variable domain and/or for the light chain variable domain, or a mutant thereof. A mutant of the authentic sequence is a nucleic acid encoding a heavy chain variable domain and/or a light chain variable domain of the above-mentioned antibodies in which one or more amino acids are deleted or exchanged with one or more other amino acids. Preferably the modification(s) are outside the complementary determining regions (CDRs) of the heavy chain variable domain and/or of the light chain variable domain of the antibody. Such a mutant nucleic acid is also intended to be a silent mutant wherein one or more nucleotides are replaced by other nucleotides with the new codons coding for the same amino acid(s). Such a mutant sequence is also a degenerated sequence. Degenerated sequences are degenerated within the meaning of the genetic code in that an unlimited number of nucleotides are replaced by other nucleotides without resulting in a change of the amino acid sequence originally encoded. Such degenerated sequences may be useful due to their different restriction sites and/or frequency of particular codons which are preferred by the specific host, particularly yeast, bacterial or mammalian cells, to obtain an optimal expression of the heavy chain variable domain and/or a light chain variable domain.

The term mutant is intended to include a DNA mutant obtained by *in vitro* or *in vivo* mutagenesis of DNA according to methods known in the art.

Recombinant DNA technology may be used to improve antibodies. Thus, chimeric antibodies may be constructed in order to decrease the immunogenicity thereof in diagnostic or therapeutic applications. Moreover, immunogenicity may be minimised by humanising the antibodies by CDR grafting [European Patent 0 239 400 (Winter)] and, optionally, framework modification [European Patent 0239400; Riechmann et al., (1988) Nature 322:323-327; and as reviewed in international patent application WO 90/07861 (Protein Design Labs)].

Recombinant nucleic acids may be employed comprising an insert coding for a heavy chain variable domain of an antibody fused to a human constant domain γ, for example γ1, γ2, γ3 or γ4, preferably γ1 or γ4. Likewise recombinant DNAs comprising an insert coding for a light chain variable domain of an antibody fused to a human constant domain κ or λ, preferably κ may also be used.

More preferably, CDR-grafted antibodies, which are preferably CDR-grafted light chain and heavy chain variable domains only, may be used. Advantageously, the heavy chain variable domain and the light chain variable domain are linked by way of a spacer group, optionally comprising a signal sequence facilitating the processing of the antibody in the host cell and/or a DNA coding for a peptide facilitating the purification of the antibody and/or a cleavage site and/or a peptide spacer and/or an effector molecule. Such antibodies are known as ScFvs.

Antibodies may moreover be generated by mutagenesis of antibody genes to produce artificial repertoires of antibodies. This technique allows the preparation of antibody libraries, as discussed further below; antibody libraries are also available commercially. Hence, artificial repertoires of immunoglobulins, preferably artificial ScFv repertoires, are used as an immunoglobulin source.

Isolated or cloned antibodies may be linked to other molecules, for example nucleic acid or protein association means by chemical coupling, using protocols known in the art (for example, Harlow and Lane, Antibodies: a Laboratory Manual, (1988) Cold Spring Harbor, and Maniatis, T., Fritsch, E. F. and Sambrook, J. (1991), Molecular Cloning: A Laboratory Manual. Cold Spring Harbor, New York, Cold Spring Harbor Laboratory Press).

## NUCLEIC ACID PROBES

As noted above, the detectable entity may comprise a nucleic acid, and such reference standard embodiments comprising nucleic acid detectable entities are suitably used as standards in *in situ* hybridisation. In such embodiments, a nucleic acid probe capable of binding to the nucleic acid detectable entity is employed to reveal its presence.

The nucleic acid probe in particular preferably comprises at least a sequence that is capable of hybridising to a sequence in the detectable entity. In particular, it may comprise at least a sequence that is complementary to such a sequence. The nucleic acid probe is preferably single stranded, and may comprise in particular single stranded DNA or single stranded RNA.

The term "hybridisation" as used in this document refers to "the process by which a strand of nucleic acid joins with a complementary strand through base pairing". Preferably, the nucleic acid probe includes sequences that are capable of hybridising under stringent conditions (for example 50°C and 0.2x SSC {1x SSC = 0.15 M NaCl, 0.015 M Na3citrate pH 7.0}) to at least a nucleotide sequence in the detectable entity. More preferably, the nucleic acid probe includes sequences that are capable of hybridising under high stringent conditions (for example 65°C and 0.1 x SSC {1x SSC = 0.15 M NaCl, 0.015 M $Na_3$citrate pH 7.0}).

Nucleic acid probes capable of selectively hybridising to a nucleotide sequence in the detectable entity, or to their complement, will be generally at least 75 %, preferably at least 85 or 90 % and more preferably at least 95 % or 98 % homologous to the corresponding complementary nucleotide sequence in the detectable entity over a region of at least 20, preferably at least 25 or 30, for instance at least 40, 60 or 100 or more contiguous nucleotides. Preferred probes comprise at least one region preferably at least 80 or 90 % and more preferably at least 95 % homologous to the nucleotide sequence in the detectable entity

The term "selectively hybridizable" means that the nucleic acid probe is capable of hybridising to the target nucleic acid sequence at a level significantly above background. The background hybridization may occur because of other nucleotide sequences present, for example, in the sample being processed for ISH. In this event, background implies a level of signal generated by interaction between the probe and a non-specific DNA member of the library which is less than 10 fold, preferably less than 100 fold as intense as the specific interaction observed with the target DNA. The intensity of interaction may be measured, for example, by radiolabelling the probe, for example with [32]P.

Hybridization conditions are based on the melting temperature (Tm) of the nucleic acid binding complex, as taught in Berger and Kimmel (1987, Guide to Molecular Cloning Techniques, Methods in Enzymology, Vol 152, Academic Press, San Diego CA), and confer a defined "stringency" as explained below.

Maximum stringency typically occurs at about Tm-5°C (5°C below the Tm of the probe); high stringency at about 5°C to 10°C below Tm; intermediate stringency at about 10°C to 20°C below Tm; and low stringency at about 20°C to 25°C below Tm. As will be understood by those of skill in the art, a maximum stringency hybridization can be used to identify probes comprising identical nucleotide sequences while an intermediate (or low) stringency hybridization can be used to identify probes comprising similar or related polynucleotide sequences.

Nucleotide sequences which are not 100 % homologous to a sequence in the detectable entity but which may be used as probes can be obtained in a number of ways. Conserved sequences can be predicted, for example, by aligning the amino acid sequences from several variants/homologues. Sequence alignments can be performed using computer software known in the art. For example the GCG Wisconsin PileUp program is widely used.

The probes may be produced recombinantly, synthetically, or by any means available to those of skill in the art. In general, probes will be produced by synthetic means, involving a step-wise manufacture of the desired nucleic acid sequence one nucleotide at a time. Techniques for accomplishing this using automated techniques are readily available in the art.

Longer nucleotide sequences for use as nucleic acid probes will generally be produced using recombinant means, for example using a PCR (polymerase chain reaction) cloning techniques. This will involve making a pair of primers (for example of about 15 to 30 nucleotides) flanking a region of the targeting sequence which it is desired to clone, bringing the primers into contact with mRNA or cDNA obtained from an animal or human cell, performing a polymerase chain reaction (PCR) under conditions which bring about amplification of the desired region, isolating the amplified fragment (for example by purifying the reaction mixture on an agarose gel) and recovering the amplified DNA. The primers may be designed to contain suitable restriction enzyme recognition sites so that the amplified DNA can be cloned into a suitable cloning vector.

The nucleic acid probes may be labelled by various means, as known in the art. For example, the probe may be labelled using radioactive labels such as $^{31}$P, $^{33}$P or $^{32}$S, or non-radioactively, using labels such as digoxigenin, or fluorescent labels, a great many of which are known in the art.

## STAINS AND DYES

Although antibodies are preferred as binding agents for use in revealing the detectable entity, other suitable stains or dyes may be used to reveal the target. For example, dyes typically used to colour fabrics may be used to stain the target or detectable entity. Such dyes will typically bind in a stoichiometric fashion, such that the more detectable entity is present, the more dye is bound. However, simple staining to reveal presence and/or location may often provide enough information.

*"Special " Stains and Dyes*

It should be understood, therefore, that the reference standard described here may also be stained not only using immunological recognition using for example antibodies, but also by means of chemical, which we refer to as "special stains".

The most common used are the general Haematoxylin-Eosin (H & E) staining, the Gomori methenamine silver stain (GMS) useful for identifying for example carbohydrates from fungi and the Periodic Acid-Schiff (PAS) stain useful for identifying for example glycogen, acid and neutral mucosubstances, fungal cell wall, basement membranes, collagen fibres and reticular fibres.

There are numerous special stains formulations, variations and combinations, including Au-chloride, Trichrome Blue, Masson's Trichrome, Prussian Blue, Giemsa, Diff-Quik, Reticulum, Congo Red, Alcian Blue, Steiner, AFB, PAP, Gram, Mucicarmine, Verhoeff-van Gieson, Elastic, Carbol Fuchsin and Golgi's stains.

It should also be understood, that some of the above-mentioned special stains will specifically stain target probes like for example some carbohydrates or hydrophobic moities, which are easily introduced into the reference standard described here. Furthermore, any combination of one or more immunological stains and one or more special staining may also be carried out.

For example, suitable stains and dyes may include any of the following: 1,4-Phenylenediamine, 2,3,5-Triphenyltetrazolium chloride, 2,4-Dinitro-5-fluoroaniline, 2-Naphthol (beta), 3,3'-Diaminobenzidine, 4-Chloro-1-naphthol, 4-Chloro-1-naphthol, Acridine Orange, Acridine Orange hydrochloride Hydrate, Silver proteinate, Alcian Blue 8GX, Alizarin, Alizarin Red S, Alkali Blue 4 B, Ammonium molybdate Tetrahydrate, Aniline hydrochloride, Auramine O, Azocarmine B, Azocarmine G, Azophloxine, Azure A, Azure B, Azure II, Azure II-Eosin, Azure Mixture sicc. Giemsa Stain, Bengal Rose B, Benzopurpurine 4B, Prussian Blue soluble, Prussian Blue insoluble, Bismarck Brown Y (G), Bismarck Brown R, Bismuth(III) nitrate basic, Lead(II) acetate Trihydrate, Lead(II) citrate TrihydrateLead(II) nitrate, Lead(II) nitrate, Lead(II) tartrate, Lead tetraacetate, Borax Carmin Solution ((acc. to Grenacher), Brilliant Green, Brilliant Cresyl Blue, 'Brilliant Cresyl Blue', Brilliant Cresyl Blue Solution, Bromocresol Green and complexometry, Bromocresol Green Sodium salt, Bromocresol Purple, Bromophenol Blue, Bromosulfalein, Bromothymol Blue Sodium salt, Carbol-Fuchsin Dye Powder, Carbol-Fuchsin Solution (according to Kinyoun), Carbol-Fuchsin Solution (according to Ziehl-Neelsen), Carbol-Gentianaviolet Solution, Carbol-Methylene Blue Solution, Carmine, Carminic acid, Celestine Blue, Quinacrine Mustard dihydrochloride, Quinoline Yellow, Chlorazol Black, Chromium(VI) oxide, Chromium(VI) oxide, Chromotrop 2 R and complexometry, Chrysoidine G, Cobaltous chloride anhydrous, Cobalt naphthenate, ~10% Co, Cyanosine, Cytochrome c from horse heart lyophil.salt-free powder, Cytochrome c from horse heart, Cytochrome c from horse heart, Cytochrome c from bovine heart, Cytochrome c from pigeon breast muscle, Differential Stain Solution, Direct Red, Direct Red 80, Fast Blue B Salt, Fast Blue BB Salt, Fast Blue RR Salt, Fast Green FCF, Fast Red 3 GL Salt, Fast Red RC Salt, Fast Violet B Salt, Eosin alcohol soluble, Eosin Yellowish, Eosin Yellowish Solution, Eosin-Hematoxylin Solution ((acc. to Ehr-lich), Eosin Methylene Blue (acc. to Leishman), Eosin Methylene Blue (acc. to Wright), Eosin Methylene Blue, Eosin Methylene Blue Solution (acc. ToWright), Eosin Methylene Blue Solution (acc. to Wright-Lillie), Eosin Scarlet, Eriochrome Red B, Erythrosin extra Bluish, Acetic acid Solution, Ethyl Violet, Evans Blue Fluka, Dye Sol. (acc. to Boroviczeny A: Toluidine Blue-Safranine fix), Dye Sol. (acc. to Boroviczeny B: EosineSolution), Ferritin from horse spleen, Fat Red Bluish, Fat Black, Fluorescein isothiocyanate, Fuchsin, Fuchsin Solution, Gallocyanine, Gentian Violet, Giemsa-Solution, Gold chloride Hydrate (~52% Au), Gold chloride Hydrate (ACS, >49% Au), Gold Solution, colloidal, Hemalaun, Hematein, Hematoxylin, Hematoxylin Solution A (acc. to Weigert), Hematoxylin Solution B (acc. to Weigert, Hematoxylin Solution (acc. to Boehmer), Hematoxylin Solution, (acc. to Delafield), Hematoxylin Solution (acc. to Mayer), Hanker-Yates Reagent, Hayem's Solution, Hesperidin, Indigocarmine, Indium(III) chloride Hydrate, Indium(III) chloride anhydrous, Iodonitrotetrazolium chloride, iso-Chloridazon solution, Janus GreenB, Potassium dichromate, Potassium hexahydroxoantimonate (V), Potassium permanganate, Potassium permanganate (Hg <0.000005%, ACS), Carmine Solution ammoniacal (acc. to Best), Carmine Solution acidic (acc. to Mayer), Nuclear Fast Red, Congo Red,indicator, Cresol Red, Cresyl Violet acetate, Crystal Violet indicator, Crystal Violet Solution, Lactophenol Blue Solution, Lanthanum nitrate Hexahydrate, Light Green SF Yellowish, Lipid Crimson, Lithium carbonate, Lugol Solution, Malachite green oxalate, May-Grünwald Solution, Metanil Yellow, Methylene Blue Zinc chloride Double salt, Methylene Blue, Methylene Blue Concentrate (acc. to Ehr-lich), Methylene Blue Solution alkaline (acc. to Löffler), Methylene green zinc double salt, Methyl Green, Methyl Orange, Methyl Violet, Morin, Mucicarmine, N-(4-Amino-2,5-diethoxyphenyl)benzamide, N,N-Dimethylaniline, N,N-Dimethyl-p-toluidine, Naphthol AS-acetate, Naphthol AS-BI-beta-D-glucuronide, (the following Naphthol derivatives are enzyme (AP) substrates), Naphthol AS-BI-phosphate Disodium salt Heptahydrate, Naphthol AS-BI-phosphate, Naphthol AS-BI-phosphate, Naphthol AS-BS-phosphate, Naphthol AS-chloroacetate, Naphthol AS-D-acetate, Naphthol AS-D-chloroacetate, Naphthol AS-E-acetate, Naphthol AS-E-phosphate, Naphthol AS-MX-acetate, Naphthol AS-MX-phosphate Disodium salt Nonahydrate, Naphthol AS-MX-phosphate, for histology, Naphthol AS-phosphate, for histology, Naphthol AS-TR-phosphate, Naphthol AS-TR-phosphate, Naphthol Blue Black, Naphthol Yellow S, Naphthol Green B and complexometry, Sodium tungstate Dihydrate, Oil of cloves, Neotetrazolium chloride, New Coccine, New Fuchsine, New Methylene Blue N, Neutral Red, Nigrosin B alcohol soluble, Nigrosin watersoluble, Nile Blue A, Nile Blue chloride, Ninhydrin, Ninhydrin, Nitrazine Yellow, Nitrotetrazolium Blue chloride, Nitrotetrazolium Blue chloride, Nitrotetrazolium Blue chlor, Orange G, Orange G Solution (alcoholic), Orcein, Orcein, Palladium(II) chloride anhydrous, Palladium(II) oxide Hydrate, Parafuchsin, Parafuchsin hydrochloride, Peroxidase from horse radish (lyoph.powd.salt-free ~100 U/mg), Phenosafranine, Phosphomolybdic acid Ammonium salt Hydrate, Phosphomolybdicacid Hydrate, Phosphomolybdic acid Sodium salt Hydrate, Phosphorus pentoxide, Phosphotungstic acid Hydrate, Phthalocyanine, Picric acid moistened with water (H2O ~40%), Pinacyanol iodide, Platinum(IV) oxide Hydrate, Ponceau BS, Ponceau S, Pyridine, Pyronine Y (G), Resorufin, Rhodamine B, Ruthenium(III) chloride anhydrous, Ruthenium Red, Safranin T, Safranin Solution (acc. to Olt), Acid Fuchsin Calcium salt, Acid Fuchsin indicator, Scarlet R, Schiff's Reagent for Aldehydes, Silver, Codex France, colloidal, Silver nitrate, Sirius Rose BB, 'Stains-all', Sudan Blue II, Sudan Orange G, Sudan Red B, Sudan Black B, Sulforhodamine B acid chloride, Tartrazine, Tetranitroblue tetrazolium chloride, Tetrazolium Blue chloride, Tetrazolium Violet, Thallium(I) nitrate, Thiazole Yellow G, adsorption indicator, Thiazolyl Blue Tetrazolium bromide, Thiocarbohydrazide, Thioflavine T, Thionine acetate, Toluidine Blue, Tropaeolin 000 No. 1, Tropaeolin 000 No. 2, Trypan Blue, Trypan BlueSolution, 0.4%, Tuerk Solution, Uranyl acetate Dihydrate, Uranyl nitrate Hexahydrate, Variamine Blue B salt, Vesuvine Solution (acc. to Neisser), Victoria Blue B, Water Blue, Weigert's Solution, Tungstosilicic acid Hydrate, Wright Stain, Xylenecyanol FF (redox indicator).

## ANTIGEN RETRIEVAL

To facilitate the specific recognition in fixed tissue, it is often necessary to retrieve or unmask the targets through pre-treatment of the specimens to increase reactivity of the majority of targets. This procedure is referred to as "antigen retrieval", "target retrieval" or "epitope retrieval", "target unmasking" or "antigen unmasking". An extensive review of antigen retrieval (antigen unmasking) may be found in Shi S-R, Cote RJ, Taylor CR. Antigen retrieval immunocytochemistry: past, present, future. J Histochem Cytochem 1997: 45(3);327-343.

Antigen retrieval or target retrieval includes a variety of methods by which the availability of the target for interaction with a specific detection reagent is maximised. The most common techniques are enzymatic digestion with a proteolytic enzyme (for example Proteinase, pronase, pepsin, papain, trypsin or neuraminidase) in an appropriate buffer or heat induced epitope retrieval (HIER) using microwave irradiation, heating in a regular oven, autoclaving or pressure cooking in an appropriately pH stabilised buffer, usually containing EDTA, EGTA, Tris-HCl, citrate, urea, glycin-HCl or boric acid.

Detergents may be added to the HIER buffer to increase the epitope retrieval or added to the dilution media and/or rinsing buffers to lower unspecific binding.

Additionally, the signal-to-noise ratio may be increased by different physical methods, including application of vacuum and ultrasound, or freezing and thawing of the sections before or during incubation of the reagents.

Endogenous biotin binding sites or endogenous enzyme activity (for example phosphatase, catalase or peroxidase) can be removed as a step in the staining procedure.

Similarly, blocking of unspecific binding sites with inert proteins like, HSA, BSA, ovalbumine, fetal calf serum or other sera, or detergents like Tween20, Triton X-100, Saponin, Brij or Pluronics is widely used. Blocking unspecific binding sites in the tissue or cells with unlabelled and target non-specific versions of the specific reagents.

## OTHER TECHNIQUES

In staining procedures using the so-called "free floating techniques", a tissue section is brought into contact with different reagents and wash buffers in suspension or freely floating in appropriate containers, for example micro centrifuge tubes.

The tissue sections can be transferred from tube to tube with different reagents and buffers during the staining procedure using for example a "fishing hook like" device, a spatula or a glass ring.

The different reagents and buffer can also be changed by gentle decantation or vacuum suction. Alternatively, containers with the tissue sections can be emptied into a special staining net, like the Coming "Netwells" and the tissue section washed before being transferred back into the tube for the next staining step.

All the individual staining procedure steps, including for example fixation, antigen retrieval, washing, incubation with blocking reagents, immuno-specific reagents and for example the enzymatic catalysed development of the coloured stains, are done while the tissue section is floating freely or withheld on nets. After development of the stain, the tissue section is mounted on slides, dried, before being counterstained and cover slipped before being analysed in for example a microscope.

Occasionally, the tissue section is mounted on slides following the critical incubation with the immuno-specific reagents. The rest of the staining process is then conducted on the slide mounted tissue sections.

The free-floating method has been used mainly on thick tissue sections. It is important that sections never dry out during the staining process.

Advantages of the free-floating method include even and good penetration of the immunohistochemical staining reagents. The free-floating method allows for high concentrations of reagents and good mixing.

## HISTOLOGICAL MATERIALS, TECHNIQUES AND ANALYSIS

There are in general two categories of histological materials. The reference standard described here may be suitably employed for analysis of each type.

The first category includes preparations, which are fresh tissues and/or cells, which generally are not fixed with aldehyde-based fixatives. These specimens commonly include biopsy materials, which may be analysed while the surgical procedure is in progress (frozen sections), cytological preparations (including e. g. touch preparations and blood smears), and tissues, which are to be histochemically analysed. Such specimens are either placed directly on a slide or cover slip, or frozen and sectioned onto slides. Such specimens are then fixed, usually with an alcohol- or acetone-based fixative, and stained.

The more common second category includes a fixed, paraffin-embedded tissue specimen, often archive material. These are often referred to as "formalin-fixed and paraffin-embedded" (FFPE) specimens. These specimens are fixed, usually using a formalin-based fixative, dehydrated by using for example xylene, embedded in a suitable embedding medium such as paraffin or plastic (for example Epon, Araldite, Lowicryl, LR White or polyacrylamide), sectioned onto a slide, deparaffinised or otherwise treated, rehydrated, and stained.

Biological samples may be treated using the cytological and histological techniques described here, prior to being stained and compared to the reference standard.

The sample may be purified or concentrated, or cells may be isolated prior to analysis. The sample may also be embedded into paraffin and sectioned prior to analysis. Such procedures are readily known to the person skilled in the art.

The sample may be mounted on a support. By the term "mounted" is meant placed on or attached to a substantially planar support. Any suitable support may be employed. Included is placing the tissue or cell sample on a support, for example for viewing on a microscope slide. The sample can be attached to further prevent it from falling or sliding off during handling of the support. The method of attachment to the support includes relying on the physical, capillary attraction, adhesives and chemically binding. The sample may be fixed or not fixed.

In particular, the support may be a glass slide, a membrane, a filter, a polymer slide, a chamber slide, a dish, or a petridish.

The sample or parts thereof may suitably be grown or cultured directly on the support prior to analysis. Examples of suitable culture media includes culture media of biological origin such as blood serum or tissue extract; chemically defined synthetic media; or mixtures thereof. Cell cultures are usually grown either as single layers of cells on for example a glass or plastic surface, in flasks or on chamber slides, or as a suspension in a liquid or semisolid medium. The cells can be transferred to and mounted onto a more suitable support, for example a glass slide. If grown on a chamber slide, which is suitable for for example viewing in a microscope, the cells can potentially remain on the support.

However, the cells need not be grown or cultured prior to analysis. Often the sample will be analysed directly without culturing. It is to be understood that samples for direct analysis may undergo the processing procedures described above.

The sample may, either directly or after having undergone one or more processing steps, be analysed in primarily two major types of methods, *in situ* methods (*in situ* analyses) and *in vitro* methods (*in vitro* analyses).

In this context, *in situ* methods are to be understood as assays, in which the morphology of the sample cells is essentially preserved. By "essentially preserved" is meant that the overall morphology is preserved, making it possible to identify some or all of the structural compositions of the tissue or cells. Examples are analysis of smears, biopsies, touch preparations and spreading of the sample onto the support. Samples may be subjected to i.a. fixation, permeabilisation, or other processing steps prior to analysis.

*In vitro* methods are to be understood as methods, in which the overall morphology is not preserved. In the case of *in vitro* methods, the sample is subjected to a treatment, which disrupts the morphology of the cell structure. Such treatments are known to the person skilled in the art and include treatment with organic solvents, treatment with strong chaotropic reagents such as high concentrations of guanidine thiocyanate, enzyme treatment, detergent treatment, bead beating, heat treatment, sonication and/or application of a French press.

## DETAILED PROCEDURES

The following section provides a detailed description of procedures for making FFPE samples, as well as antibody staining and detection. It is taken from the reference textbook Antibodies : A Laboratory Manual by Ed Harlow (Editor), David Lane (Editor) (1988, Cold Spring Harbor Laboratory Press, ISBN 0-87969-314-2), 1855.

*Preparing Paraformaldehyde*

Standard commercial 37 % solutions of formaldehyde are stabilised with 10-15 % methanol to inhibit polymerisation of the formaldehyde to paraformaldehyde. For most fixatives, paraformaldehyde should be used in place of the commercial formaldehyde. Paraformaldehyde dissolves in water, releasing formaldehyde in the process. To prepare a 4 % solution of paraformaldehyde, add 8 g to 100 mL of water. Heat to 60°C in a fume hood. Add a few drops of 1 N NaOH to help dissolve. When the solid has completely dissolved, let the solution cool to room temperature, add 100 mL of 2x PBS. This stock solution should be prepared fresh daily.

*Preparing Paraffin Embedded Tissue Sections*

1. Cut small blocks approximately 1 cm$^2$ x 0.4 cm. 2. Place in either freshly prepared 4 % paraformaldehyde or Bouin's fixative (To prepare 1 L, dissolve 2 g of picric acid in 500 mL of deionized water. Filter through Whatman No. 1, or equivalent. Add 20 g of paraformaldehyde, and heat to 60°C in a fume hood. Add a few drops of 1 N NaOH to dissolve. Cool and add 500 mL of 2x PBS). 3. Incubate for 2 hr. to overnight. 4. Follow standard paraffin embedding procedures. 5. Collect 4 $\mu$m sections onto clean glass slides. 6. Place in 60°C oven for 30 min. 7. Dewax in xylene. Change two times, 3 min. each. 8. Rehydrate by passing through graded alcohols (two changes, absolute ethanol, 3 min. each, followed by two changes, 95 % ethanol, 3 min. each). 9. Rinse in water.

Antibodies can now be applied to the specimen, as described below.

Where peroxidase labelled detection methods are to be used, it may be necessary to block or inhibit endogenous enzyme activity within the specimen before the application of antibody. To block endogenous peroxidase activity, the specimen is incubated with a solution of 4 parts methanol to 1 part of 3 % hydrogen peroxide for 20 min. Hydrogen peroxide is generally supplied as a 30 % solution and should be stored at 4°C, at which it will last about 1 month. For specimens containing high peroxidase activities, such as spleen or bone marrow, better results may be obtained by using a solution of 0.1 % phenylhydrazine hydrochloride in Phosphate Buffered Saline (PBS).

*Fixation*

All fixation protocols must (1) prevent antigen leakage, (2) permeabilize the cell to allow access of the antibody, (3) keep the antigen in such a form that it can be recognised efficiently by the antibody, and (4) maintain the cell structure.

A wide range of fixatives are in common use, and the correct choice of method will depend on the nature of the antigen being examined and on the properties of the antibody preparation. Fixation methods fall generally into two classes, organic solvents and cross-linking reagents. Organic solvents such as alcohols and acetone remove lipids and dehydrate the cells, precipitating the proteins on the cellular architecture. Cross-linking reagents form intermolecular bridges, normally through free amino groups, thus creating a network of linked antigens. Both methods may denature protein antigens, and for this reason, antibodies prepared against denatured proteins may be more useful for cell staining. In some instances, anti-denatured protein antibodies are the only ones that can work.

*Fixing Attached Cells in Paraformaldehyde or Glutaraldehyde*

Fixation in protein cross-linking reagents such as paraformaldehyde or glutaraldehyde preserves cell structure better than organic solvents but may reduce the antigenicity of some cell components. Simple fixation with paraformaldehyde or glutaraldehyde does not allow access of the antibody to the specimen and therefore is followed by a permeabilization step using an organic solvent or non-ionic detergent. Using the organic solvent is easy, but it can destroy certain elements of the cell architecture, although the prior fixation with paraformaldehyde does help to preserve the cellular structure. If preservation of cell structure is important, the best first choice would be to use a non-ionic detergent.

1. Prior to the staining, prepare either the paraformaldehyde or glutaraldehyde solution. For paraformaldehyde, prepare a 4 % solution. For glutaraldehyde, prepare a 1 % solution (electron microscopic grade) in PBS. **Caution** Glutaraldehyde is toxic. Work in a fume hood. 2. Wash the coverslip, slide, or plate gently in PBS. 3. For paraformaldehyde, incubate in a 4 % solution for 10 min. at room temperature. For glutaraldehyde, incubate in a 1 % solution for 1 hr. at room temperature in a fume hood. 4. Wash the cells twice with PBS. At this stage, cells fixed with glutaraldehyde can be removed from the fume hood. 5. Permeabilize the fixed cells by incubating in any of the following: (i) 0.2 % Triton X-100 in PBS for 2 min. at room temperature. Some antigens may need as long as 15 min. Check this for each antigen; (ii) Methanol for 2 min. at room temperature; or (iii) Acetone for 30 sec. at room temperature (for cells grown on tissue culture plates, use 50 % acetone/50 % methanol). (**Optional**) For glutaraldehyde, block free reactive aldehyde groups by incubating with 0.2 M ethanolamine pH 7.5 for 2 hr. at room temperature or by incubating with three changes of 5 min. each with 0.5 mg/mL sodium borohydride in PBS.

In some cases this may also help paraformaldehyde-fixed cells, but in general is not necessary. 6. Rinse gently in PBS with four changes over 5 min.

The sample is now ready for the application of antibodies, as described below.

*Unmasking Hidden Epitopes with Proteases*

Fixation in formaldehyde or glutaraldehyde may mask or change some epitopes. These epitopes can often be re-exposed by a gentle incubation of the sample in proteases. Trypsin works well. Incubate the specimen in a 0.1 % trypsin, 0.1 % $CaCl_2$, 20 mM Tris pH 7.8 solution for 2-20 min. at room temperature. Stop the digestion by rinsing the specimen under the cold tap for 5 min.

*Binding Antibodies to Attached Cells*

Antibodies generally are applied directly to the area of the cells of tissues that is being studied.

1. Cells are fixed and washed. Place coverslips, slides or plates in a humidified chamber. Slides or coverslips can be placed in a petri dish containing a water-saturated filter. Coverslips are best placed on a layer of parafilm; this helps to stop the antibody solution from rolling off the edge of the coverslip and makes it easy to pick up the coverslips with fine forceps, as the parafilm is compressible. 2. Add the first antibody solution. All dilutions must be carried out in protein-containing solutions. For example, use PBS containing 3 % **BSA. For unlabelled primary antibodies:** Monoclonal antibodies are best applied as tissue culture supernatants (specific antibody concentration of 20-50 mg/L, use neat). Ascites fluids, purified monoclonal and polyclonal antibodies, and crude polyclonal sera should be tested at a range of dilutions aimed at producing specific antibody concentrations between 0.1-10 mg/L. If the specific antibody concentration of the antibody sample is unknown, prepare and test 1/10, 1/100, 1/1000, and 1/10,000 dilutions of the starting material. **For labelled primary antibodies:** Primary antibodies can be labelled with enzymes, fluorochromes, or iodine. They should be assayed at several dilutions in preliminary tests to determine the correct working range. Too-high concentrations will yield high backgrounds; too-low concentration will depend on both the abundance of the antigen under study and the specificity of the antibody. 3. Incubate the coverslips, slides, or plates for a minimum of 30 min. at room temperature in the humidified chamber. For some reactions, prolonged incubations of up to 24 hr. can increase sensitivity. 4. Wash in three changes of PBS over 5 min. This buffer may be supplemented with 1 % Triton X-100 or NP-40 to help with any background problems.

If the first antibody is labelled, the specimen is now ready for the detection step. Otherwise:

5. Apply the labelled secondary reagent. It is essential to carry out all dilutions in a protein-containing solution such as 3 % BSA in PBS or 1 % immunoglobulin in PBS (prepared from the same species as the detection reagent). Useful secondary reagents include anti-immunoglobulin antibodies, protein A, or protein G. They can be labelled with enzymes, fluorochromes, gold, or iodine. Labelled secondary reagents can be purchased from several suppliers or can be prepared. **For enzyme-labelled reagents:** If using a commercial preparation, test dilutions of the secondary antibodies 1/50 to 1/1000. Alkaline phosphatase-labelled reagents should be handled using Tris-buffered saline, not PBS. **For fluorochrome-labelled reagents:** If using commercial preparations, test dilutions between 1/10 to 1/300. **For gold-labelled reagents:** Wash the gold particles once in PBS. Dilute in PBS containing 1 % gelatin and add to the specimen. **For iodine-labelled reagents:** Add the iodinated antibody at approximately 0.1 mg/L. Usually, specific activities between 10 and 100 Ci/g are used.

6. Incubate with the labelled secondary reagent for a minimum of 20 min. at room temperature in the humidified chamber. For gold-labelled reagents, observe periodically under the microscope until a satisfactory signal is obtained. 7. Wash in three changes of PBS (or Tris saline) over 5 min.

The specimen is now ready for the detection step.

*Detection Using Enzyme-Labelled Reagents*

Enzyme-labelled reagents are detected using soluble chromogenic substrates that precipitate following enzyme action, yielding an insoluble coloured product at the site of enzyme-localisation (Avrameas and Uriel 1966; Nakane and Pierce 1967a,b; Avrameas 1972). A range of substrates is available for each enzyme, and the following protocols represent some of the most useful alternatives. A wide range of conjugated reagents are available commercially or can be prepared as described. Enzymes can be coupled to anti-immunoglobulin antibodies, protein A, protein G, avidin, or streptavidin.

*Horseradish Peroxidase-Labelled Reagents*

A range of substrates are useful, including diaminobenzidine, chloronaphthol, and aminoethylcarbazole. In preferred embodiments, the use of diaminobenzidine is indicated.

*Diaminobenzidine*

Diaminobenzidine (DAB) is the most commonly used substrate and one of the most sensitive for horseradish peroxidase. It yields an intense brown product that is insoluble in both water and alcohol. DAB staining is compatible with a wide range of common histological stains.

1. Dissolve 6 mg of DAB (use DAB tetrahydrochloride) in 10 mL of 0.05 M Tris buffer pH 7.6. 2. Add 0.1 mL of 3 % solution of $H_2O_2$ in $H_2O$. $H_2O_2$ generally is supplied as a 30 % solution and should be stored at 4°C, at which it will last about 1 month. 3. If a precipitate appears, filter through Whatman No. 1 filter paper (or equivalent). 4. Apply to specimen, incubate for 1-20 min. Stop the reaction by washing in water. (**Optional**) Counterstain if necessary. 5. Mount in DPX.

*DiaminobenzidinelMetal*

The diaminobenzidine substrate for horseradish peroxidase can be made more sensitive by adding metal salts such as cobalt or nickel to the substrate solution. The reaction product is slate gray to black, and the products are stable in both water and alcohol. DAB/metal staining is compatible with a wide range of common histological stains.

1. Dissolve 6 mg of DAB (use DAB tetrahydrochloride) in 9 mL of 0.05 M Tris buffer pH 7.6. 2. Add 1 mL of a 0.3 % W/V stock solution of nickel chloride in $H_2O$ (the same amount of cobalt chloride can be used as an alternative). 3. Add 0.1 mL of a 3 % solution of $H_2O_2$ in $H_2O$. $H_2O$ generally is supplied as a 30 % solution and should be stored at 4°C, at which it will last about 1 month. 4. If a precipitate appears, filter through Whatman No. 1 filter paper (or equivalent). 5. Apply to specimen, incubate for 1-20 min. Stop the reaction by washing in $H_2O$. (**Optional**) Coun-

terstain if necessary. 6. Mount in DPX.

**COUPLING**

The detectable entity may be attached or coupled to the compact particle by a number of methods. For example, the detectable entity may be coupled to the compact particle by the use of cyanogen bromide.

Chemical crosslinkers are used to covalently modify proteins for studying ligand-receptor interactions, conformational changes in tertiary structure, or for protein labeling. Crosslinkers are divided into homobifunctional crosslinkers, containing two identical reactive groups, or heterobifunctional crosslinkers, with two different reactive groups. Heterobifunctional crosslinkers allow sequential conjugations, minimizing polymerization.

*Homobifunctional*

| Reagent | code No. | Modified Group | Solubility | Comments | Refs |
|---|---|---|---|---|---|
| BMME | 442635-Y | -SH | DMF, Acetone | Homobifunctional crosslinker useful for formation of conjugates via thiol groups. | Weston, P.D., et al. 1980. Biochem. Biophys Acta. 612, 40. |
| BSOCOE S | 203851-Y | -NH2 | Water | Base cleavable crosslinker useful for studying receptors and mapping surface polypeptide antigens on lymphocytes. | Howard, A.D., et al. 1985. J. Biol. Chem.260, 10833. |
| DSP | 322133-Y | -NH2 | Water | Thiol cleavable crosslinker used to immobilize proteins on supports containing amino groups. | Lee, W.T., and Conrad, D.H. 1985. J. Immunol. 134, 518. |
| DSS | 322131-Y | -NH2 | Water | Non-cleavable, membrane impermeable crosslinker widely used for conjugating radiolabeled ligands to cell surface receptors and for detecting conformational changes in membrane proteins. | D'Souza, S.E., et al. 1988. J. Biol. Chem.263, 3943. |

(continued)

| Reagent | code No. | Modified Group | Solubility | Comments | Refs |
|---------|----------|----------------|------------|----------|------|
| EGS | 324550-Y | -NH2 | DMSO | Hydroxylamine cleavable reagent for crosslinking and reversible immobilization of proteins through their primary amine groups. Useful for studying structure-function relationships. | Geisler, N., et al. 1992. Eur. J. Biochem.206, 841. 14. Moenner, M., et al. 1986. Proc. Natl. Acad. Sci. USA83, 5024. |
| EGS, Water Soluble | 324551-Y | -NH2 | Water | Water soluble version of EGS that reacts rapidly with dilute proteins at neutral pH. Crosslinked proteins are readily cleaved with hydroxylamine at pH 8.5 for 3-6 hours, 37°C. | Yanagi, T., et al. 1989. Agric. Biol. Chem.53, 525. |
| Glutarald ehyde | 354400-Y | -OH | Water | Used for crosslinking proteins and polyhydroxy materials. Conjugates haptens to carrier proteins; also used as a tissue fixative. | Harlow, E., and Lane, D. 1988. Antibodies: A Laboratory Manual, Cold Spring Harbor Publications, N.Y., p. 349. |
| SATA | 573100-Y | -NH2 | DMSO | Introduces protected thiols via primary amines. When treated with hydroxylamine, yields a free sulhydryl group that can be conjugated to maleimide-modified proteins. | Duncan, R.J.S., et al. 1983. Anal. Biochem.132, 68. |

*Heterobifunctional*

| Reagent | code No. | Modified Group | Solubility | Comments | Refs |
|---------|----------|----------------|------------|----------|------|
| GMBS | 442630-Y | -NH2, -SH | DMSO | Heterobifunctional crosslinker useful for preparing enzyme- | Kitagwa, T., et al. 1983. J. Biochem.94, |

(continued)

| Heterobifunctional | | | | | |
|---|---|---|---|---|---|
| **Reagent** | **code No.** | **Modified Group** | **Solubility** | **Comments** | **Refs** |
| | | | | antibody conjugates (for example beta-gal-IgG) and for immobilizing enzymes on solid supports. | 1160.19. Rusin, K.M., et al. 1992. Biosens. Bioelectron.7, 367. |
| MBS | 442625-Y 442626-Y | -NH2, -SH -NH2, -SH | DMSO, Water | Thiol cleavable, heterobifunctional reagent especially useful for preparing peptide-carrier conjugates and conjugating toxins to antibodies. | Green, N., et al. 1982. Cell 28, 477. |
| PMPI | 528250-Y | -SH2, -OH | DMSO, DMF | Used in the preparation of alkaline phosphatase conjugates of estradiol, progesterone, serine-enriched peptides, and vitamin B12. | Aithal, H.N., et al. 1988. J. Immunol. Methods 112, 63. |
| SMCC | 573114-Y 573115-Y | -NH2, -SH -NH2, -SH | DMF, AN Acetonitri le Water | Heterobifunctional reagent for enzyme labeling of antibodies and antibody fragments. The cyclohexane bridge provides extra stability to the maleimide group. Ideal reagent for preserving enzyme activity and antibody specificity after coupling. | Annunziato, M.E., et al. 1993. Bioconjugate Chem. 4, 212. |

(continued)

| Heterobifunctional | | | | | |
|---|---|---|---|---|---|
| Reagent | code No. | Modified Group | Solubility | Comments | Refs |
| SPDP | 573112-Y | -NH2, -SH | DMF, AN Acetonitri le | Introduces protected thiol groups to amine groups. Thiolated proteins can be coupled to a second molecule via an iodoacetamide or maleimide group, or to a second pyridyldisulfide containing molecule. | Caruelle, D., et al. 1988. Anal. Biochem. 173, 328. |

Each of these reagents may be obtained from a number of manufacturers, for example, from Calbiochem (code No. in column 2), or Piece Chemical Company.

The compact particle may be activated prior to coupling, to increase its reactivity. In preferred embodiments, the compact particle is activated using chloroacetic acid followed by coupling using EDAC/NHS-OH. Compact particles may also be activated using hexane di isocyanate to give primary amino group. Such activated compact particles may be used in combination with any hetero bifunctional cross linker. The compact particle in certain embodiments is activated using divinyl sulfon. Such activated compact particles comprise moieties which can react with amino or thiol groups, on a peptide, for example.

The compact particle may also be activated using tresyl chloride, giving moieties which are capable of reacting with amino or thiol groups. The compact particle may also be activated using cyanogen chloride, giving moieties which can react with amino or thiol groups

**PEPTIDE COUPLING**

In preferred embodiments, the detectable entity comprises a peptide. Coupling of peptides to compact particle is described in detail in this section.

Peptides can be obtained by solid phase synthesis methods. The first stage of the technique, first introduced by Merrifield (R.B. Merrifield, Solid Phase Peptide Synthesis. The synthesis of a Tetrapeptide., J. Am. Chem. Soc. 85, page 2149-2154, (1963) and R.B. Merrifield, Solid Phase Synthesis, Science 232, page 341-347, (1986)) consists of peptide chain assembly with protected amino acid derivatives on a polymeric support. The second stage of the technique is the cleavage of the peptide from the support with the concurrent cleavage of all side chain protecting groups to give the crude free peptide. To achieve larger peptides, these processes can be repeated sequentially.

The flexibility of the method allows the synthesis of long, short and branched peptides, including peptides with natural and un-natural occurring amino acids, different linkers and so-called spacers. The spacers typically being of polyethylenglycol, PEG derivatives or polyalkanes or homo poly amino acids. The solid phase synthesis method allows for the preparation of peptides terminated with reactive functionalities, for example free thiols, for chemo selective coupling schemes to the compact particle material.

A sequence of amino acids can be repeated in the final peptide sequence to enhance the immunoreactivity with a specific antibody. The repetitive and reactive sequence can be spaced with irrelevant amino acid sequences in a linear peptide. Also, by synthesizing branched or dendritic peptide constructs, like the multiple antigen peptides (MAP), the immuno reactivity can be enhanced.

For a review of the general methodology, including the different chemical protection schemes and solid and soluble supports, see for example G. Barany, N. Kneib-Cordonier, D. G. Mullen, Solid-phase peptide synthesis: A silver anniversary report, Int. J. Peptide Protein Res. 30, page 705-739, (1987), and G.B. Fields, R. L. Noble, Solid phase peptide synthesis utilizing 9-fluorenylmethoxycarbonyl amino acids, Int. J. Peptide Protein Res. 35, page 161-214 (1990)

Other methods for obtaining peptides include enzymatic fragment ligation, genetic engineering techniques as for example site-directed mutagenesis. Genetic engineering of oligonucleotides, PCR-products, or cloned fragments of DNA material encoding relevant amino acid sequence using standard DNA cloning techniques has been a well established methods of obtaining polypeptides. Alternatively, the peptides can be obtained after isolation from natural sources, such as by protein purification and digestion.

Conjugation of the target molecule (for example, peptide) may be achieved by forming covalent bonds or using strong binding pairs, for example ion binding, biotinavidin. Examples of other binding entities than streptavidin, avidin and derivatives and biotin and biotin analogues, are the leucine zipper domain of AP-1 (Jun and fos), hexa-his (metal chelate moiety), hexa-hat GST (glutathione S-Transferase) glutathione affinity, trivalent vancomycin, D-Ala-D-Ala, lectines that binds to a diversity of compounds, including carbohydrates, lipids and proteins, for example Con A (Canavalia ensiformis), concanavalin A and WGA (Whet germ agglutinin) and tetranectin or Protein A or G. These and other methods are well known to any skilled in the art of conjugation.

Covalent conjugation confers several advantages, including increased resistance to degradation.

The coupling method useful for conjugation is dependent on the chemical structure of the target and the compact particle involved. Typical chemical reagents used are so-called zero length cross linkers, homobifunctional, heterobi functional or polymeric cross linkers.

Zero length cross linkers like 1-Ethyl-3-(3-dimethylaminopropyl) carbodiimide (EDAC) or 1-cyclohexyl-3-(2-morpholinoethyl)carbodiimide metho-p-toluenesulfonate (CHMC) and other carbodiimides can facilitate direct coupling between for example Glu or Asp to Lysine residues and for example the N terminus of a peptide.

Homobifunctional cross linkers like glutar(di)aldehydes, imidates, bis-diazotized benzidines, bis(imido esters), bis(succinimidyl esters), diisocyanates, diacid chlorides, divinylsulfone or similar, allows amino or hydroxyl groups to be bound covalent together through a short linker molecule. Formaldehyde or glutar(di)aldehyde can also facilitate cross-linking between compact particle and peptide.

The use of heterobifunctional cross linkers is described in more detail for cross linking peptides to a compact particle by a methodology known to any skilled in the art of conjugation.

Heterobifunctional cross linkers have the advantage of providing greater control over the cross-linking than methods which rely on for example homobifunctional cross linkers.

The most common schemes for forming a heteroconjugate involve the indirect coupling of an amine group on one bio molecule to a thiol group on a second bio molecule, usually by a two- or three-step reaction sequence. The high reactivity of thiols and their relative rarity in many biomolecules make thiol groups ideal targets for controlled chemical cross-linking.

If a thiol group is not present, thiol goups can be introduced by several methods. One common method including the use of succinimidyl 3-(2-pyridyldithio)propionate (SPDP) followed by reduction of the 3-(2-pyridyldithio)propionyl conjugate with DTT or TCEP. Reduction releases the chromophore 2-pyridinethione, which can be used to determine the degree of thiolation.

Alternatively, the degree of thiolation can be measured using 5,5'-dithiobis-(2-nitrobenzoic acid) (DTNB, Ellman's reagent) which stoichiometrically yields the chromophore 5-mercapto-2-nitrobenzoic acid upon reaction with a thiol group.

Heterobifunctional cross linkers typically contain an activated carboxyl group at one end which can react with amino groups and a maleimido or iodoacetamide group at the opposite end which reacts readily with the sulfhydryl group of cysteine residues.

Two frequently used heterobifunctional crosslinkers are N-gamma-Maleimidobutyryloxysuccinimide ester (GMBS) and Succinimidyl 4-(N-maleimidomethyl)cyclohexane-1-carbonate (SMCC).

It should be understood, that the cross linker may contain a photoactivated reactive moiety. The photo reactive moiety acts as a masked reactive group. By using a photoactive coupling method, it is possible to bring the target molecule into specific part of for example the compact particle before using the photo reactive group for the covalent coupling.

Typically, the peptide is synthesized with a single cysteine residue at either the Nor C-termini. Alternatively, the internal Cys residues or Cys residues on a linker can be used. If the peptide contains no thiol group, then one or more can be introduced using one of several thiolation methods, typically by modifying one of the amino groups.

It should be understood that coupling of target probes, like for example peptides, are not limited by the use of thiol selective coupling schemes.

Other useful chemical moieties for both chemo selective or random conjugation schemes include carboxyl, hydroxyl, aromatic, phenolic or amino groups. Especially amino groups are useful, as they are very reactive at relevant pH, can form strong chemical bonds and are widely distributed in biological material.

The possibility to employ conjugation schemes using the amino group in the N-termini of peptides, including the amino group in the side chain of lysine or polylysine is of special relevance to the compositions and methods described here.

The cross linker is first reacted with the amino groups on the compact particle, followed by removal of the unreacted cross linker using for example a decanting or centrifugation. The activated carrier is then reacted with the Cys-containing peptide. Excess peptide is removed using for example a desalting column, dialysis, filtration or centrifugation. The amount of peptide or cross linker attached can be assessed by various direct or indirect analytical methods.

The conjugation sequence can be reversed by first attaching the heterobifunctional cross linker to the peptide, before attaching to thiols on the compact particle.

During conjugation reaction, the free thiols are often protected against spontaneous oxidation by the addition of EDTA, EGTA or tributylphosphine or similar or by using a protective atmosphere.

Other methods of covalent cross-linking include the use of homo or heterofunctionel polymeric cross linkers. Examples of reagents include tresyl or vinylsulfone activated dextrans or activated polyacrylic acid polymers or derivatives. Especially divinyl is preferred for activation of for example hydroxyl groups on compact particles, as the resulting second vinylsulfone is highly reactive towards thiols.

The amount of coupled peptide can be determined by several methods, including incorporating one beta-alanine residue immediately adjacent to the cysteine residue on the peptide. Amino acid analysis may then be used to determine the amount of beta-alanine present after purification of the resulting conjugate.

The cross linkers may offer the possibility to include a tracer or detectable moiety. This moiety can be used to measure the amount of cross linker bound to the bio molecule. The tracer can be fluorescent, radioactive, a hapten or any other detectable molecule.

**POLYMERS**

The embedding medium may be produced by polymerisation of monomers, as described in detail in this section.

Polymers made from polymerisable monomers have wide spread applications. For example, polymers are used as additives for coating applications, such as paints and adhesives.

Polymers are prepared by polymerising one or more types of polymerisable monomers, such as by emulsion polymerisation, solution polymerisation, suspension polymerisation or bulk polymerisation. The monomer(s) may be polymerised in the presence of optional ingredients such as any one of emulsifiers, stabilisers, surface active agents, initiators (such as photoinitiators), inhibitors, dispersants, oxidising agents, reducing agents, viscosity modifiers, catalysts, binders, activators, accelerators, tackifiers, plasticizers, saponification agents, chain transfer agents, surfactants, fillers, dyes, metal salts, and solvents.

There are numerous references on polymerisation of polymerisable monomers. For example, some teachings may be found in "Emulsion Polymerization: Theory and Practice" by D. C. Blackley (published by Wiley in 1975) and "Emulsion Polymerization" by F. A. Bovey et al. (published by Interscience Publishers in 1965). For example, a polymer can be prepared from monomers such as methyl acrylate, ethyl acrylate, butyl acrylate, 2-ethylhexyl acrylate, decyl acrylate, methyl methacrylate, ethyl methacrylate, butyl methacrylate, styrene, butadiene, ethylene, vinyl acetate, vinyl esters, $C_9$, $C_{10}$ and $C_{11}$ tertiary monocarboxylic acids, vinyl chloride, vinyl pyridine, vinyl pyrrolidine, vinylidene chloride, acrylonitrile, chloroprene, acrylic acid, methacrylic acid, itaconic acid, maleic acid and fumaric acid.

Examples of further teachings on polymerisation of polymerisable monomers may be found in "Vinyl and Related Polymers" by C.E. Schildknecht (New York: John Wiley & Sons 1952) and "Monomeric Acrylic Esters" by E.H. Riddle (New York: Reinhold Publishing Corp. 1954), and by A.G. Alexander (J Oil Colour Chemists' Association [1962] 45 12) and G.G. Greth and J.E. Wilson (J Appl Polymer Sci [1961] 5 135).

More recent teachings regarding polymerisation methods may be found in EP-A-0622378, EP-A-0634428, EP-A-0623632, EP-A-0635522, EP-A-0633273, EP-A-0632157, EP-A-0630908, EP-A-0630641, EP-A-0628614, EP-A-0628610, EP-A-0622449, EP-A-0626430 and EP-A-0625529.

By the term "cross-linker" we mean a compound which increases the degree of cross-linking of a monomer during polymerisation when compared to the polymerisation of the monomer in the absence of the cross-linker.

The monomers may be provided in the form of a polymerisable composition. The polymerisable composition may also comprise conventional additional components such as any one or more of emulsifiers, stabilisers, surface active agents, initiators (such as photoinitiators), inhibitors, dispersants, oxidising agents, reducing agents, viscosity modifiers, catalysts, binders, activators, accelerators, tackifiers, plasticizers, saponification agents, chain transfer agents, cross-linking agents, surfactants, fillers, dyes, metal salts, and solvents.

By way of example, the surfactants and dispersants can be salts of fatty rosin and naphthenic acids, condensation products of naphthalene sulphonic acid and formaldehyde of low molecular weight, carboxylic polymers and copolymers of the appropriate hydrophile-lipophile balance, higher alkyl sulfates, such as sodium lauryl sulfate, alkyl aryl sulfonates, such as dodecylbenzene sulfonate, sodium or potassium isopropylbenzene sulfonates or isopropylnaphthalene sulfonates; sulfosuccinates, such as sodium dioctylsulfosuccinate alkali metal higher alkyl sulfosuccinates, *for example* sodium octyl sulfosuccinate, sodium N-methyl-N-palmitoyl-taurate, sodium oleyl isethionate, alkali metal salts of alkylarylpolyethoxyethanol sulfates or sulfonates, *for example* sodium t-octylphenoxy-polyethoxyethyl sulfate having 1 to 5 oxyethylene units. Typical polymerisation inhibitors that can be used include hydroquinone, monomethyl ether, benzoquinone, phenothiazine and methylene blue.

In a preferred embodiment the dye is selected from the group consisting of 2-hydroxybenzophenone, oxidiazoles, salicylic acid, resorcinol monobenzoate, benzotriazole, preferably 2H-benzotriazole, benzothiazoloazine, preferably 2N-benzothiazoloazine, $\alpha$-cyano-$\beta$-phenylcinnamic acid, polyalkypiperidine and derivatives thereof.

Preferably, the dye is selected from benzotriazole, in particular 2H-benzotriazole and derivatives thereof.

The composition may comprise one or more additional comonomers. Examples of the one or more additional comonomers that can be used include one of: (alkyl and cycloalkyl) acrylates; (alkyl and cycloalkyl) methacrylates; free-radical polymerisable olefinic acids, including alkoxy-, alkylphenoxy-, alkylphenoxy-(polyethyleneoxide)-, vinyl ester-, amine substituted (including quaternary ammonium salts thereof), nitrile-, halo-, hydroxy-, and acid substituted (for example phospho- or sulpho-) derivatives thereof; and other suitable ethylenically unsaturated polymerisable moieties; including combinations thereof. Preferably the alkyl and cycloalkyl groups contain up to 20 carbon atoms, for example ($C_1$-$C_{20}$ alkyl and $C_1$-$C_{20}$ cycloalkyl) acrylates, and ($C_1$-$C_{20}$ alkyl and $C_1$-$C_{20}$ cycloalkyl) methacrylates. In more detail, typical comonomers include any one of methyl acrylate, ethyl acrylate, n-propyl acrylate, isopropyl acrylate, n-butyl acrylate, isobutyl acrylate, t-butyl acrylate, isobornyl acrylate, pentyl acrylate, hexyl acrylate, octyl acrylate, iso-octyl acrylate, nonyl acrylate, lauryl acrylate, stearyl acrylate, eicosyl acrylate, 2-ethylhexyl acrylate, cyclohexyl acrylate, cycloheptyl acrylate, methyl methacrylate, ethyl methacrylate, hydroxymethylacrylate, hydroxymethylmethacrylate, propyl methacrylate, n-butyl methacrylate, t-butyl methacrylate, isobutyl methacrylate, pentyl methacrylate, hexyl methacrylate, cyclohexyl methacrylate, 2-ethylhexyl methacrylate, isobornyl methacrylate, heptyl methacrylate, cycloheptyl methacrylate, octyl methacrylate, iso-octyl methacrylate, nonyl methacrylate, decyl methacrylate, lauryl methacrylate, eicosyl methacrylate, dodecyl acrylate, pentadecyl acrylate, cetyl acrylate, stearyl acrylate, eicosyl acrylate, isodecyl acrylate, vinyl stearate, nonylphenoxy-(ethyleneoxide)$_{1-20}$ acrylate, octadecene, hexadecene, tetradecene, dodecene, dodecyl methacrylate, pentadecyl methacrylate, cetyl methacrylate, stearyl methacrylate, eicosyl methacrylate, isodecyl methacrylate, nonylphenoxy-(ethyleneoxide)$_{1-20}$ methacrylate, acrylic acid, methacrylic acid, fumaric acid, crotonic acid, itaconic acid, fumaric anhydride, crotonic anhydride, itaconic anhydride, maleic acid, maleic anhydride, styrene, alphamethyl styrene, vinyl toluene, acrylonitrile, methacrylonitrile, ethylene, vinyl acetate, vinyl chloride, vinylidene chloride, acrylamide, methacrylamide, methacrylamide 2-cyanoethyl acrylate, 2-cyanoethyl methacrylate, dimethylaminoethyl methacrylate, dimethylaminopropyl methacrylate t-butylaminoethyl methacrylate, glycidyl acrylate, glycidyl methacrylate, glyceryl acrylate, glyceryl methacrylate, benzyl acrylate, benzyl methacrylate, phenyl acrylate, phenyl methacrylate, vinyl pyridine, vinyl pyrrolidine, siloxanes, silanes and mixtures thereof. Other polymerisable monomers are disclosed in US-A-2879178, US-A-3037006, US-A-3502627, US-A-3037969 and US-A-3497485.

Preferred comonomers include any one of glyceryl methacrylate (GMA), (2,2 dimethyl-1,3-dioxolan-4-yl) methyl methacrylate (GMAK), hydroxy ethyl methacrylate (HEMA), methacrylic acid, acrylic acid, GYMA, N-vinyl pyrrolidone, alkyl methacrylates (such as $C_{1-20}$ alkyl methacrylates, more preferably $C_{1-15}$ alkyl methacrylates, more preferably $C_{1-10}$ alkyl methacrylates, more preferably $C_{1-5}$ alkyl methacrylates, such as methyl methacrylate), alkyl acrylates (such as $C_{1-20}$ alkyl acrylates, more preferably $C_{1-15}$ alkyl acrylates, more preferably $C_{1-10}$ alkyl acrylates, more preferably $C_{1-5}$ alkyl acrylates, such as methyl acrylate), aryl methacrylates, aryl acrylates, diacetone acrylamide, acrylamide, methacrylamide, N-alkyl acrylamides (such as $C_{1-20}$ N-alkyl acrylamides, more preferably $C_{1-15}$ N-alkyl acrylamides, more preferably $C_{1-10}$ N-alkyl acrylamides, more preferably $C_{1-5}$ N-alkyl acrylamides, such as methyl acrylamide), N-alkyl methacrylamides (such as $C_{1-20}$ N-alkyl methacrylamides, more preferably $C_{1-15}$ N-alkyl methacrylamides, more preferably $C_{1-10}$ N-alkyl methacrylamides, more preferably $C_{1-5}$ N-alkyl methacrylamides, such as methyl methacrylamide), vinyl acetate, vinyl esters, styrene, other substituted olefins, N-dialkyl acrylamides (such as $C_{1-20}$ N-dialkyl acrylamides, more preferably $C_{1-15}$ N-dialkyl acrylamides, more preferably $C_{1-10}$ N-dialkyl acrylamides, more preferably $C_{1-5}$ N-dialkyl acrylamides, such as N N dimethyl acrylamide), N-dialkyl methacrylamides (such as $C_{1-20}$ N-dialkyl methacrylamides, more preferably $C_{1-15}$ N-dialkyl methacrylamides, more preferably $C_{1-10}$ N-dialkyl methacrylamides, more preferably $C_{1-5}$ N-dialkyl methacrylamides, such as N N dimethyl methacrylamide), 3-methacryloxypropyl tris (trimethysilyl siloxy) silane (TRIS monomer), fluoro substituted alkyl and aryl acrylates and methacrylates (preferably wherein the alkyl is $C_{1-20}$ alkyl, more preferably $C_{1-15}$ alkyl, more preferably $C_{1-10}$ alkyl, more preferably $C_{1-5}$ alkyl), and combinations thereof.

More preferred comonomers include any one of glyceryl methacrylate (GMA), (2,2 dimethyl-1,3-dioxolan-4-yl) methyl methacrylate (GMAK), 2-hydroxy ethyl methacrylate (2-HEMA), methacrylic acid, acrylic acid and glycidyl methacrylate, or combinations thereof.

The lists of comonomers also include substituted derivatives of those monomers, such as halogenated monomers, especially fluorinated monomer derivatives, and acetal and ketal derivatives.

The polymerisable monomer of the composition and the one or more additional comonomers may be selected so that the composition consists essentially of GMA and HEMA.

Any typical, suitable polymerisation method may be used. The preferred method is free radical polymerisation, thermal or UV initiated.

**FURTHER ASPECTS**

Further aspects and embodiments are now set out in the following numbered Paragraphs:

Paragraph 1. A reference standard for a detectable entity, the reference standard comprising a support medium, preferably an embedding medium, a compact particle having a compact shape supported by the medium, and a quantity of detectable entity attached to the compact particle.

Paragraph 2. A reference standard for a detectable entity, the reference standard comprising a compact particle having a compact shape with a quantity of detectable entity attached thereto, in which the compact particle is of biological, preferably cellular origin.

Paragraph 3. A reference standard according to Paragraph 1 or 2, in which a detectable amount of the detectable entity is present in a defined region in a cross section of the reference standard.

Paragraph 4. A reference standard according to Paragraph 1, 2 or 3, in which the detectable entity adopts a compact shape, preferably an unextended or non-elongate shape; in the support medium.

Paragraph 5. A reference standard according to any preceding Paragraph, in which the compact shape is such that the ratio of the longest dimension to the shortest dimension is less than 5:1, preferably less than 2:1

Paragraph 6. A reference standard according any preceding Paragraph, in which the compact shape comprises a particulate, uniform or regular shape.

Paragraph 7. A reference standard according to any preceding Paragraph, in which the compact shape comprises a sphere shape, an ovoid shape, an ellipsoid shape, a disc shape, a cell shape, a pill shape or a capsule shape.

Paragraph 8. A reference standard according to any preceding Paragraph, in which the detectable entity is heterologous to the compact particle.

Paragraph 9. A reference standard according to any preceding Paragraph, in which the detectable entity is chemically coupled to the compact particle.

Paragraph 10. A reference standard according to any preceding Paragraph, in which the compact particle comprises a cell.

Paragraph 11. A reference standard according to Paragraph 10, in which the cell does not express the detectable entity.

Paragraph 12. A reference standard according to Paragraph 10 or 11, in which the cell is selected from the group consisting of: a virus, a bacterial cell, a eukaryotic cell, an insect cell, an animal cell, a mammalian cell, a mouse cell and a human cell.

Paragraph 13. A reference standard according to Paragraph 10, 11 or 12, in which the cell comprises an insect cell, preferably an Sf9 cell, or a mammalian cell, preferably a Chinese Hamster Ovary (CHO) cell.

Paragraph 14. A reference standard according to any of Paragraphs 1 to 9, in which the compact particle comprises an organelle.

Paragraph 15. A reference standard according to Paragraph 14, in which the organelle comprises a mitochondrion, a plastid, a chloroplast, or a nucleus, preferably derived from a cell as set out in any of Paragraphs 11 to 13.

Paragraph 16. A reference standard according to any of Paragraphs 1 to 9, in which the detectable entity is substantially free of cellular material.

Paragraph 17. A reference standard according to any of Paragraphs 1 to 9 and 16, in which the compact particle comprises a microbead or a micelle.

Paragraph 18. A reference standard according to any preceding Paragraph, in which the compact shape has a dimension of less than 100μm, preferably less than 50μm, more preferably less than 20μm, most preferably less than 10μm.

Paragraph 19. A reference standard according to any preceding Paragraph, in which the defined region is present in at least one other cross section of the reference standard, preferably comprising a similar amount of detectable entity.

Paragraph 20. A reference standard according to any preceding Paragraph, in which the support medium comprises an embedding medium, in which the detectable entity is embedded.

Paragraph 21. A reference standard according to any preceding Paragraph, in which the detectable entity comprises a diagnostically relevant target.

Paragraph 22. A reference standard according to any preceding Paragraph, in which the detectable entity comprises an antigen, an epitope, a peptide, a polypeptide, a protein, a nucleic acid, or two or more or a plurality of any of the above, or combinations of one or more of the above.

Paragraph 23. A reference standard according to any preceding Paragraph, in which the detectable entity is selected from the group consisting of: a hapten, a biologically active molecule, an antigen, an epitope, a protein, a polypeptide, a peptide, an antibody, a nucleic acid, a virus, a virus-like particle, a nucleotide, a ribonucleotide, a deoxyribonucleotide, a modified deoxyribonucleotide, a heteroduplex, a nanoparticle, a synthetic analogue of a nucleotide, a synthetic analogue of a ribonucleotide, a modified nucleotide, a modified ribonucleotide, an amino acid, an amino acid analogue, a modified amino acid, a modified amino acid analogue, a steroid, a proteoglycan, a lipid, a carbohydrate, a dye, and mixtures, fusions, combinations or conjugates of the above.

Paragraph 24. A reference standard according to any preceding Paragraph, in which the detectable entity comprises any one or more of HER2, oestrogen receptor (ER), PR, p16, Ki-67 and Epidermal Growth Factor Receptor (EGFR) protein, and nucleic acids encoding such.

Paragraph 25. A reference standard according to any preceding Paragraph, in which the presence and/or quantity of the detectable entity is revealable by a binding agent, preferably a labelled binding agent.

Paragraph 26. A reference standard according to Paragraph 25, in which the binding agent is selected from the group consisting of: an antibody, preferably an antibody capable of specific binding to the detectable entity, a nucleic acid such as a DNA or an RNA, preferably a nucleic acid capable of specific binding to the detectable entity, a protein nucleic acid (PNA), a dye, a special stain, Au-chloride, Haematoxylin-Eosin (H & E), Gomori methenamine silver stain (GMS), Periodic Acid-Schiff (PAS) stain, Trichrome Blue, Masson's Trichrome, Prussian Blue, Giemsa, Diff-Quik, Reticulum, Congo Red, Alcian Blue, Steiner, AFB, PAP, Gram, Mucicarmine, Verhoeff-van Gieson, Elastic, Carbol Fuchsin and Golgi's stains.

Paragraph 27. A reference standard according to any preceding Paragraph, in which the presence of the detectable entity in a cell, tissue, organ or organism is indicative of a disease or a condition.

Paragraph 28. A reference standard according to any preceding Paragraph, in which the defined region includes a reference area, the reference area comprising the detectable entity at a pre-defined amount.

Paragraph 29. A reference standard according to Paragraph 28, in which the amount of the detectable entity in the reference area is compared to the amount of the detectable entity in a sample to determine the presence, quantity or concentration of the detectable entity in the sample.

Paragraph 30. A reference standard according to any preceding Paragraph, in which the reference standard is in the shape of a rectangular box.

Paragraph 31. A reference standard for a detectable entity, comprising: (a) an embedding medium in a preferably substantially rectangular box shape; and (b) a cell with a quantity of detectable entity coupled thereto.

Paragraph 32. A reference standard according to any preceding Paragraph, which comprises two or more compact particles, each having detectable entity attached thereto.

Paragraph 33. A reference standard according to any preceding Paragraph, which comprises two or more different detectable entities, each of which is attached to the same or different compact particle.

Paragraph 34. A reference standard according to any preceding Paragraph, which comprises two or more compact particles comprising different amounts of detectable entity on each.

Paragraph 35. A reference standard according to any preceding Paragraph, in which a planar section of the reference standard comprises a plurality of areas on which are presented the detectable entity at different density.

Paragraph 36. A reference standard according to any preceding Paragraph, in which a planar section of the reference standard comprises a first area comprising the detectable entity substantially at a diagnostically significant density.

Paragraph 37. A reference standard according to any preceding Paragraph, further comprising a control comprising a compact particle which comprises substantially no detectable entity.

Paragraph 38. A reference standard according to any preceding Paragraph, in which the embedding medium is selected from the group consisting of: ice, wax, paraffin, acrylic resin, methacrylate resin, epoxy, Epon, Araldite, Lowicryl, K4M and LR White and Durcupan.

Paragraph 39. A reference standard for a detectable entity comprising an surrounding medium together with a quantity of detectable entity located in the surrounding medium in a defined amount, in which the detectable entity adopts a compact shape in the surrounding medium.

Paragraph 40. A planar section, preferably a transverse planar section, preferably of substantially uniform thickness, of a reference standard according to any preceding Paragraph.

Paragraph 41. A support, preferably a slide such as a microscope slide, comprising a planar section according to Paragraph 40 mounted thereon.

Paragraph 42. A kit comprising a reference standard according to any preceding Paragraph, together with a binding agent capable of specific binding to the detectable entity, optionally together with instructions for use.

Paragraph 43. A reference standard, kit or a planar section according to any preceding Paragraph, in which the reference standard has been stained, preferably with an antibody or a nucleic acid probe.

Paragraph 44. A diagnostic kit for detecting the presence or amount of a detectable entity in a biological sample, comprising: (a) a reference standard, planar section or slide according to any preceding Paragraph; (b) a binding agent capable of specific binding to the detectable entity; and optionally (c) instructions for use.

Paragraph 45. A combination of a reference standard, planar section, support, kit or diagnostic kit according to any of Paragraphs 1 to 44 together with a therapeutic agent capable of treating or alleviating at least one of the symptoms of a disease or condition in an individual.

Paragraph 46. A combination according to Paragraph 45, in which the individual is diagnosed as suffering from or susceptible to the disease or condition, if the amount of detectable entity in the biological sample or component is similar to or greater than that in the reference standard.

Paragraph 47. A diagnostic kit according to Paragraph 44 or a combination according to Paragraph 45 or 46, in which the binding agent or therapeutic agent comprises an antibody against the detectable entity.

Paragraph 48. Use of a reference standard, a planar section or a kit according to any preceding Paragraph, for determining the presence or amount of a detectable entity in a biological sample.

Paragraph 49. A method of comparing the amount of a detectable entity in a biological sample with a reference standard, the method comprising the steps of:

Paragraph (a) providing a biological sample and obtaining a first signal indicative of the amount of detectable entity in the biological sample, or a component thereof;

Paragraph (b) providing a reference standard, planar section, support, kit or diagnostic kit according to any of Paragraphs 1 to 44;

Paragraph (c) obtaining a second reference signal indicative of the amount of detectable entity in the reference standard or planar section thereof; and

Paragraph (d) comparing the first signal obtained in (a) against the reference signal.

Paragraph 50. A method according to Paragraph 49, in which the detectable signal is selected from the group consisting of: radiation, optical density, reflectance, radioactivity, fluorescence, enzymatic activity.

Paragraph 51. A method according to Paragraph 49 or 50, in which the reference standard or planar section thereof is subjected to the same one or more steps or conditions, preferably substantially all, as the biological sample.

Paragraph 52. A method according to Paragraph 49, 50 or 51, in which the reference standard or planar section thereof is processed through one or more, preferably all, of the following steps: mounting onto a slide, baking, deparaffination, rehydration, antigen retrieval, blocking, exposure to antibody, exposure to primary antibody, exposure to nucleic acid probe, washing, exposure to secondary antibody-enzyme conjugate, exposure to enzyme substrate, exposure to chromogen substrate, and counter staining.

Paragraph 53. A method or use according to Paragraphs 48 to 52, in which the biological sample comprises a cell, tissue or organ, preferably a cell, tissue or organ of an organism suspected of suffering a disease or condition.

Paragraph 54. A method of diagnosis of a disease or a condition in an individual, the method comprising the steps of:

Paragraph (a) obtaining a biological sample from the individual; and

Paragraph (b) comparing the amount of a detectable entity in a biological sample or component thereof with a reference standard, in a method according to Paragraph 49;

Paragraph in which preferably the individual is diagnosed as suffering from or susceptible to the disease or condition, if the amount of detectable entity in the biological sample or component is similar to or greater than that in the reference standard.

Paragraph 55. A method of treatment of a disease or a condition in an individual, the method comprising the steps of diagnosing the disease or condition in an individual in a method according to Paragraph 54, and administering a therapeutic agent to the individual.

Paragraph 56. A method of treatment according to Paragraph 55, in which the therapeutic agent comprises an antibody capable of binding to the detectable entity.

Paragraph 57. A method of assessing the effectiveness or success of a procedure, the method comprising the steps of:

Paragraph (a) providing a reference standard according to any of Paragraphs 1 to 39, in which a detectable property of the detectable entity is changed as a result of the procedure;

Paragraph (b) conducting the procedure on the reference standard; and

Paragraph (c) detecting a change in the detectable property of the detectable entity.

Paragraph 58. A method according to Paragraph 57, in which a detectable property of the detectable entity is changed as a result of a successful procedure, which change in the detectable property of the detectable entity is detected to establish that the procedure is successful.

Paragraph 59. A method according to Paragraph 57, in which a detectable property of the detectable entity is changed as a result of an unsuccessful procedure, which change in the detectable property of the detectable entity is detected to establish that the procedure is not successful.

Paragraph 60. A method of validating a procedure according to Paragraph 57, 58 or 59, in which the procedure is selected from the group consisting of: an *in situ* hybridisation procedure, an immunohistochemical procedure, deparaffination, antigen retrieval, blocking, endogenous biotin blocking, endogenous enzyme blocking, a washing step, incubation with revealing agent such as a primary antibody, incubation with secondary visualisation components, chromogen staining, staining information acquisition and analysis.

Paragraph 61. A method according to any of Paragraphs 57 to 60, in which the procedure is an antigen retrieval procedure, and in which the detectable property of the detectable entity comprises the masking or unmasking of one or more epitopes.

Paragraph 62. A method according to any of Paragraphs 57 to 61, in which the detectable entity in the reference standard is modified to mask one or more epitopes, some or all of which are unmasked in an antigen retrieval procedure which is successful.

Paragraph 63. A method according to any of Paragraphs 57 to 60, in which the procedure is an deparaffination procedure, and in which the detectable property of the detectable entity comprises the presence or quantity of detectable entity in the reference standard following the deparaffination procedure.

Paragraph 64. A method according to any of Paragraphs 57 to 60 and 63, in which the detectable entity in the reference standard is soluble in the deparaffination medium, and in which at least a portion, preferably all, of the detectable entity is removed following a successful deparaffination procedure.

Paragraph 65. Use of a reference standard as Paragraphed in any preceding Paragraph as an antigen retrieval validation standard, a deparaffination standard, a blocking validation standard, a washing validation standard, a primary antibody validation standard, a secondary antibody validation standard, a calibration standard, or a diagnostic standard.

Paragraph 66. A method of producing a reference standard for a detectable entity, the method comprising the steps of: (a) providing a support medium, preferably an embedding medium; (b) providing a compact particle having a compact shape; (c) attaching a quantity of detectable entity to the compact particle and (d) supporting or embedding the compact particle in the medium.

Paragraph 67. A method of producing a reference standard for a detectable entity, the method comprising the steps of: providing a compact particle of biological, preferably cellular origin, and attaching a quantity of detectable entity to the compact particle.

Paragraph 68. A method of producing a reference standard for a detectable entity, the method comprising supporting a compact particle having a compact shape and a quantity of detectable entity attached thereto in a support medium.

Paragraph 69. A method of producing a reference standard for a detectable entity, the method comprising the steps of: (a) providing an embedding medium; (b) forming a quantity of detectable entity in a generally compact shape by attachment to a compact particle; and (c) embedding the detectable entity in the embedding medium.

Paragraph 70. A method according to any of Paragraphs 66 to 69, which further comprises attaching a quantity of a second detectable entity to the compact particle, or to a second compact particle.

Paragraph 71. A method according to any of Paragraphs 66 to 70, which further comprises attaching a second different quantity of the or each detectable entity to the or each compact particle.

Paragraph 72. A method according to any of Paragraphs 66 to 71, in which the support medium comprises an embedding medium, and the or each compact particle is supported by embedding in the embedding medium.

Paragraph 73. A method according to any of Paragraphs 66 to 72, in which the or each detectable entity is covalently coupled to its respective compact particle.

Paragraph 74. A reference standard for a detectable entity, comprising a detectable entity attached to a cell and supported by a support medium.

Paragraph 75. A method of producing a reference standard for a detectable entity, the method comprising the steps of providing a cell, attaching or covalently coupling a quantity of detectable entity to the cell, and embedding the cell in an embedding medium.

Paragraph 76. A reference standard according to Paragraph 74 or a method according to Paragraph 75, in which the cell does not express the detectable entity.

Paragraph 77. A method or reference standard according to Paragraph 74, 75 or 76, in which the cell is selected from the group consisting of: a virus, a bacterial cell, a eukaryotic cell, an insect cell, preferably an Sf9 cell, an animal cell, a mammalian cell, preferably a Chinese Hamster Ovary (CHO) cell, a mouse cell and a human cell.

Paragraph 78. An artificial cell or organelle comprising a detectable entity attached to a compact particle having a compact shape.

Paragraph 79. A method of making an artificial cell or organelle comprising a detectable entity, the method comprising providing a compact particle having a compact shape, and attaching a quantity of detectable entity to the compact particle.

Paragraph 80. A modified cell or organelle comprising a detectable entity coupled to a cell, or a component thereof, which preferably does not express the detectable entity.

Paragraph 81. A method of making an modified cell or organelle comprising a detectable entity, the method comprising providing a cell or a component thereof which does not express the detectable entity, and coupling a quantity of detectable entity to the cell or component.

Paragraph 82. A method of establishing a cellular distribution of detectable entity in a reference standard, the method comprising providing a cell or a component thereof which does not express a detectable entity, coupling a quantity of detectable entity to the cell or component, and supporting the cell or component in a support medium.

Paragraph 83. A reference standard substantially as hereinbefore described with reference to and as shown in the accompanying drawings.

Paragraph 84. A planar section preferably of substantially uniform thickness of a reference standard substantially as hereinbefore described with reference to and as shown in the accompanying drawings.

Paragraph 85. Use of a reference standard or a planar section for determining the presence or amount of a detectable entity in a biological sample, such use substantially as hereinbefore described with reference to and as shown in the accompanying drawings.

Paragraph 86. A method of determining the amount of a detectable entity in a biological sample substantially as hereinbefore described with reference to and as shown in the accompanying drawings.

Paragraph 87. A method of diagnosis of a disease or a condition in an individual substantially as hereinbefore described with reference to and as shown in the accompanying drawings.

## EXAMPLES

To illustrate the broad and general utility of the present invention, several reference systems have been prepared and evaluated, including the use of different kinds of cells, intact whole cells or isolated nucleic membranes, pre fixed, non fixed or permabilized, several different targets, mixtures with irrelevant target, and with graded staining intensities and various visualization systems.

The reference systems have been evaluated as various cytological preparations by microscope and in a flow cytometer - and as formalin fixed and paraffin embedded (FFPE) preparations evaluated in bright field or fluorescence microscope.

## Example 0. Materials and Methods

In the following, some of the general procedures are described:

### A. Isolation of CHO cells

Chinese hamster ovary cells (CHO cells) are obtained from ATCC (cat No.: CLL-61) and grown in Gibco F-12K Nutrient Mixture media containing 10% foetal calf serum (FCS), penicillin and streptomycin. Cells are cultured at 37°C with 5% $CO_2$. At confluence, the cells are dislodged by adding trypsin-EDTA and then washed in F-12K media without FCS. Cells are counted in a hemacytometer (NucleoCounter, Chemometec, Denmark)

The cells are isolated from media by gently centrifugation at 800 rpm (approx. 90 x g) at 20-24 °C for 5-10 minuts. The cell pellet is re-suspended in 10 mM $NaH_2PO_4$/ $Na_2HPO_4$, (MERCK cat no. 6580 and cat no. 6346) pH 7.2, 0.145 M NaCl MERCK cat no. 6404 (PBS)(0.33 mL per 10 mill cells) followed by a centrifugation.

The intact cells are used directly for coupling of the target or further treated to obtain the nuclear membrane.

### B. Isolation of sf9 cells

Sf9 insect cells (Fall armyworm, Spodoptera frugiperda pupal ovary, ECACC 89070101) are grown as suspension culture in spinner flasks in media (InVitrogen Cat. No. 11605-045 grace) containing 10% FCS, glutamine and antibiotics at 27 C, in more than 20% oxygen with no special access to $CO_2$.

The cells are isolated from the media by centrifugation and wash, as described above for the CHO cells. The intact cells are used directly for coupling of the target or further treated to obtain the nuclear membrane.

### C. Isolation of nuclear membranes

The nuclear preparation is made according to Edgar Schreiber et.al. (1989), Nucleic Acids research, Vol. 17, Number 15, page 6419.

Cell cytoplasmic membrane lysis (not nuclear envelope lysis) is carefully controlled by re-suspension of the cell pellet in 4-8 °C cold 10 mM HEPES (Sigma, cat. no. H-3375, pH 7.9, 10 mM KCl, 0.2 mM EDTA (MERCK cat. no. 1.08418), and 1 mM DDT (Sigma cat. no D-0632) in an 1:6 vol:vol ratio. The suspension of approximately 150 million cells is left on ice in 4.5 ml of hypotonic buffer for an interval of 10 to 15 minutes followed by 10 seconds of very gentle vortex mixing.

Nonidet P-40 detergent (BDH, cat. no.56009) is added to a final concentration of 0.15 v/v % followed by further 10 seconds of gentle vortex mixing.

The nuclear membranes are visually inspected to be more than 90% intact by phase contrast bright field microscopy and pelleted at 1000 rpm (approx. 140 x g ) at 4-8 °C for 5 minutes.

Recovery of intact nuclear membranes is more than 130 million, according to the cell count in the hemacytometer.

The nuclear membranes are used for coupling different targets.

### D. General procedure for coupling peptide epitopes to intact cells or nuclear membranes

Approx. 30 million nuclei or cell pellet are resuspended in 1.00 mL cold 25 mM $Na_2CO_3$, pH 8.0, 100 mM NaCl, 5 mM $MgCl_2$, and 1 mg/mL D-Glucose (MERCK cat no. 1.08342)(hereafter called the coupling buffer).

The nuclei or cells are washed twice by centrifugation at 800 rpm (approx. 90 x g) at 20 to 24 °C for 10 minutes. Followed by re-suspension in 1 mL (0.33 mL per 10 mill cells) cold coupling buffer.

The suspension of approx. 30 mill nuclei or cells is activated with a hetero bifunctional cross-linker (N-($\gamma$-Maleimidobutyryloxy)-succinimide ester) ('GMBS', cat no. 22309, Pierce Chemical Company, 0.84 mg/ml in dimethylformamide) for 20 minutes at 30 to 37 °C.

The reaction is quenched by addition of 3 fold molar excess of L-Glycine (Merck cat. no. 1.04201, 1.0 mg/mL) per hetero bi functional linker for 5 to 10 minutes at 30 to 37 °C.

The nuclei or cells are again washed twice by centrifugation at 800 rpm (approx. 90 x g) at 20 to 24 °C for 10 minutes. Followed by re-suspension in 1 mL (0.33 mL per 10 mill cells) cold coupling buffer.

Following the washing step, the activated nuclei are resuspended in the general coupling buffer and conjugated with the particular peptide containing a thiol functionality.

A solution of the selected peptide is added and incubated with the activated cell suspension for 20 minutes at 30 to 37 °C.

After washing by centrifugation and resuspension twice in the general coupling buffer the re-suspended pellet are transferred to further FFPE or cytological processing and immunocytochemistry evaluation.

*General FFPE preparations*

*Embedding in agarose gel*

The total volume of the cell suspension is measured. The same amount of an agarose solution (2.0 % by weight in deionised water, HSA 1000 protein grade, FMC BioPolymer/Litex, obtained from Medinova Scientific A/S, Hellerup, Denmark) is heated to 60 °C in a water bath. The cell suspension is heated to 60 °C for a few minutes before being added the agarose solution. The warm mixture is very gently mixed for a few seconds.

The warm liquid gel slurry is quickly drawn up in a long single use plastic pipette (Transfer Pipettes, cat. No. 262, Coming Samco Corporation, San Fernando, USA). Any air bobbles is allowed to escape before the pipette is cooled in a water bath.

The tip of the plastic pipette is cut off and the solidified gel squeezed out and into cold water. The gel cylinders of approximately 3 mm in diameter and 40 mm in length is collected and cut into pieces of approximately 15 mm in length with a surgical knife.

*Fixation*

Each gel piece is transferred to a container (30 mL Polystyrene Nunc/Nalgene test tube) with Neutral buffered formalin, NBF (20 mL, 10 mM $NaH_2PO_4$/ $Na_2HPO_4$, (MERCK cat no. 6580 and cat no. 6346), 0.145 M NaCl (Merck cat no. 6404), pH 7,0, adjusted to 4 % formaldehyde from a 37 % Formaldehyde (Merck code No. 4003), and left overnight in a ventilated laboratory hood (18 hr., at room temperature)

*Dehydration and paraffin embedding*

The gel pieces containing the cells are gently wrapped in microscope lens cleansing paper (Leica catalog no. 8060861) and placed in a marked plastic histocapsule (Sekura, Japan, ProHosp: Mega-cassette code No. 59040, approximately 32x26x10 mm), before being dehydrated and embedded in paraffin.

The gel embedded cells are dehydrated by sequential treating with 70% ethanol twice for 45 minutes, 96% ethanol twice for 45 minutes, 99% ethanol twice for 45 minutes, xylene twice for 45 minutes, before being transferred to melted paraffin (Merck code No. 7337.9020, melting point 56-58°C) and left over night (12-16 hours) at 60°C. The paraffin-infiltrated pieces are transferred to fresh warm paraffin and left there for additional 60 minutes before being embedded with paraffin in a cast (Sekura, ProHosp 4166 mega, approximately 31x23x13 mm) and cooled to form the final paraffin blocks.

The marked paraffin blocks containing the embedded reference material are stored cold (2-8 °C) and dark before being cut, mounted, deparaffinated and stained.

*Cutting, mounting and deparaffination*

The paraffin blocks are mounted in a microtome (Leica 0355 model RM2065, Feather S35 knives, set at 5.0 micrometer). The first few mm are cut away before the paraffin sections are cut in 5-micrometer thickness at room temperature and collected. The paraffin sections are gently stretched on a 45-60 °C hot water bath before being collected and mounted onto marked microscope glass slides (Superfrost plus, Menzel-gläser code No. 041300). The slides are dried, baked in an oven at 60°C, and excess and melted paraffin wiped away with a tissue.

The slides are deparaffinated by successive incubating twice in xylene for 2-5 min., twice in 96 % ethanol for 2-5 min., twice in 70 % ethanol for 2-5 min. and once in TBS (50 mM Tris-HCl (Tris(hydroxymethyl)aminomethan p.a., Crystal Chem inc., II, USA), 150 mM NaCl, pH 7.6) for 5 min.

*Antigen retrieval and blocking*

The slides are treated with an antigen retrieval buffer (from the DakoCytomation p16 research kit for histology, product no. OA315) for 40 minutes at 95-98 °C in a water bath and allowed to cool to room temperature for another 20 minutes.

The slides are washed gently with a wash buffer (Dakocytomation, catalogue no. S3006, here after called the "wash buffer") for 5 min., followed by incubation with a 3% hydrogen peroxide solution for 5 minutes to quench endogenous peroxide activity. (Peroxidase-Blocking Solution DakoCytomation code no. S 2023), before being washed once with TBS for 5 min.

To ensure good coverage of reagent on the material, the area on the slide with reference material is encircled with a silicone rubber barrier ("DakoPen", DakoCytomation code No. S 2002).

The slides are transferred to a rack in a small and closed chamber to avoid drying out during the following procedural steps.

*Immunovisualisation*

The overall procedure being first incubating with a primary antibody against the relevant target. Followed by washing, incubation with a polymeric dextran conjugate mixture containing horseradish peroxidase and secondary goat antibody and staining with a HRP chromogen.

In more detail, the immunovisualisation of the cells is done using the primary antibody against the target in question (200 micro liters per slide for FFPE preparations and 300 micro liters per slide for cytological preparations).

All slides are washed in the wash buffer for 5 min., followed by incubation with Envision+1HRP conjugate (Dakocytomation, Envision, code No. K4001, 200 micro liters per slide from FFPE preparations and 300 micro liters per slide from cytological preparations) for 30 min.

The slides are washed gently three times in the wash buffer for 5 min., followed by incubation with a diaminobenzidine chromogenic substrate system (DAB+, DakoCytomation code No. K 3468) for 10 min. for FFPE preperations according to the product instructions.

For cytological preperations, the chromogenic substrate is incubated twice for 5 minutes.

All the slides are washed with the distilled water in 5 minutes, before being counterstained with Hematoxylin in 5 minutes and washed in tap water in 5 minutes, according to the product instructions (DakoCytomation, product no S3301)

The slides are cover slipped using an aqueous mounted media, Faramount (DakoCytomation code No. S 3025), and examined in a bright field microscope (Leica DM LB) at 10x or 40x magnification, using light strength setting 8. The slides are digitally photographed (Olympus DP50-CU) and the pictures white background corrected.

The cytological preparations is done according to one of the three slide based methods: Cytospin, Autocyte/ TriPath or ThinPrepTM.

*General cytological preparations using a "cytospin" technique*

Total cell counts are obtained using a hemocytometer. The cell suspension is adjusted to 0.5 to 1.0 million cells per milliliter. Aliquots of cells is pelleted onto glass slides using a "cytospin 2" (Shandon Scientific, Cheshire, United Kingdom).

The glass microscope slides is mounted in the slide clip together with filter card and finally the cytofunnel. The assembled slide system is placed in the centrifuge before the cell suspension is added (50 or 100 microliters per slide), the centrifuge lid closed and the centrifuge run at 800 rounds per minute for 5 minutes.

The assembled slide system is removed from the centrifuge and the slide with the cell preparation detached from the filter card and cytofunnel.

The slides are dried for 30 minutes at room temperature. The cells mounted on the slide are spray fixed with the alcohol and glycerol containing Mercofix (Merck, product no. 77-323-2 and 77-323-1) and allowed to dry for 10 minutes at room temperature.

The slides could be stored frozen for maximum 3 weeks before further processing.

The slides are antigen retrieved, blocked and immunovisualized as described above for the FFPE preparations.

*General cytological preparations using a Autocyte/Tripath techniques*

The overall procedure is done as instructed in the manual "Manual preparation of LBC based on AutoCyte principle" (Cytyc Corporation, MA, USA). The reagents, centrifuge tubes and filters are all purchased from Cytyc (Cytyc Corporation, MA, USA).

The cell sample is separated from any debris by centrifugation, before being collected on a filter device, mounted onto slides and fixed using a buffered alcohol solution.

In more detail, the slides (Cytyc Thin Prep microscope slides, product No. 70214-001) are treated with the "Slidecoate-reagent" for 10 min. before being dried in the air for 30 min.

The cell suspension (approx 2-4 ml) and "Density Reagent" (4 ml) is mixed in a centrifuge tube using a vortex mixer.

The filter pump is placed in the sample container, and pushed all the way down. The sample container with the pump is placed on top of the centrifuge tube that contained the Density Reagent. The tube is centrifuged for 2 min at 200 rpm.

The mixture is divided in 2 layers and the top layer carefully removed by a pipette and discarded. The sample is centrifuged again for 2 min at 500 rpm followed by decanting the liquid from the cell pellet in the bottom of the tube.

The cell pellet is resuspended in water (2.00 ml).

The coated slides are placed in the AutoCyte slide rack and the Prep Settling chamber device mounted on top.

The cell suspension (400 micro liters) is added to each chamber and the mixture allowed to sediment for 10 minutes. Excess liquid is decanted and the slide removed from the chamber.

The slides are dried for 60 minutes. The cells mounted on the slide are spray fixed with the alcohol and glycerol containing Mercofix (Merck, product no. 77-323-2 and 77-323-1) and allowed to dry for 10 minutes at room temperature. The slides could be stored for maximum 3 weeks before further processing.

The slides are antigen retrieved, blocked and immunovisualized as described above for the FFPE preparations.

*General cytological preparations using Thin Prep™ techniques.*

The overall procedure is done as instructed in the manual for ThinPrep 2000, (Cytyc Corporation, MA, USA).

In summary, the sample vial containing the cells is placed into the apparatus. Under the control of the instrument's microprocessor, the following steps are automatically performed: First, a gentle dispersion step broke up the sample and thoroughly mixed the sample. A series of negative pressure pulses are generated which draw fluid though a TransCyt® Filter to collect a thin, even layer of diagnostic cellular material. The cellular material is then transferred to a glass slide using computer controlled mechanical positioning and positive air pressure. The slide is then ejected into a cell fixative bath containing buffered methanol, before being stained and evaluated.

*General fluorescent staining of cytological preparations evaluated in a flowcytometer*

The total cell counts are obtained using the hemocytometer. The cells are stained using indirect or directly staining method.

Approximately 1.0 million cells in suspension are centrifuged at 13 G for 5 minutes at room temperature in Falcon tubes (Becton Dickinson product no. 352052). The pellet is added 10 microliter of either a negative antibody control, unlabelled specific antibody or a fluorescent-labelled specific antibody solution (diluted 1 to 20 in PBS).

The suspension is vortex mixed for 5 to 10 seconds (MT2 Minishaker, IKA-Werke GmbH & Co., Staufen, Germany) at approx 3000 rounds per minute before static incubation at 4 °C for 30 minutes in the dark. The tube is added 2 milliliters of PBS, vortex mixed for 5 to 10 seconds, centrifuged at 13 G in 5 minutes at room temperature. The tagged cell pellets are added 400 microliter PBS buffer, vortex mixed for 5 to 10 seconds before being analysed on a flowcytometer.

The directly labelled cells are analysed on a standard flowcytometer (FACS Calibur, Master description 4CS-E1822, Becton Dickinson Immunocytometry Systems, Erembodegem, Belgium).

For indirect labelling, the fluorescent-labelled secondary antibody is added by repeating the procedure above before being analysed on the flowcytometer.

The reference cells are analysed and evaluated using the standard software (Becton Dickinson Cell Quest version 3.3). The data are presented by plotting the forward Scatter (FSC-H) versus the side scatter (SSC-H), the logarithm fluorescent channel (e.g. FL-1) versus the counts and the forward scatter (FSH-H) versus the logarithm fluorescent channel (e.g. FL-1).

In the following the peptides used are described:

*Peptides*

The peptides used as target in the examples are all obtained from NeoSystems, Strasbourg, France or Novartis, Basel, Switzerland.

*Peptide no 1*

Ki-67 peptide (NeoSystems, lot no. SP011749C) contains an epitope to the MIB1™ antibody (Dakocytomation Ki67 clone MIB-1, Trade Mark). The epitope for the monoclonal antibody MIB1™ has been elucidated to be included in the 18-meric amino acid sequence -Ala-Gly-Phe-Lys-Glu-Leu-Phe-Gln-Thr-Ala-Gly-Phe-Lys-Glu-Leu-Phe-Gln-Thr. The peptide has an Mw of 2164.5 Da.

*Peptide no 2*

Biotinylated Ki-67 peptide (NeoSystems, lot no. SP010864B) contains an epitope to the MIB1™ antibody similar to peptide no 1 and containing a Cystein in the C-terminus for coupling purposes and a Biotinyl derivative in the Amino-terminus. The peptide has an Mw of Mw 2390.8 Da.

*Peptide no 3*

HER2 peptide (NeoSystems, lot no. 991729) contains an epitope for the polyclonal antibody against HER2/neu (DakoCytomation A0485 rabbit antibody).

The Rabbit antibody recognize several linear amino acid sequences in the intracellular part of the HER2/neu receptor tyrosine kinase protein designated aa 1242Thr to aa 1255Val and the peptide contain an additional spaced Cystein in the C-terminus for coupling purposes. The peptide has an Mw of 1676.9 Da.

*Peptide no 4*

p16 peptide (NeoSystems, lot no. SP021294) contains an epitope for the monoclonal antibody E6H4 elucidated to be included in the C-terminus part of the Human p16-INK4 protein designated aa 144Arg to aa 151Pro (Swiss-Protein accession No. P42771), synthesized as a two times repetitive amino acid sequence, Fluorescein labeled in the Amino-terminus and containing an additional spaced Cystein in the C-terminus for coupling purposes. The peptide has an Mw of Mw 2201.3 Da.

*Peptide no 5*

p16 peptide (NeoSystems, lot no. SP0010864) same amino acid sequence as peptide no 4, biotin labeled in the amino-terminus and containing a spaced Cystein in the C-terminus for coupling purposes. The peptide has an Mw of Mw 2390.3 Da.

*Peptide no 6*

Ki-67 peptide (NeoSystems, lot no. SP0211991) contains an epitope to the MIB1™ antibody similar to peptide no 1, synthesized as a two times repetitive amino acid sequence, and containing a Cystein in the C-terminus for coupling purposes. The peptide has an Mw of Mw 1817.2 Da.

*Peptide no 7*

HER3 peptide (NeoSystems, lot no. SP000432) contains an epitope for the polyclonal antibody against HER3.

The antibody recognize several linear amino acid sequences in the intracellular part of the HER3 receptor near aa sequence 1289. The peptide has an Mw of 1617 Da.

*Peptide no 8*

Phosphonated HER2 peptide (Novartis, NVP-ABN-379-A1-1) contains an epitope for the polyclonal antibody against the intracellular part of the HER2/neu receptor tyrosine kinase protein designated aa 1242Thr to aa 1255Val, phosphonated at amino acid position 1248Tyrosine and containing an additional spaced Cystein in the C-terminus for coupling purposes. The peptide has an Mw of 1753.8 Da.

## *Experimental Support*

### 1. Use of Ki-67 peptide as target and Horse Radish Peroxidase (HRP) EnVision/DAB staining of isolated nuclei from CHO cells in a cytospin cytological preparation

10 million Chinese Hamster Ovary (CHO) cell nuclei are isolated and activated as described in general above.

Activation is made with 0.50 μMolar "GMBS" (0.14 mg, 10 mg/ml DMF), quenched with L-Glycin, and final reacted with 0.125 μMolar (0.29 mg) of Ki-67 peptide (peptide no 2, epitope to MIB1™ antibody, MW 2390.8 Da), containing a Cystein in the C-teminus and a Biotinyl derivative in the Amino-terminus.

The cells is treated as cytospin cytological preparation and mounted on slides as described above in the general procedure.

Immunovisualisation of nuclei is done as described above in the general section. In summary, the primary monoclonal antibody is clone MIB1™ (DakoCytomation, 7240, dilution 1:200), followed by incubation with Goat anti Mouse Immunoglobulins and horse radish peroxidase labelled onto a dextran polymer (DakoCytomation Envision, code No. K4001), and finally a diaminobenzidine chromogenic substrate system (DakoCytomation DAB*plus*, code No. 3468).

An immunohistochemically negative hematoxylen counterstaining staining is processed in parallel to distinctly visualise nuclei.

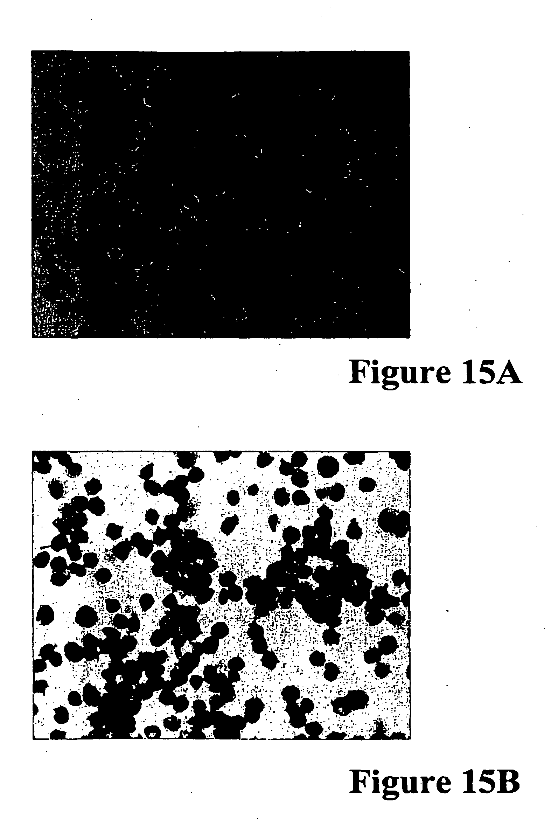

Figure 15A shows the homogeneous hematoxylin stain of the nucleolus.

Figure 15B shows the DAB Immunostaining as homogeneously distributed to the cell nuclear membrane and lamina, to the chromatin and to the nucleolus. Both pictures are taken at 20-time magnification.

In conclusion, the target peptide can be coupled to the isolated nuclei. The hematoxilin stain further illustrates the quality of the isolated nuclei.

**2. Use of the Ki-67 peptide as target and Horse Radish Peroxidase (HRP) Envision/DAB staining of isolated Nuclei from CHO cells in a formalin fixed and paraffin embedded (FFPE) preparation**

10 million Chinese Ovary Cell nuclei are isolated and activated as described earlier. Activation is made with 1.0 μMolar 'GMBS', followed by reaction with 3 μMolar (3 x molar excess) L-Glycin to 'GMBS', and final reaction with 0.1 μMolar of Ki-67 peptide (peptide no 2, epitope to MIB1™ antibody, MW 2390.8 Da), containing a Cystein in the C-terminus and a Biotinyl derivative in the Amino-terminus.

The cells are treated as FFPE preparation, mounted on slides and immunovisualized as described in example 1 and in the general procedure.

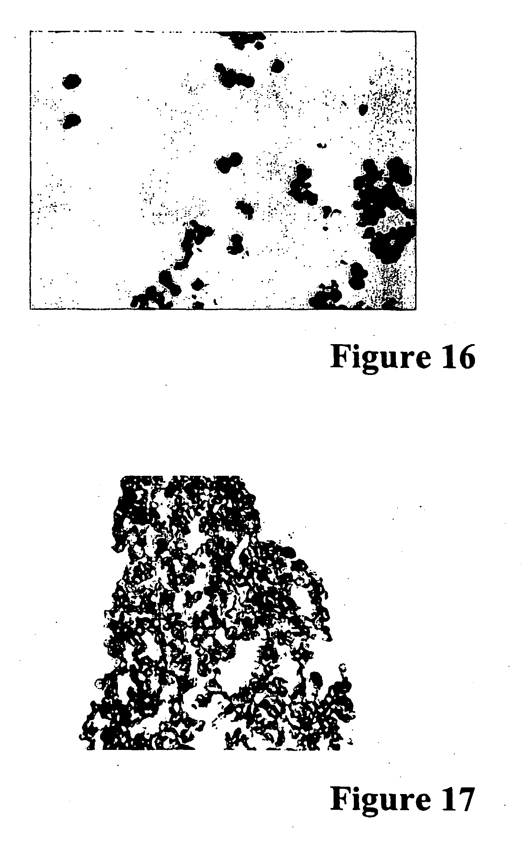

Figure 16 is a photograph of the stained CHO cells taken at 20 times magnification. A very strong immunostaining is observed to both the cell nuclear membrane and lamina, to the chromatin and to the nucleus. The staining clearly illustrates the possibility of staining standard formalin fixed and paraffin embedded (FFPE) whole cells.

**3. Effect of added detergents during activation and conjugation of the Ki-67 peptide target and HRP Envision/DAB staining of isolated Nuclei from CHO cells in a FFPE tissue preparation**

The activated nuclei are reacted with 0.02 μmolar of Ki-67 peptide (peptide no 1, epitope to the MIB1™ antibody containing a Cystein in the C-terminus, MW 2164.5 Da), with 0.1 v/v % Nonidet P-40 and 0.1 v/v % Pluronics F-127 (BASF), added in the conjugation mixture.

The cells are treated as a FFPE preparation, mounted on slides and immunovisualized as described above.

Figure 17 is a photograph of the stained CHO cells taken at 20 times magnification.

The intensity of the immunostaining is markedly reduced as compared to the staining in example 2.

The staining pattern indicate, that using detergents during conjugation increases the penetration of reagents into the cellular material and makes the staining more evenly distributed between the individual cells. Furthermore, the cell distribution and the overall appearance of the stain are more homogenous.

**4. Use of the Ki-67 peptide as target and alkaline Phosphatase (AP)/fast Red LSAB+ Immunostaining of isolated nuclei from CHO cells in a cytospin cytological tissue preparation and evaluated by both bright field and fluorescence microscopy**

In short, about 30 million Chinese Ovary Cell nuclei are isolated, activated and conjugated as described earlier. Activation is made with 3.0 μmolar 'GMBS' (0.84 mg), followed by reaction with 9.0 μMolar (3 x molar excess) L-Glycin to 'GMBS', and final reaction with 0.06 μmolar (0.13 mg) of Ki-67 peptide (peptide no 1, epitope to the MIB1™ antibody, MW 2164.5 Da), containing a cystein in the C-terminus.

The cells is treated as cytospin cytological preparation and mounted on slides as described above in the general procedure.

Immunovisualisation of nuclei is done by incubation with the primary monoclonal antibody is clone MIB1™ (Dakocytomation 7204), as in the general procedure. This is followed by incubation for 1 h with biotinylated Goat anti Mouse secondary antibody and alkaline phosphatase conjugated streptavidin (DAKOcytomation LSAB+, code No. K5005), and finally Vector Red chromogenic substrate system (DakoCytomation code No. SK-5 100C), all according to the product instructions.

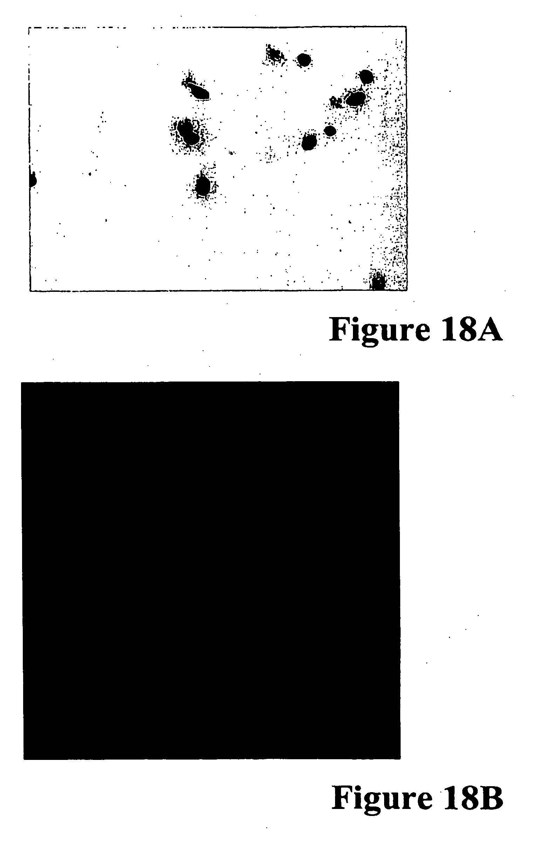

Figure 18A is a photograph of the Vector Red stained CHO cells taken at 20 times magnification. Similar to the HRP Envision staining system, the AP LSAB streptavidine-biotin vizualization system gave the expected staining.

The strong crisp red immunostaining is observed to both the cell nuclear membrane and lamina, to the chromatin and to the nucleolus.

The same preparation is evaluated in a fluorescence microscopy (Leica DMRA, with Sensys Photometrics Camera) using a TRITC Pinkel filter kit (Chroma Technology Corp.) and is depicted in Figure 18B.

The use of the fluorescent stain markedly enhanced the observed intensity of the immunostaining as compared to bright field microscopy.

**5. Effect of prefixing intact CHO cells prior to activation and conjugation of the HER2 peptide target**

This Example illustrates the effect of prefixing intact CHO cells prior to activation and conjugation of the HER2 peptide target. The resulting cells are treated both as cytospin cytological or FFPE preparations, HRP Envision/DAB stained by the Herceptest™ protocol and compared to the MDA-175 and SK-BR-3 control cell lines.

In short, about 20 million Chinese Ovary Cells are isolated as described earlier in Example 1 - except for the lysis step.

The cells are divided into two equally sized populations. The first population is prefixed using 0.7 v/v % final concentration of para-formaldehyd in a PBS buffer, pH 7.2, and left for 15 minutes on ice before the activation with GMBS.

The prefixed and non-prefixed cell populations are hereafter processed in parallel.

Activation is made witch 1.0 $\mu$Molar 'GMBS', followed by reaction with 3.0 $\mu$Molar (3 x molar excess) L-Glycin to 'GMBS', and final reaction with 0.25 $\mu$Molar HER2/*neu* peptide (0.42 mg) (peptide no 3, epitope to DakoCytomation A0485 antibody, MW 1674.8), containing a Cystein in the C-terminus.

The intact and peptide modified CHO cells is divided into two populations, and treated as cytospin cytological or as FFPE preparation, respectively, and mounted on slides as described above in the general procedure.

The intact CHO cells are immunovisualized using the HercepTest immunosystem (DakoCytomation K5204), which include the primary polyclonal antibody (DakoCytomation A0485) directed against the HER2/*neu* receptor protein.

The FFPE reference cells (MDA-175 and SK-BR-3 cell lines) included in the Herceptest kit, is treated and stained in parallel. The slides are all photographed at 20-time magnification.

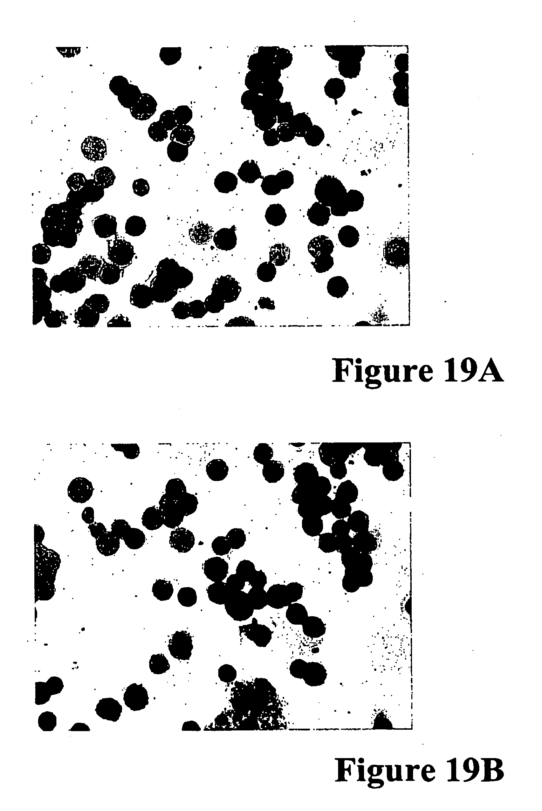

Figure 19A is the HRP/DAB stained cytospin preparation without a prefixation step, and Figure 19B with a prefixation step.

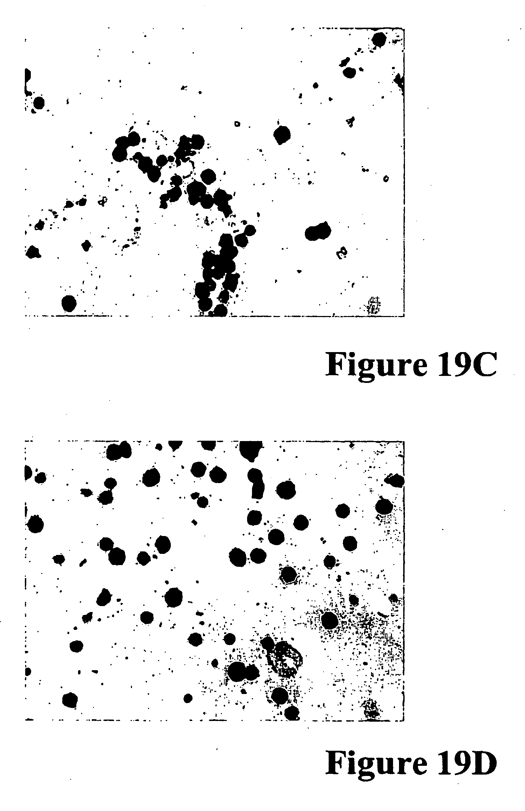

Figure 19C is the HRP/DAB stained FFPE preparation without a prefixation step, and Figure 19D with a prefixation step.

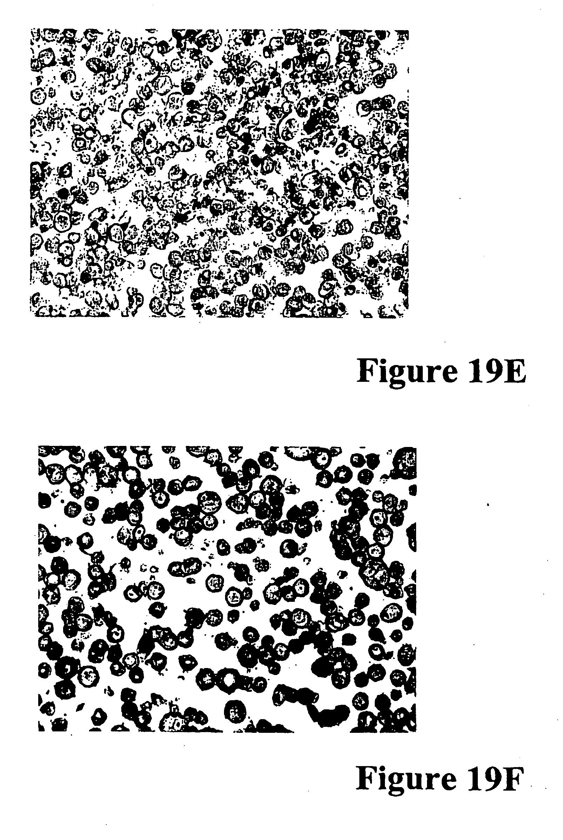

Figure 19E is the HRP/DAB stained MDA-175 (score +1) Herceptest FFPE reference cells, and Figure 19F is the HRP/DAB stained SK-BR-3 (score +3) Herceptest FFPE reference cells.

For both the cytospin and the FFPE preperations, it can seen that the stabilisation by fixation prior to the chemical modification of cell material results in a more well defined staining of the cellular components as compared to non-fixed cells.

The increased homogenisity and well defined staining can be advantageous in some applications in cytochemistry as the reference cells can be used to guide the interpreter to reach the correct scoring. Compared to the stained reference cells from the Dakocotymation Herceptest, the inter cell homogenisity is better and the preparation contained less cellular debris.

**6. Effect of using different levels of target peptide during conjugation to obtain graded staining of isolated nuclei from CHO cells in cytospin preparations**

The target in this Example is Ki-67 peptide and immunovizualized by Horse Radish Peroxidase (HRP) Envision/DAB staining.

In short, about 10 million Chinese Ovary Cell nuclei are isolated and activated as described earlier in example 1 and 2.

The nuclei population is divided into three populations. Activation is made by reaction with 0.5 $\mu$Molar 'GMBS', followed by reaction with 1.5 $\mu$Molar (3 x molar excess) L-Glycin to 'GMBS' and reacted with either (A) 0.125 $\mu$Molar Ki-67 peptide (peptide 5, Mw 2390.3 Da) (B) 0.50 $\mu$Molar Ki-67 peptide (peptide 5, Mw 2390.3 Da) or (C) 0.50 $\mu$Molar irrelevant HER2 peptide (peptide no 3, Mw 1676.9 Da). Both peptides contain a Cystein in the C-terminus.

The three nuclei populations is treated as cytospin cytological preparation and mounted on slides and immunovisualized using the primary monoclonal antibody against Ki-67 (Clone MIB-1, DakoCytomation 7240), as described in the general procedure.

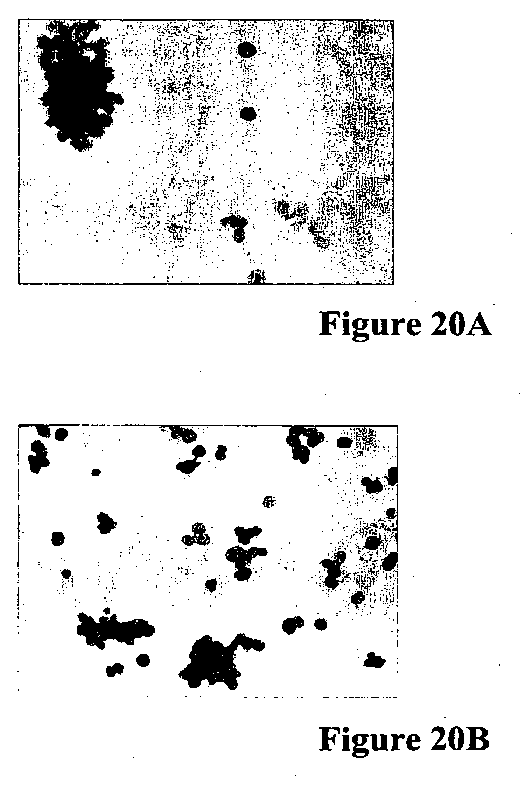

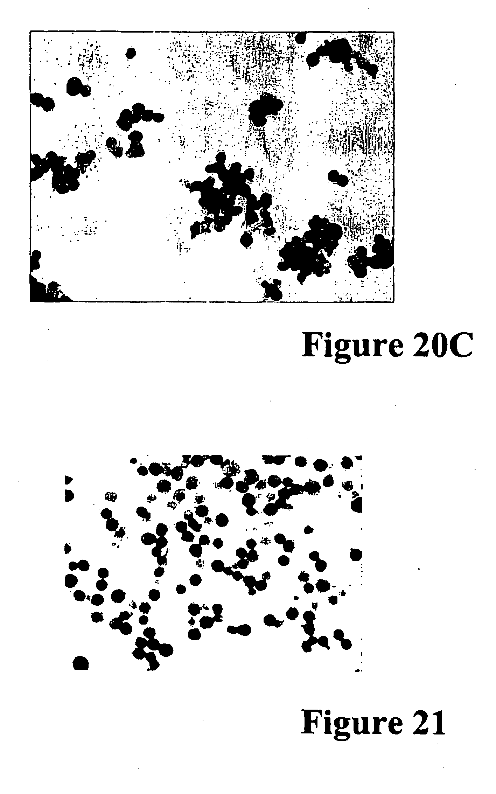

Figure 20 is photographs of the stained CHO nuclei taken at 20 times magnification: Nuclei coupled with the irrelevant HER2 peptide (Figure 20A), nuclei coupled with the low concentration (0.125 $\mu$Molar, Figure 20B), and high concentration of Ki-67 peptide (0.50 $\mu$Molar, Figure 20C).

Figure 20A shows the low non-specific staining of the nuclei modified with the irrelevant peptide.

Figure 20B and Figure 20C shows different staining levels (about 2+ and 3+, respectively). The preparations are somewhat inhomogeneous and contain some debris.

The two pictures shows that the intensity of the immunostaining and by this the cytochemically scoring can be adjusted by the concentration of the peptide during coupling.

The nuclei preparation is useful as references for targets normally located in the nucleus, as the size and morphology will be similar as viewed in the microscope.

**7. Use of a double p16 epitope motif peptide as target and Horse Radish Peroxidase (HRP) Envision/DAB staining of pre-fixed intact sf9 cells in a FFPE preparation**

In this Example, prior to chemically activation, the cells are treated with DakoCytomation IntraStain™, which is a permeabilization reagent.

200 million intact sf9 cells are isolated without a lysis step as described previously, pre-fixed using 4.0 v/v % final concentration of para-formaldehyd in a PBS buffer, pH 7.2 on ice for 20 minutes, washed, followed by treatment with DakoCytomation IntraStain™ (0.4 ml/mill cells) for 20 minutes at room temperature, followed by one washing step. The cells are in general activated as described earlier.

Activation is made with 10 µMolar 'GMBS', followed by reaction with 30 µMolar (3 x molar excess) L-Glycin, and final reaction with 0.01 µMolar of peptide no 4 (MW 2201.3), with a double epitope to the p16 antibody and containing a fluorescein moiety in the N-terminus and a cystein in the C-terminus.

The cells are treated as FFPE preparation, mounted on slides and immunovisualized using the primary monoclonal antibody against p16 (clone p16 E6H4, DakoCytomation, 0.62 micro gram/ml), as described in the general procedure.

Figure 21 is a photograph of the stained sf9 cells taken at 20 times magnification.

An immunostaining is observed throughout the individual cells. The staining pattern being homogeneous to the various parts (membrane, cytoplasm and nucleus) of the cells. The inter cell staining intensity range from weakly stained (1+) to strongly stained (4+) cells.

The staining pattern illustrates the positive effect of the intrastain reagent to help transport reagents to the various parts of the cells. The resulting stain is not only localized on e.g. the cell membrane.

**8. Use of the p16 peptide as target and Horse Radish Peroxidase (HRP) Envision/DAB staining of pre-fixed intact sf9 cells, in a FFPE preparation**

In this Example, the cells are treated with DakoCytomation IntraStain™ and coupled with known mixtures of the relevant double p16 motif peptide and an irrelevant peptide to obtain specific staining with different intensities.

200 million sf9 cells are isolated, prefixed and intrastain treated as in the previous example 124.

The cell population is divided into two equally sized populations (A and B) and activated in parallel as described earlier. Activation is made with 10 µMolar 'GMBS', followed by reaction with 30 µMolar (3 x molar excess) L-Glycin.

Population A is reaction with a premixed solution of 0.01 µMolar of peptide no 4 (same as in example 7 above.) and 0.30 µMolar (population A) or 0.10 µMolar (population B) of a irrelevant peptide (peptide No 6, which is a double epitopic-motif K167 peptide (Mw 1817.2 Da) both containing a cystein in the C-terminus.

The cells are treated as FFPE preparation, mounted on slides and immunovisualized using the primary monoclonal antibody against p16 (clone p16 E6H4, DakoCytomation), as described in the general procedure.

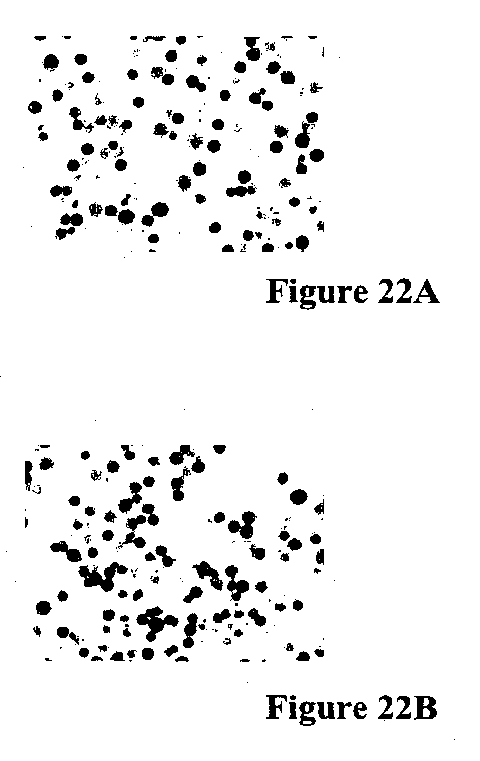

Figure 22A (using 30 equivalent irrelevant peptide) and Figure 22B (using 10 equivalent irrelevant peptide) are photographs of the stained sf9 cells taken at 20 times magnification.

Staining of population (A) using a negative control antibody (DakoCytomation N 1698) gave no detectable DAB staining.

The staining intensity is scored to 0.5 - 1+ in Figure 22A and 1 - 1.5+ in Figure 22B.

By comparing Figure 22A (30 equivalent irrelevant peptide) and Figure 22B 10 equivalent irrelevant peptide) with Figure 21 (no irrelevant peptide), it can be seen that the staining intensity is reduced by the addition of the irrelevant peptide.

Additionally, the inter cell staining level homogeneity is improved by the addition of the irrelevant peptide in the reaction mixture.

In conclusion, it is possible to lower the staining intensity by addition of irrelevant peptide in the coupling mixture. The obtained weak staining level of 0.5 to 1.0 is important, as IHC assays often need threshold values in this range.

**9. Reproducibility in preparation of in total four batches of the pre-fixed and intrastain treated intact sf9 cells with the double p16 peptide as target and the use of an irrelevant peptide**

The cells being evaluated for peptide density in a flowcytometer and treated as FFPE preparations and Horse Radish Peroxidase (HRP) Envision/DAB stained.

300 million intact sf9 cells are isolated, prefixed and intrastain treated as in example 124. Activation is made with 165 µMolar 'GMBS', followed by reaction with 400 µMolar (3 x molar excess) L-Glycin, and final reaction with a premixed solution of 0.015 µMolar of peptide no 4 (MW 2201.3), with a double epitope to the p16 antibody and containing a fluorescein moiety in the N-terminus and a Cystein in the C-terminus and 0.15 µMolar (10 time excess) irrelevant peptide (peptide no. 6) double-epitopic-motif KI67 peptide (1817.2 Da), containing a cystein in the C-terminus.

One tenth of the cell population is fixed (0,7 v/v %paraformaldegyd in PBS buffer, pH 7.2, 15 minutes on ice) and evaluated in flow cytometry for the level of fluorescein peptide coupling. The average fluorescent signal (FL1) per cell is plotted after 100000 events.

The rest of the cells are treated as FFPE preparation, mounted on slides and immunovisualized using the primary monoclonal antibody against p16 (clone p16 E6H4, DakoCytomation), as described in the general procedure.

The same laboratory personnel repeated the entire procedure from the first isolation of the cells to staining and final cover slipping four times in parallel.

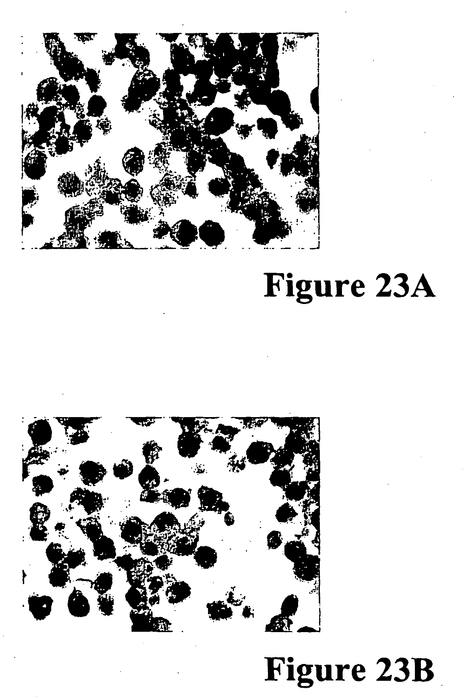

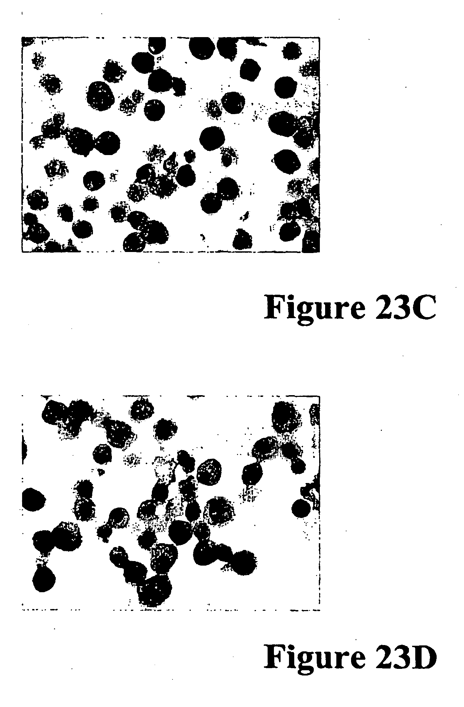

Figures 23A, B, C and D are photographs of the four preparations of stained sf9 cells taken at 40 times magnification.

The staining intensity is scored to +1 for all four preparations - the staining pattern and localization being almost identical for all preparations.

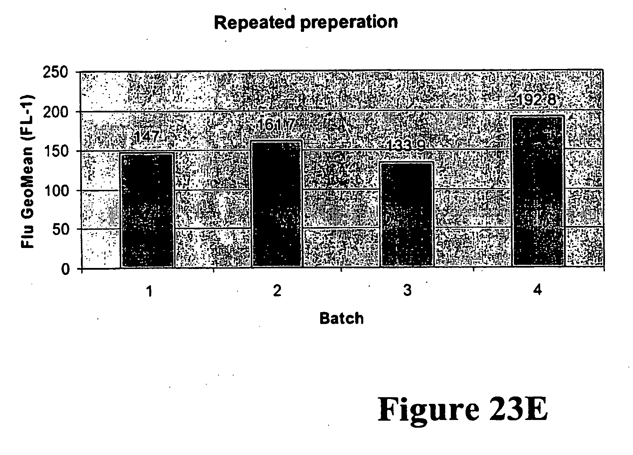

The average fluorescent signal densities for fluorescein (FL1) for the four preparations are summarized in Figure 23E The mean fluorescein signal ranged from 133.9 to 192.8 units for the four preparations.

In conclusion, the procedure could be reproduced four times to give the same DAB staining intensity. The measurement of the fluorescent signal from the relevant peptide can be used as an independent method for estimating the average target density on the cells.

The observed variation in target density as seen in the flow cytometer method could not be detected by the DAB/HRP immunostaining.

## 10. Use of the p16 peptide as target and Horse Radish Peroxidase (HRP) Envision/DAB staining of pre-fixed intact sf9 cells, in a FFPE preparation

In this Example, the cells are treated with DakoCytomation IntraStain™ and coupled with different cross linker concentrations and mixtures of the relevant double p16 motif peptide and an irrelevant Ki76 peptide to obtain specific staining with different intensities. The ratio between cross linker and total peptide concentration kept constant.

300 million sf9 cells are isolated, prefixed and intrastain treated as in example 124. The cell population is divided into three equally sized populations (A, B and C) and activated in parallel as described earlier.

Activation is made with (A) 60 µMolar, (B) 105 µMolar or (C) 165 µMolar "GMBS" respectively, followed by reaction with 3 equivalents L-Glycin with respect to GMBS: (A) 180 µMolar (B) 325 µMolar and (C) 495 µMolar L-glycine.

The maleimide activated cells is reacted with different ratios of the relevant p16 (peptide no 4 (MW 2201.3), and irrelevant Ki67 peptide (no 6, 1817.2 Da), both containing a Cystein in the C-terminus:

(A) 0.015 µMolar peptide no 4 and 0.045 µMolar peptide no 6, (B) 0.015 µMolar peptide no 4 and 0.090 µMolar peptide no 6 and (C) 0.015 µMolar peptide no 4 and 0.150 µMolar peptide no 6.

In summary, the L-Glycine to GMBS concentration ratio and the GMBS to total peptide concentration ratio is kept constant at 3.

The relevant p16 peptide concentration is kept constant at 0.015 µMolar, and the relevant to irrelevant peptide concentration ratio is 3, 6 and 10 respectively.

The cells are treated as FFPE preparation, mounted on slides and immunovisualized using the primary monoclonal antibody against p16 (clone p16 E6H4, DakoCytomation), as described in the general procedure.

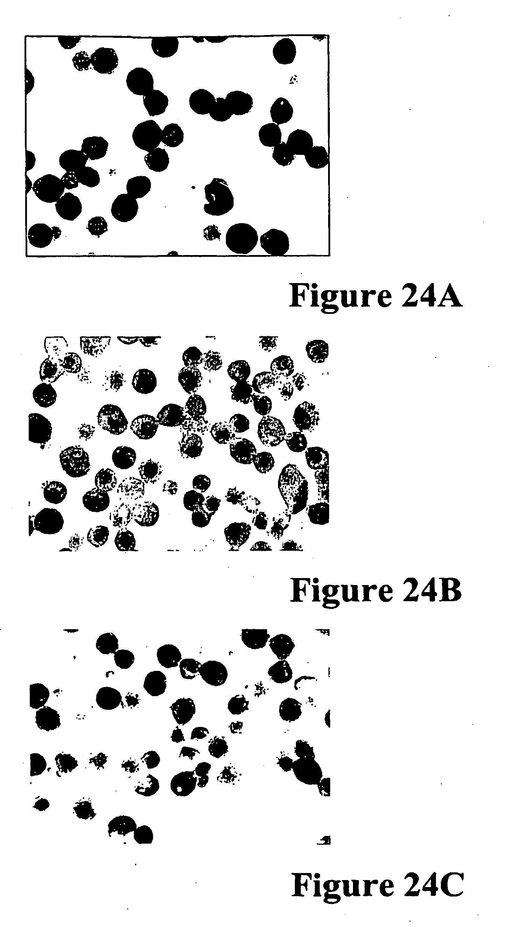

Figures 24A, B and C are photographs of the HRP/DAB stained FFPE sf9 cells taken at 40 times magnification with (A) 3x excess irrelevant peptide, (B) 6x excess irrelevant peptide and (C) 10x excess irrelevant peptide, respectively.

The staining intensity, as viewed in the microscope, is scored to (A) 3+ (with very few 1+), (B) .1.0-2.0+, and (C) 0.5-1.0+, respectively.

Additionally, the staining pattern and localization is the same for the three preparations.

In conclusion, by simple variation of the cross linker, specific target and irrelevant peptide concentrations during activation and coupling, the staining level can be controlled. The diagnostically relevant level can be adjusted to 0.5 -1.0+, which is expected to be a diagnostically desired threshold staining value for e.g. the p 16 target in cervical samples.

## 11. Use of p16 peptide as target and Horse Radish Peroxidase (HRP) Envision/DAB staining of pre-fixed intact sf9 cells, in a ThinPrep or Autocyte/TriPath cytological preparation

The cells being treated with DakoCytomation IntraStain™ and coupled with a mixture of the relevant double p16 motif peptide and an irrelevant Ki67 peptide.

200 million intact sf9 cells are isolated, prefixed and intrastain treated as in example 124. Activation is made with 20 µMolar 'GMBS', followed by reaction with 60 µMolar (3 x molar excess) L-Glycin, and final reaction with a premixed solution of 0.04 µMolar of peptide no 4 (MW 2201.3) and 0.4 µMolar irrelevant KI67 peptide (no 6, 1817.2 Da), containing a Cystein in the C-terminus.

The cells are treated as ThinPrep or manually treated Autocyte/TriPath preparation as described in the general section.

Both preparations are immunovisualized using the primary monoclonal antibody against p16 (clone p16 E6H4, DakoCytomation), as described in the general procedure.

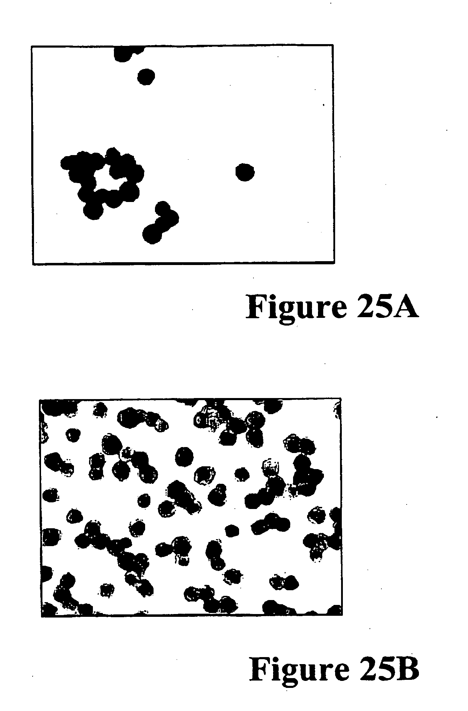

Figures 25A, B are photographs of the (A) ThinPrep or (B) Autocyte/TriPath treated and stained sf9 cells taken at 20 times magnification.

The ThinPrep preparation pictured in Figure 25A is highly stained and scored at 3-4+.

The Autocyte/TriPath preparation pictured in Figure 25B is scored at 0 - 2+, with some cells being scored to 2+ and some almost appear unstained.

The two methods of preparation gave very different overall appearance of cells and staining level.

In conclusion, the widely used ThinPrep or Autocyte/TriPath general methods of treating cytological samples can treat the reference material. The different procedures resulted in different staining levels and overall appearance for the model system chosen.

## 12. Use of HER2 neu peptide as target and fluorescent staining of whole prefixed Chinese Ovary Cells (CHO) cells evaluated by flowcytometry

30 million Chinese Ovary Cells (CHO) are isolated as described earlier in experimental 1 - except for the lysis step.

The cells are prefixed using 0.7 v/v % final concentration of para-formaldehyd in a PBS buffer, pH 7.2, and left for 15 minutes on ice before the activation with GMBS.

Activation is made with 1.0 $\mu$Molar 'GMBS', followed by reaction with 3.0 $\mu$Molar (3 x molar excess) L-Glycin to 'GMBS', and final reaction with 0.25 $\mu$Molar HER2/*neu* peptide (peptide no 3, epitope to Dakocytomation A0485 antibody, MW 1674.8), containing a Cystein in the C-terminus.

The cell population is stained and evaluated using a flowcytometer following the general flowcytometer procedures described previously.

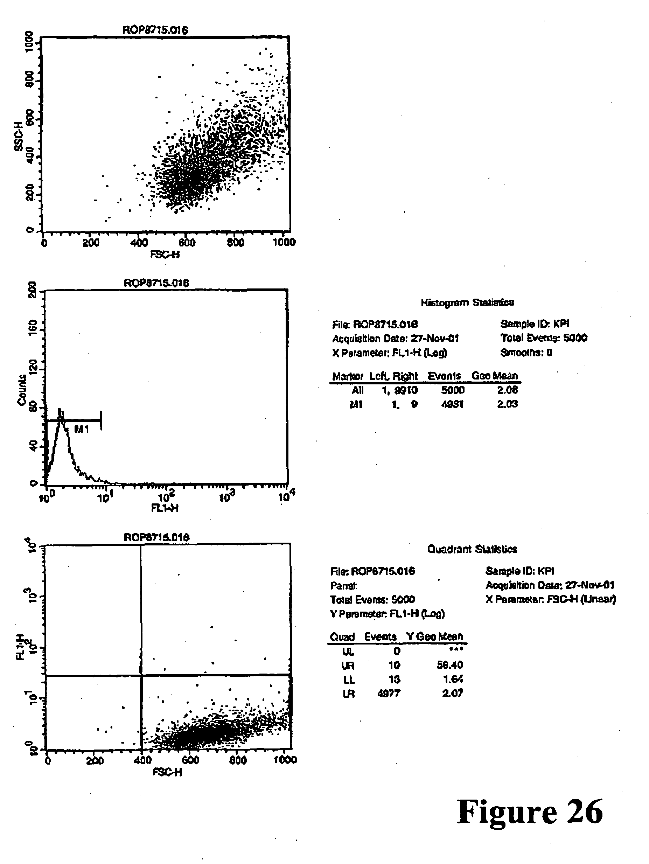

Figure 26A, B and C shows the unstained CHO control cells.

The FL1 channel fluorescence is very low (Figure 26C, lower right)

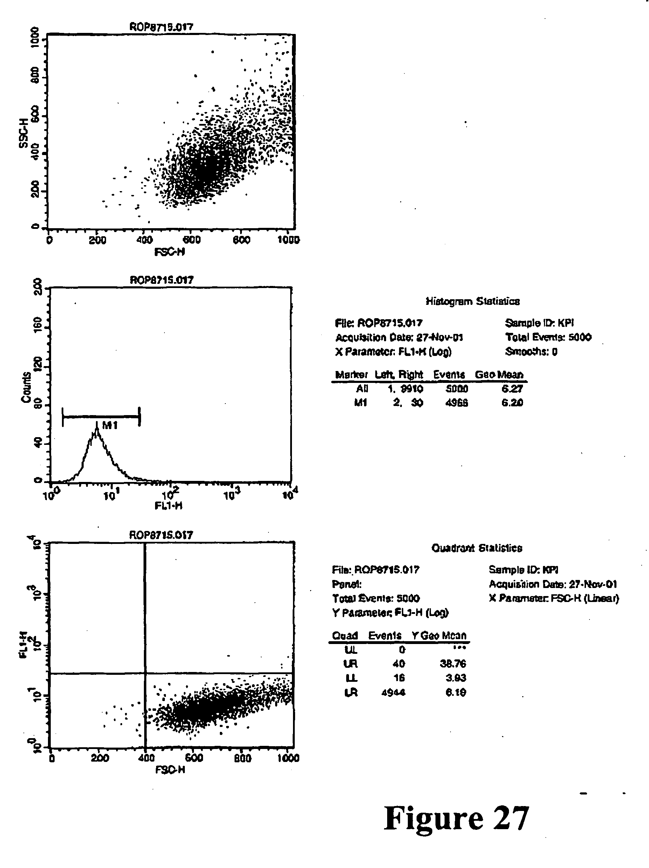

Figures 27A, B and C show the HER2 modified, CHO cells incubated with Rabbit F(ab)$_2$ negative control immunoglobulin pool (DakoCytomation X0929) and a fluorescein isothiocyanate (FITC) labelled Swine F(ab)$_2$ Anti-Rabbit Immunoglobulins/FITC (DakoCytomation F0054). ,

The FL1 channel fluorescence is very low (Figure 27C, lower right), indicating low unspecific background.

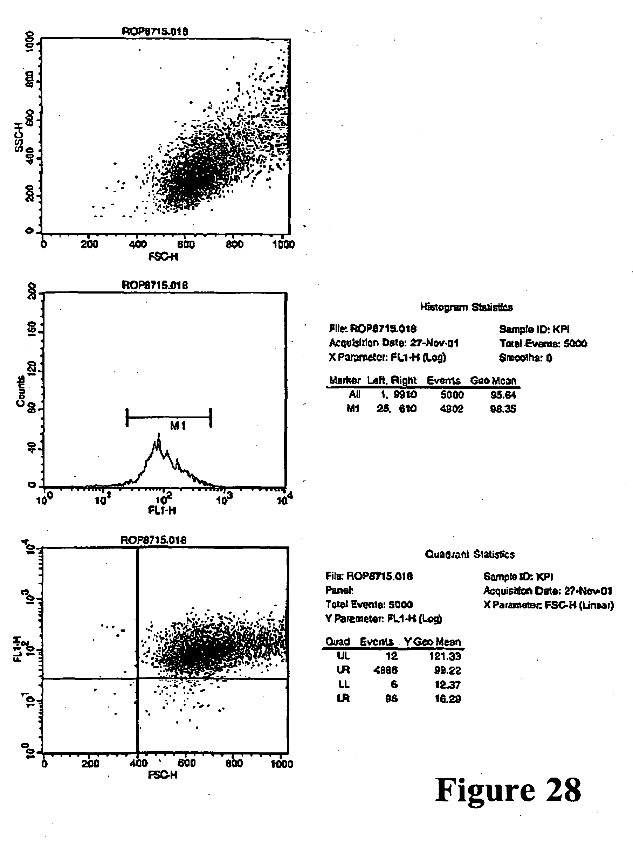

Figure 28A, B and C show the HER2 modified CHO cells incubated with Rabbit Anti-HER2/*neu* (DakoCytomation A0485) and Swine F(ab)$_2$ Anti-Rabbit Immunoglobulins/ FITC (DakoCytomation F0054).

Figure 28C (UpperRight) shows very high FL1 fluorescence from the HER2/neu peptide expression of positive cells immuno-labelled with Rabbit Anti-HER2/*neu* and Swine F(ab)$_2$ Anti-Rabbit Immunoglobulins/FITC.

In conclusion, the cells can be stained and evaluated by flowcytometry. The non-specific background is very low as indicated by the staining using non-specific antibody of the same origin as the specific antibody. Also, the auto fluorescence from the cells alone is low.

## 13. Use of the HER2 neu peptide as target and fluorescent staining of prefixed whole Chinese Ovary Cells (CHO) cells evaluated by flowcytometry

In this Example, the prefixation is milder and the amount of target peptide being lower than in the previous example. The homogenecity of the cell is compared.

20 million Chinese Ovary Cells (CHO) are isolated as described earlier in experimental 1- except for the lysis step. The cells is prefixed using 0.5 v/v % final concentration of para-formaldehyd in a PBS buffer, pH 7.2, and left for 15 minutes on ice before the activation with GMBS.

Activation is made with 1.0 $\mu$Molar 'GMBS', followed by reaction with 3.0 $\mu$Molar (3 x molar excess) L-Glycin to 'GMBS', and final reaction with 0.20 $\mu$Molar HER2/*neu* peptide (peptide no. 3, epitope to Dakocytomation A0485 antibody, MW 1674.8), containing a Cystein in the C-terminus.

The cell population is stained and evaluated following the general flowcytometer procedures described previously.

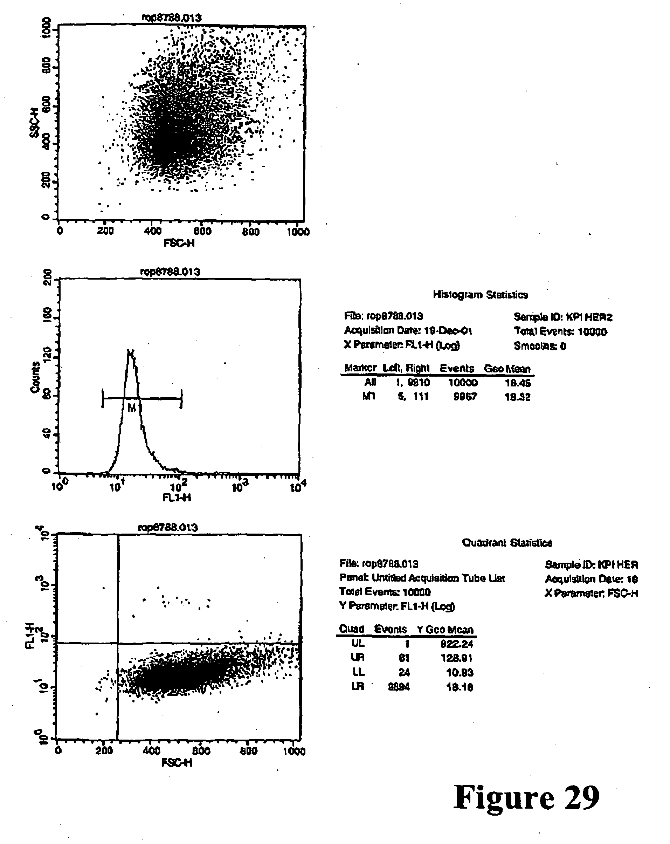

Figure 29 shows the unstained CHO control cells.

The FL1 channel fluorescence is very low (Figure 29C, lower right)

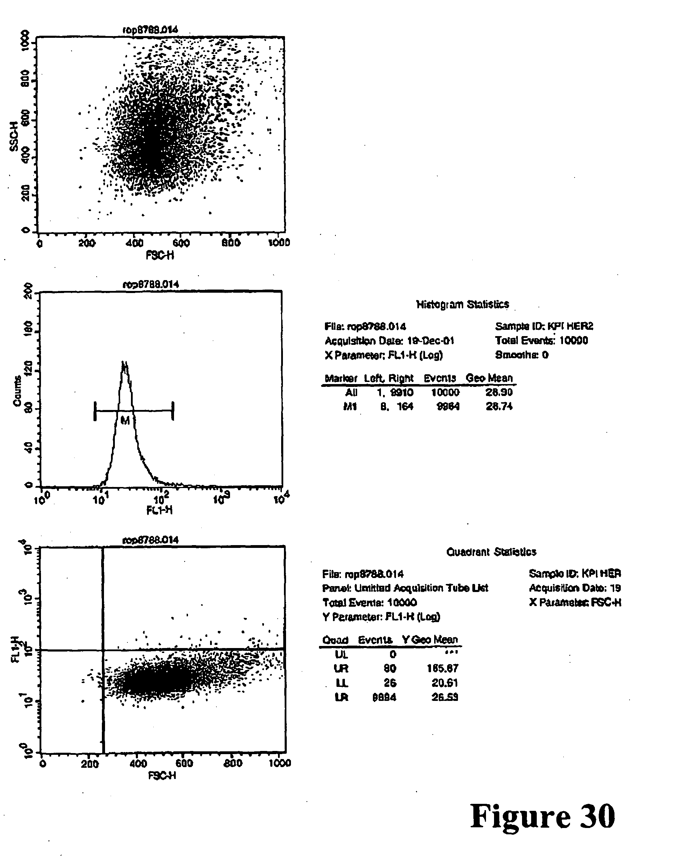

Figure 30A, B and C show the HER2 modified CHO cells incubated with Rabbit F(ab)$_2$ negative control immunoglobulin pool (DakoCytomation X0929) and a fluorescein isothiocyanate (FITC) labelled Swine F(ab)$_2$ Anti-Rabbit

Immunoglobulins/FITC (DakoCytomation F0054). The FL1 channel fluorescence is very low (Figure 30C, lower right), indicating low unspecific background as in the previous example.

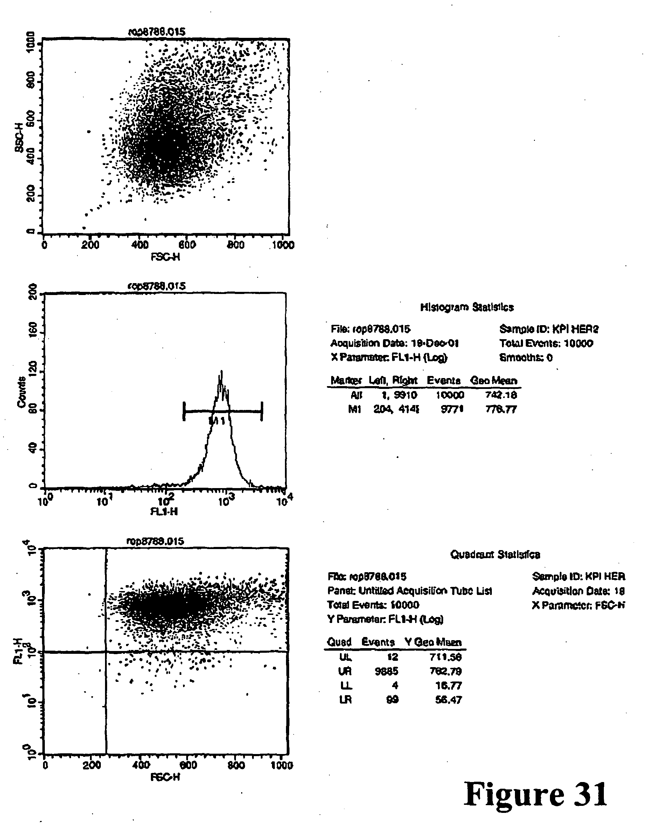

Figure 31A, B and C show the HER2 modified CHO cells incubated with Rabbit Anti-HER2/*neu* (DakoCytomation A0485) and Swine F(ab)$_2$ Anti-Rabbit Immunoglobulins/ FITC (DakoCytomation F0054).

Figure 31C (UpperRight) shows high FL1 fluorescence from the HER2/neu peptide expression of positive cells immuno-labelled with Rabbit Anti-HER2/*neu* and Swine F(ab)$_2$ Anti-Rabbit Immunoglobulins/FITC.

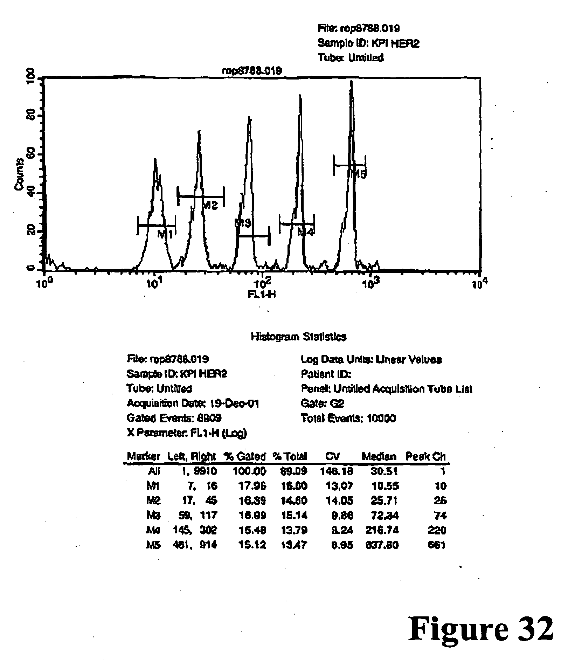

Figure 32 shows the histogram from a flow set of calibration microbeads used to ensure quality assurance and reduce variability of flow cytometry data.

Compared to the previous example (example 13), the auto fluorescence and non specific background are at about the same low level. The specific signal is somewhat higher. This indicates significant influence from the prefixation conditions on the final fluorescent signal strength.

The forward and side scatter plots (Figure 29A, 30A, 31 A) indicate a more inhomogeneous cell population with respect to size and granularity than in the preparation using a higher concentration during the prefixation step (Figure 26A, 27A, 28A).

## 14. Use of the HER2 neu peptide as target and fluorescent staining of whole prefixed Chinese Ovary Cells (CHO) cells evaluated by flow cytometry

Different cross linker concentrations is used during activation. A non-relevant peptide is included.

In short, about 20 million CHO Cells are isolated as described earlier in experimentals A and B, and activated with a differentiated level of 'GMBS' (0.04 µM, 0.20 µM, and 1.00 µM, respectively), reacted with 3 x molar excess L-Glycin to 'GMBS' and finally reacted with 0.04 µM HER2/neu peptide (peptide no. 3, epitope to Dakocytomation A0485 antibody) or irrelevant HER3 peptide (peptide no. 7, Mw 1617 Da) both containing a Cystein in the C-terminus.

The cell populations is stained and evaluated following the general flowcytometer procedures described previously.

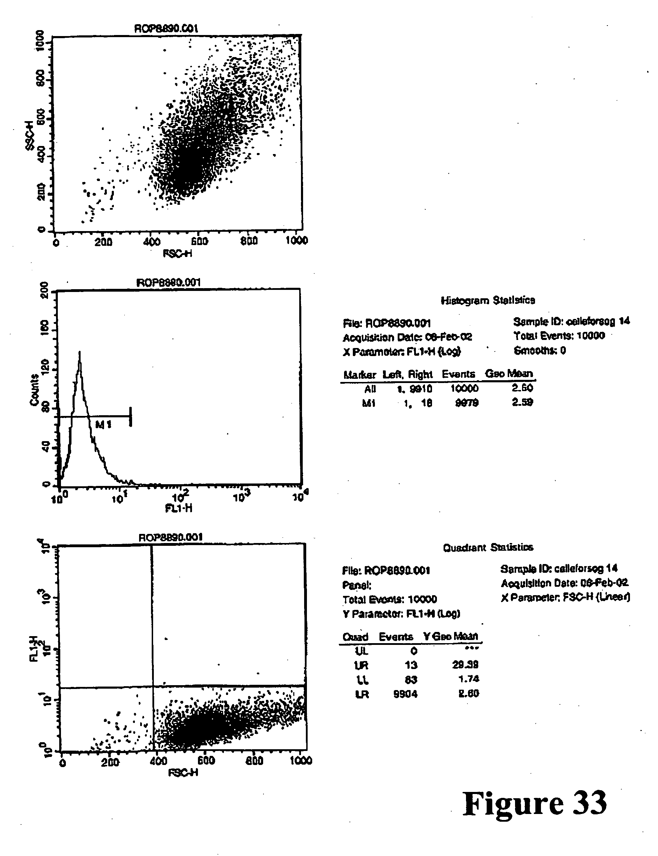

Figure 33 shows the unstained CHO control cells prepared using the high GMBS level (1.00 µM) and the HER2 target. The FL1 channel fluorescence is low (Figure 11C, lower right)

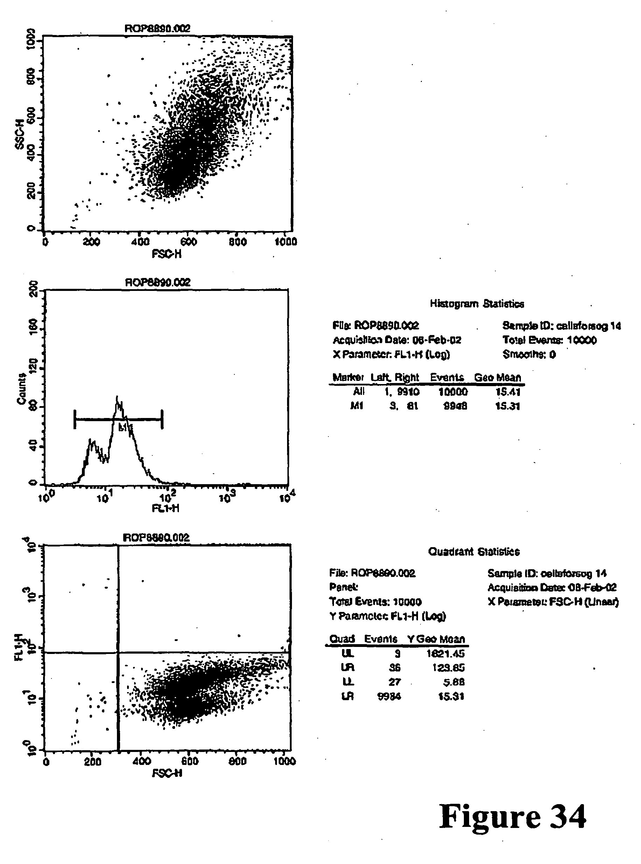

Figure 34 shows CHO cells prepared using the high GMBS level (1.00 µM) and the HER2 target. Incubated with Rabbit F(ab)$_2$ negative control immunoglobulin pool (DakoCytomation X0929) and a fluorescein isothiocyanate (FITC) labelled Swine F(ab)$_2$ Anti-Rabbit Immunoglobulins/FITC (DakoCytomation F0054)

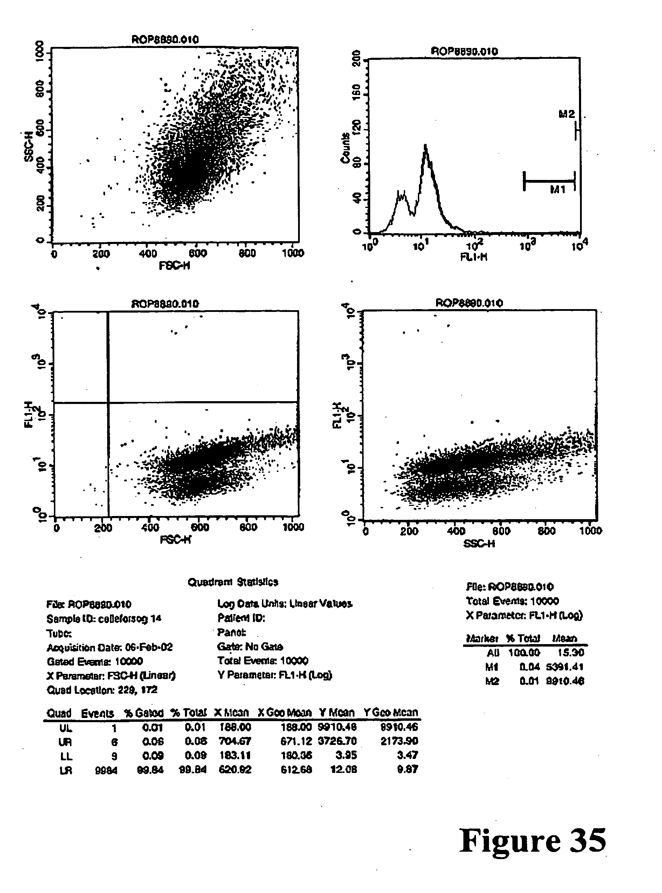

Figure 35 shows low fluorescence from CHO cells, prepared using the high GMBS level (1.00 µM) and the irrelevant HER3 target and directly immuno-labelled with fluorescein isothiocyanate (FITC) labelled Rabbit antibody, specific for the HER2/*neu* protein receptor (DAKOCYTOMATION).

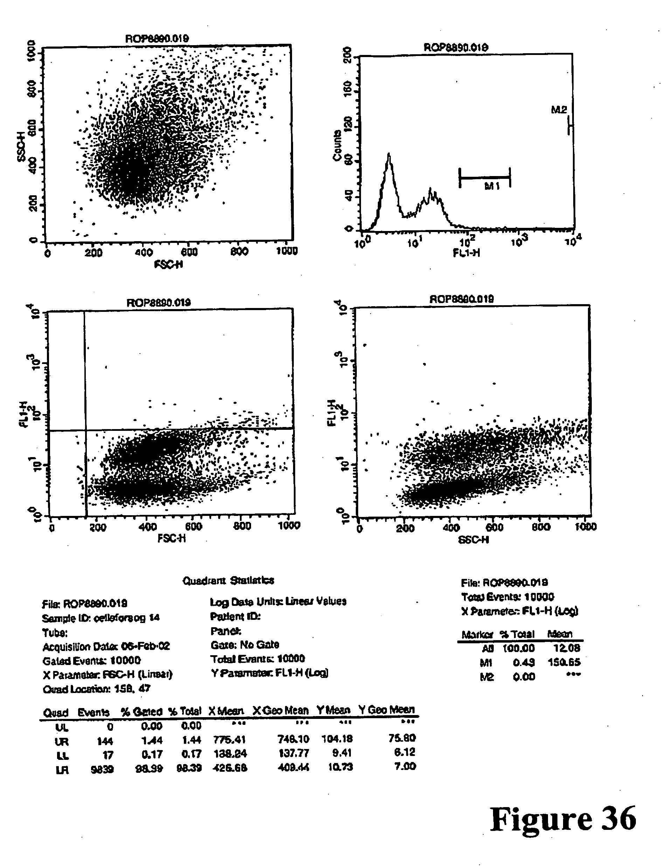

Figure 36, 37 and 38 shows the data from the flowcytometer after directly immuno-labelling of the three different HER2 modified cell populations (0.04 µM, 0.20 µM, and 1.00 µM GMBS, respectively), with fluorescein isothiocyanate (FITC) labelled Rabbit antibody, specific for the HER2/*neu* protein receptor (FITC labelled Dakocytomation A40485).

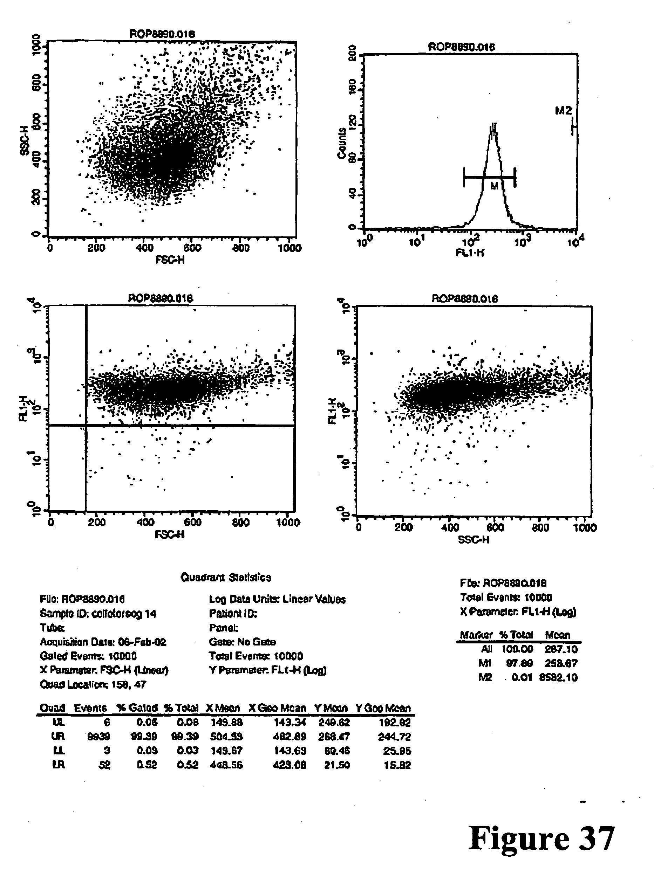

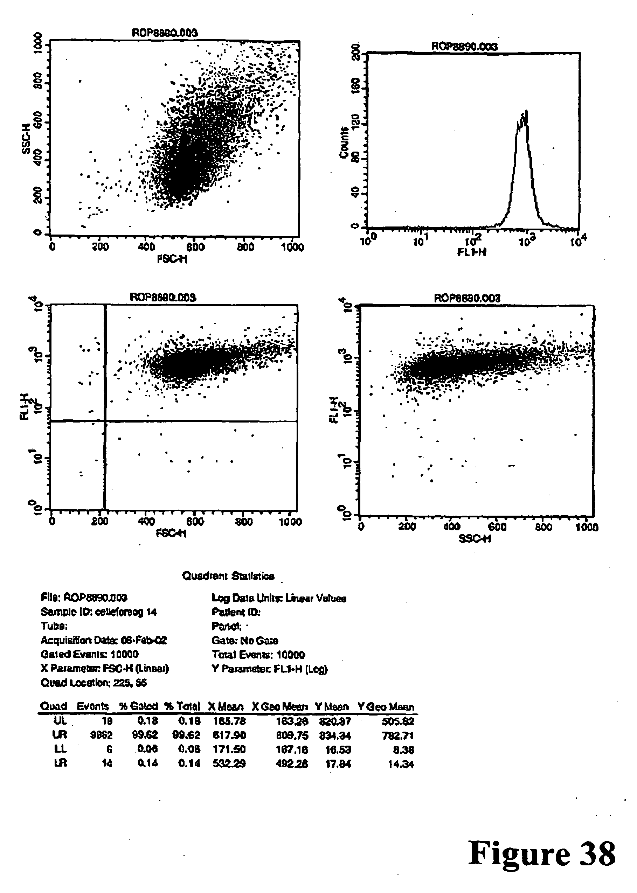

In summary, the Figure 36 shows low, Figure 37 shows medium and Figure 38 shows high staining signal.

In conclusion, the concentration of the crosslinker during activation can be used to adjust the resulting specific fluorescent staining signal. This is important for obtaining reference cells with various staining intensities.

Autofluorescence is low as illustrated in Figure 11. The non-specific signal is low, as illustrated in Figure 12 and 13.

Though, it can clearly be seen that the populations seems to be divided in two (Figure 34 and 35).

## 15. Use of the HER2/neu peptide or phosphylated HER2 as target and fluorescent staining of whole prefixed sf9 cells evaluated by flowcytometry

40 million Sf9 Cells are isolated as described earlier, pre-fixed using 0.50 v/v % final concentration of para-formaldehyde, activated with 2.0 µM 'GMBS', reacted with 3 x molar excess L-Glycin to 'GMBS' and finally divided into two populations and reacted with either (A) 0.08 µM HER2/neu peptide 'SP991729' (peptide no. 3, 1676.9 Da, epitope to Dakocytomation A0485 Rabbit antibody) or (B) 0.08 µM PhosphoTyrosine containing HER2/neu peptide 'NVP-ABN-379-A1-1' (peptide no. 8, Mw 1753.8 Da, epitope to Dakocytomation DAK-H2-PY-1248 Monoclonal antibody), both containing a cystein in the C-terminus.

The two cell populations is stained and evaluated following the general flowcytometer procedures described previously.

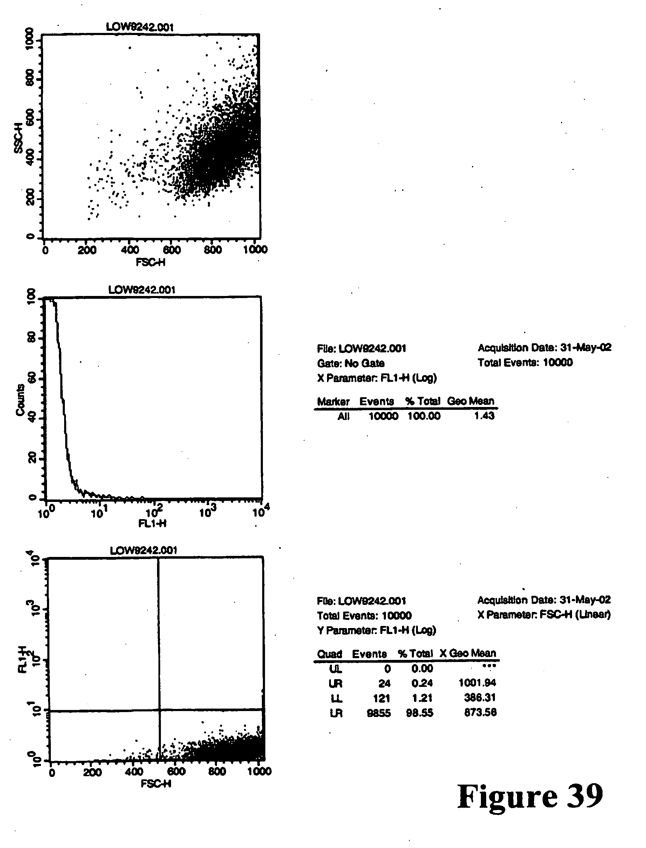

Figure 39 shows the unstained HER2 Sf9 control cells (population A).

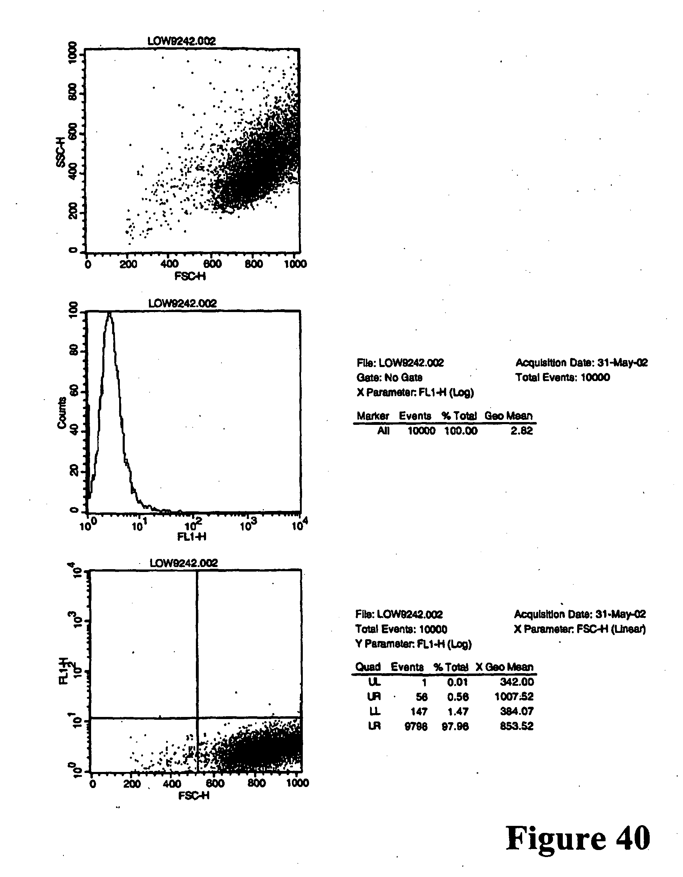

Figure 40 shows the HER2 Sf9 control cells (population A) incubated with Rabbit negative control immunoglobulin pool (DakoCytomation X0903) and Swine F(ab)$_2$ Anti-Rabbit Immunoglobulins/FITC (DakoCytomation F0054).

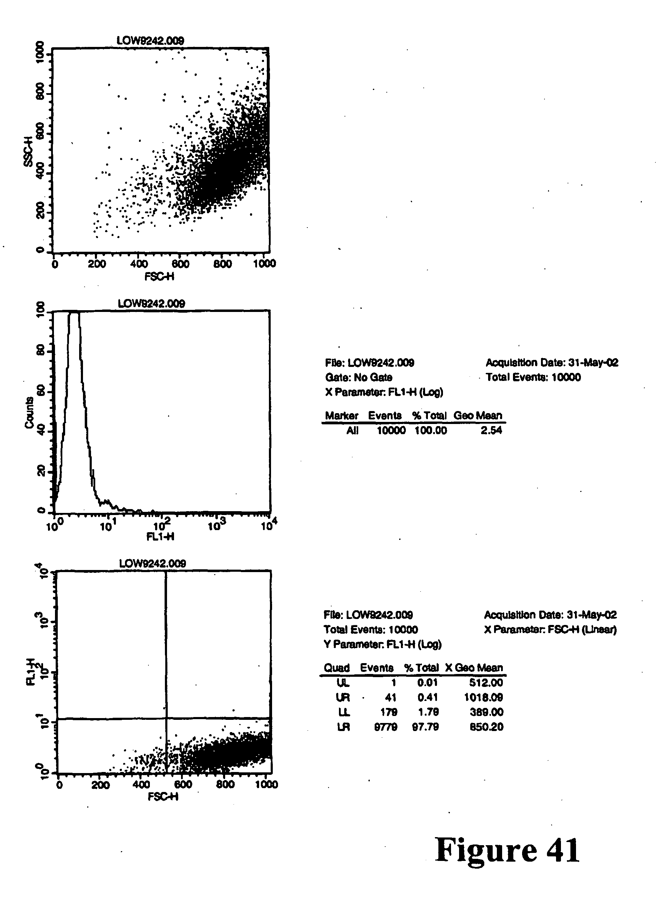

Figure 41 shows the unstained PhosphoHER2 peptide Sf9 control cells (population B).

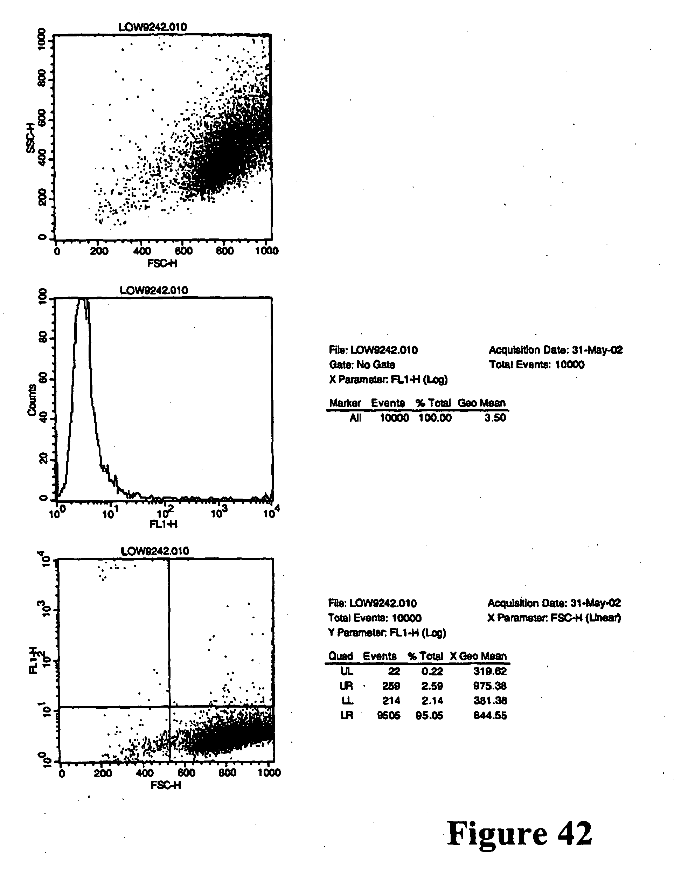

Figure 42 shows the PhosphoHER2 peptide Sf9 control cells (B) incubated with Mouse IgG1 negative control immunoglobulin (Dakocytomation X0931) and Rabbit F(ab)₂ Anti-Mouse Immunoglobulins/FITC (Dakocytomation F0313).

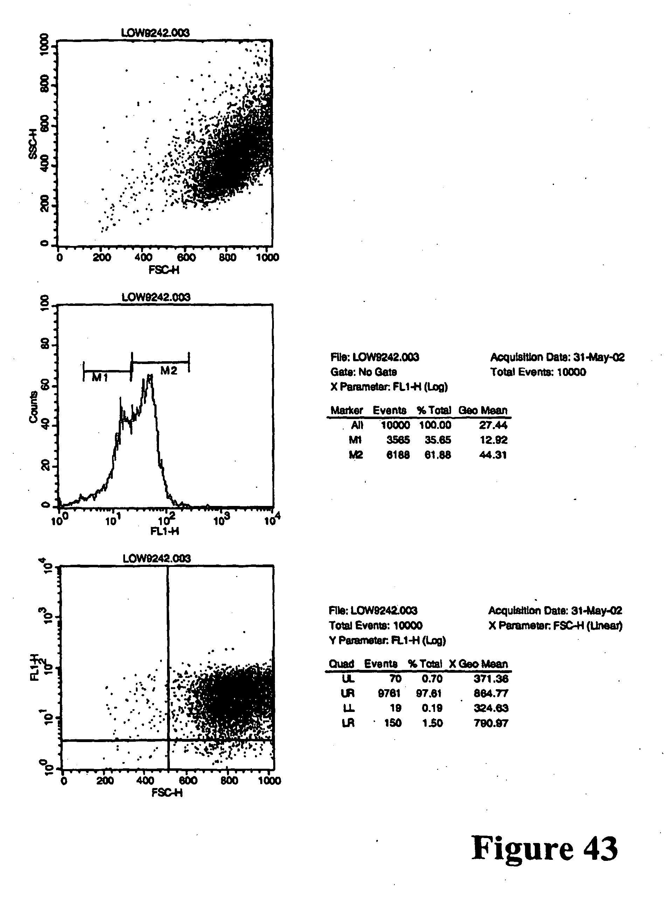

Figure 43 shows the specific staining of the HER2 Sf9 control cells (population A).

Figure 43A shows the distribution of cells in Forward Scatter (FSC-H) vs. Side Scatter (SSC-H) dot blot and Figure 43B shows two populations with different expression levels of the HER2/neu peptide.

Figure 43C shows high fluorescence from the HER2/*neu* peptide expression of positive cells incubated with Rabbit Anti-HER2/*neu* (Dakocytomation A0485, 0.1 g/L) and Swine F(ab)₂ Anti-Rabbit Immunoglobulins/ FITC (Dakocytomation F0054).

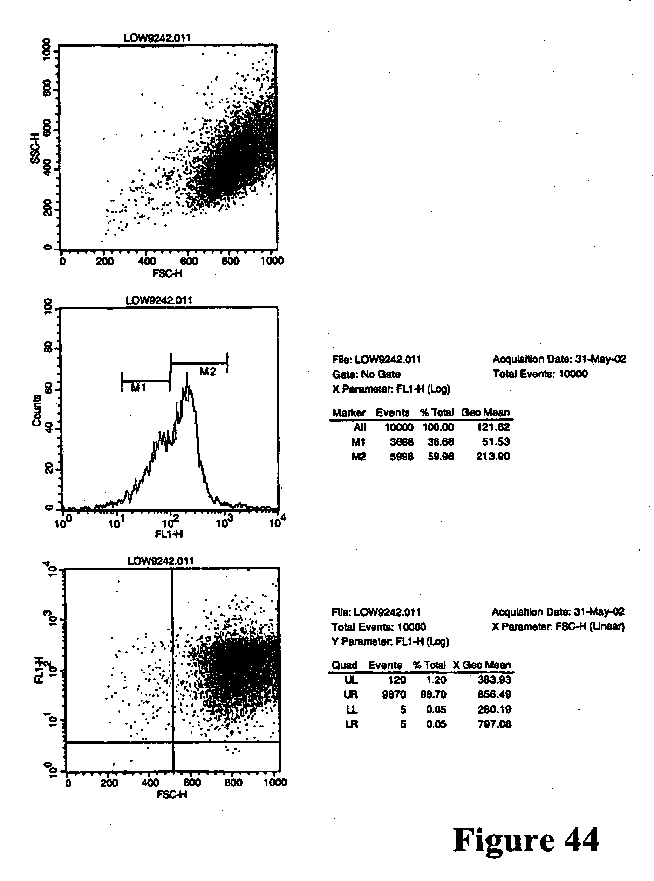

Figure 44 shows the specific staining of the PhosphoHER2 peptide Sf9 control cells (B).

Figure 44A shows the distribution of cells in Forward Scatter (FSC-H) vs. Side Scatter (SSC-H) dot blot and Figure 44B shows two populations with different expression levels of the PhosphoTyrosine containing HER2/neu peptide.

Figure 44C shows high fluorescence from the phosphylated HER2 peptide expression of positive cells incubated with Monoclonal Anti-Phosphorylation-State-Specific HER2-PY1248 (Dakocytomation DAK-H2-PY-1248, 0.1 g/L) and Rabbit F(ab)₂ Anti-Mouse Immunoglobulins/ FITC (Dakocytomation F0313).

The auto fluorescence and non-specific staining are low (Figure 39, 40, 41 and 42). The specific staining illustrates the possibility of specifically staining the two HER derivates, which both and together has diagnostic interest.

## 16. Use of FITC as target and Horse Radish Peroxidase (HRP) EnVision/DAB staining of artificial PEGA cells, in a FFPE preparation

In this example, man-made artificial resins are used as reference system. The resin is an FITC modified amino polyethylene glycol (PEGA) resin. The resin has a cell like shape and is characterized by it special ability to swell in both organic and aqueous solvents.

The Amino PEGA resin (Amino polyethylene glycol resin, Novabiochem, San Diego, USA, code No. 01-64-0100) appears as lightly yellow spherical particles. The modification with FITC (fluorescein-5-isothiocyanate, 'Isomer I', Molecular Probes code No. F-1906), is done by direct coupling to a portion of the amino groups in an organic solvent.

In short, the modification is done by washing 1.0 g of the PEGA resin 3 times with Toluene (LabScan, Denmark, code No. H6518, 2 minutes, 10 ml/gram room temperature), followed by reaction with 1 mM or 10 mM FITC (1% in NMP, LabScan, Denmark) and 10mM Triethylamine (Aldrich code No. 13,206-0).

After 17 hours mixing by turning upside down, the reactions are stopped by washing 5 times with water. In parallel, unmodified PEGA resin is also washed.

The modified and unmodified washed PEGA resin are embedded separately in an agarose solution (2% agarose by weight, HSA 1000 protein grade, in 0,05% PEI (Fluka code No. 03880) TBS solution (DakoCytomation, K5325, pH 7.5, in 1 liter water) and 100 mg resin is well mixed 130μl water and furthermore added 400μl 60-62°C warm agarose solution and quickly hereafter drawn up in a 1ml syringe. After the agarose has cooled down, the tip of the syringe is cut of with a sharp knife and the solidified gel is gently spurting into water.

The gel pieces are wrapped in microscope lens cleansing paper as described in general previously.

Fixation, dehydration and some part of the paraffin embedding are made automatically in an automatic tissue processor Shandon Excelsior (Thermo Shandon, Ax-Lab, Vedbaer, Denmark). The histocapsules with the gel embedded resin are transferred into the chamber and fixated for 4 hours in neutral buffered formalin (3.7 % in PBS) at room temperature. Dehydrated in 6 steps with increasing amount of ethanol, from 70% ethanol to 99,9% ethanol, each step takes 30 minutes with vacuum at 30°C. Afterwards, the gel embedded resin is treated 3 times 20 minutes with xylene with vacuum at 30°C. To finish the process, it is treated 3 times 80 minutes with 60-62°C melted paraffin with vacuum. When finished, it is left in 60-62°C melted paraffin overnight.

The histocapsules with the resin are transferred to fresh 60-62°C melted paraffin and embedded in paraffin blocks as described in general above.

The FFPE block of the FITC modified or unmodified PEGA resin is cut, mounted on Poly-L-Lysin coated microscope glass (Electron Microscopy Sciences, code No. 63410-01), and deparaffinated as described in general above.

The immunovisualisation of the FITC modified PEGA resin as well as the unmodified PEGA resin is done as previously described in the general procedure, included controls of primary antibody, the visualisation system and the resin. In summary, the primary polyclonal antibody (F(ab')) is Rabbit anti-FITC-HRP (DakoCytomation code No. P5100, dilution 1:50), negative control of primary antibody is Rabbit IgG (DakoCytomation code No. X0936, dilution 1:1000). Followed by Goat anti-Mouse/Goat anti-Rabbit immunoglobulins and horseradish peroxidase labelled dextran polymer (DakoCytomation code No. K5007) and for negative control of the visualisation system Goat anti-Mouse immunoglobulins and horseradish peroxidase labelled dextran polymer (DakoCytomation code No. K4006). Finally a diaminobenzidine chromogenic substrate system (DakoCytomation code No. K5007).

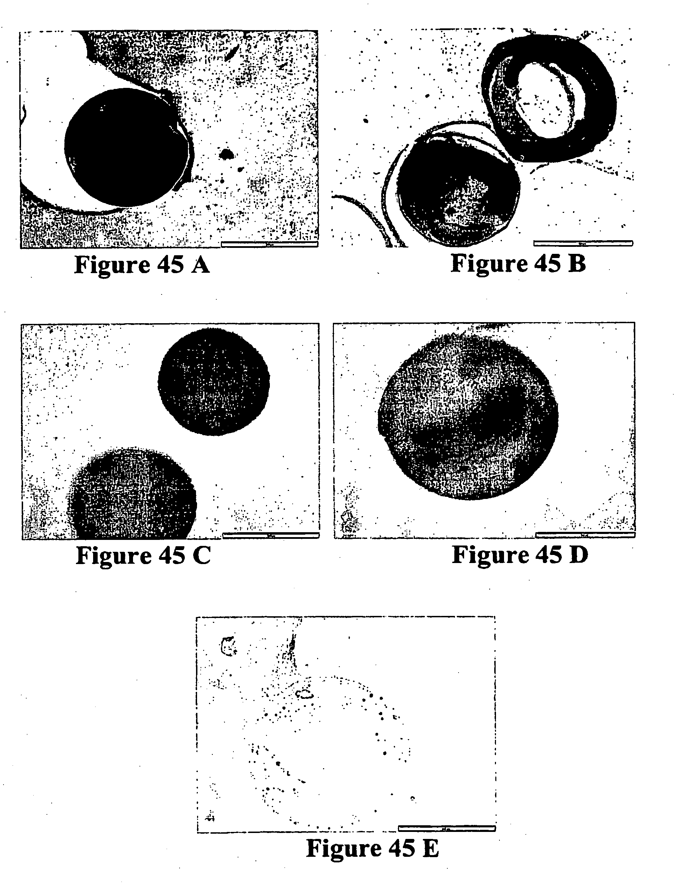

Figure 45A, 45B and 45C are photomicrographs of the DAB stained PEGA resins in a bright field microscope, all taken at 20 times magnification.

Figure 45A and 45B is the DAB stained PEGA resin coupled with 1 mM and 10 mM FITC, respectively.

Figure 45C and 45D is the stained PEGA using a negative control primary antibody and the negative En Vision control, respectively, both on the highly FITC modified PEGA resin.

Figure 45E is the unmodified; PEGA resin DAB stained with anti Fitc and En Vision.

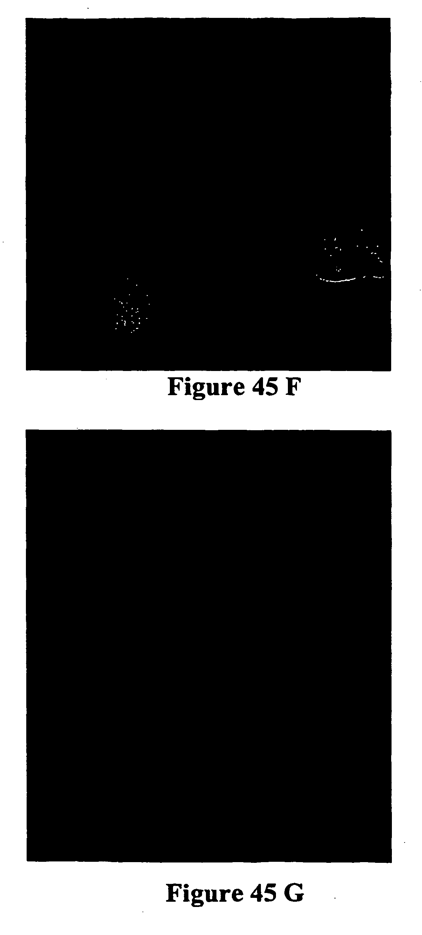

Figure 45F is a representative of photomicrographs of 10mM FITC modified resin (the same as Figure 45B) and Figure 45G is the unmodified resin, both taken in a fluorescent microscope, at 16 times magnification.

In general, an immonustaining is observed throughout the individual resin. Some resin beads appears to be hollow in the centre. The staining intensity is scored to 1½-2 in Figure 45A (1mM FITC) and scored to 2-2½ in Figure 45A (10mM FITC).

The negative control of antibody and control of secondary visualisations system are both scored to ½ and the staining intensity of the unmodified resin is scored to 0. This indicates a very low general background due to the resin and the modification.

Further, the fluorescence pictures show some inhomogeneous modification of the resin. The unmodified resin had very low auto fluorescence.

In conclusion, it is possible to use an artificial cell-like resin and modified it with a well-known general hapten, immunovisualises it with a DAB staining and evaluate in bright field light microscope.

The staining intensity is clearly graded for the high and low degree of hapten modification (Figure 45A and 45B), and indicates the possibility to make a reference material with a low staining intensity, which is clearly distinguishable from the background.

The material could be useful for validation of the visualizations system or the entire manual or automated staining process.

**Example 17. Use of Rabbit immunoglobulin as target and Horse Radish Peroxidase (HRP) EnVision/DAB staining of artificial agarose cells, in a FFPE preparation**

In this example, a man-made artificial carbohydrate matrix is used as a control of the visualisations system. The matrix is a Rabbit IgG modified divinyl sulfone activated matrix of spherical agarose beads, which has a cell like shape.

In short, the modification of the amino-, hydroxyl- and thiol reactive vinyl sulfone activated matrix (Mini Leak, Kem-En-T'ec, Copenhagen, code No.1012) with Rabbit IgG (DakoCytomation code No. X0936) is done as a direct coupling in aqueous buffer.

In more detail, the modification is done by washing 1.7 g of the Mini Leak matrix 3 times with water (2 minutes, 10 ml/gram room temperature), followed by reaction with 0,65 mg (0.50 mg/ml matrix) or 1,95 mg (1.50 mg/ml matrix) dialysed Rabbit IgG (DakoCytomation Code no. X0936, 0,1M NaCl, some Carbonate at pH 9,0).

After 17½ hours at room temperature mixing by turning upside down, is it quenched with Ethanolamin (in all 0.010 M, pH 9.0) After 30 minutes, the matrix is washed 3 times in water (2 minutes, 10 ml/gram room temperature). In parallel, unmodified Mini Leak matrix is also washed in water.

The two IgG modified and one unmodified washed Mini Leak matrix are embedded separately in an agarose solution as described above.

The gel pieces are wrapped in microscope lens cleansing paper as described in general above.

Fixation, dehydration and some part of the paraffin embedding are made automatically in an automatic tissue processor Shandon Excelsior as previously described.

The histocapsules with the matrix are transferred to fresh 60-62°C melted paraffin and embedded in paraffin blocks as described in general above.

The FFPE block of the Rabbit IgG modified or unmodified Mini Leak matrix is cut, mounted on Poly-L-Lysin coated microscope glass (Electron Microscopy Sciences, code No. 63410-01), and deparaffinated as described in general above.

The immunovisualisation of the Rabbit IgG modified Mini Leak matrix as well as the unmodified Mini leak matrix is done as previously described in the general procedure, included controls of the visualisation system and the matrix. In summary, no primary antibody is used. Visualisation by Goat anti-Mouse/Goat anti-Rabbit immunoglobulins and horseradish peroxidase labelled dextran polymer (DakoCytomation code No. K5007) and for negative control of the visualisation system Goat anti-Mouse immunoglobulins and horseradish peroxidase labelled dextran polymer (DakoCytomation code No. K4006). Finally a diaminobenzidine chromogenic substrate system (DakoCytomation code No. K5007).

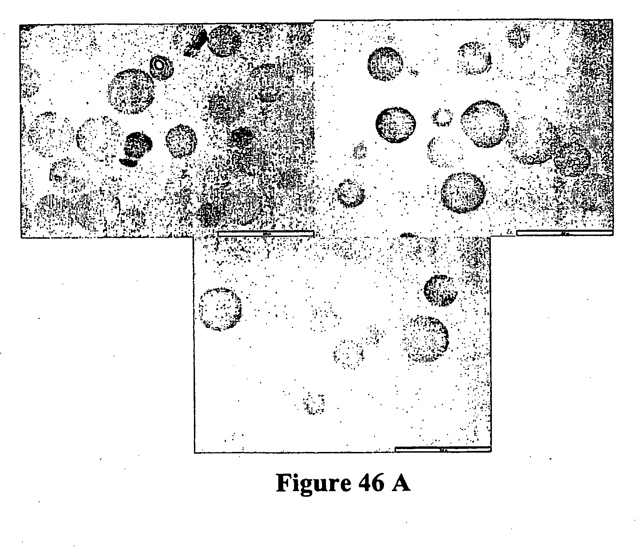

Figure 46, 47 and 48 are all photomicrographs of the DAB stained Mini Leak as viewed in a bright field microscope.

Figure 46A and 46B is representative photomicrographs of the DAB stained Mini Leak matrix modified with 0.5 mg Rabbit IgG/ml, taken at 10 and 20 times magnification, respectively.

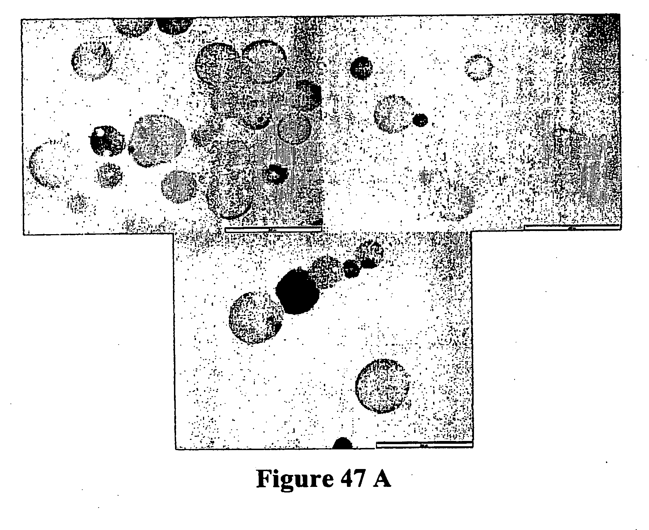

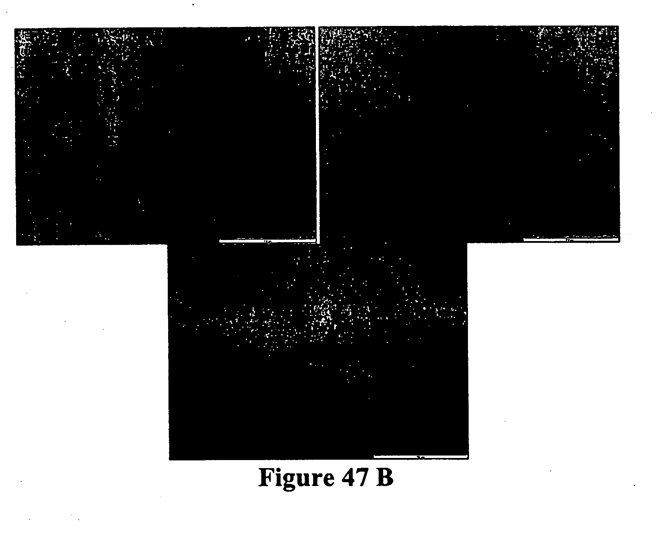

Figure 47A and 47B is representative photomicrographs of the DAB stained Mini Leak matrix modified with 1.5 mg Rabbit IgG/ml, taken at 10 and 20 times magnification, respectively.

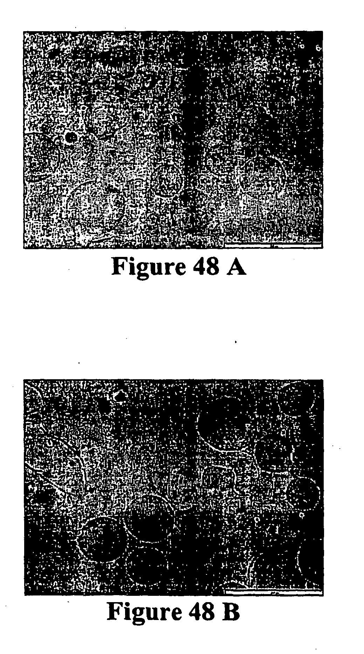

Figure 48A is the EnVision negative control using goat anti mouse conjugates, and Figure 48B is a photomicrograph of the EnVision HRP and DAB stained unmodified Mini Leak matrix, all taken at 10 times magnification.

The staining intensity is scored to ½-1 in figure 46 (0,5mg/mi matrix Rabbit IgG) and ½-1 scored in Figure 47 (1,5mug Rabbit IgG mg/ml matrix). The overall staining intensity is estimated at 10 time magnification.

An immunostaining is observed throughout the individual matrix. The low score matrix (Figure 47) appear to have few beads with higher staining intensity.

The staining intensity is slightly higher on the outer surface or edge than in the interior of the beads. The different sizes of individual beads are due to both the cutting process and could be due to uneven size distribution of the original beads. A few beads have small cavities in the middle. No significant debris is seen and the beads all appear spherical, indicating a good quality of paraffin infiltration and subsequent cutting.

The control of visualisations system is scored to 0 and control of the matrix is scored to less than ½, Figure 48A and 48B, respectively.

In conclusion, it is possible to use a simple affinity material as artificial cell-like reference and modified it with a Rabbit immunoglobulin, immunovisualises it with a DAB staining and evaluate in bright field light microscope.

The native and unmodified matrix gave very low background staining intensity due to the nature of the matrix.

The above reference material could be useful for validation of the functionality of the secondary visualization system. Furthermore, the staining intensity is low and in the relevant level for many IHC diagnostic kits.

**18. Use of HER2 peptide as target and Horse Radish Peroxidase (HRP) EnVision/DAB staining of artificial cells, in a FFPE preparation**

In this example, the mini leak matrix is coupled with HER2 peptide and an irrelevant peptide to obtain a specific staining to be used as a reference of the primary reagent.

The modification of the Mini Leak matrix with HER2 peptide (peptide no 3, epitope to DakoCytomation A0485 antibody) and a p16 peptide (peptide no. 4) is done by direct coupling between the thiol containing peptide and the vinylsulfon-activated matrix in an aqueous solvent.

The p16 peptide acts as an irrelevant peptide in this example. The concentration of target peptide is varied and the total concentration of peptides is constant for the couplings.

In more detail, the 6 different modifications is done by washing 0,85 g of the Mini Leak matrix 2 times in 50mM HEPES, 2,5 mM EDTA pH 8 (20 minutes, 10 ml/gram room temperature), followed by reaction with

a) 0,0 g/l HER2 and 0,0g/l P16

b) 0,01g/l HER2 and 0,19g/l P16,

c) 0,05g/l HER2 and 0,15g/l P16,

d) 0,10g/l HER2 and 0,10g/l P16,

e) 0,15g/l HER2 and 0,05g/l P16 or

f) 0,19g/l HER2 and 0,01g/l P16, respectively

In total a combined peptide concentration of 0,20 g/l, in a Hepes buffer (100mM HEPES, 5mM EDTA, 5% DMF, pH 8,0)

After 17 hours at room temperature mixed by shaking, the beads are quenched with 1/10 volume 0,1M Ethanolamin pH 9,0. After 30 minutes, the matrix is washed 2 times in. 50mM HEPES, 2,5M EDTA pH 8,0 (20 minutes, 10 ml/gram room temperature). In parallel, unmodified Mini Leak matrix is also washed.

The modified and unmodified washed Mini Leak matrix are embedded separately in an agarose solution, and the gel pieces are wrapped in microscope lens cleansing paper as described previously.

Fixation, dehydration and the first part of the paraffin embedding are made automatically in an automatic tissue processor Shandon Excelsior as previously described, however is was fixated in 0,4% NBF instead of a 3,7% NBF fixation.

The histocapsules with the matrix are transferred to fresh 60-62°C melted paraffin and embedded in paraffin blocks as described in general above.

The FFPE block of the HER2 peptide modified or unmodified Mini Leak matrix is cut, mounted on Poly-L-Lysin coated microscope glass, and deparaffinated as described in general above.

The immunovisualisation of the HER2 peptide modified Mini Leak matrix as well as the unmodified Mini leak matrix is done manually according to the protocol of the HercepTest™ (DakoCytomation code no. K5205).

The immonuvisualisation include control of the primary antibody, as well as of the visualisation system, the coupling, the matrix and a control slide with different HER2 reference cell lines supplied with the HercepTest™ kit.

In summary, the primary antibody is Rabbit anti-Human HER2 protein and Rabbit IgG is used as negative control. Visualisation done by Goat anti-Rabbit immunoglobulins and horseradish peroxidase labelled dextran polymer. Negative control of the visualisation system is done by a Goat anti-Mouse immunoglobulins and horseradish peroxidase labelled dextran polymer (DakoCytomation code No. K4006). Finally a diaminobenzidine chromogenic substrate system is used.

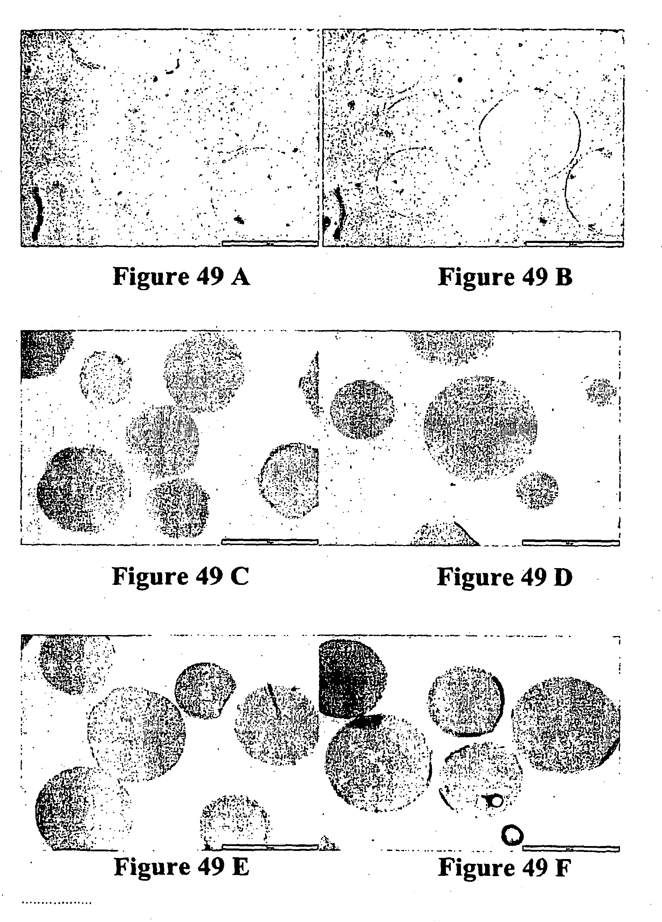

Figure 49A to Figure 49F are all photomicrographs of the DAB stained Mini Leak coupled with (a) 0,0 g/l, (b) 0,01g/l, (c) 0,05g/l, (d) 0,10g/l, (e) 0,15g/l or (f) 0,19g/l HER2 target peptide, respectively. All photomicrograph were taken at 20 times magnification in a bright field microscope.

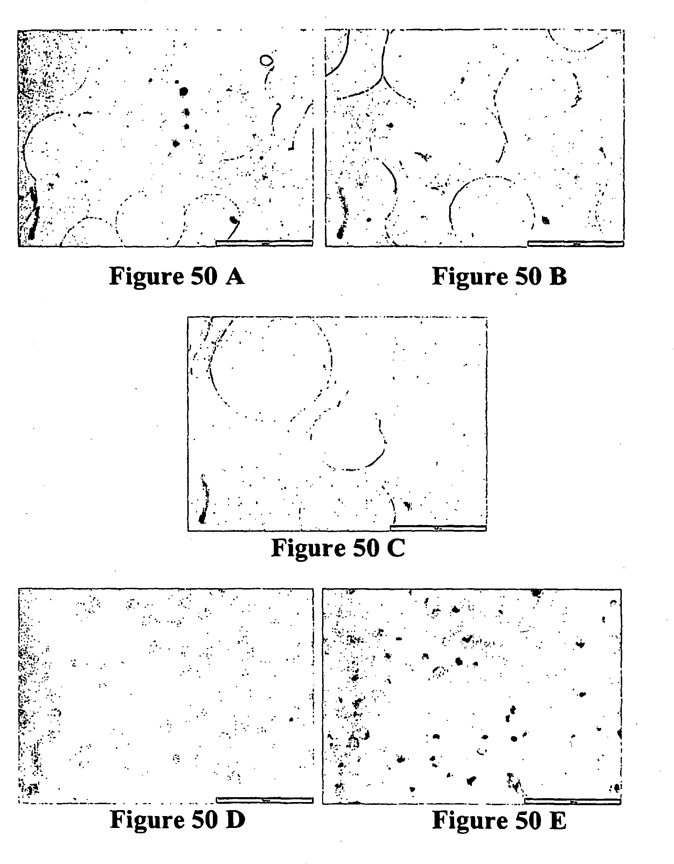

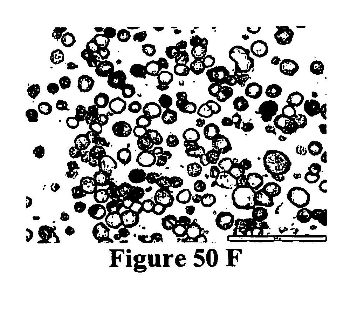

Figure 50 is the various control stainings: Negative primary antibody control (Figure 50A) and Envision negative control (Figure 50B), both on 0.19 g/l HER2 mini leak matrix. Figure 50 C is the unmodified Mini leak matrix stained with the Herceptest. Figure 50 D, E and F is the HercepTest™ control slides.

An immonustaining very similar to a homogeneous cytoplasmic staining is observed throughout the positive stained individual matrix cells.

The staining intensity is scored to 0+ in Figure 49B (0,01g/l HER2) and approximately 1+ in Figure 49 C, D, E and F (0,05-0,19g/l HER2). The four 1+ slides had increasing numbers of stronger intensity stained individual cells, but the overall staining score was approximately 1+.

The control of the coupling, the visualisations system, the antibody and the matrix are all scored to 0+ in Figure 50 A, B and C. The HercepTest™ control slide is scored to 0, 1+ and 3+ as specified in the kit instructions.

The native and unmodified matrix gave very low background staining intensity due to the nature of the matrix.

In conclusion, it is possible to use the artificial cell-like matrix and modified it with a small target as HER2 peptide in different concentration, immunovisualises it with a DAB staining and evaluate in bright field light microscope. The level of peptide concentration during the covalent modification of the matrix gave graded immuno staining.

### 19. Use of p16 peptide as target and Horse Radish Peroxidase (HRP) EnVision/DAB staining of artificial cells, in a FFPE preparation

In this example, the mini leak matrix is coupled with a p 16 peptide and an irrelevant HER2 peptide similar to the previous example.

The modification of the Mini Leak matrix with p 16 peptide and an irrelevant peptide (HER2), and the FFPE preparation is described in the previous example.

The immunovisualisation of the p16 peptide modified Mini Leak matrix as well as the unmodified Mini leak matrix is done manually according to the protocol of the CINtec™ p16$^{INK4a}$ cytology kit (DakoCytomation code no. K5339).

The immonuvisualisation is included control of the primary antibody, as well as of the visualisation system, the coupling and the matrix.

In summary, the primary antibody is Mouse Anti-Human P16$^{INK4a}$ and the negative control is Mouse IgG2a. Visualisation is done by Goat anti-Mouse immunoglobulins and horseradish peroxidase labelled dextran polymer. Negative control of the visualisation system is Goat anti-Rabbit immunoglobulins and horseradish peroxidase labelled dextran polymer (DakoCytomation code No. K4003). Finally a diaminobenzidine chromogenic substrate system is used.

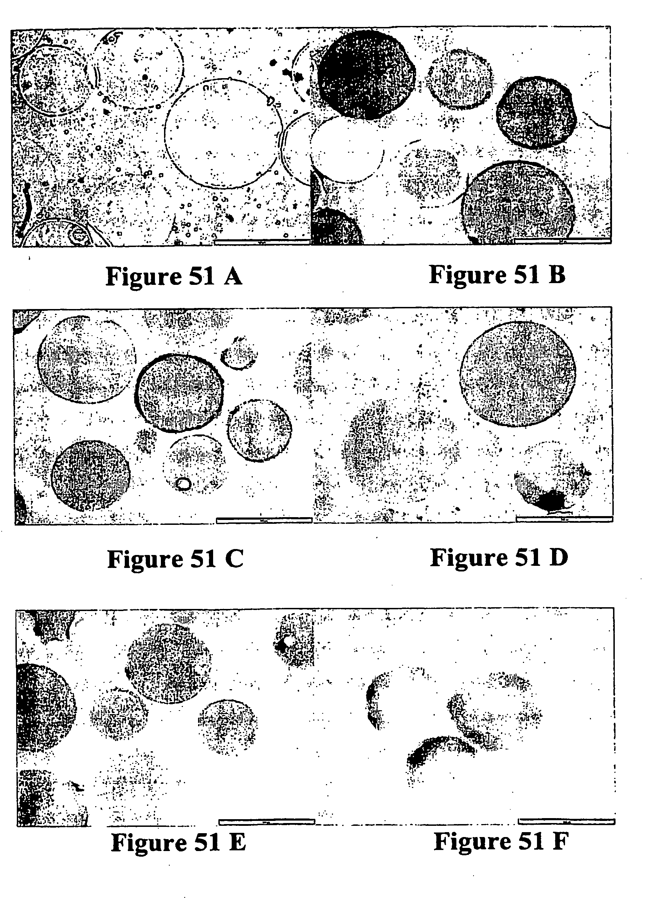

Figure 51 is pictures of the CINtec™ p16$^{INK4a}$ DAB stained Mini Leak matrix in a bright field microscope, all taken at 20 times magnification.

Figures 51 A to Figure 51 F are all photomicrographs of the DAB stained Mini Leak coupled with (a) 0,0 g/l, (b) 0,19g/l, (c) 0,15g/l, (d) 0,10g/l, (e) 0,05g/l or (f) 0,01g/l p16 target peptide, respectively.

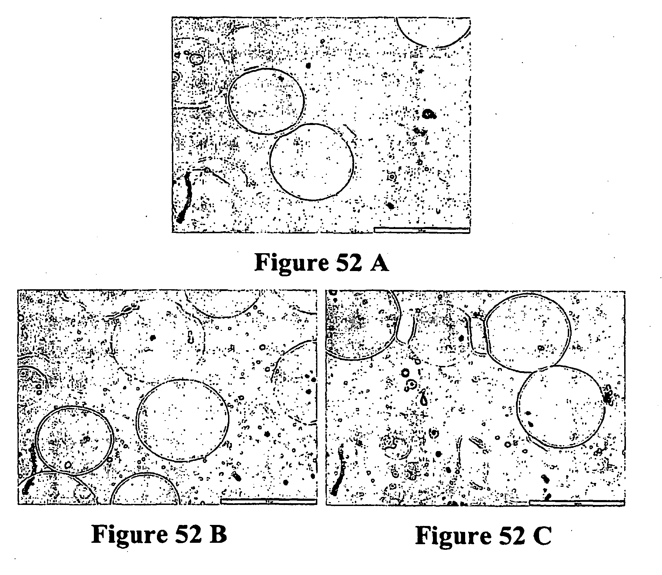

Figure 52 is the various control stainings: Negative primary antibody control (Figure 52 A) and Envision negative control (Figure 50 B), both on 0.19 g/l HER2 mini leak matrix. Figure 52 C is the unmodified Mini leak matrix stained with the CINtec™ p16$^{INK4a}$ kit.

As in the previous example, a homogeneous immonostaining is observed throughout the individual matrix.

The staining intensity is scored to ½+ to 3+ in Figure 51F (0,01g/l p16), from 1+ to 3+ in Figure 51 B, C, D, and E (from 0,19 down to 0,05 g/l p16). The staining intensity was somewhat inhomogeneous distributed between the individual cells.

The control of the coupling, the visualisations system, the antibody and the matrix are all scored to 0 (Figure 52 A-C), but some background staining was observed in agarose between the individual beads.

In conclusion, it is possible to use the artificial cell-like matrix and modified it with a small target as the p16 peptide, immunovisualises it with a DAB staining and evaluate in bright field light microscope.

**20. Size measurement of artificial cells.**

The following example summarizes size measurement of the artificial cells made in example 16 and 18.

In more detail, the FFPE blocks with Fitc modified PEGA resin (example 16, coupled with 10 mM FITC) and p16/HER2 modified Mini leak matrix (example 18, Mini leak matrix modified with 0,15g/l HER2 and 0,05g/l P16) was cut, mounted and stained as previously described. The size was calculated at 20-time magnification relatively to the calibrated scale bar.

The ten largest PEGA beads on 5 slides were measured in two directions (horizontal and perpendicular). The largest was 221 micrometers and the average of the ten larges beads was 173 micrometers. Table 1 A below summarizes the results.

Table 1 A

| Bead | Diameter Horizont. | Perpend. | Average |
|------|-----------|----------|---------|
| #1 | 195μm | 200μm | 198μm |
| #2 | 179μm | 167μ m | 173μm |
| #3 | 239μm | 236μm | 138μm |
| #4 | 152μm | 152μm | 152μm |
| #5 | 176μm | 136μm | 156μm |
| #6 | 176μm | 155μm | 166μm |
| #7 | 218μm | 224μm | 221μm |
| #8 | 167μm | 170μm | 169μm |
| #9 | 191μm | 158μm | 175μm |
| #10 | 182μm | 173μm | 178μm |

Ten slides with p16 modified Mini leak matrix were evaluated. Each individual artificial cell was size measured. The largest was 152 micrometers. The average of 20 cells was 123 micrometers. Table 1 B below summarizes the results for the ten HER2 mini leak slides.

Table 1B

| Slide | Diameter 1 | 2 | 3 | Average |
|-------|-----------|-----|-----|---------|
| #1 | 124μm | 115μm | 106μm | 115μm |
| #2 | 106μm | 82μm | 115μm | 101μm |
| #3 | 133μm | 136μm | - | 135μm |
| #4 | 127μm | 127μm | - | 127μm |
| #5 | 121μm | 127μm | - | 124μm |
| #6 | 136μm | 118μm | - | 127μm |
| #7 | 124μm | 124μm | - | 124μm |
| #8 | 130μm | - | - | 130μm |
| #9 | 152μm | - | - | 152μm |
| #10 | 130μm | 130μm | - | 130μm |

As the individual beads are cut at various places, the size of the cells vary greatly. The largest beads or cells were 221 micrometers, and could easily be seen in the viewing field of the bright field microscope.

Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended. Such modifications of the described modes for carrying out the invention only actually belong to the present invention in as much as they fall within the scope of the claims.

**Claims**

1. A reference standard for a detectable entity, the reference standard comprising an embedding medium, a compact particle having a compact shape with a quantity of detectable entity chemically coupled thereto and supported by the embedding medium, in which the compact particle is a biological, preferably a cellular compact particle.

2. A reference standard for a detectable entity, the reference standard comprising an embedding medium, a compact particle having a compact shape with a quantity of detectable entity coupled thereto and supported by the embedding medium, in which the compact particle is a non-biological compact particle having cell-like dimensions.

3. A reference standard according to Claim 1 or 2, in which a detectable amount of the detectable entity is present in a defined region in a cross section of the reference standard.

4. A reference standard according to Claim 1, 2 or 3, in which the detectable entity adopts a compact shape, preferably an unextended or non-elongate shape, in the embedding medium.

5. A reference standard according to any preceding claim, in which the compact shape is such that the ratio of the longest dimension to the shortest dimension is less than 5:1, preferably less than 2:1

6. A reference standard according any preceding claim, in which the compact shape comprises a particulate, uniform or regular shape.

7. A reference standard according to any preceding claim, in which the compact shape comprises a sphere shape, an ovoid shape, an ellipsoid shape, a disc shape, a cell shape, a pill shape or a capsule shape.

8. A reference standard according to any preceding claim, in which the detectable entity is heterologous to the compact particle.

9. A reference standard according to Claim 1 or any of Claims 3 to 8 as dependent thereon, in which the compact particle comprises a cell or a virus.

10. A reference standard according to Claim 9, in which the cell or virus does not express the detectable entity.

11. A reference standard according to Claim 9 or 10, in which the cell is selected from the group consisting of: a bacterial cell, a eukaryotic cell, an insect cell, an animal cell, a mammalian cell, a mouse cell and a human cell.

12. A reference standard according to Claim 9, 10 or 11, in which the cell comprises an insect cell, preferably an Sf9 cell, or a mammalian cell, preferably a Chinese Hamster Ovary (CHO) cell.

13. A reference standard according to Claim 1 or any of Claims 3 to 8 as dependent thereon, in which the compact particle comprises an organelle.

14. A reference standard according to Claim 13, in which the organelle comprises a mitochondrion, a plastid, a chloroplast, or a nucleus, preferably derived from a cell as set out in any of Claims 10 to 12.

15. A reference standard according to any of Claims 1 to 8, in which the detectable entity is substantially free of cellular material.

16. A. reference standard according to Claim 2 or any of Claims 3 to 8 as dependent thereon, in which the compact particle comprises a microbead or a micelle.

17. A reference standard according to any preceding claim, in which the compact shape has a dimension of less than $1000\mu m$, preferably less than $500\mu m$, preferably less than $200\mu m$, preferably less than $100\mu m$, preferably less than

50μm, more preferably less than 20μm, most preferably less than 10μm.

18. A reference standard according to any preceding claim, in which the defined region is present in at least one other cross section of the reference standard, preferably comprising a similar amount of detectable entity.

19. A reference standard according to any preceding claim, in which the detectable entity is embedded in the embedding medium.

20. A reference standard according to any preceding claim, in which the detectable entity comprises a diagnostically relevant target.

21. A reference standard according to any preceding claim, in which the detectable entity comprises an antigen, an epitope, a peptide, a polypeptide, a protein, a nucleic acid, or two or more or a plurality of any of the above, or combinations of one or more of the above.

22. A reference standard according to any preceding claim, in which the detectable entity is selected from the group consisting of: a hapten, a biologically active molecule, an antigen, an epitope, a protein, a polypeptide, a peptide, an antibody, a nucleic acid, a virus, a virus-like particle, a nucleotide, a ribonucleotide, a deoxyribonucleotide, a modified deoxyribonucleotide, a heteroduplex, a nanoparticle, a synthetic analogue of a nucleotide, a synthetic analogue of a ribonucleotide, a modified nucleotide, a modified ribonucleotide, an amino acid, an amino acid analogue, a modified amino acid, a modified amino acid analogue, a steroid, a proteoglycan, a lipid, a carbohydrate, a dye, and mixtures, fusions, combinations or conjugates of the above.

23. A reference standard according to any preceding claim, in which the detectable entity comprises any one or more of HER2, oestrogen receptor (ER), progesterone receptor (PR), p16, Ki-67, c-kit, laminin 5 gamma 2 chain and Epidermal Growth Factor Receptor (EGFR) protein, nucleic acids encoding such, and post-translationally modified forms, preferably phosphorylated forms of such.

24. A reference standard according to any preceding claim, in which the presence and/or quantity of the detectable entity is revealable by a binding agent, preferably a labelled binding agent.

25. A reference standard according to Claim 24, in which the binding agent is selected from the group consisting of: an antibody, preferably an antibody capable of specific binding to the detectable entity, a nucleic acid such as a DNA or an RNA, preferably a nucleic acid capable of specific binding to the detectable entity, a protein nucleic acid (PNA), a dye, a special stain, Au-chloride, Haematoxylin-Eosin (H & E), Gomori methenamine silver stain (GMS), Periodic Acid-Schiff (PAS) stain, Trichrome Blue, Masson's Trichrome, Prussian Blue, Giemsa, Diff-Quik, Reticulum, Congo Red, Alcian Blue, Steiner, AFB, PAP, Gram, Mucicarmine, Verhoeff-van Gieson, Elastic, Carbol Fuchsin and Golgi's stains.

26. A reference standard according to any preceding claim, in which the presence of the detectable entity in a cell, tissue, organ or organism is indicative of a disease or a condition.

27. A reference standard according to any preceding claim, in which the defined region includes a reference area, the reference area comprising the detectable entity at a pre-defined amount.

28. A reference standard according to Claim 27, in which the amount of the detectable entity in the reference area is compared to the amount of the detectable entity in a sample to determine the presence, quantity or concentration of the detectable entity in the sample.

29. A reference standard according to any preceding claim, in which the reference standard is in the shape of a rectangular box.

30. A reference standard for a detectable entity, comprising: (a) an embedding medium in a preferably substantially rectangular box shape; and (b) a cell with a quantity of detectable entity coupled thereto.

31. A reference standard according to any preceding claim, which comprises two or more compact particles, each having detectable entity coupled thereto.

32. A reference standard according to any preceding claim, which comprises two or more different detectable entities, each of which is coupled to the same or different compact particle.

33. A reference standard according to any preceding claim, which comprises two or more compact particles comprising different amounts of detectable entity on each.

34. A reference standard according to any preceding claim, in which a planar section of the reference standard comprises a plurality of areas on which are presented the detectable entity at different density.

35. A reference standard according to any preceding claim, in which a planar section of the reference standard comprises a first area comprising the detectable entity substantially at a diagnostically significant density.

36. A reference standard according to any preceding claim, further comprising a control comprising a compact particle which comprises substantially no detectable entity.

37. A reference standard according to any preceding claim, in which the embedding medium is selected from the group consisting of: ice, wax, paraffin, acrylic resin, methacrylate resin, epoxy, Epon, Araldite, Lowicryl, K4M and LR White and Durcupan.

38. A planar section, preferably a transverse planar section, preferably of substantially uniform thickness, of a reference standard according to any preceding claim.

39. A support, preferably a slide such as a microscope slide, comprising a planar section according to Claim 38 mounted thereon.

40. A kit comprising a reference standard according to any preceding claim, together with a binding agent capable of specific binding to the detectable entity, optionally together with instructions for use.

41. A reference standard, kit or a planar section according to any preceding claim, in which the reference standard has been stained, preferably with an antibody or a nucleic acid probe.

42. A diagnostic kit for detecting the presence or amount of a detectable entity in a biological sample, comprising: (a) a reference standard, planar section or slide according to any preceding claim; (b) a binding agent capable of specific binding to the detectable entity; and optionally (c) instructions for use.

43. A combination of a reference standard, planar section, support, kit or diagnostic kit according to any of Claims 1 to 42 together with a therapeutic agent capable of treating or alleviating at least one of the symptoms of a disease or condition in an individual.

44. A combination according to Claim 43, in which the individual is diagnosed as suffering from or susceptible to the disease or condition, if the amount of detectable entity in the biological sample or component is similar to or greater than that in the reference standard.

45. A diagnostic kit according to Claim 42 or a combination according to Claim 43 or 44, in which the binding agent or therapeutic agent comprises an antibody against the detectable entity.

46. Use of a reference standard, a planar section or a kit according to any preceding claim, for determining the presence or amount of a detectable entity in a biological sample.

47. A method of comparing the amount of a detectable entity in a biological sample with a reference standard, the method comprising the steps of:

   (a) providing a biological sample and obtaining a first signal indicative of the amount of detectable entity in the biological sample, or a component thereof;
   (b) providing a reference standard, planar section, support, kit or diagnostic kit according to any of Claims 1 to 42;
   (c) obtaining a second reference signal indicative of the amount of detectable entity in the reference standard or planar section thereof; and
   (d) comparing the first signal obtained in (a) against the reference signal.

**48.** A method according to Claim 47, in which the detectable signal is selected from the group consisting of radiation, optical density, reflectance, radioactivity, fluorescence, enzymatic activity.

**49.** A method according to Claim 47 or 48, in which the reference standard or planar section thereof is subjected to the same one or more steps or conditions, preferably substantially all, as the biological sample.

**50.** A method according to Claim 47,48 or 49, in which the reference standard or planar section thereof is processed through one or more, preferably all, of the following steps: mounting onto a slide, baking, deparaffination, rehydration, antigen retrieval, blocking, exposure to antibody, exposure to primary antibody, exposure to nucleic acid probe, washing, exposure to secondary antibody-enzyme conjugate, exposure to enzyme substrate, exposure to chromogen substrate, and counter staining.

**51.** A method or use according to Claims 46 to 50, in which the biological sample comprises a cell, tissue or organ, preferably a cell, tissue or organ of an organism suspected of suffering a disease or condition.

**52.** A method of obtaining an indication suitable for the diagnosis of a disease or a condition in an individual, the method comprising:

comparing the amount of a detectable entity in a biological sample from an individual, or component thereof, with a reference standard, by a method according to Claim 47;
in which preferably the individual is indicated as likely to be suffering from or susceptible to the disease or condition, if the amount of detectable entity in the biological sample or component is similar to or greater than that in the reference standard.

**53.** A reference standard according to any of Claims 1 to 37 for use in a method of treatment of a disease or a condition in an individual, the method comprising the steps of obtaining an indication suitable for the diagnosis of the disease or condition in an individual by a method according to Claim 52, and administering a therapeutic agent to the individual.

**54.** A reference standard according to Claim 53 for a use as specified therein, in which the therapeutic agent comprises an antibody capable of binding to the detectable entity.

**55.** A method of assessing the effectiveness or success of a procedure, the method comprising the steps of:

(a) providing a reference standard according to any of Claims 1 to 37, in which a detectable property of the detectable entity is changed as a result of the procedure;
(b) conducting the procedure on the reference standard; and
(c) detecting a change in the detectable property of the detectable entity.

**56.** A method according to Claim 55, in which a detectable property of the detectable entity is changed as a result of a successful procedure, which change in the detectable property of the detectable entity is detected to establish that the procedure is successful.

**57.** A method according to Claim 55, in which a detectable property of the detectable entity is changed as a result of an unsuccessful procedure, which change in the detectable property of the detectable entity is detected to establish that the procedure is not successful.

**58.** A method of validating a procedure according to Claim 55, 56 or 57, in which the procedure is selected from the group consisting of: an *in situ* hybridisation procedure, an immunohistochemical procedure, deparaffination, antigen retrieval, blocking, endogenous biotin blocking, endogenous enzyme blocking, a washing step, incubation with revealing agent such as a primary antibody, incubation with secondary visualisation components, chromogen staining, staining information acquisition and analysis.

**59.** A method according to any of Claims 55 to 58, in which the procedure is an antigen retrieval procedure, and in which the detectable property of the detectable entity comprises the masking or unmasking of one or more epitopes.

**60.** A method according to any of Claims 55 to 59, in which the detectable entity in the reference standard is modified to mask one or more epitopes, some or all of which are unmasked in an antigen retrieval procedure which is successful.

**61.** A method according to any of Claims 55 to 58, in which the procedure is an deparaffination procedure, and in which the detectable property of the detectable entity comprises the presence or quantity of detectable entity in the reference standard following the deparaffination procedure.

**62.** A method according to any of Claims 55 to 58 and 61, in which the detectable entity in the reference standard is soluble in the deparaffination medium, and in which at least a portion, preferably all, of the detectable entity is removed following a successful deparaffination procedure.

**63.** Use of a reference standard as claimed in any preceding claim as an antigen retrieval validation standard, a deparaffination standard, a blocking validation standard, a washing validation standard, a primary antibody validation standard, a secondary antibody validation standard, a calibration standard, or a diagnostic standard.

**64.** A method of producing a reference standard for a detectable entity, the method comprising the steps of: (a) providing an embedding medium; (b) providing a compact particle having a compact shape, in which the compact particle is a biological, preferably a cellular compact particle; (c) chemically coupling a quantity of detectable entity to the compact particle and (d) supporting or embedding the compact particle in the medium.

**65.** A method of producing a reference standard for a detectable entity, the method comprising the steps of: (a) providing an embedding medium; (b) providing a compact particle having a compact shape, in which the compact particle is a non-biological compact particle having cell-like dimensions; (c) coupling a quantity of detectable entity to the compact particle and (d) supporting or embedding the compact particle in the medium.

**66.** A method of producing a reference standard for a detectable entity, the method comprising the steps of: providing a compact particle having a compact shape of biological, preferably cellular origin, chemically coupling a quantity of detectable entity to the compact particle, and supporting or embedding the compact particle in an embedding medium.

**67.** A method of producing a reference standard for a detectable entity, the method comprising the steps of: providing a compact particle having a compact shape, in which the compact particle is a non-biological compact particle having cell-like dimensions, coupling a quantity of detectable entity to the compact particle, and supporting or embedding the compact particle in an embedding medium.

**68.** A method of producing a reference standard for a detectable entity, the method comprising supporting a compact particle having a compact shape and cell-like dimensions having a quantity of detectable entity chemically coupled thereto in an embedding medium, in which the compact particle is a biological, preferably a cellular compact particle.

**69.** A method of producing a reference standard for a detectable entity, the method comprising supporting a compact particle having a compact shape and cell-like dimensions having a quantity of detectable entity coupled thereto in an embedding medium, in which the compact particle is a non-biological compact particle having cell-like dimensions.

**70.** A method of producing a reference standard for a detectable entity, the method comprising the steps of: (a) providing an embedding medium; (b) forming a quantity of detectable entity in a generally compact shape and cell-like dimensions by (i) chemically coupling to a compact particle which is a biological, preferably a cellular compact particle, or (ii) coupling to a compact particle which is a non-biological compact particle having cell-like dimensions; and (c) embedding the detectable entity in the embedding medium.

**71.** A method according to any of Claims 64 to 70, which further comprises coupling a quantity of a second detectable entity to the compact particle, or to a second compact particle.

**72.** A method according to any of Claims 64 to 71, which further comprises coupling a second different quantity of the or each detectable entity to the or each compact particle.

**73.** A method according to any of Claims 64 to 72, in which the or each compact particle is supported by embedding in the embedding medium.

**74.** A method according to any of Claims 64 to 73, in which the or each detectable entity is covalently coupled to its respective compact particle.

**75.** A reference standard for a detectable entity, comprising a detectable entity chemically coupled to a cell or a virus supported by an embedding medium.

**76.** A method of producing a reference standard for a detectable entity, the method comprising the steps of providing a cell or a virus, chemically coupling a quantity of detectable entity to the cell or virus, and embedding the cell or virus in an embedding medium.

**77.** A reference standard according to Claim 75 or a method according to Claim 76, in which the cell or virus does not express the detectable entity.

**78.** A method or reference standard according to Claim 75, 76 or 77, in which the cell is selected from the group consisting of: a bacterial cell, a eukaryotic cell, an insect cell, preferably an Sf9 cell, an animal cell, a mammalian cell, preferably a Chinese Hamster Ovary (CHO) cell, a mouse cell and a human cell.

**79.** A method of establishing a cellular distribution of detectable entity in a reference standard, the method comprising providing a cell or a component thereof which does not express a detectable entity, chemically coupling a quantity of detectable entity to the cell or component, and supporting the cell or component in an embedding medium.


**Patentansprüche**

**1.** Vergleichsstandard für ein detektierbares Objekt, wobei der Vergleichsstandard ein Einbettungsmedium, ein kompaktes Partikel mit einer kompakten Gestalt mit einer Anzahl chemisch damit verknüpftem und von dem Einbettungsmedium gestütztem detektierbarem Objekt umfaßt, wobei das kompakte Partikel ein biologisches, vorzugsweise ein zelluläres kompaktes Partikel ist.

**2.** Vergleichsstandard für ein detektierbares Objekt, wobei der Vergleichsstandard ein Einbettungsmedium, ein kompaktes Partikel mit einer kompakten Gestalt mit einer Anzahl damit verknüpftem und von dem Einbettungsmedium gestütztem detektierbarem Objekt umfaßt, wobei das kompakte Partikel ein nicht-biologisches kompaktes Partikel mit zellähnlichen Dimensionen ist.

**3.** Vergleichsstandard nach Anspruch 1 oder 2, wobei eine detektierbare Menge des detektierbares Objekts in einem definierten Bereich in einem Querschnitt des Vergleichsstandards vorliegt.

**4.** Vergleichsstandard nach Anspruch 1, 2 oder 3, wobei das detektierbare Objekt eine kompakte Gestalt, vorzugsweise eine nicht ausgedehnte oder nicht langgestreckte Gestalt, in dem Einbettungsmedium annimmt.

**5.** Vergleichsstandard nach einem der vorangehenden Ansprüche, wobei die kompakte Gestalt so ist, daß das Verhältnis der längsten Dimension zu der kürzesten Dimension geringer ist als 5:1, vorzugsweise geringer als 2:1.

**6.** Vergleichsstandard nach einem der vorangehenden Ansprüche, wobei die kompakte Gestalt eine partikuläre, gleichmäßige oder reguläre Gestalt umfaßt.

**7.** Vergleichsstandard nach einem der vorangehenden Ansprüche, wobei die kompakte Gestalt eine sphäroide Gestalt, eine eiförmige Gestalt, eine ellipsoide Gestalt, eine scheibenförmige Gestalt, eine zellförmige Gestalt, eine pillenförmige Gestalt oder eine kapselförmige Gestalt umfaßt.

**8.** Vergleichsstandard nach einem der vorangehenden Ansprüche, wobei das detektierbare Objekt zu dem kompakten Partikel heterolog ist.

**9.** Vergleichsstandard nach Anspruch 1 oder einem der Ansprüche 3 bis 8 sofern abhängig davon, wobei das kompakte Partikel eine Zelle oder einen Virus umfaßt.

**10.** Vergleichsstandard nach Anspruch 9, wobei die Zelle oder das Virus das detektierbare Objekt nicht exprimiert.

**11.** Vergleichsstandard nach Anspruch 9 oder 10, wobei die Zelle ausgewählt ist aus der Gruppe, bestehend aus: einer Bakterienzelle, einer Eukaryotenzelle, einer Insektenzelle, einer Tierzelle, einer Säugetierzelle, einer Mauszelle und einer menschlichen Zelle.

12. Vergleichsstandard nach Anspruch 9, 10 oder 11, wobei die Zelle eine Insektenzelle umfaßt, vorzugsweise eine Sf9-Zelle, oder eine Säugetierzelle, vorzugsweise eine Ovarialzelle des chinesischen Hamsters (CHO-Zelle).

13. Vergleichsstandard nach Anspruch 1 oder einem der Ansprüche 3 bis 8 sofern abhängig davon, wobei das kompakte Partikel eine Organelle umfaßt.

14. Vergleichsstandard nach Anspruch 13, wobei die Organelle ein Mitochondrium, ein Plastid, einen Chloroplasten oder einen Kern umfaßt, vorzugsweise abgeleitet aus einer Zelle, wie sie in einem der Ansprüche 10 bis 12 angegeben ist.

15. Vergleichsstandard nach einem der Ansprüche 1 bis 8, wobei das detektierbare Objekt im wesentlichen frei von zellulärem Material ist.

16. Vergleichsstandard nach Anspruch 2 oder einem der Ansprüche 3 bis 8 sofern abhängig davon, wobei das kompakte Partikel ein Mikrokügelchen oder eine Mizelle umfaßt.

17. Vergleichsstandard nach einem der vorangehenden Ansprüche, wobei die kompakte Gestalt eine Dimension von weniger als 1000 µm, vorzugsweise weniger als 500 µm, vorzugsweise weniger als 200 µm, vorzugsweise weniger als 100 µm, vorzugsweise weniger als 50 µm, noch bevorzugter weniger als 20 µm, am meisten bevorzugt weniger als 10 µm, aufweist.

18. Vergleichsstandard nach einem der vorangehenden Ansprüche, wobei der definierte Bereich in wenigstens einem anderen Querschnitt des Vergleichsstandards vorliegt, vorzugsweise umfassend eine vergleichbare Menge des detektierbaren Objekts.

19. Vergleichsstandard nach einem der vorangehenden Ansprüche, wobei das detektierbare Objekt in dem Einbettungsmedium eingebettet ist.

20. Vergleichsstandard nach einem der vorangehenden Ansprüche, wobei das detektierbare Objekt ein diagnostisch relevantes Ziel umfaßt.

21. Vergleichsstandard nach einem der vorangehenden Ansprüche, wobei das detektierbare Objekt ein Antigen, ein Epitop, ein Peptid, ein Polypeptid, ein Protein, eine Nukleinsäure oder zwei oder mehr oder eine Vielzahl von einem der obigen der oder Kombinationen von einem oder mehreren der obigen umfaßt.

22. Vergleichsstandard nach einem der vorangehenden Ansprüche, wobei das detektierbare Objekt ausgewählt ist aus der Gruppe, bestehend aus: einem Hapten, einem biologisch aktiven Molekül, einem Antigen, einem Epitop, einem Protein, eine Polypeptid, einem Peptid, einem Antikörper, einer Nukleinsäure, einem Virus, einem Virus-ähnlichen Partikel, einem Nukleotid, einem Ribonukleotid, einem Desoxyribonukleotid, einem modifizierten Desoxyribonukleotid, einem Heteroduplex, einem Nanopartikel, einem synthetischen Analogon eines Nukleotids, einem synthetischen Analogon eines Ribonukleotids, einem modifizierten Nukleotid, einem modifizierten Ribonukleotid, einer Aminosäure, einem Aminosäure-Analogon, einer modifizierten Aminosäure, einem modifizierten Aminosäure-Analogon, einem Steroid, einem Proteoglycan, einem Lipid, einem Kohlenhydrat, einem Farbstoff und Gemischen, Fusionen, Kombinationen oder Konjugaten der obigen.

23. Vergleichsstandard nach einem der vorangehenden Ansprüche, wobei das detektierbare Objekt eines oder mehrere unter HER2, Östrogenrezeptor (ER), Progesteronrezeptor (PR), p16, Ki-67, c-kit, Laminin-5-Gamma-2-Kette und epidermalen-Wachstumsfaktorrezeptor- (EGFR-) Protein, Nukleinsäuren, die solches codieren, und post-translational modifizierte Formen, vorzugsweise phosphorylierte Formen, davon umfaßt.

24. Vergleichsstandard nach einem der vorangehenden Ansprüche, wobei die Gegenwart und/oder die Menge des detektierbaren Objekts erkennbar ist durch ein bindendes Mittel, vorzugsweise ein markiertes bindendes Mittel.

25. Vergleichsstandard nach Anspruch 24, wobei das bindende Mittel ausgewählt ist aus der Gruppe, bestehend aus: einem Antikörper, vorzugsweise einem Antikörper, der in der Lage ist, an das detektierbare Objekt spezifisch zu binden, einer Nukleinsäure, wie zum Beispiel einer DNA oder einer RNA, vorzugsweise einer Nukleinsäure, die in der Lage ist, an das detektierbare Objekt spezifisch zu binden, einer Proteinnukleinsäure (PNA), einem Farbstoff, einem speziellen Färbemittel, Au-Chlorid, Hämatoxylin-Eosin (H & E), Gomori'sche Methenamin-Silber-Färbung (GMS), Perjodsäure-Schiff- (PAS-) Färbung, Trichrom-Blau-, Masson'sche Trichrom-, Preußisch-Blau-, Giemsa-, Diff-Quick-, Reticulum-, Kongorot-, Alcianblau-, Steiner-, AFB-, PAP-, Gram-, Mucicarmin-, Verhoeff-van-Gieson-, Elastic-, Carbolfuchsin- und Golgi-Färbungen.

26. Vergleichsstandard nach einem der vorangehenden Ansprüche, wobei die Gegenwart des detektierbaren Objekts in einer Zelle, einem Gewebe, einem Organ oder einem Organismus ein Hinweis auf eine Erkrankung oder einen Zustand ist.

27. Vergleichsstandard nach einem der vorangehenden Ansprüche, wobei der definierte Bereich einen Vergleichsbereich einschließt, wobei der Vergleichsbereich das detektierbare Objekt in einer vorbestimmten Menge umfaßt.

28. Vergleichsstandard nach Anspruch 27, wobei die Menge des detektierbaren Objekts in dem Vergleichsbereich mit der Menge des detektierbaren Objekts in einer Probe verglichen wird, um die Gegenwart, Menge oder Konzentration des detektierbaren Objekts in der Probe zu bestimmen.

29. Vergleichsstandard nach einem der vorangehenden Ansprüche, wobei der Vergleichsstandard in der Form einer rechteckigen Box vorliegt.

30. Vergleichsstandard für ein detektierbares Objekt, umfassend: (a) ein Einbettungsmedium in einer vorzugsweise im wesentlichen rechteckigen Boxform und (b) eine Zelle mit einer damit verknüpften Menge an detektierbarem Objekt.

31. Vergleichsstandard nach einem der vorangehenden Ansprüche, welcher zwei oder mehr kompakte Partikel umfaßt, die jeweils damit verknüpftes detektierbares Objekt aufweisen.

32. Vergleichsstandard nach einem der vorangehenden Ansprüche, welcher zwei oder mehr verschiedene detektierbare Objekte umfaßt, von denen jedes mit dem gleichen oder mit verschiedenen kompakten Partikeln verknüpft ist.

33. Vergleichsstandard nach einem der vorangehenden Ansprüche, welcher zwei oder mehr kompakte Partikel umfaßt, die darauf jeweils verschiedene Mengen an detektierbarem Objekt umfassen.

34. Vergleichsstandard nach einem der vorangehenden Ansprüche, bei dem ein planarer Schnitt des Vergleichsstandards eine Mehrzahl von Bereichen umfaßt, auf denen das detektierbare Objekt in unterschiedlicher Dichte präsentiert wird.

35. Vergleichsstandard nach einem der vorangehenden Ansprüche, bei dem ein planarer Schnitt des Vergleichsstandards einen ersten Bereich umfaßt, der das detektierbare Objekt im wesentlichen in einer diagnostisch signifikanten Dichte umfaßt.

36. Vergleichsstandard nach einem der vorangehenden Ansprüche, welcher weiterhin eine Kontrolle umfaßt, welche ein kompaktes Partikel umfaßt, welches im wesentlichen kein detektierbares Objekt umfaßt.

37. Vergleichsstandard nach einem der vorangehenden Ansprüche, bei dem das Einbettungsmedium ausgewählt ist aus der Gruppe, bestehend aus: Eis, Wachs, Paraffin, Acrylharz, Metacrylatharz, Epoxy, Epon, Araldit, Lowicryl, K4M und LR-Weiß und Durcupan.

38. Planarer Schnitt, vorzugsweise ein planarer Querschnitt, vorzugsweise von im wesentlichen einheitlicher Dicke, eines Vergleichsstandards nach einem der vorangehenden Ansprüche.

39. Träger, vorzugsweise ein Objektträger, wie zum Beispiel ein Objektträger für die Mikroskopie, umfassend einen planaren Schnitt nach Anspruch 38, der darauf aufgebracht ist.

40. Kit, umfassend einen Vergleichsstandard nach einem der vorangehenden Ansprüche, gemeinsam mit einem bindendes Mittel, welches in der Lage ist, an das detektierbare Objekt spezifisch zu binden, wahlweise gemeinsam mit Anweisungen für die Verwendung.

41. Vergleichsstandard, Kit oder planarer Schnitt nach einem der vorangehenden Ansprüche, wobei der Vergleichsstandard gefärbt wurde, vorzugsweise mit einem Antikörper oder einer Nukleinsäuresonde.

42. Diagnostisches Kit für die Detektion der Gegenwart oder der Menge eines detektierbaren Objekts in einer biologischen Probe, umfassend: (a) einen Vergleichsstandard, planaren Schnitt oder Objektträger nach einem der vorangehenden Ansprüche, (b) ein bindendes Mittel, welches in der Lage ist, an das detektierbare Objekt spezifisch zu binden, und wahlweise (c) Anweisungen für die Verwendung.

43. Kombination eines Vergleichsstandards, eines planaren Schnitts, eines Trägers, eines Kits oder eines diagnostischen Kits nach einem der Ansprüche 1 bis 42 zusammen mit einem therapeutischen Mittel, das in der Lage ist, wenigstens eines der Symptome einer Krankheit oder eines Zustands in einem Individuum zu behandeln oder zu mildern.

44. Kombination nach Anspruch 43, wobei für das Individuum diagnostiziert wird, daß es unter der Krankheit oder dem Zustand leidet oder hierfür anfällig ist, wenn die Menge des detektierbaren Objekts in der biologischen Probe oder Komponente ähnlich zu der oder größer als die in dem Vergleichsstandard ist.

45. Diagnostisches Kit nach Anspruch 42 oder Kombination nach Anspruch 43 oder 44, wobei das bindende Mittel oder therapeutische Mittel ein Antikörper gegen das detektierbare Objekt umfaßt.

46. Verwendung eines Vergleichsstandards, eines planaren Schnitts oder eines Kits nach einem der vorangehenden Ansprüche bei der Bestimmung der Gegenwart oder der Menge eines detektierbaren Objekts in einer biologischen Probe.

47. Verfahren zum Vergleichen der Menge eines detektierbaren Objekts in einer biologischen Probe mit einem Vergleichsstandard, wobei das Verfahren die Stufen umfaßt, bei denen man:

(a) eine biologische Probe bereitstellt und ein erstes Signal, das auf die Menge des detektierbaren Objekts in der biologischen Probe oder einer Komponente davon hinweist, erhält,
(b) einen Vergleichsstandard, einen planaren Schnitt, einen Träger, ein Kit oder ein diagnostisches Kit nach einem der Ansprüche 1 bis 42 bereitstellt,
(c) ein zweites Vergleichssignal, das auf die Menge des detektierbaren Objekts in dem Vergleichsstandard oder dem planaren Schnitt davon hinweist, erhält und
(d) das erste in (a) erhaltene Signal mit dem Vergleichssignal vergleicht.

48. Verfahren nach Anspruch 47, wobei das detektierbare Signal ausgewählt ist aus der Gruppe, bestehend aus: Strahlung, optische Dichte, Reflexionsgrad, Radioaktivität, Fluoreszenz, enzymatische Aktivität.

49. Verfahren nach Anspruch 47 oder 48, wobei der Vergleichsstandard oder der planare Schnitt davon der gleichen einen oder den gleichen mehreren Stufen oder Bedingungen, vorzugsweise im wesentlichen allen, wie die biologische Probe unterworfen wird.

50. Verfahren nach Anspruch 47, 48 oder 49, wobei der Vergleichsstandard oder der planare Schnitt davon durch einen oder mehrere, vorzugsweise alle, der folgenden Stufen bearbeitet wird: Aufbringen auf einen Objektträger, Backen, Entparaffinieren, Rehydrieren, Auffindung des Antigens, Blockieren, Exponieren gegenüber Antikörper, Exponieren gegenüber primären Antikörper, Exponieren gegenüber Nukleinsäuresonde, Waschen, Exponieren gegenüber sekundärem Antikörper-Enzymkonjugat, Exponieren gegenüber Enzymsubstrat, Exponieren gegenüber Chromogensubstrat und Gegenfärben.

51. Verfahren oder Verwendung nach den Ansprüchen 46 bis 50, wobei die biologische Probe eine Zelle, ein Gewebe oder ein Organ, vorzugsweise eine Zelle, ein Gewebe oder ein Organ eines Organismus, der im Verdacht steht, unter einer Krankheit oder einem Zustand zu leiden, umfaßt.

52. Verfahren zum Erhalten eines Hinweises, der für die Diagnose einer Erkrankung oder eines Zustands in einem Individuum geeignet ist, wobei das Verfahren umaßt, daß man:

die Menge eines detektierbaren Objekts in einer biologischen Probe aus einem Individuum oder in einer Komponente davon mit einem Vergleichsstandard nach einem Verfahren gemäß Anspruch 47 vergleicht, wobei es vorzugsweise für das Individuum ein Hinweis ist, daß es wahrscheinlich unter der Erkrankung oder dem Zustand leidet oder hierfür empfänglich ist, wenn die Menge des detektierbaren Objekts in der biologischen Probe oder in der Komponente ähnlich zu der oder größer als die in dem Vergleichsstandard ist.

**53.** Vergleichsstandard nach einem der Ansprüche 1 bis 37 für die Verwendung bei einem Verfahren zur Behandlung einer Erkrankung oder eines Zustands in einem Individuum, wobei das Verfahren die Stufen umfaßt, bei denen man mit einem Verfahren nach Anspruch 52 einen Hinweis erhält, der geeignet ist für die Diagnose der Erkrankung oder des Zustands in einem Individuum, und dem Individuum ein therapeutisches Mittel verabreicht.

**54.** Vergleichsstandard nach Anspruch 53 für eine Verwendung, wie darin angegeben, wobei das therapeutische Mittel einen Antikörper umfaßt, der in der Lage ist, an das detektierbare Objekt zu binden.

**55.** Verfahren zur Bestimmung der Wirksamkeit oder des Erfolgs einer Methode, wobei das Verfahren die Stufen umfaßt, bei denen man:

(a) einen Vergleichsstandard nach einem der Ansprüche 1 bis 37 bereitstellt, wobei eine detektierbare Eigenschaft des detektierbaren Objekts als ein Ergebnis der Methode verändert wird,
(b) die Methode mit dem Vergleichsstandard durchführt und
(c) eine Veränderung der detektierbaren Eigenschaft des detektierbaren Objekts erfaßt.

**56.** Verfahren nach Anspruch 55, wobei eine detektierbare Eigenschaft des detektierbaren Objekts als ein Ergebnis einer erfolgreichen Methode verändert wird, wobei die Veränderung der detektierbaren Eigenschaft des detektierbaren Objekts erfaßt wird, um festzustellen, daß die Methode erfolgreich ist.

**57.** Verfahren nach Anspruch 55, wobei eine detektierbare Eigenschaft des detektierbaren Objekts als ein Ergebnis einer nicht erfolgreichen Methode verändert wird, wobei die Veränderung der detektierbaren Eigenschaft des detektierbaren Objekts erfaßt wird, um festzustellen, daß die Methode nicht erfolgreich ist.

**58.** Verfahren zum Validieren einer Methode nach Anspruch 55, 56 oder 57, wobei die Methode ausgewählt ist aus der Gruppe, bestehend aus: einer in-situ-Hybridisierungsmethode, einer immunhistochemischen Methode, Entparaffinierung, Auffindung eines Antigens, Blockieren, endogenes Biotin-Blockieren, endogenes Enzym-Blockieren, einer Waschstufe, Inkubation mit einem Erkennungsmittel, wie zum Beispiel einem primären Antikörper, Inkubation mit sekundären Visualisierungskomponenten, Chromogenfärbung, Akquisition und Analyse von Färbungsinformationen.

**59.** Verfahren nach einem der Ansprüche 55 bis 58, wobei die Methode eine Antigen-Auffindungsmethode ist, und wobei die detektierbare Eigenschaft des detektierbaren Objekts das Maskieren oder Demaskieren von einem oder mehreren Epitopen umfaßt.

**60.** Verfahren nach einem der Ansprüche 55 bis 59, wobei das detektierbare Objekt in dem Vergleichsstandard modifiziert ist, um ein oder mehrere Epitope, von denen manche oder alle nicht maskiert sind, in einer Antigen-Auffindungsmethode, die erfolgreich ist, zu maskieren.

**61.** Verfahren nach einem der Ansprüche 55 bis 58, wobei die Methode eine Entparaffinierungs-Methode ist, und wobei die detektierbare Eigenschaft des detektierbaren Objekts die Gegenwart oder Menge des detektierbaren Objekts in dem Vergleichsstandard im Anschluß an die Entparaffinierungs-Methode umfaßt.

**62.** Verfahren nach einem der Ansprüche 55 bis 58 und 61, wobei das detektierbare Objekt in dem Vergleichsstandard in dem Entparaffinierungsmedium löslich ist und wobei wenigstens ein Teil des, vorzugsweise das gesamte detektierbare Objekt im Anschluß an eine erfolgreiche Entparaffinierungs-Methode entfernt wird.

**63.** Verwendung eines Vergleichsstandards, wie beansprucht in einem der vorangehenden Ansprüche, als ein Antigen-Auffindungs-Validierungsstandard, ein Entparaffinierungsstandard, ein Blockierungs-Validierungsstandard, ein Wasch-Validierungsstandard, ein Validierungsstandard für primäre Antikörper, ein Validierungsstandard für sekundäre Antikörper, ein Kalibrierungsstandard oder ein diagnostischer Standard.

**64.** Verfahren zur Herstellung eines Vergleichsstandards für ein detektierbares Objekt, wobei das Verfahren die Stufen umfaßt, bei denen man: (a) ein Einbettungsmedium bereitstellt, (b) ein kompaktes Partikel mit einer kompakten Gestalt bereitstellt, wobei das kompakte Partikel ein biologisches, vorzugsweise ein zelluläres kompaktes Partikel ist, (c) eine Menge an detektierbarem Objekt chemisch mit dem kompakten Partikel verknüpft und (d) das kompakte Partikel in dem Medium stützt oder einbettet.

**65.** Verfahren zur Herstellung eines Vergleichsstandards für ein detektierbares Objekt, wobei das Verfahren die Stufen umfaßt, bei denen man: (a) ein Einbettungsmedium bereitstellt, (b) ein kompaktes Partikel mit einer, kompakten Gestalt bereitstellt, wobei das kompakte Partikel ein nicht-biologisches kompaktes Partikel mit zell-ähnlichen Dimensionen ist, (c) eine Menge an detektierbarem Objekt mit dem kompakten Partikel verknüpft und (d) das kompakte Partikel in dem Medium stützt oder einbettet.

**66.** Verfahren zur Herstellung eines Vergleichsstandards für ein detektierbares Objekt, wobei das Verfahren die Stufen umfaßt, bei denen man ein kompaktes Partikel mit einer kompakten Gestalt eines biologischen, vorzugsweise zellulären, Ursprung bereitstellt, eine Menge an detektierbarem Objekt mit dem kompakten Partikel chemisch verknüpft und das kompakte Partikel in einem Einbettungsmedium stützt oder einbettet.

**67.** Verfahren zur Herstellung eines Vergleichsstandards für ein detektierbares Objekt, wobei das Verfahren die Stufen umfaßt, bei denen man: ein kompaktes Partikel mit einer kompakten Gestalt bereitstellt, wobei das kompakte Partikel ein nicht-biologisches kompaktes Partikel mit zell-ähnlichen Dimensionen ist, eine Menge an detektierbarem Objekt mit dem kompakten Partikel verknüpft und das kompakte Partikel in einem Einbettungsmedium stützt oder einbettet.

**68.** Verfahren zur Herstellung eines Vergleichsstandards für ein detektierbares Objekt, wobei das Verfahren umfaßt, daß man ein kompaktes Partikel mit einer kompakten Gestalt und zellähnlichen Dimensionen, welches eine Menge an detektierbarem Objekt chemisch damit verknüpft aufweist, in einem Einbettungsmedium stützt, wobei das kompakte Partikel ein biologisches, vorzugsweise ein zelluläres, kompaktes Partikel ist.

**69.** Verfahren zur Herstellung eines Vergleichsstandards für ein detektierbares Objekt, wobei das Verfahren umfaßt, daß man ein kompaktes Partikel mit einer kompakten Gestalt und zellähnlichen Dimensionen, welches eine Menge an detektierbarem Objekt damit verknüpft aufweist, in einem Einbettungsmedium stützt, wobei das kompakte Partikel ein nicht-biologisches kompaktes Partikel mit zell-ähnlichen Dimensionen ist.

**70.** Verfahren zur Herstellung eines Vergleichsstandards für ein detektierbares Objekt, wobei das Verfahren die Stufen umfaßt, bei denen man: (a) ein Einbettungsmedium bereitstellt, (b) eine Menge an detektierbarem Objekt in einer im allgemeinen kompakten Gestalt und in zellähnlichen Dimensionen ausbildet, indem man (i) es mit einem kompakten Partikel, welches ein biologisches, vorzugsweise ein zelluläres, kompaktes Partikel ist, chemisch verknüpft oder (ii) es mit einem kompakten Partikel, welches ein nicht-biologisches kompaktes Partikel mit zellähnlichen Dimensionen ist, verknüpft und (c) das detektierbare Objekt in dem Einbettungsmedium einbettet.

**71.** Verfahren nach einem der Ansprüche 64 bis 70, wobei dieses weiterhin umfaßt, daß man eine Menge eines zweiten detektierbaren Objekts mit dem kompakten Partikel oder mit einem zweiten kompakten Partikel verknüpft.

**72.** Verfahren nach einem der Ansprüche 64 bis 71, welches weiterhin umfaßt, daß man eine zweite unterschiedliche Menge des oder jedes detektierbaren Objekts mit dem oder jedem kompakten Partikel verknüpft.

**73.** Verfahren nach einem der Ansprüche 64 bis 72, wobei das oder jedes kompakte Partikel durch Einbettung in dem Einbettungsmedium gestützt ist.

**74.** Verfahren nach einem der Ansprüche 64 bis 73, wobei das oder jedes detektierbare Objekt mit dessen jeweiligem kompakten Partikel kovalent verknüpft ist.

**75.** Vergleichsstandard für ein detektierbares Objekt, umfassend ein chemisch mit einer Zelle oder einem Virus verknüpftes detektierbares Objekt, welches von einem Einbettungsmedium gestützt ist.

**76.** Verfahren zur Herstellung eines Vergleichsstandards für ein detektierbares Objekt, wobei das Verfahren die Stufen umfaßt, bei denen man eine Zelle oder einen Virus bereitstellt, eine Menge eines detektierbaren Objekts mit der Zelle oder dem Virus chemisch verknüpft und die Zelle oder das Virus in einem Einbettungsmedium einbettet.

**77.** Vergleichsstandard nach Anspruch 75 oder Verfahren nach Anspruch 76, wobei die Zelle oder das Virus das detektierbare Objekt nicht exprimiert.

**78.** Verfahren oder Vergleichsstandard nach Anspruch 75, 76 oder 77, wobei die Zelle ausgewählt ist aus der Gruppe, bestehend aus: einer Bakterienzelle, einer eukaryotischen Zelle, einer Insektenzelle, vorzugsweise einer Sf9-Zelle, einer Tierzelle, einer Säugetierzelle, vorzugsweise einer Ovarialzelle des chinesischen Hamsters (CHO-Zelle), einer Mauszelle und einer menschlichen Zelle.

**79.** Verfahren zur Feststellung einer zellulären Verteilung eines detektierbaren Objekts in einem Vergleichsstandard, wobei das Verfahren umfaßt, daß man eine Zelle oder eine Komponente davon bereitstellt, welche ein detektierbares Objekt nicht exprimiert, eine Menge an detektierbarem Objekt mit der Zelle oder der Komponente chemisch verknüpft und die Zelle oder die Komponente in einem Einbettungsmedium stützt.

**Revendications**

**1.** Echantillon de référence pour une entité détectable, l'échantillon de référence comprenant un milieu d'inclusion, une particule compacte ayant une forme compacte avec une quantité d'une entité détectable couplée chimiquement à celle-ci et portée par le milieu d'inclusion, dans lequel la particule compacte est une particule compacte biologique, de préférence une particule compacte cellulaire.

**2.** Echantillon de référence pour une entité détectable, l'échantillon de référence comprenant un milieu d'inclusion, une particule compacte ayant une forme compacte avec une quantité d'une entité détectable couplée à celle-ci et portée par le milieu d'inclusion, dans lequel la particule compacte est une particule compacte non biologique ayant des dimensions proches de celles d'une cellule.

**3.** Echantillon de référence suivant la revendication 1 ou 2, dans lequel une quantité détectable de l'entité détectable est présente dans une région définie dans une section transversale de l'échantillon de référence.

**4.** Echantillon de référence suivant la revendication 1, 2 ou 3, dans lequel l'entité détectable acquiert une forme compacte, de préférence une forme non étendue ou non allongée, dans le milieu d'inclusion.

**5.** Echantillon de référence suivant l'une quelconque des revendications précédentes, dans lequel la forme compacte est telle que le rapport de la plus grande dimension à la plus petite dimension est inférieur à 5:1, de préférence inférieur à 2:1.

**6.** Echantillon de référence suivant l'une quelconque des revendications précédentes, dans lequel la forme compacte comprend une forme de particule, une forme uniforme ou une forme régulière.

**7.** Echantillon de référence suivant l'une quelconque des revendications précédentes, dans lequel la forme compacte comprend une forme de sphère, une forme ovoïde, une forme d'ellipsoïde, une forme de disque, une forme de cellule, une forme de pilule ou une forme de capsule.

**8.** Echantillon de référence suivant l'une quelconque des revendications précédentes, dans lequel l'entité détectable est hétérologue vis-à-vis de la particule compacte.

**9.** Echantillon de référence suivant la revendication 1 ou l'une quelconque des revendications 3 à 8 en dépendant, dans lequel la particule compacte comprend une cellule ou un virus.

**10.** Echantillon de référence suivant la revendication 9, dans lequel la cellule ou le virus n'exprime pas l'entité détectable.

**11.** Echantillon de référence suivant la revendication 9 ou 10, dans lequel la cellule est choisie dans le groupe consistant en : une cellule bactérienne, une cellule eucaryotique, une cellule d'insecte, une cellule animale, une cellule de mammifère, une cellule de souris et une cellule humaine.

**12.** Echantillon de référence suivant la revendication 9, 10 ou 11, dans lequel la cellule comprend une cellule d'insecte, de préférence une cellule Sf9, ou une cellule de mammifère, de préférence une cellule d'ovaire de hamster chinois (CHO).

**13.** Echantillon de référence suivant la revendication 1 ou l'une quelconque des revendications 3 à 8 en dépendant, dans lequel la particule compacte comprend un organite.

**14.** Echantillon de référence suivant la revendication 13, dans lequel l'organite comprend une mitochondrie, un plastide, un chloroplaste ou un noyau, dérivé de préférence d'une cellule indiquée dans l'une quelconque des revendications 10 à 12.

**15.** Echantillon de référence suivant l'une quelconque des revendications 1 à 8, dans lequel l'entité détectable est pratiquement dépourvue de matière cellulaire.

**16.** Echantillon de référence suivant la revendication 2 ou l'une quelconque des revendications 3 à 8 en dépendant, dans lequel la particule compacte comprend une microbille ou une micelle.

**17.** Echantillon de référence suivant l'une quelconque des revendications précédentes, dans lequel la forme compacte a une dimension inférieure à 1000 $\mu$m, de préférence inférieure à 500 $\mu$m, de préférence inférieure à 200 $\mu$m, de préférence inférieure à 100 $\mu$m, de préférence inférieure à 50 $\mu$m, mieux encore inférieure à 20 $\mu$m, notamment inférieure à 10 $\mu$m.

**18.** Echantillon de référence suivant l'une quelconque des revendications précédentes, dans lequel la région définie est présente dans au moins une autre section transversale de l'échantillon de référence, comprenant de préférence une quantité similaire de l'entité détectable.

**19.** Echantillon de référence suivant l'une quelconque des revendications précédentes, dans lequel l'entité détectable est incluse dans le milieu d'inclusion.

**20.** Echantillon de référence suivant l'une quelconque des revendications précédentes, dans lequel l'entité détectable comprend une cible intéressante à des fins de diagnostic.

**21.** Echantillon de référence suivant l'une quelconque des revendications précédentes, dans lequel l'entité détectable comprend un antigène, un épitope, un peptide, un polypeptide, une protéine, un acide nucléique ou bien deux, plus de deux ou une pluralité de n'importe laquelle des entités précitées, ou des associations d'une ou plusieurs des entités précitées.

**22.** Echantillon de référence suivant l'une quelconque des revendications précédentes, dans lequel l'entité détectable est choisie dans le groupe consistant en : un haptène, une molécule biologiquement active, un antigène, un épitope, une protéine, un polypeptide, un peptide, un anticorps, un acide nucléique, un virus, une particule de type viral, un nucléotide, un ribonucléotide, un désoxyribonucléotide, un désoxyribonucléotide modifié, un hétéroduplex, une nanoparticule, un analogue synthétique d'un nucléotide, un analogue synthétique d'un ribonuléotide, un nucléotide modifié, un ribonucléotide modifié, un aminoacide, un analogue d'aminoacide, un aminoacide modifié, un analogue d'aminoacide modifié, un stéroïde, un protéoglycane, un lipide, un glucide, un colorant, et des mélanges, des fusions, des associations ou des conjugués des entités précitées.

**23.** Echantillon de référence suivant l'une quelconque des revendications précédentes, dans lequel l'entité détectable comprend une ou plusieurs quelconques des entités consistant en HER2, un récepteur d'oestrogène (ER), un récepteur de progestérone (PR), p16, Ki-67, c-kit, la chaîne gamma 2 de la laminine 5 et la protéine du type récepteur de facteur de croissance épidermique (EGFR), les acides nucléiques codant pour ces entités, et leurs formes modifiées en post-traduction, de préférence leurs formes phosphorylées.

**24.** Echantillon de référence suivant l'une quelconque des revendications précédentes, dans lequel la présence et/ou la quantité de l'entité détectable peut être révélée par un agent de liaison, de préférence un agent de liaison marqué.

**25.** Echantillon de référence suivant la revendication 24, dans lequel l'agent de liaison est choisi dans le groupe consistant en : un anticorps, de préférence un anticorps capable de se lier spécifiquement à l'entité détectable, un acide nucléique tel qu'un ADN ou un ARN, de préférence un acide nucléique capable de se lier spécifiquement à l'entité détectable, un acide nucléique protéique (PNA), un colorant, une matière tinctoriale particulière, le chlorure de Au, l'hématoxyline-éosine (H & E), le colorant à l'argent-méthénamine de Gomori (GMS), le colorant à l'acide périodique de Schiff (PAS), le bleu de trichrome, le trichrome de Masson, le bleu de Prusse, le colorant Giemsa, le colorant Diff-Quik, le Reticulum, le rouge Congo, le bleu Alcian, le Steiner, l'AFB, le PAP, le colorant de Gram, la musicarmine, le colorant de Verhoeff-van Gieson, l'Elastic, la carbol-fuchsine et les colorants de Golgi.

**26.** Echantillon de référence suivant l'une quelconque des revendications précédentes, dans lequel la présence de l'entité détectable dans une cellule, un tissu, un organe ou un organisme est une indication d'une maladie ou d'une affection.

27. Echantillon de référence suivant l'une quelconque des revendications précédentes, dans lequel la région définie comprend une zone de référence, la zone de référence comprenant l'entité détectable en une quantité prédéfinie.

28. Echantillon de référence suivant la revendication 27, dans lequel la quantité de l'entité détectable dans la zone de référence est comparée à la quantité de l'entité détectable dans un échantillon pour déterminer la présence, la quantité ou la concentration de l'entité détectable dans l'échantillon.

29. Echantillon de référence suivant l'une quelconque des revendications précédentes, ledit échantillon de référence étant sous forme d'un boîtier rectangulaire.

30. Echantillon de référence pour une entité détectable, comprenant : (a) un milieu d'inclusion sous forme d'une boîte de préférence substantiellement rectangulaire ; et (b) une cellule avec une quantité d'une entité détectable couplée à celle-ci.

31. Echantillon de référence suivant l'une quelconque des revendications précédentes, qui comprend deux ou plus de deux particules compactes, chacune comprenant une entité détectable couplée à celle-ci.

32. Echantillon de référence suivant l'une quelconque des revendications précédentes, qui comprend deux ou plus de deux entités détectables différentes, dont chacune est couplée à la même particule compacte ou à une particule compacte différente.

33. Echantillon de référence suivant l'une quelconque des revendications précédentes, qui comprend deux ou plus de deux particules compactes comprenant des quantités différentes de l'entité détectable sur chacune.

34. Echantillon de référence suivant l'une quelconque des revendications précédentes, dans lequel une section plane de l'échantillon de référence comprend une pluralité de zones sur lesquelles sont présentées l'entité détectable à une densité différente.

35. Echantillon de référence suivant l'une quelconque des revendications précédentes, dans lequel une section plane de l'échantillon de référence comprend une première zone comprenant l'entité détectable substantiellement à une densité significative à des fins de diagnostic.

36. Echantillon de référence suivant l'une quelconque des revendications précédentes, comprenant en outre un témoin comprenant une particule compacte qui ne comprend pratiquement aucune entité détectable.

37. Echantillon de référence suivant l'une quelconque des revendications précédentes, dans lequel le milieu d'inclusion est choisi dans le groupe consistant en : la glace, une cire, la paraffine, une résine acrylique, une résine de méthacrylate, une résine époxy, l'Epon, l'Araldite, le Lowicryle, le K4M et le LR White et le Durcupan.

38. Section plane, de préférence section plane transverse, de préférence d'une épaisseur substantiellement uniforme, d'un échantillon de référence suivant l'une quelconque des revendications précédentes.

39. Support, de préférence une lame telle qu'une lame pour microscope, comprenant une section plane suivant la revendication 38, montée sur celui-ci.

40. Kit comprenant un échantillon de référence suivant l'une quelconque des revendications précédentes, conjointement avec un agent de liaison capable de se lier spécifiquement à l'entité détectable, éventuellement conjointement avec des instructions d'utilisation.

41. Echantillon de référence, kit ou section plane suivant l'une quelconque des revendications précédentes, l'échantillon de référence ayant été coloré, de préférence avec un anticorps ou une sonde d'acide nucléique.

42. Kit de diagnostic pour détecter la présence ou la quantité d'une entité détectable dans un échantillon biologique, comprenant : (a) un échantillon de référence, une section plane ou une lame suivant l'une quelconque des revendications précédentes ; (b) un agent de liaison capable de se lier spécifiquement à l'entité détectable ; et facultativement (c) des instructions d'utilisation.

43. Association d'un échantillon de référence, d'une section plane, d'un support, d'un kit ou d'un kit de diagnostic suivant l'une quelconque des revendications 1 à 42 et d'un agent thérapeutique permettant de traiter ou de soulager au moins un des symptômes d'une maladie ou d'une affection chez un individu.

44. Association suivant la revendication 43, dans laquelle l'individu est diagnostiqué comme souffrant de, ou sensible à, la maladie ou l'affection, si la quantité d'entité détectable dans l'échantillon biologique ou le constituant biologique est similaire ou supérieure à celle dans l'échantillon de référence.

45. Kit de diagnostic suivant la revendication 42 ou association suivant la revendication 43 ou 44, dans lequel l'agent de liaison ou l'agent thérapeutique comprend un anticorps contre l'entité détectable.

46. Utilisation d'un échantillon de référence, d'une section plane ou d'un kit suivant l'une quelconque des revendications précédentes, pour la détermination de la présence ou de la quantité d'une entité détectable dans un échantillon biologique.

47. Méthode pour comparer la quantité d'une entité détectable dans un échantillon biologique avec un échantillon de référence, la méthode comprenant les étapes consistant à :

(a) fournir un échantillon biologique et obtenir un premier signal indiquant la quantité de l'entité détectable dans l'échantillon biologique, ou d'un de ses constituants ;
(b) fournir un échantillon de référence, une section plane, un support, un kit ou un kit de diagnostic suivant l'une quelconque des revendications 1 à 42 ;
(c) obtenir un second signal de référence indiquant la quantité de l'entité détectable dans l'échantillon de référence ou sa section plane ; et
(d) comparer le premier signal obtenu en (a) au signal de référence.

48. Méthode suivant la revendication 47, dans laquelle le signal détectable est choisi dans le groupe consistant en : un rayonnement, une densité optique, une réflectance, une radioactivité, une fluorescence et une activité enzymatique.

49. Méthode suivant la revendication 47 ou 48, dans laquelle l'échantillon de référence ou sa section plane est soumis à une ou plusieurs étapes ou conditions, de préférence pratiquement la totalité, identiques à celles de l'échantillon biologique.

50. Méthode suivant la revendication 47, 48 ou 49, dans laquelle l'échantillon de référence ou sa section plane est traité par une ou plusieurs, de préférence la totalité, des étapes suivantes : le montage sur une lame, une cuisson, un déparaffinage, une réhydratation, une récupération d'antigène, un blocage, une exposition à un anticorps, une exposition à un anticorps primaire, une exposition à une sonde d'acide nucléique, un lavage, une exposition à un conjugué anticorps secondaire-enzyme, une exposition à substrat d'enzyme, une exposition à un substrat chromogène et une contre-coloration.

51. Méthode ou utilisation suivant les revendications 46 à 50, dans laquelle l'échantillon biologique comprend une cellule, un tissu ou un organe, de préférence une cellule, un tissu ou un organe d'un organisme supposé souffrir d'une maladie ou affection.

52. Méthode d'obtention d'une indication convenable pour le diagnostic d'une maladie ou d'une affection chez un individu, la méthode comprenant :

la comparaison de la quantité d'une entité détectable dans un échantillon biologique provenant d'un individu, ou d'un de ses constituants, à celle d'un échantillon de référence, par une méthode suivant la revendication 47 ; dans laquelle, de préférence, l'individu est indiqué comme susceptible de souffrir de, ou sensible à, la maladie ou l'affection, si la quantité de l'entité détectable dans l'échantillon biologique ou le constituant biologique est similaire ou supérieure à celle dans l'échantillon de référence.

53. Echantillon de référence suivant l'une quelconque des revendications 1 à 37, destiné à être utilisé dans une méthode de traitement d'une maladie ou d'une affection chez un individu, la méthode comprenant les étapes d'obtention d'une indication convenable pour le diagnostic de la maladie ou de l'affection chez un individu par une méthode suivant la revendication 52, et d'administration d'un agent thérapeutique à l'individu.

54. Echantillon de référence suivant la revendication 53, pour une utilisation telle que spécifiée ici, l'agent thérapeutique comprenant un anticorps capable de se lier à l'entité détectable.

55. Méthode pour évaluer l'efficacité ou le succès d'un mode opératoire, la méthode comprenant les étapes consistant à :

(a) fournir un échantillon de référence suivant l'une quelconque des revendications 1 à 37, une propriété détectable de l'entité détectable étant modifiée en résultat de la mise en oeuvre du mode opératoire ;
(b) mettre en oeuvre le mode opératoire sur l'échantillon de référence ; et
(c) détecter une variation de la propriété détectable de l'entité détectable.

56. Méthode suivant la revendication 55, dans laquelle une propriété détectable de l'entité détectable est modifiée en résultat d'un mode opératoire couronné de succès, ladite modification de la propriété détectable de l'entité détectable étant détectée pour établir que le mode opératoire est couronné de succès.

57. Méthode suivant la revendication 55, dans laquelle une propriété détectable de l'entité détectable est modifiée en résultat d'un mode opératoire infructueux, ladite modification de la propriété détectable de l'entité détectable étant détectée pour établir que le mode opératoire n'est pas couronné de succès.

58. Méthode pour valider un mode opératoire suivant la revendication 55, 56 ou 57, dans laquelle le mode opératoire est choisi dans le groupe consistant en : un mode opératoire d'hybridation in situ, un mode opératoire d'immunohistochimie, un déparaffinage, une récupération d'antigène, un blocage, un blocage de biotine endogène, un blocage d'enzyme endogène, une étape de lavage, une incubation avec un révélateur tel qu'un anticorps primaire, une incubation avec des constituants de visualisation secondaires, une coloration avec un agent chromogène, l'acquisition d'une information de coloration et une analyse.

59. Méthode suivant l'une quelconque des revendications 55 à 58, dans laquelle le mode opératoire est un mode opératoire de récupération d'antigène, et dans laquelle la propriété détectable de l'entité détectable comprend le masquage ou le démasquage d'un ou plusieurs épitopes.

60. Méthode suivant l'une quelconque des revendications 55 à 59, dans laquelle l'entité détectable dans l'échantillon de référence est modifiée pour masquer un ou plusieurs épitopes, dont une partie ou la totalité est démasquée dans un mode opératoire de récupération d'antigène qui est couronné de succès.

61. Méthode suivant l'une quelconque des revendications 55 à 58, dans laquelle le mode opératoire est un mode opératoire de déparaffinage, et dans laquelle la propriété détectable de l'entité détectable comprend la présence ou la quantité de l'entité détectable dans l'échantillon de référence après le mode opératoire de déparaffinage.

62. Méthode suivant l'une quelconque des revendications 55 à 58 et 61, dans laquelle l'entité détectable dans l'échantillon de référence est soluble dans le milieu de déparaffinage, et dans laquelle au moins une partie, de préférence la totalité, de l'entité détectable est éliminée après un mode opératoire de déparaffinage couronné de succès.

63. Utilisation d'un échantillon de référence suivant l'une quelconque des revendications précédentes comme échantillon de validation de récupération d'antigène, échantillon de déparaffinage, échantillon de validation de blocage, échantillon de validation de lavage, échantillon de validation d'anticorps primaire, échantillon de validation d'anticorps secondaire, échantillon d'étalonnage ou échantillon de diagnostic.

64. Méthode pour la production d'un échantillon de référence pour une entité détectable, la méthode comprenant les étapes consistant à : (a) fournir un milieu d'inclusion ; (b) fournir une particule compacte ayant une forme compacte, ladite particule compacte étant une particule compacte biologique, de préférence une particule compacte cellulaire ; (c) coupler chimiquement une quantité de l'entité détectable à la particule compacte et (d) porter ou inclure la particule compacte dans le milieu.

65. Méthode pour la production d'un échantillon de référence pour une entité détectable, la méthode comprenant les étapes consistant à : (a) fournir un milieu d'inclusion ; (b) fournir une particule compacte ayant une forme compacte, ladite particule compacte étant une particule compacte non biologique ayant des dimensions proches de celles d'une cellule ; (c) coupler une quantité de l'entité détectable à la particule compacte et (d) porter ou inclure la particule compacte dans le milieu.

66. Méthode pour la production d'un échantillon de référence pour une entité détectable, la méthode comprenant les étapes consistant à : fournir une particule compacte ayant une forme compacte d'origine biologique, de préférence d'origine cellulaire, coupler chimiquement une quantité de l'entité détectable à la particule compacte, et porter ou inclure la particule compacte dans un milieu d'inclusion.

**67.** Méthode pour la production d'un échantillon de référence pour une entité détectable, la méthode comprenant les étapes consistant à : fournir une particule compacte ayant une forme compacte, ladite particule compacte étant une particule compacte non biologique, ayant des dimensions proches de celles d'une cellule, coupler une quantité de l'entité détectable à la particule compacte, et porter ou inclure la particule compacte dans un milieu d'inclusion.

**68.** Méthode pour la production d'un échantillon de référence pour une entité détectable, la méthode comprenant le support d'une particule compacte ayant une forme compacte et des dimensions proches de celles d'une cellule, comprenant une quantité de l'entité détectable couplée chimiquement à celle-ci dans un milieu d'inclusion, dans laquelle la particule compacte est une particule compacte biologique, de préférence une particule compacte cellulaire.

**69.** Méthode pour la production d'un échantillon de référence pour une entité détectable, la méthode comprenant le support d'une particule compacte ayant une forme compacte et des dimensions proches de celles d'une cellule, comprenant une quantité de l'entité détectable couplée à celle-ci dans un milieu d'inclusion, dans laquelle la particule compacte est une particule compacte non biologique ayant des dimensions proches de celles d'une cellule.

**70.** Méthode pour la production d'un échantillon de référence pour une entité détectable, la méthode comprenant les étapes consistant à : (a) fournir un milieu d'inclusion ; (b) former une quantité de l'entité détectable sous une forme généralement compacte et en des dimensions proches de celles d'une cellules par (i) couplage chimique à une particule compacte qui est une particule compacte biologique, de préférence une particule compacte cellulaire, ou (ii) couplage à une particule compacte qui est une particule compacte non biologique ayant des dimensions proches de celles d'une cellule ; et (c) inclure l'entité détectable dans le milieu d'inclusion.

**71.** Méthode suivant l'une quelconque des revendications 64 à 70, qui comprend en outre le couplage d'une quantité d'une seconde entité détectable à la particule compacte, ou à une seconde particule compacte.

**72.** Méthode suivant l'une quelconque des revendications 64 à 71, qui comprend en outre le couplage d'une seconde quantité différente de l'entité détectable ou de chaque entité détectable à la ou à chaque particule compacte.

**73.** Méthode suivant l'une quelconque des revendications 64 à 72, dans laquelle la ou chaque particule compacte est portée par inclusion dans le milieu d'inclusion.

**74.** Méthode suivant l'une quelconque des revendications 64 à 73, dans laquelle la ou chaque entité détectable est couplée par covalence à sa particule compacte respective.

**75.** Echantillon de référence pour une entité détectable, comprenant une entité détectable couplée chimiquement à une cellule ou à un virus porté par un milieu d'inclusion.

**76.** Méthode pour la production d'un échantillon de référence pour une entité détectable, la méthode comprenant les étapes consistant à fournir une cellule ou un virus, à coupler chimiquement une quantité de l'entité détectable à la cellule ou au virus, et à inclure la cellule ou le virus dans un milieu d'inclusion.

**77.** Echantillon de référence suivant la revendication 75 ou méthode suivant la revendication 76, dans lequel la cellule ou le virus n'exprime pas l'entité détectable.

**78.** Méthode ou échantillon de référence suivant la revendication 75, 76 ou 77, dans lequel la cellule est choisie dans le groupe consistant en : une cellule bactérienne, une cellule eucaryotique, une cellule d'insecte, de préférence une cellule Sf9, une cellule animale, une cellule de mammifère, de préférence une cellule d'ovaire de hamster chinois (CHO), une cellule de souris et une cellule humaine.

**79.** Méthode pour établir une distribution cellulaire d'une entité détectable dans un échantillon de référence, la méthode comprenant les étapes consistant à fournir une cellule ou un de ses constituants qui n'exprime pas une entité détectable, coupler chimiquement une quantité de l'entité détectable à la cellule ou au constituant, et assurer le support de la cellule ou du constituant dans un milieu d'inclusion.

# Figure 1

A                    B

C                    D

A

B

C

D

Figure 2

**Figure 3**

# Figure 4

A

B

C

A

B

Figure 5

Deparafination
Rehydration
Antigen retrieval
Specific Staining

Figure 6

**Figure 7**

Figure 8

D

E

Figure 8

F

A

Figure 9

B

**Tissue**

**Staining**

# Figure 10

**Figure 11**

Activation of particles

↓

**Coupling of targets**

↓

Embedding in
agarose & fixation

Handling

↓

Embedding in paraffin

↓

Cutting and mounting onto
standard slides

Standard
IHC
Unit
Operations

↓

Standard "IHC" staining

Particles (e.g., cells) washed

↓

*Chemically* modified with dyes, peptides etc.

↓

Embedded in an agarose gel

↓

Fixed with formalin

↓

Dehydrated

↓

Embedded in paraffin

↓

Cut and treated as FFPE sample

**Figure 12**

"GMBS"

(A)

(B)

Peptide-SH

(C)

S—Peptide

(D)

**Figure 13**

Figure 14A

Figure 14B

Figure 14C

**Figure 14D**

**Figure 14E**

Figure 14F

Figure 15A

Figure 15B

**Figure 16**

**Figure 17**

**Figure 18A**

**Figure 18B**

**Figure 19A**

**Figure 19B**

**Figure 19C**

**Figure 19D**

**Figure 19E**

**Figure 19F**

**Figure 20A**

**Figure 20B**

**Figure 20C**

**Figure 21**

**Figure 22A**

**Figure 22B**

**Figure 23A**

**Figure 23B**

Figure 23C

Figure 23D

**Repeated preperation**

Figure 23E

**Figure 24A**

**Figure 24B**

**Figure 24C**

**Figure 25A**

**Figure 25B**

Histogram Statistics

File: ROP8715.016 Sample ID: KPI
Acquisition Date: 27-Nov-01 Total Events: 5000
X Parameter: FL1-H (Log) Smooths: 0

| Marker | Left, Right | Events | Geo Mean |
|---|---|---|---|
| All | 1, 9910 | 5000 | 2.08 |
| M1 | 1, 9 | 4931 | 2.03 |

Quadrant Statistics

File: ROP8715.016 Sample ID: KPI
Panel: Acquisition Date: 27-Nov-01
Total Events: 5000 X Parameter: FSC-H (Linear)
Y Parameter: FL1-H (Log)

| Quad | Events | Y Geo Mean |
|---|---|---|
| UL | 0 | *** |
| UR | 10 | 58.40 |
| LL | 13 | 1.64 |
| LR | 4977 | 2.07 |

Figure 26

**Figure 27**

Histogram Statistics

File: ROP8715.018                          Sample ID: KPI
Acquisition Date: 27-Nov-01                Total Events: 5000
X Parameter: FL1-H (Log)                   Smooths: 0

| Marker | Left, Right | Events | Geo Mean |
|--------|-------------|--------|----------|
| All    | 1, 9910     | 5000   | 95.64    |
| M1     | 25, 610     | 4902   | 98.35    |

Quadrant Statistics

File: ROP8715.018                          Sample ID: KPI
Panel:                                     Acquisition Date: 27-Nov-01
Total Events: 5000                         X Parameter: FSC-H (Linear)
Y Parameter: FL1-H (Log)

| Quad | Events | Y Geo Mean |
|------|--------|------------|
| UL   | 12     | 121.33     |
| UR   | 4886   | 99.22      |
| LL   | 6      | 12.37      |
| LR   | 96     | 16.29      |

**Figure 28**

**Histogram Statistics**

File: rop8788.013                        Sample ID: KPI HER2
Acquisition Date: 19-Dec-01              Total Events: 10000
X Parameter: FL1-H (Log)                 Smooths: 0

| Marker | Left, Right | Events | Geo Mean |
|--------|-------------|--------|----------|
| All    | 1, 9810     | 10000  | 18.45    |
| M1     | 5, 111      | 9967   | 18.32    |

**Quadrant Statistics**

File: rop8788.013                        Sample ID: KPI HER
Panel: Untitled Acquisition Tube List    Acquisition Date: 18
Total Events: 10000                      X Parameter: FSC-H
Y Parameter: FL1-H (Log)

| Quad | Events | Y Geo Mean |
|------|--------|------------|
| UL   | 1      | 922.24     |
| UR   | 81     | 128.91     |
| LL   | 24     | 10.93      |
| LR   | 9894   | 18.18      |

# Figure 29

Figure 30

**Histogram Statistics**

File: rop8788.015                           Sample ID: KPI HER2
Acquisition Data: 18-Dec-01                  Total Events: 10000
X Parameter: FL1-H (Log)                     Smooth: 0

| Marker | Left, Right | Events | Geo Mean |
|--------|-------------|--------|----------|
| All    | 1, 9910     | 10000  | 742.18   |
| M1     | 204, 4141   | 9771   | 776.77   |

**Quadrant Statistics**

File: rop8788.015                           Sample ID: KPI HER
Panel: Untitled Acquisition Tube List       Acquisition Date: 18
Total Events: 10000                         X Parameter: FSC-H
Y Parameter: FL1-H (Log)

| Quad | Events | Y Geo Mean |
|------|--------|------------|
| UL   | 12     | 711.56     |
| UR   | 9885   | 762.79     |
| LL   | 4      | 16.77      |
| LR   | 99     | 56.47      |

# Figure 31

File: rop8788.019
Sample ID: KPI HER2
Tube: Untitled

Histogram Statistics

File: rop8788.019
Sample ID: KPI HER2
Tube: Untitled
Acquisition Date: 19-Dec-01
Gated Events: 8909
X Parameter: FL1-H (Log)

Log Data Units: Linear Values
Patient ID:
Panel: Untitled Acquisition Tube List
Gate: G2
Total Events: 10000

| Marker | Left, Right | % Gated | % Total | CV | Median | Peak Ch |
|--------|-------------|---------|---------|--------|--------|---------|
| All | 1, 9910 | 100.00 | 89.09 | 146.18 | 30.51 | 1 |
| M1 | 7, 16 | 17.96 | 16.00 | 13.07 | 10.55 | 10 |
| M2 | 17, 45 | 16.39 | 14.60 | 14.05 | 25.71 | 26 |
| M3 | 59, 117 | 16.99 | 15.14 | 9.86 | 72.34 | 74 |
| M4 | 145, 302 | 15.48 | 13.79 | 8.24 | 216.74 | 220 |
| M5 | 461, 914 | 15.12 | 13.47 | 8.95 | 837.80 | 661 |

# Figure 32

**Figure 33**

## Histogram Statistics

File: ROP8890.002      Sample ID: cellsforsog 14
Acquisition Date: 06-Feb-02      Total Events: 10000
X Parameter: FL1-H (Log)      Smooths: 0

| Marker | Left, Right | Events | Geo Mean |
|--------|-------------|--------|----------|
| All | 1, 9910 | 10000 | 15.41 |
| M1 | 3, 81 | 9948 | 15.31 |

## Quadrant Statistics

File: ROP8890.002      Sample ID: cellsforsog 14
Panel:      Acquisition Date: 08-Feb-02
Total Events: 10000      X Parameter: FSC-H (Linear)
Y Parameter: FL1-H (Log)

| Quad | Events | Y Geo Mean |
|------|--------|------------|
| UL | 3 | 1821.45 |
| UR | 36 | 123.85 |
| LL | 27 | 5.88 |
| LR | 9934 | 15.31 |

# Figure 34

**Figure 35**

**Figure 36**

**Quadrant Statistics**

File: ROP8890.016     Log Data Units: Linear Values
Sample ID: cellcforeog 14     Patient ID:
Tube:     Panel:
Acquisition Date: 06-Feb-02     Gate: No Gate
Gated Events: 10000     Total Events: 10000
X Parameter: FSC-H (Linear)     Y Parameter: FL1-H (Log)
Quad Location: 158, 47

| Quad | Events | % Gated | % Total | X Mean | X Geo Mean | Y Mean | Y Geo Mean |
|------|--------|---------|---------|--------|------------|--------|------------|
| UL | 6 | 0.06 | 0.06 | 149.88 | 143.34 | 249.82 | 192.82 |
| UR | 9939 | 99.39 | 99.39 | 504.53 | 482.89 | 268.47 | 244.72 |
| LL | 3 | 0.03 | 0.03 | 149.67 | 143.69 | 60.46 | 25.95 |
| LR | 52 | 0.52 | 0.52 | 448.56 | 423.08 | 21.50 | 15.82 |

File: ROP8890.016
Total Events: 10000
X Parameter: FL1-H (Log)

| Marker | % Total | Mean |
|--------|---------|------|
| All | 100.00 | 267.10 |
| M1 | 97.89 | 258.67 |
| M2 | 0.01 | 8582.10 |

**Figure 37**

Quadrant Statistics

File: ROP8890.003
Sample ID: celleforsog 14
Tube:
Acquisition Date: 06-Feb-02
Gated Events: 10000
X Parameter: FSC-H (Linear)
Quad Location: 225, 56

Log Data Units: Linear Values
Patient ID:
Panel:
Gate: No Gate
Total Events: 10000
Y Parameter: FL1-H (Log)

| Quad | Events | % Gated | % Total | X Mean | X Geo Mean | Y Mean | Y Geo Mean |
|---|---|---|---|---|---|---|---|
| UL | 18 | 0.18 | 0.18 | 165.78 | 163.26 | 820.37 | 505.82 |
| UR | 9962 | 99.62 | 99.62 | 617.90 | 609.75 | 834.34 | 782.71 |
| LL | 6 | 0.06 | 0.06 | 171.50 | 167.16 | 16.53 | 8.38 |
| LR | 14 | 0.14 | 0.14 | 532.29 | 492.26 | 17.84 | 14.34 |

# Figure 38

File: LOW9242.001 — Acquisition Date: 31-May-02
Gate: No Gate — Total Events: 10000
X Parameter: FL1-H (Log)

| Marker | Events | % Total | Geo Mean |
|--------|--------|---------|----------|
| All | 10000 | 100.00 | 1.43 |

File: LOW9242.001 — Acquisition Date: 31-May-02
Total Events: 10000 — X Parameter: FSC-H (Linear)
Y Parameter: FL1-H (Log)

| Quad | Events | % Total | X Geo Mean |
|------|--------|---------|------------|
| UL | 0 | 0.00 | ••• |
| UR | 24 | 0.24 | 1001.94 |
| LL | 121 | 1.21 | 386.31 |
| LR | 9855 | 98.55 | 873.58 |

**Figure 39**

File: LOW9242.002          Acquisition Date: 31-May-02
Gate: No Gate             Total Events: 10000
X Parameter: FL1-H (Log)

| Marker | Events | % Total | Geo Mean |
|--------|--------|---------|----------|
| All    | 10000  | 100.00  | 2.82     |

File: LOW9242.002          Acquisition Date: 31-May-02
Total Events: 10000        X Parameter: FSC-H (Linear)
Y Parameter: FL1-H (Log)

| Quad | Events | % Total | X Geo Mean |
|------|--------|---------|------------|
| UL   | 1      | 0.01    | 342.00     |
| UR   | 56     | 0.56    | 1007.52    |
| LL   | 147    | 1.47    | 384.07     |
| LR   | 9796   | 97.96   | 853.52     |

**Figure 40**

File: LOW9242.009
Gate: No Gate
X Parameter: FL1-H (Log)

Acquisition Date: 31-May-02
Total Events: 10000

| Marker | Events | % Total | Geo Mean |
|---|---|---|---|
| All | 10000 | 100.00 | 2.54 |

File: LOW9242.009
Total Events: 10000
Y Parameter: FL1-H (Log)

Acquisition Date: 31-May-02
X Parameter: FSC-H (Linear)

| Quad | Events | % Total | X Geo Mean |
|---|---|---|---|
| UL | 1 | 0.01 | 512.00 |
| UR | 41 | 0.41 | 1018.09 |
| LL | 179 | 1.79 | 389.00 |
| LR | 9779 | 97.79 | 850.20 |

# Figure 41

File: LOW9242.010                    Acquisition Date: 31-May-02
Gate: No Gate                         Total Events: 10000
X Parameter: FL1-H (Log)

| Marker | Events | % Total | Geo Mean |
|--------|--------|---------|----------|
| All    | 10000  | 100.00  | 3.50     |

File: LOW9242.010                    Acquisition Date: 31-May-02
Total Events: 10000                  X Parameter: FSC-H (Linear)
Y Parameter: FL1-H (Log)

| Quad | Events | % Total | X Geo Mean |
|------|--------|---------|------------|
| UL   | 22     | 0.22    | 319.62     |
| UR   | 259    | 2.59    | 975.38     |
| LL   | 214    | 2.14    | 381.36     |
| LR   | 9505   | 95.05   | 844.55     |

# Figure 42

Figure 43

Figure 44

**Figure 45 A**

**Figure 45 B**

**Figure 45 C**

**Figure 45 D**

**Figure 45 E**

**Figure 45 F**

**Figure 45 G**

**Figure 46 A**

**Figure 47 A**

**Figure 47 B**

**Figure 48 A**

**Figure 48 B**

**Figure 49 A**

**Figure 49 B**

**Figure 49 C**

**Figure 49 D**

**Figure 49 E**

**Figure 49 F**

**Figure 50 A**

**Figure 50 B**

**Figure 50 C**

**Figure 50 D**

**Figure 50 E**

**Figure 50 F**

**Figure 51 A**          **Figure 51 B**

**Figure 51 C**          **Figure 51 D**

**Figure 51 E**          **Figure 51 F**

**Figure 52 A**

**Figure 52 B**

**Figure 52 C**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0062064 A
- EP 0345953 A
- WO 9105263 A, Battifora
- WO 0023799 A, Smith
- US 4376110 A
- EP 0623679 A
- EP 0368684 A
- EP 0436597 A
- EP 0239400 A, Winter
- WO 9007861 A
- EP 0622378 A
- EP 0634428 A
- EP 0623632 A
- EP 0635522 A

- EP 0633273 A
- EP 0632157 A
- EP 0630908 A
- EP 0630641 A
- EP 0628614 A
- EP 0628610 A
- EP 0622449 A
- EP 0626430 A
- EP 0625529 A
- US 2879178 A
- US 3037006 A
- US 3502627 A
- US 3037969 A
- US 3497485 A

### Non-patent literature cited in the description

- **SOMPURAM et al.** *Clin. Chem.,* 2002, vol. 48 (3), 410
- **J. SAMBROOK ; E. F. FRITSCH ; T. MANIATIS.** Molecular Cloning: A Laboratory Manual. Cold Spring Harbor Laboratory Press, 1989
- **AUSUBEL, F. M. et al.** Current Protocols in Molecular Biology. John Wiley & Sons, 1995
- **B. ROE ; J. CRABTREE ; A. KAHN.** DNA Isolation and Sequencing: Essential Techniques. John Wiley & Sons, 1996
- **J. M. POLAK ; JAMES O'D. MCGEE.** In situ Hybridization: Principles and Practice. Oxford University Press, 1990
- Oligonucleotide Synthesis: A Practical Approach. Irl Press, 1984
- Methods of Enzymology: DNA Structure Part A: Synthesis and Physical Analysis of DNA. **D. M. J. LILLEY ; J. E. DAHLBERG.** Methods in Enzymology. Academic Press, 1992
- Using Antibodies : A Laboratory Manual : Portable Protocol. Cold Spring Harbor Laboratory Press, 1999
- Antibodies : A Laboratory Manual. Cold Spring Harbor Laboratory Press, 1988, 1855
- Handbook of Drug Screening. Marcel Dekker, 2001
- Lab Ref: A Handbook of Recipes, Reagents, and Other Reference Tools for Use at the Bench. Cold Spring Harbor Laboratory, 2002
- **O'REILLY, D.R. ; L.K. MILLER ; V.A. LUCKOW.** *Baculovirus Expression Vectors,* 1994

- **VAUGHN JL et al.** *The establishment of two cell lines from the insect Spodoptera frugiperda (Lepidoptera; Noctuidae). In Vitro,* 1977, vol. 13, 213-217
- **PUCK TT et al.** Genetics of somatic mammalian cells III. Long-term cultivation of euploid cells from human and animal subjects. *J. Exp. Med.,* 1958, vol. 108, 945-956
- **FLEISHMAN RA.** *Trends Genet,* 1993, vol. 9, 285-90
- **BLECHMAN JM et al.** *J Biol Chem,* 1993, vol. 268, 4399-4406
- **PAPAYANNOPOULOU et al.** *Blood,* 1991, vol. 78, 1403-12
- **KIKUTANI et al.** Proceedings of the 5th International Workshop and Conference. Oxford University Press, 03 November 1993, 1855-64
- **BÜHRING et al.** Proceedings of the 5th International Workshop and Conference. Oxford University Press, 03 November 1993, 1882-8
- **BÜHRING et al.** *Br J Haematol,* 1992, vol. 82, 287-94
- **DECLINE et al.** *J Cell Sci,* 2000, vol. 114, 811-231
- **SALO, S.** Function of the γ2 chain in epithelial adhesion and migration, and expression in epithelial cells and carcinomas. *academic dissertation,* 1999
- **SKYLDBERG et al.** *J Natl Cancer Inst,* 1999, vol. 91, 1882-7
- **NORDSTRÖM et al.** *Int J Gynecol Cancer,* 2002, vol. 12, 105-9
- **PYKE et al.** *Cancer Res,* 1995, vol. 55, 4132-9

- **MÄÄTTÄ et al.** *J Pathol,* 1999, vol. 188, 361-8
- **COUSSENS et al.** *Science,* 06 December 1985, vol. 230 (4730), 1132-9
- **SPIVAK-KROIZMAN et al.** *J Biol Chem.,* 25 April 1992, vol. 267 (12), 8056-63
- **KING et al.** *J Biol Chem.,* 05 August 1986, vol. 261 (22), 10073-8
- **PLOWMAN et al.** *Proc Natl Acad Sci USA.,* July 1990, vol. 87 (13), 4905-9
- **PONGLIKITMONGKOL et al.** *EMBO J.,* November 1988, vol. 7 (11), 3385-8
- **LAZENNEC et al.** *Gene,* 12 December 1995, vol. 166 (2), 243-7
- **WATERMAN et al.** *Mol Endocrinol.,* January 1988, vol. 2 (1), 14-21
- **GREEN et al.** *Nature,* 1986, vol. 320, 134-139
- **GREENE et al.** *Science,* 1986, vol. 231, 1150-1154
- **MISRAHI et al.** *Biochem. Biophys. Res. Commun.,* 1987, vol. 143, 740-748
- **CONNEELY et al.** *J Soc Gynecol Investig.,* January 2000, vol. 7 (1), 25-32
- **MOTE et al.** *J Clin Endocrinol Metab.,* August 1999, vol. 84 (8), 2963-71
- **BOGENRIEDER et al.** *Hautarzt.,* February 1998, vol. 49 (2), 91-100
- **UCHIDA et al.** *Leuk Lymphoma.,* March 1998, vol. 29 (1-2), 27-35
- **GERADTS et al.** *Cancer Res.,* 15 December 1995, vol. 55 (24), 6006-11
- **SCHLUTER et al.** *J. Cell. Biol,* 1993, vol. 123, 513-522
- **CARLIN et al.** *Proc Natl Acad Sci USA.,* August 1982, vol. 79 (16), 5026-30
- **KONDO et al.** *Cytogenet Cell Genet.,* 1993, vol. 35 (1), 9-14
- **REVIS-GUPTA et al.** *Proc Natl Acad Sci USA.,* 15 July 1991, vol. 88 (14), 5954-8
- **HUANG et al.** *J Biol Chem.,* 14 March 2003, vol. 278 (21), 18902-13
- **M, HSU S ; LEWIS G et al.** Inhibitory effects of combinations of HER-2/neu antibody and chemotherapeutic agents used for treatment of human breast cancers. *Oncogene,* 1999, vol. 18, 2241-2251
- **ARGIRIS A ; DIGIOVANNA M.** Synergistic interactions between tamoxifen and Herceptin. *Proc Am Assoc Cancer Res,* 2000, vol. 41, 718
- **PIETRAS RJ ; FENDLY BM ; CHAZIN VR et al.** Antibody to HER-2/neu receptor blocks DNA repair after cisplatin in human breast and ovarian cancer cells. *Oncogene,* 1994, vol. 9, 1829-1838
- **BASELGA J ; NORTON L ; ALBANELL J et al.** Recombinant humanized anti-HER2 antibody (Herceptinú) enhances the antitumor activity of paclitaxel and doxorubicin against HER2/neu overexpressing human breast cancer xenografts. *Cancer Res,* 1994, vol. 58, 2825-2831
- **SLIWKOWSKI MX ; LOFGREN JA ; LEWIS GD et al.** Nonclinical studies addressing the mechanism of action of trastuzumab. *Semin Oncol.,* 1999, vol. 26 (12), 60-70
- **LEWIS GD ; FIGARI I ; FENDLY B et al.** Differential responses of human tumor cell lines to anti-p185HER2 monoclonal antibodies. *Cancer Immunol Immunother,* 1993, vol. 37, 255-263
- **PEGRAM MD ; BALY D ; WIRTH C et al.** Antibody dependent cell-mediated cytotoxicity in breast cancer patients in Phase III clinical trials of a humanized anti-HER2 antibody. *Proc Am Assoc Cancer Res,* 1997, vol. 38, 602
- **LARSSON.** Immunocytochemistry: Theory and Practice. CRC Press inc, 1988
- Immunochemical Protocols, vol 80. Methods in Molecular Biology. Humana Press, 1998, vol. 80
- **ALTMANN et al.** *Glycoconj J 1999,* 1999, vol. 16, 109-23
- **KOST ; CONDREAY.** *Curr Opin Biotechnol,* 1999, vol. 10, 428-33
- **KOHLER ; MILSTEIN.** *Nature,* 1975, vol. 256, 495-497
- **HARLOW ; LANE.** Antibodies: a Laboratory Manual. Cold Spring Harbor, 1988
- **RIECHMANN et al.** *Nature,* 1988, vol. 322, 323-327
- **MANIATIS, T. ; FRITSCH, E. F. ; SAMBROOK, J.** Molecular Cloning: A Laboratory Manual. Cold Spring Harbor Laboratory Press, 1991
- **BERGER ; KIMMEL.** Guide to Molecular Cloning Techniques, Methods in Enzymology. Academic Press, 1987, vol. 152
- **SHI S-R ; COTE RJ ; TAYLOR CR.** Antigen retrieval immunocytochemistry: past, present, future. *J Histochem Cytochem,* 1997, vol. 45 (3), 327-343
- **WESTON, P.D. et al.** *Biochem. Biophys Acta,* 1980, vol. 612, 40
- **HOWARD, A.D. et al.** *J. Biol. Chem.,* 1985, vol. 260, 10833
- **LEE, W.T. ; CONRAD, D.H.** *J. Immunol.,* 1985, vol. 134, 518
- **D'SOUZA, S.E. et al.** *J. Biol. Chem.,* 1988, vol. 263, 3943
- **GEISLER, N. et al.** *Eur. J. Biochem.,* 1992, vol. 206, 841
- **MOENNER, M. et al.** *Proc. Natl. Acad. Sci. USA,* 1986, vol. 83, 5024
- **YANAGI, T. et al.** *Agric. Biol. Chem.,* 1989, vol. 53, 525
- **HARLOW, E. ; LANE, D.** Antibodies: A Laboratory Manual. Cold Spring Harbor Publications, 1988, 349
- **DUNCAN, R.J.S. et al.** *Anal. Biochem.,* 1983, vol. 132, 68
- **KITAGWA, T. et al.** *J. Biochem.,* 1983, vol. 94
- **RUSIN, K.M. et al.** *Biosens. Bioelectron.,* 1992, vol. 7, 367

- **GREEN, N. et al.** 442626-Y -SH Water heterobifunctional reagent. *Cell,* 1982, vol. 28, 477
- **AITHAL, H.N. et al.** OH DMF alkaline phosphatase conjugates. *J. Immunol. Methods,* 1988, vol. 112, 63
- **ANNUNZIATO, M.E. et al.** *Bioconjugate Chem.,* 1993, vol. 4, 212
- **CARUELLE, D. et al.** SH Acetonitri to amine groups. Thiolated. *Anal. Biochem.,* 1988, vol. 173, 328
- **R.B. MERRIFIELD.** Solid Phase Peptide Synthesis. The synthesis of a Tetrapeptide. *J. Am. Chem. Soc.,* 1963, vol. 85, 2149-2154
- **R.B. MERRIFIELD.** *Solid Phase Synthesis, Science,* 1986, vol. 232, 341-347
- **G. BARANY ; N. KNEIB-CORDONIER ; D. G. MULLEN.** Solid-phase peptide synthesis: A silver anniversary report. *Int. J. Peptide Protein Res.,* 1987, vol. 30, 705-739
- **G.B. FIELDS ; R. L. NOBLE.** Solid phase peptide synthesis utilizing 9-fluorenylmethoxycarbonyl amino acids. *Int. J. Peptide Protein Res.,* 1990, vol. 35, 161-214
- **D. C. BLACKLEY.** Emulsion Polymerization: Theory and Practice. Wiley, 1975
- **F. A. BOVEY et al.** Emulsion Polymerization. Interscience Publishers, 1965
- **C.E. SCHILDKNECHT.** Vinyl and Related Polymers. John Wiley & Sons, 1952
- **E.H. RIDDLE.** Monomeric Acrylic Esters. Reinhold Publishing Corp, 1954
- **A.G. ALEXANDER.** J Oil Colour Chemists' Association. 1962, vol. 45, 12
- **G.G. GRETH ; J.E. WILSON.** J Appl Polymer Sci. 1961, vol. 5, 135
- **EDGAR SCHREIBER.** *Nucleic Acids research,* 1989, vol. 17 (15), 6419